# EUROPEAN PATENT SPECIFICATION

(11) **EP 1 987 359 B1**
(45) Date of publication and mention of the grant of the patent: **18.03.2015**
(21) Application number: 07711616.8
(22) Date of filing: 21.02.2007
(51) Int. Cl.: G01N 33/574, G01N 33/68, A61K 38/00, C07K 16/00, C12N 15/62

(54) **VASCULAR TUMOR MARKERS**
MARKER FÜR GEFÄSSTUMORE
MARQUEURS DE TUMEURS VASCULAIRES

(30) Priority: 22.02.2006 EP 06003644
(43) Date of publication of application: 05.11.2008
(60) Divisional application: 09009981.3
(73) Proprietor: Philogen S.p.A., 53100 Siena (IT)
(72) Inventor: NERI, Dario, CH-8107 Buchs (CH); RÖSLI, Chistoph, CH-8142 Uitikon-Waldegg (CH); RAYBAK, Jascha-Nikolai, 79618 Rheinfelden(Baden) (DE)
(74) Representative: Kasche, André
(86) International application number: PCT/EP2007/001490
(87) International publication number: WO 2007/096142

(56) References cited:
- EP-A- 1 524 524
- WO-A-01/57062
- WO-A-03/016471
- WO-A-2005/062055
- WO-A2-2004/106495
- US-A1- 2005 106 644
- SHAO RONG ET AL: "Acquired expression of periostin by human breast cancers promotes tumor angiogenesis through up-regulation of vascular endothelial growth factor receptor 2 expression." MOLECULAR AND CELLULAR BIOLOGY MAY 2004, vol. 24, no. 9, May 2004 (2004-05), pages 3992-4003, XP002435244 ISSN: 0270-7306
- VAN WIJNGAARDEN JENS ET AL: "Identification of differentially expressed genes in a renal cell carcinoma tumor model after endostatin-treatment." LABORATORY INVESTIGATION; A JOURNAL OF TECHNICAL METHODS AND PATHOLOGY NOV 2004, vol. 84, no. 11, November 2004 (2004-11), pages 1472-1483, XP002435245 ISSN: 0023-6837
- BRENTNALL TERESA A ET AL: "Pancreatic cancer proteomics: The proteins that underlie invasion, metastasis, and immunologic escape" GASTROENTEROLOGY, vol. 128, no. 4, Suppl. 2, April 2005 (2005-04), page A531, XP008079355 & ANNUAL MEETING OF THE AMERICAN-GASTROENTEROLOGICAL-ASSOCIATION/D IGEST IVE-DISEASE-WEEK; CHICAGO, IL, USA; MAY 14 -19, 2005 ISSN: 0016-5085
- RYBAK JASCHA-N ET AL: "IN VIVO PROTEIN BIOTINYLATION FOR IDENTIFICATION OF ORGAN-SPECIFIC ANTIGENS ACCESSIBLE FROM THE VASCULATURE" NATURE METHODS, XX, XX, vol. 2, no. 4, April 2005 (2005-04), pages 291-298, XP008074383 ISSN: 1548-7091
- SCHEURER SIMONE B ET AL: "Identification and relative quantification of membrane proteins by surface biotinylation and two-dimensional peptide mapping" PROTEOMICS, vol. 5, no. 11, July 2005 (2005-07), pages 2718-2728, XP002418330 ISSN: 1615-9853

## Description

The present invention relates to a method for identifying neovascular structures in mammalian tissue as claimed in claim 1. The disclosure also relates to a method for identifying diseases or conditions associated with neovascularization, methods for targeting and/or imaging neovascular structures and methods for targeting diseases or conditions associated with neovascularization. Furthermore, the disclosure is directed to the the use of novel and/or known ligands, preferably antibodies, directed against novel and/or known target proteins for identifying tumor cells in mammalian tissue, preferably mammalian kidney tissue, more preferably mammalian vascular kidney tissue. The present disclosure also relates to novel ligands, preferably antibodies, fusion proteins comprising said ligands or antibodies, pharmaceutical and diagnostic compositions comprising said ligands, antibodies or fusion proteins, diagnostic and therapeutic methods as well as novel proteins and corresponding polynucleotides, vectors and host cells.

### The prior art

It is well known in the field of oncology that the growth of solid tumors depends on their capacity to acquire a supporting blood supply. Anti-angiogenics that prevent vascularization at an early stage have been a promising anti-tumor approach. A more recent therapeutic concept is the targeted destruction of established tumor vasculature. Vascular targeting has already been shown to be an effective antitumor strategy in animal models (Neri, D. and Bicknell, R., Nature reviews. Cancer, vol. 5, 436-446, June 2005) and clinical testing for a number of promising compounds has started. Targeting the established tumor vasculature presents an alternative, possibly complementary and certainly wide-ranging therapy.

It has long been known that the endothelium and surrounding stroma in tumors differs from that in normal tissue, but only recently have these differences begun to be characterized at the molecular level. Proteins that are expressed on the endothelial cells or in the surrounding stroma of tumors have been suggested for therapeutic targeting. (Neri and Bicknell, 2005, *supra*)*.* For example, the toxin ricin was conjugated to high-affinity antibodies directed to a mouse MHC class II antigen in solid tumors. The conjugate was injected into mice intravenously and the antibody delivered the ricin specifically to the tumor endothelium, where it was internalized, eliciting cell death with a subsequent collapse of the vasculature and eradication of the solid tumor (Burrows, F.J. and Thorpe, P.E., PNAS USA 90, 8996-9000 (1993). Proteins expressed specifically on the tumor vasculature but not on the vasculature of normal tissues can not only be used for antitumor targeting but also for diagnostic in particular imaging purposes.

For identifying tumor vascular targets most studies are based on *in vitro* endothelial cell isolates, that are exposed to culture conditions thought to mimic those in normal and tumor tissues and a range of molecular techniques were then employed to identify differentially expressed genes. Although differences in gene expression were apparent, it proved difficult to identify the differentially expressed proteins on the molecular level. Another popular approach has been to raise antibodies to different endothelial structures leading to the identification of new endothelial markers but failed to identify differentially expressed genes, possibly because such proteins are a minor component of the abundant components on the cell surface.

In another recent approach the vasculature has also been targeted *in vivo* with antibodies directed to vascular antigens. In another recent *in vivo* targeting approach the present inventors identified accessible antigens in normal organs and in tumors based on the terminal perfusion of tumor-bearing mice with reactive ester derivatives of biotin (Rybak et al., Nat. Methods 2, 291, April 2005).

Tumor-specific vascular targets provide important tumor-diagnostic information and also allow for specifically targeting antitumor compounds. The specific accumulation at the tumor vasculature actively reduces the toxic side effects that are typically associated with the anti-tumor compounds at other locations in the normal tissue and, consequently, allows for the reduction of the concentration of the toxic agents. Moreover, tumor vasculature-specific antitumor agents can be micro-injected in the arterial in-flow of blood into a solid tumor, attach to the vasculature and, thereby, provide a minimum of toxic outflow.

In summary, vascular targets for tumors in general and, in particular, for specific tumors, organ-specific tumors, etc. provide an important tool for the diagnosis and therapy of tumors.

It is the object of the present invention to identify neovascular structures in mammalian tissues, in particular, in mature tissues. Another object is the identification of a disease or condition related to neovascularization in a mammal. A further object is the provision of methods for targeting and/or imaging neovascular structures in mammalian tissues, in particular mature tissues, more particular in tissues affected by a disease. Also, it is the an object to provide specific tumor targets and uses therefore. Another object is the provision of kidney-specific tumor targets, in particular vascular kidney tumor targets.

The present disclosure provides novel polypeptide targets for identifying neovascular structures, in particular neovascular structures in diseases associated with neovascularisation in mammalian tissue such as tumors, macular degeneration, arthritis and atherosclerosis.

Neovasculature structures, as defined herein, are endothelial cells, extracellular matrix, pericytes, other components of the stroma and/or diseased cells in the close proximity of vessels. Such neo-vasculature structures can be found in tumors but also in other angiogenesis-related disorders such as, for example, macular degeneration, arteriosclerosis, rheumatoid arthritis etc.

These new vascular polypeptide targets are selected from the group consisting of:
(1) Periostin [precursor] including isoforms thereof and new splice variants A, B, D, E, (2) putative G-protein coupled receptor 42 including isoforms thereof (3) solute carrier family 2, facilitated glucose transporter member 1, (4) Versican core protein [precursor], (5) CEACAM3 including isoforms thereof, (6) Fibromodulin, (7) Peroxidasin homolog [fragment], (8) probable G-protein coupled receptor 37 [precursor], (9) Protein sidekick-1 [precursor], (10) Alpha1A-voltage-dependent calcium channel, (11) EMILIN2 protein [fragment], (12) Down syndrome critical region protein 8 including isoforms thereof, (13) probable G-protein coupled receptor 113 [precursor], (14) ANXA4 protein [fragment] including isoforms thereof, (15) uromodulin-like 1 [precursor] including isoforms thereof, (16) scavenger receptor class F member 2 [precursor], (17) Sushi domain-containing protein 2 [precursor], (18) tumor protein, translationally controlled 1, (19) putative G-protein coupled receptor Q8TDU0, (20) hypothetical protein DKFZp686K0275 [fragment], (21) Transmembrane protein TMEM55A, (22) hypothetical protein Q8WYY4, (23) Family with sequence similarity 116, member A, (24) UPF0240 protein C6orf66, (25) CDNA FLJ45811 fis, clone NT2RP7014778, (26) hypothetical protein DKFZp779O1248, (27) Beta-ureidopropionase, (28) hypothetical protein DKFZp434F1919 including isoforms thereof, (29) Cysteine-rich with EGF-like domain protein 2 [precursor] including isoforms thereof, (30) UPF0378 family protein KIAA0100 [precursor] (31) potassium voltage-gated channel subfamily H member 1 including isoforms thereof.

Some of the above vascular targets are known proteins, whereas others have been postulated to be proteins from the identification of nucleotide sequences that may code such a protein. A list of (i) the above thirty-one proteins and (ii) the corresponding accession numbers of available amino acid and nucleotide sequences encoding them (Swiss. Prot.) as well as (iii) sequence identification numbers (SEQ ID NOs) relating to the sequences listed further below are provided in the following Table 1.

**Table 1**

| # | **Name** (synonyms) *(comments)* | **Swiss Prot**. **Acc**. **No.** | **SEQ ID No.: AA, NA**** |
|---|---|---|---|
| 1 | **Periostin [precursor]** (PN, Osteoblast-specific factor 2, OSF-2) | Q15063 | 1, 2 |
| | *(Other isoforms*:) | | |
| | **OTTHUMP00000018269** (Periostin, osteoblast specific factor, isoform CRA_a, POSTN protein) | Q5VSY8 | 3, 4 |
| | **OTTHUMP00000018270** | Q5VSY7 | 5, 6 |
| | **OTTHUMP00000018271** | Q5VSY6 | 7, 8 |
| | **Variant A** | | 9, 10 |
| | **Variant B** | | 11, 12 |
| | **Variant D** | | 13, 14 |
| | **Variant E** | | 15, 16 |
| 2 | **Putative G-protein coupled receptor 42** | O15529 | 17, 18 |
| | (*Other isoforms:)* | | |
| | **Free fatty acid receptor 3** (G-protein coupled receptor 41) | O14843 | 19, 20 |
| 3 | **Solute carrier family 2, facilitated glucose transporter member 1** (Glucose transporter type 1, erythrocyte/brain; HepG2 glucose transporter) | P11166 | 21, 22 |
| 4 | **Versican core protein [precursor]** (Large fibroblast proteoglycan, chondroitin sulfate proteoglycan core protein 2, PG-M, glial hyaluronate-binding protein, GHAP) | P13611 | 23, 24 |
| 5 | **CEACAM3** | Q6UY47 | 25, 26 |
| | *(Other isoforms:)* | | |
| | **Carcinoembryonic antigen-related cell adhesion molecule 21** | Q3KPI0 | 27, 28 |
| | **R29124_1** | O75296 | 29, 30 |
| 6 | **Fibromodulin** (Fibromodulin, isoform CRA_a) | Q8IV47 | 31, 32 |
| 7 | **Peroxidasin homolog [fragment]** (Melanoma-associated antigen MG 50) | Q92626 | 33, 34 |
| 8 | **Probable G-protein coupled receptor 37 [precursor]** (endothelin B receptor-like protein 1, ETBR-LP-1, Parkin-associated endothelin receptor-like receptor, PAELR) | O15354 | 35, 36 |
| 9 | **Protein sidekick-1 [precursor]** *(formerly identified as FLJ00154 protein)* | Q8TEN9 | 37, 38 |
| 10 | **Alpha1A-voltage-dependent calcium channel** | Q9NS88 | 39,40 |
| 11 | **EMILIN2 protein [fragment]** | Q8N5L1 | 41, 42 |
| 12 | **Down syndrome critical region protein 8** (Malignant melanoma-associated protein 1, MMA-1, MTAG2 protein) | Q96T75 | 43, 44 |
| | *(Other isoforms:)* | | |
| | **Malignant melanoma-associated protein (Down syndrome critical region gene 8, isoform CRA_b)** | Q6EXA9 | 45 |
| | | Q684H4 | 46 |
| | **Malignant melanoma-associated protein** | Q96T75-2 | 47 |
| | **MTAG6** | Q96T75-3 | 48 |
| | **MMA-1a** | Q96T75-4 | 49 |
| | **MMA-1b** | | |
| 13 | **Probable G-protein coupled receptor 113 [precursor]** (G-protein coupled receptor PGR23) | Q8IZF5 | 50,51 |
| 14 | **ANXA4 protein [fragment]** (Hypothetical protein ANXA4, proliferation-inducing protein 28) | Q6LES2 | 52, 53 |
| | *(Other isoforms*:) | | |
| | **Annexin A4** (Annexin IV, Lipocortin IV, Endonexin I, Chromobindin-4, Protein II, P32.5, Placental anticoagulant protein II, PAP-II, PP4-X, 35-beta calcimedin, carbohydrate-binding protein P33/P41, P33/41) | P09525 | 54, 55 |
| 15 | **Uromodulin-like 1 [precursor]** (Olfactorin) | Q5DID0 | 56, 57 |
| | *(Other isoforms:)* | | |
| | **Q5DID0-2** | Q5DID0-2 | 58 |
| | **UMOLD1S** | Q5DID0-3 | 59 |
| | **UMOLD1L** | Q5DID0-4 | 60 |
| 16 | **Scavenger receptor class F member 2 [precursor]** (Scavenger receptor expressed by endothelial cells 2 protein, SREC-II, SRECRP-1) | Q96GP6 | 61, 62 |
| 17 | **Sushi domain-containing protein 2 [precursor]** | Q9UGT4 | 63,64 |
| | *(formerly identified **as** BK65A6.2)* | | |
| 18 | **Tumor protein, translationally controlled** 1 | Q5W0H4 | 65, 66 |
| 19 | **Putative G-protein coupled receptor Q8TDU0** (HCG2044627) | Q8TDU0 | 67,68 |
| 20 | **Hypothetical protein DKFZp686K0275 [fragment]** | Q7Z3A1 | 69, 70 |
| 21 | **Transmembrane protein TMEM55A** | Q8N4L2 | 71, 72 |
| | (hypothetical protein TMEM55A, EC 3.1.3- type II phosphatidylinositol-4,5-biphosphate 4-phosphatase, Ptdlns-4,5-P2 4-Ptase II) | | |
| 22 | **Hypothetical protein Q8WYY4** | Q8WYY4 | 73, 74 |
| 23 | **Family with sequence similaritiy 116, member** A (Hypothetical protein FLJ34969) | Q81WF6 | 75, 76 |
| 24 | **UPF0240 protein C6orf66** | Q9P032 | 77, 78 |
| 25 | **CDNA FLJ45811 fis, clone NT2RP7014778** *(formerly identified as hypothetical protein FLJ45811)* | Q6ZS59 | 79, 80 |
| 26 | **Hypothetical protein DKFZp779O1248** | Q6AHZ8 | 81, 82 |
| 27 | **Beta-ureidopropionase** (EC 3.5.1.6 beta-alanine synthase, N-carbamoyl-beta-alanine amidohydrolase, BUP-1) | Q9UBR1 | 83, 84 |
| 28 | **Hypothetical protein DKFZp434F1919** (Hypothetical protein MDS025, Coiled-coil domain containing 90B, CUA003) | Q9GZU6 | |
| | *(Other isoform:)* | | |
| | **MDS011** (Coiled-coil domain containing 90B, MDS025) | Q9GZT6 | 87, 88 |
| 29 | **Cysteine-rich with EGF-like domain protein** 2 **[precursor]** (CRELD2 protein) *(Q9BU47, Q86UC0 and Q6UXH1 were formerly separate entries in Swiss Prot.)* | Q6UXH1 | 89, 90 |
| | (Other isoforms:) | | |
| | **Alpha** | Q6UXH1-2 | 91 |
| | **Beta** | Q6UXH1-3 | 92 |
| | **Gamma** | Q6UXH1-4 | 93 |
| | **Epsilon** | Q6UXH1-5 | 94 |
| | **Zeta** | Q6UXH1-6 | 95 |
| 30 | **UPF0378 family protein KIAA0100 [precursor]** (breast cancer overexpressed gene 1 protein, antigen Mlaa-22) *(*formerly identified as hypothetical protein DKFZp686M0843)* | Q5H9T4 | 96, 97 |
| 31 | **Potassium voltage-gated channel subfamily H member 1** (Voltage-gated potassium channel subunit Kv10.1, Ether-a-go-go potassium channel 1, hEAG1. h-eag) *(*formerly identified as Potassium voltage-gated channel subfamily H member 1 (KCNH1))* | 095259 | 98, 99 |
| | *(Other isoforms:)* | | |
| | **hEAG** | 095259-2 | 100 |

| | | | |
|---|---|---|---|
| ** previously identified under this name in the priority document* ** *AA, NA = amino acid sequence, nucleic acid sequence* WO 03/016471 A discloses a method of identifying a metastasis of breast cancer to bone by means of antibodies binding the periostin splice variant L, which encompasses the motif EIPVTVY. US 2005/0106644 A1 teaches the upregulation of TAT163 polynucleotides in kidney tumor tissue. However, upregulaton is only assessed on the mRNA level with cDNA probes and relative to a "universal epithelial control sample". A TAT163 polypeptide product was never verified for any actual tumor-related upregulation. | | | |

The terms "[fragment]" and "[precursor]" in the context of the above listed proteins are part of their actual name in the database entry of the respective protein.

Furthermore, it is common knowledge in the art that database entries sometimes contain minor sequencing errors and may be subject to revisions and changes. In addition, proteins can be subject to posttranslational modifications and differential splicing.

The above new vascular polypeptide targets were identified by an *ex vivo* vascular perfusion of surgically removed kidneys with a biotinylation reagent that labels vascular accessible primary amine-containing structures with biotin. The isolation, characterization and subsequent comparison of the many biotin-labelled amine structures in the vasculature of kidneys with and without tumors eventually led to the identification of the above vascular tumor targets. For details of the identification procedure see the example 1 below.

The above vascular targets now allow for the preparation of vascular target-specific ligands. Ligands include antibodies, antibody fragments or functional derivatives thereof as well as antibody-like binding molecules, peptides, small organic molecules, aptamers and other binding molecules as described below having a binding affinity to one of the above-listed proteins in table 1.

These vascular target-specific ligands are useful for the methods and uses provided.

In a first aspect the present invention relates to a method for identifying neovascular structures in mammalian tissue according to claim 1.

The term "mature tissue", as it is used herein, is understood to mean fully differentiated tissue from born mammals, preferably adult mammals and specifically excludes prenatal tissue.

Another aspect of the present invention provides for a method for identifying a a kidney tumor, preferably a human kidney tumor according to claim 2.

The present invention also encompasses a method for targeting and/or imaging neovascular structures in mammalian tissue according to claim 3.

Preferably, said mammalian tissue is mature mammalian kidney tissue, more preferably human mature tissue, most preferably kidney tissue.

A further aspect of the invention is directed to a method for targeting and/or imaging a tissue affected by a a kidney tumor, more preferably a human kidney tumor according to claim 4.

While monoclonal antibodies and their derivatives are still the preferred binding molecules/ligands for pharmaceutical biotechnological applications, other classes of binding molecules/ligands with antibody-like binding properties have increasingly been used as alternatives to antibodies for many applications. Such functional analogues include aptamers (Brody EN, Gold L., Aptamers as therapeutic and diagnostic agents. J. Biotechnol. 2000 Mar., 74(1):5-13. Review), small globular proteins engineered (e.g., by mutagenesis of loops) to recognize cognate antigens (e.g., anticalins, affibodies, ankyrin repeats, etc. [Binz HK, Amstutz P, Pluckthun A; Engineering novel binding proteins from nonimmunoglobulin domains. Nat Biotechnol. 2005 Oct., 23(10):1257-68. Review.]). Globular proteins having antibody-like proteins can be derived from large libraries of mutants, e.g. be panned from large phage display libraries and can be isolated in analogy to regular antibodies. Also, antibody-like binding proteins can be obtained by combinatorial mutagenesis of surfaces-exposed residues in globular proteins. Moreover, low molecular weight synthetic organic molecules can be used as vascular tumor targeting agents, provided that they have sufficient binding affinity and specificity for the antigen as well as suitable pharmacokinetic properties.

Therefore, in another aspect, the present disclosure relates to the use of at least one ligand, preferably at least one antibody, fragment or functional derivative thereof, having specific binding affinity to a protein selected from Table 1 for identifying neovascular structures, preferably for identifying tumors, in mammalian tissue.

In a preferred example the at least one ligand, preferably an antibody, fragment or functional derivative thereof, has specific binding affinity to a protein selected from: 1A, 1B, 1 D, 1E, 2, 5, 7-13, 15-17, 19-23, 25-30.

The proteins in table 1 above and the preferred proteins listed directly above were specifically identified in neo-vasculature structures of human tumor tissue.

Therefore, in a more preferred example the present disclosure relates to the use for identifying tumors in human tissue.

The proteins of table 1 were identified in neo-vasculature structures of human kidney tumor tissue. Therefore, the present disclosure relates to the use according to the invention for identifying neovascular structures, in particular for identifying tumors, in mammalian kidney tissue, preferably human kidney tissue.

Most preferred the proteins for identifying neo-vasculature structures in mammalian kidney tissue, preferably human kidney tissue, are selected from the group consisting of: 1, 2, 4 - 13, 15 - 31.

All of the proteins in table 1 were specifically identified in neo-vasculature structures of human kidney tumors. They represent specific targets in kidneys that are accessible from the blood stream. Therefore, in a most preferred example the present disclosure relates to the use of at least one ligand, preferably at least one antibody, fragment or functional derivative thereof, having specific binding affinity to a protein selected from Table 1 for identifying neovascular structures, in particular tumors, in mammalian vascular kidney tissue, preferably human vascular kidney tissue.
The above uses provide for tumor diagnostic methods *in vitro* and *in vivo.* For example, ligands such as antibodies having specific binding affinity to at least one of the neo-vasculature tumor targets of table 1 may be contacted with cells, tissue and/or organs under conditions that allow for the binding of said ligands, preferably antibodies, to their corresponding target protein. Ligand-bound, preferably antibody-bound cells, tissue and/or organs are then identified as tumor or tumor-associated cells, tissue and/or organs. The identification of the bound ligands/antibodies may be performed by any of the many routine techniques available to the skilled person that have become routine in the art such as, e.g. secondary antibodies or the identification of markers conjugated to the ligands/antibodies such as radiolabels and chemical labels. The step of contacting the ligands/antibodies and/or the identification of ligand/antibody-bound tumor cells, tissue and/or organs may be performed *in vivo*, e.g. by a radio-imaging method. However, the contacting step may also be performed *in vivo* in a mammal, subsequently isolating cells, tissue and/or the organ of interest and identifying antibody-bound tumor cells *in vitro*/*ex vivo.*

Preferably, said ligands/antibodies are used to identify tumor cells *in vitro* only. The term *"in vitro*" is meant to indicate that the use of said ligands/antibodies according to the invention is limited to methods that are not practiced on the human or animal body and therefore do not violate Art. 53 (c) EPC.

In another aspect the present disclosure is also directed to a ligand, preferably an antibody, fragment or functional derivative thereof, having specific binding affinity to a protein selected from the group consisting of the proteins in table 1 above.
Preferably, the ligand, preferably an antibody, fragment or functional derivative has a specific binding affinity to a protein selected from the group consisting of:
(1) Periostin splice variants A, B, D, E, (5) CEACAM3 including isoforms thereof, (7) Peroxidasin homolog [fragment], (9) Protein sidekick-1 [precursor], (12) Down syndrome critical region protein 8 including isoforms thereof, (13) probable G-protein coupled receptor 113 [precursor], (15) uromodulin-like 1 [precursor] including isoforms thereof, (16) scavenger receptor class F member 2 [precursor], (17) Sushi domain-containing protein 2 [precursor], (19) putative G-protein coupled receptor Q8TDU0, (20) hypothetical protein DKFZp686K0275 [fragment], (21) Transmembrane protein TMEM55A, (22) hypothetical protein Q8WYY4, (23) Family with sequence similarity 116, member A, (25) CDNA FLJ45811 fis, clone NT2RP7014778, (28) hypothetical protein DKFZp434F1919 including isoforms thereof, (30) UPF0378 family protein KIAA0100 [precursor].

The term "specific binding affinity" as it is used herein is to be understood to mean that the ligand/antibody specifically binds to the target protein with signifcant affinity and not to other proteins with significant affinity that are also located in the same environment, i.e. assay system, diagnostic or therapeutic setting *in vivo* or *in vitro,* in an organ, e.g. kidney, and under the same conditions, e.g. pH, temperature, buffer, etc. In general, a binding specificity is tested by performing a binding assay with a specific target molecule and with a large number of non-related substances. Furthermore, functional tests, immunohistochemistry and other procedures can be used to assess the binding specificity of a certain ligand (e.g. an antibody).

For many bioassays (e.g. ELISA) based on ligands, e.g. antibodies or globular proteins, capable of specific binding, a dissociation constant of 1 micromolar or lower is required to yield detectable binding signals which are often associated with a specific binding mode. Preferably, the ligands/antibodies for use in the present invention have a specific binding affinity corresponding to a dissociation constant of less than about 5, preferably about 1 or less micromolar (µM), more preferably about 0,1 µM or less, most preferably about 1 nM or less or even 1 pM or less.

Ligands such as antibodies and fragments are routinely available by hybridoma technology (Kohler, G. and Milstein, C. Nature 256, 495-497, 1975), antibody phage display (Winter et al., Annu. Rev. Immunol. 12, 433-455, 1994), ribosome display (Schaffitzel et al., J. Immunol. Methods, 231, 119-135, 1999) and iterative colony filter screening (Giovannoni et al., Nucleic Acids Res. 29, E27, 2001) once the target antigen is available. Typical proteases for fragmenting antibodies into functional products are well-known. Other fragmentation techniques can be used as well as long as the resulting fragment has a specific high affinity and, preferably, a dissociation constant in the micromolar to picomolar range.

The vascular tumor targeting performance of antibody fragments in scFv format has been shown to crucially depend (at least for a micromolar to picomolar dissociation constant) on the affinity of the antibody to the target. For example, the high affinity antibody fragment scFv(L19), specific to the EDB domain of fibronectin, a marker of angiogenesis, was shown to target tumor neo-vasculature more efficiently than the parental antibody fragment scFv(E1), with a lower affinity for the antigen [Viti F, Tarli L, Giovannoni L, Zardi L, Neri D.; Increased binding affinity and valence of recombinant antibody fragments lead to improved targeting of tumoral angiogenesis. Cancer Res. 1999 Jan 15;59(2):347-52.]. In certain cases, binding avidity (e.g., associated with certain homobivalent antibody formats) can compensate for a moderate monomeric binding affinity [Nielsen UB, Adams GP, Weiner LM, Marks JD; Targeting of bivalent anti-ErbB2 diabody antibody fragments to tumor cells is independent of the intrinsic antibody affinity. Cancer Res. 2000 Nov. 15, 60(22):6434-40.].

A very convenient antibody fragment for targeting applications is the single-chain Fv fragment, in which a variable heavy and a variable light domain are joined together by a polypeptide linker. Other antibody fragments for vascular targeting applications include Fab fragments, Fab₂ fragments, miniantibodies (also called small immune proteins), tandem scFv-scFv fusions, as well as scFv fusions with suitable domains (e.g. with the Fc portion of an immunoglobulin). For a review on certain antibody formats, please see Holliger P, Hudson PJ.; Engineered antibody fragments and the rise of single domains. Nat Biotechnol. 2005 Sep., 23(9): 1126-36. Review.

The term "functional derivative" of an antibody is meant to include any antibody or fragment thereof that has been chemically modified in its amino acid sequence, e.g. by addition, substitution and/or deletion of amino acid residue(s) and/or has been chemically modified in at least one of its atoms and/or functional chemical groups, e.g. by additions, deletions, rearrangement, oxidation, reduction, etc. as long as the derivative has substantially the same binding affinity to the corresponding antigen from table 1 and, preferably, has a dissociation constant in the micro-, nano- or picomolar range. A most preferred derivative of the antibodies for use is an antibody fusion protein that will be defined in more detail below. The antibody, fragment or functional derivative thereof according to the invention is one that is selected from the group consisting of polyclonal antibodies, monoclonal antibodies, chimeric antibodies, humanized antibodies, CDR-grafted antibodies, Fv-fragments, Fab-fragments and Fab₂-fragments and antibody-like binding proteins.

Next to said ligands, preferably antibodies, fragments and derivatives a further aspect is directed to fusion proteins comprising a ligand, preferably an antibody, fragment or functional derivative thereof.

The term "fusion protein" as it is used is meant to encompass all conjugates, wherein a ligand/antibody, fragment or functional derivative according to the present invention is somehow bound to any further component such as, e.g. polypeptide, signal factor, e.g. interleukin, protein, sugar moiety, nucleotide, small biologically active molecule, toxin, label, radiolabel, etc. by, e.g. covalent and/or non-covalent, e.g. ionic bonds.

Preferably, the fusion protein additionally comprises a component having anti-tumor activity. This will greatly facilitate the selectivity as well as the specificity of the anti-tumor compound, thus, allowing for reducing the effective amount thereof to be administered to a patient in need thereof as well as for reducing the toxic side effects associated with said compound.

Intact monoclonal antibodies represent a well-established class of pharmaceuticals with a broad therapeutic potential for various indications. The constant portion of the antibody often contributes to the therapeutic potential and glycosylation can influence bioactivity (Li H, Sethuraman N, Stadheim TA, Zha D, Prinz B, Ballew N, Bobrowicz P, Choi BK, Cook WJ, Cukan M, Houston-Cummings NR, Davidson R, Gong B, Hamilton SR, Hoopes JP, Jiang Y, Kim N, Mansfield R, Nett JH, Rios S, Strawbridge R, Wildt S, Gerngross TU; Optimization of humanized IgGs in glycoengineered Pichia pastoris. Nat Biotechnol. January 2006). Furthermore, a number of vascular targeting antibody derivatives can be considered for pharmaceutical intervention. They include antibody conjugates with radionuclides, photosensitizers, liposomes and drugs, as well as antibody-based fusion proteins with pro-coagulant agents, cytokines, chemokines, toxins, Fc fusions, as well as bispecific antibodies.

More preferably, the fusion protein comprises a component having anti-tumor activity that is selected from the group consisting of intact antibodies, Fc-containing antibody fragments or Fc-functional derivatives thereof, radionucleotides, photosensitizers, liposomes, drugs, pro-coagulatory agents, cytokines, chemokines, toxins as well as bispecific antibodies.

It is well established that ligands such as antibody derivatives can contribute to the diagnosis and/or molecular imaging of a disease. The most established avenues for the macroscopic imaging of ligand/antibody localization *in vivo* include the use of radiolabeled ligands/antibodies (e.g., for PET or SPECT applications) and the use of ligands/antibodies labeled with infrared fluorophores (e.g., for superficial fluorescence imaging, for endoscopic imaging, for diffuse optical tomography, etc.). Moreover, ligand/antibody-microbubble conjugates (to be used as contrast agents in ultrasound-based imaging procedures; Joseph S, Olbrich C, Kirsch J, Hasbach M, Briel A, Schirner M.; A real-time in vitro assay for studying functional characteristics of target-specific ultrasound contrast agents. Pharm Res. 2004 Jun., 21(6):920-6.) and/or ligand/antibody-conjugates for enhancing MRI imaging (Kiessling F, Heilmann M, Lammers T, Ulbrich K, Subr V, Peschke P, Waengler B, Mier W, Schrenk HH, Bock M, Schad L, Semmler W. Synthesis and characterization of HE-24.8: a polymeric contrast agent for magnetic resonance angiography. Bioconjug Chem. 2006 Jan-Feb;17(1):42-51.) can also be used.

In another more preferred example, the fusion protein comprises a component having diagnostic activity, i.e. allowing for the selective identification of the antibody component *in vivo* and/or *e*x *vivo.*

Preferably, the component having diagnostic activity is selected from the group consisting of radiolabels, fluorophores, biotin, chelated metals or metal compounds, and microbubbles.

The fusion protein having an anti-tumor component is useful for preparing a medicament that is efficiently targeted to the vasculature of tumors, preferably to kidney tumors. Therefore, a further aspect relates to the use of a fusion protein for preparing a medicament for the treatment of cancer in a mammal, preferably in a human.

Preferably, said medicament is for the treatment of kidney cancer, preferably human kidney cancer.

The fusion protein comprising a component having diagnostic activity is useful for preparing a medicament that is efficiently targeted to the vasculature of tumors, preferably to kidney tumors. Therefore, a further aspect relates to the use of a fusion protein for preparing a diagnostic composition for the identification of tumors in a mammal, preferably a human.

Preferably, said diagnostic composition is for the identification of tumors in a mammalian kidney, preferably a human kidney.

Another aspect relates to a pharmaceutical composition, comprising a ligand, preferably an antibody, fragment or derivative thereof, or a fusion protein and a pharmaceutically acceptable carrier and/or diluent.

A further aspect relates to a diagnostic composition, comprising a ligand, preferably an antibody, fragment or derivative thereof, or a fusion protein.
Another aspect is directed to a method for identifying tumors in mammalian tissue, preferably human tissue, comprising:
(i) contacting a ligand, preferably an antibody, fragment or functional derivative thereof and/or a fusion protein comprising a ligand, preferably an antibody, fragment or functional derivative thereof, having specific binding affinity to at least one protein selected from the group listed above in table 1. with mammalian tissue of interest, preferably human tissue of interest, *in vivo* and/or ex *vivo* under conditions that allow for the specific binding of said ligand and/or fusion protein to at least one of said proteins 1 to 31, and
(ii) identifying specifically bound ligand and/or fusion protein.

Preferably, the at least one tissue protein is selected from the group consisting of: (All numbers according to table 1) 1A, 1B, 1D, 1E, 2, 5, 7-13, 15-17, 19-23, 25-30.

More preferably, the at least one tissue protein is selected from the group consisting of: 1-2, 4-13, 15-31, and the mammalian tissue of interest is kidney tissue.

Also, it is preferred that the mammalian tissue of interest is vascular kidney tissue, preferably human vascular kidney tissue.

More preferably, said steps (i) and/or (ii) of the method are performed *ex vivo,* i.e. *in vitro.*

Most preferred, the method is a method of imaging neovascular structures, in particular a method of imaging tumor cells, preferably kidney tumors, more preferably vascular kidney tumors *in vivo.*

The present disclosure also identifies a number of novel proteins that have utility as novel tumor markers, novel kidney-specific tumor markers, novel and specific markers of the vasculature of kidney tumors, and that can be used as antigens for providing selective and high affinity antibodies as diagnostic and therapeutic means.

### Four novel splice variants of periostin

Periostin is a 90 kDa protein initially identified as osteoblast-specific factor-2 (OSF-2, also called PN), secreted by osteoblasts (Takeshita et al., Biochem J, 294 (Pt 1), 271-8, 1993). Tai and colleagues produced a monoclonal anti-periostin antibody by hybridoma technology and detected, by Western blotting, expression of the human periostin protein in the adrenal glands, lung, thyroid, uterus, vagina, ovary, testis, prostate, and in the gastrointestinal tract, with a preferential expression in the stomach and colorectum, while lower levels were noted in the small intestine and esophagus (Tai et al., Carcinogenesis, 26, 908-15, 2005).

There have been observations of periostin being associated in a number of cancers. However, Periostin has not been associated with kidney tumors in the prior art.

For human periostin, Takeshita and colleagues (Takeshita, Kikuno et al., Biochem J, 294 (Pt 1), 271-8, 1993) have reported that five alternative spliced transcripts can be produced, and that all splicing events of periostin occur within the C-terminal region. The same group has found in the mouse four possible isoforms of periostin generated by a combination of six different cassettes (Horiuchi, Amizuka et al., J. Bone Miner Res., 14, 1239-49, 1999). The function of the isoforms have not yet been elucidated. Litvin and colleagues identified another isoform of mouse periostin and termed it periostin-like-factor (PLF) (Litvin et al., J. Cell Biochem., 92, 1044-61, 2004). A sequence analysis of the full-length PLF cDNA and predicted aa sequence showed that it most resembles Horuichi's isoform 3 of mouse periostin (Litvin, Selim et al., J Cell Biochem, 92, 1044-61, 2004).

The present disclosure demonstrates for the first time that periostin is a protein overexpressed in kidney cancer. Therefore, it can be used as an excellent kidney tumor marker readily accessible from the bloodstream and, thus, is also a useful target for ligand-based tumor targeting strategies.

An immunohistochemical analysis with an anti-periostin antibody further proved that periostin was highly overexpressed in the tumor stroma of renal clear cell carcinoma compared to normal kidney tissue.

A PCR amplification of the C-terminal region of periostin revealed at least eight different splice variants. In public protein databases (Expasy and NCBI), only four different isoforms of human periostin are described (the full-length form and the three splice isoforms Q5VSY8, Q5VSY7 and Q5VSY6). Next to all published isoforms, four new isoforms, termed isoforms A, B, D and E (see SEQ ID NO: 9, 11, 13, 15, respectively) were identified. The corresponding analysis showed a different distribution of isoform transcripts in the various tissues. It was found that the transcripts of periostin are only weakly expressed (or barely detectable) in normal adult kidney cDNA, but could be amplified from the clear cell carcinoma specimen in isoforms of different length. This finding is compatible with the identification of periostin in a proteomic analysis in the tumor tissue only. Fetal kidney was also positive for periostin, but the distribution of the isoforms was different from that registered in all the tumor tissues examined. Expression of periostin transcripts can also be seen in normal adult brain and liver. However, the distribution of isoforms of periostin in brain and liver cDNA libraries showed differences between tumor, fetal and normal adult specimens. The smallest detected transcript of periostin was predominantly expressed in tumor specimens, being present in at least four different kidney and liver tumors, but undetectable in normal specimens and only barely detectable in normal adult brain and fetal kidney.

Surprisingly, a mass spectrometric analysis revealed three peptides EIPVTVYKPIIKK, EIPVTVYRPTLTK and IITGPEIK, which are isoform-specific, because they encompass junctions of two exons which only exist in certain isoforms.

It is expected that ligands, preferably antibodies directed/raised against "junction peptides" existing only in the novel periostin isoforms specifically expressed in tumors (or even a particular type of tumor) will provide a very powerful tool for selective targeting and destruction of these tumors.

In one aspect, the present disclosure is directed to a periostin splice variant protein, fragment or functional derivative, comprising a peptide having the amino acid EIPVTVYGPEIK.

Preferably, this aspect relates to a periostin splice variant protein A, B, D or E having an amino acid sequence selected from SEQ ID NOs: 9, 11, 13, 15, respectively, a fragment or a functional derivative thereof, wherein the amino acid sequence of the fragment or functional derivative thereof comprises at least 20, preferably at least 30, more preferably at least 50 amino acids, and most preferably at least 75 amino acids, and
a) wherein the fragment or functional derivative of SEQ ID NO: 9 has an amino acid sequence reflecting deletions in SEQ ID NO: 1 in positions 670-756, or in positions 670-756 and 783-810;
b) wherein the fragment or functional derivative of SEQ ID NO: 11 has an amino acid sequence reflecting deletions in SEQ ID NO: 1 in positions 670-726 and 784-810 or in positions 784-810; preferably, this splice variant comprises a peptide having the amino acid sequence EIPVTVYGPEIK.
c) wherein the fragment or functional derivative of SEQ ID NO: 13 has an amino acid sequence reflecting a deletion in SEQ ID NO: 1 in positions 670-756;
d) wherein the fragment or functional derivative of SEQ ID NO: 15 has an amino acid sequence comprising the amino acid valine in position 421 in SEQ ID NO: 15 and an amino acid sequence reflecting a deletion in SEQ ID NO: 1 in positions 671-697.

A preferred example relates to a periostin splice variant according to the invention having at least 80, preferably 85, more preferably 90, most preferably at least 95 or 98 % amino acid sequence identity to the above protein, fragment or functional derivative according to the invention, wherein the sequence is not a sequence of any one of SEQ ID NO: 1, 3, 5 and 7.

Furthermore, the present disclosure relates to a polynucleotide encoding an above described protein, fragment or functional derivative of any one of the 4 novel splice variants.

A functional derivative of a protein is meant to encompass any amino acid sequence- and/or chemical derivative, that has substantially sufficient accessible amino acid residues to establish the same binding affinity to an antibody that has an affinity to the original protein. Preferably, the functional derivative is one that has deletions (including N-terminal or C-terminal truncations), additions and/or substitutions, more preferably conservative amino acid substitutions.

A fragment or a functional derivative according to the present invention has at least 10, at least 20, at least 30, at least 40, at least 50, at least 75 or at least 100 amino acids of the original full length protein.

For determining the sequence identity among polypeptides, the skilled person can revert to a number of standard algorithms known to those of skill in the art. Preferably, the BLAST programs at http://www.expasy.org/tools/bfast/ and http://www.ncbi.nlm.nih.gov/BLAST/Blast.cgi?CMD=Web&LAYOUT=TwoWindow s&AUTO_FORMAT=Semiauto&ALIGNMENTS=250&ALIGNMENT_VIEW=Pairwi se&CDD_SEARCH=on&CLIENT=web&DATABASE=nr&DESCRIPTIONS=500& ENTREZ_QUERY=%28none%29&EXPECT=10&FILTER=L&FORMAT_OBJECT =Alignment&FORMAT_TYPE=HTML&I_THRESH=0.005&MATRIX_NAME=BLO SUM62&NCBI_GI=on&PAGE=Proteins&PROGRAM=blastp&SERVICE=plain&S ET_DEFAULTS.x=41&SET_DEFAULTS.y=5&SHOW_OVERVIEW=on&END_OF _HTTPGET=Yes&SHOW_LINKOUT=yes&GET_SEQUENCE=yes, more preferably with the default settings, are used to identify the amino acid sequence identity of a protein, protein fragment or protein derivative.

In some instances the present disclosure also provides novel polynucleotides encoding the proteins, fragments or functional derivatives thereof characterized in that they have the ability to hybridize to a specifically referenced nucleic acid sequence under stringent conditions. Next to common and/or standard protocols in the prior art for determining the ability to hybridize to a specifically referenced nucleic acid sequence under stringent conditions, it is preferred to analyse and determine the ability to hybridize to a specifically referenced nucleic acid sequence under stringent conditions by comparing the nucleotide sequences of the two proteins, which may be found in gene databases (e.g. http://www.ncbi.nlm.nih.gov/entrez/query.fcgi?db =nucleotide) with alignment tools (e.g., http://www.ncbi.nlm.nih.gov/blast/Blast.cgi?CMD=Web&LAYOUT =TwoWindows&AUTO_FORMAT=Semiauto&PAGE=Nucleotides&NCBI_GI=yes &FILTER=L&HITLIST_SIZE=100&SHOW_OVERVIEW=yes&AUTO_FORMAT=y es&SHOW LINKOUT=yes).

The term "polynucleotide encoding a protein" as it is used is meant to include allelic variations and redundancies in the genetic code.

Furthermore, the present disclosure provides new proteins, fragments and derivatives thereof, as well as nucleotides encoding them in accordance with any of the claims 40 to 93.

More specifically the present disclosure provides proteins 5 (3 x), 7, 9, 12 (6 x), 13, 15 (4 x), 16, 19-23, 25, 28 (2 x), 30, 31 (2x) having an amino acid sequence as indicated in the corresponding SEQ ID NO: (see Table 1 for assignment), fragments or functional derivatives thereof.

Furthermore, in a preferred example the present disclosure relates to proteins, fragments or functional derivatives thereof having at least 70, preferably 80, more preferably 90, most preferably at least 95 % amino acid sequence identity to the above proteins 5 (3 x), 7, 9, 12 (6 x), 13, 15 (4 x), 16, 19-23, 25, 28 (2 x), 30, 31 (2x), fragments or functional derivatives thereof.

Moreover, the present disclosure is directed to polynucleotides encoding any one of the above proteins 5 (3 x), 7, 9, 12 (6 x), 13, 15 (4 x), 16, 19-23, 25, 28 (2 x), 30, 31 (2x), fragments or functional derivatives thereof, mentioned above in accordance with the present invention, having the ability to hybridize to the corresponding nucleic acid sequence (see Table 1 for assignment) encoding the complete protein under stringent conditions.

Also, the present disclosure encompasses vectors comprising the polynucleotides encoding proteins, fragments and functional derivatives as well as host cells comprising said proteins, fragments and functional derivatives and/or vectors.

Last but not least, a further aspect is directed to methods for recombinantly producing proteins, fragments and functional derivatives employing polynucleotides, vectors and/or host cells.

In the following the target proteins that have demonstrated their utility as tumor targets, in particular vascular kidney tumor targets are briefly discussed.

### Protein # 2

The identified peptide can be derived from one or both of two protein isoforms (Table 1: 2): Free fatty acid receptor 3 (014843) and/or putative G-protein coupled receptor 42 (015529), respectivley.

Within family A of the G protein-coupled receptor gene superfamily (also classified as family 1), there is a phylogenetically related group of ∼90 receptors that respond to an unusually wide variety of ligand types, considering the relatively close similarity of their primary sequences (Bockaert and Pin, Embo J, 18, 1723-9, 1999).

Free fatty acid receptor 3 was detected in adipose in all three published studies on this receptor (Brown et al., J. Biol. Chem., 278, 11312-9, 2003; Le Poul et al., J. Biol. Chem., 278, 25481-9, 2003; Xiong et al., Proc. Natl. Acad. Sci. U S A. 101, 1045-50, 2004). So far, an antibody for discriminating between GPR41 and GPR42 is unknown.

An expression or even overexpression of either of these proteins in tumors has not yet been reported.

### Protein # 3

Solute carrier family 2, facilitated glucose transporter member 1 (SLC2A1) (=Glucose transporter type 1, erythrocyte/brain (GLUT1)) (P11166)

Increased glucose uptake is one of the major metabolic changes found in malignant tissue. This uptake is mediated by glucose transporter (Glut) proteins, which are membrane proteins responsible for the transport of glucose across cellular membranes. These human glucose transporters have a distinct tissue distribution and contribute to the disposal of glucose under various conditions (Pessin and Bell, Annu Rev Physiol, 54, 911-30, 1992). A family of seven glucose transporters has been cloned. Among these, Glut1 (which is also called solute carrier family 2, facilitated glucose transporter member 1 (SLC2A1)) is expressed in erythrocytes, the blood-brain barrier, the perineurium of peripheral nerves and the placenta (Froehner et al., J. Neurocytol., 17, 173-8, 1988; Pardridge et al., J Biol Chem, 265, 18035-40, 1990; Pessin and Bell, Annu. Rev. Physiol., 54, 911-30, 1992; Takata et al., Cell Tissue Res, 267, 407-12, 1992). Glut1 has been associated with a number of tumors in the prior art. Glut1 was also shown by immunohistochemistry to be expressed in kidney cancer (Nagase et al., J Urol, 153, 798-801, 1995; North et al., Clin Neuropathol, 19, 131-7, 2000).

Glut1 is a protein readily accessible from the bloodstream, indicating this tumor marker to be a useful target for ligand-based tumor targeting strategies.

### Protein # 4

Versican core protein [precursor] (13611) is a large extracellular matrix proteoglycan that is present in a variety of tissues and plays a role in the regulation of cell adhesion and survival, cell proliferation, cell migration and extracellular matrix assembly (Wight, Curr Opin Cell Biol, 14, 617-23, 2002). In addition, there is evidence that versican is overexpressed in angiogenesis and in tumors.

The present disclosure surprisingly demonstrates the expression of the versican core protein in kidney cancer, indicating this tumor marker to be a useful target for ligand-based tumor targeting strategies.

### Protein # 5

Surprisingly, the peptide SDPLKLTVK was identified in tumor only but not in normal kidney samples. This peptide is part of three different sequence entries in the SwissProt database: CEACAM3 with 292 amino acid residues (Q6UY47), carcinoembryonic antigen-related cell adhesion molecule (gene name: CEACAM21) with 293 amino acid residues (Q3KPI0), and R29124_1 with 235 amino acid residues (075296). The existence of these proteins was postulated based on DNA sequence analysis, but was never experimentally proven. The three sequences share more than 98% identity with a maximum of 3 mismatches. These data indicates that the three sequences either belong to the same protein and the differences are due to sequencing errors, or that the sequences correspond to different isoforms of the same protein.

The sequences exhibit significant similarity to other proteins of the CEACAM family, a subgroup of the human carcinoembryonic Ag (CEA) protein family (Beauchemin et al., Exp Cell Res, 252, 243-9, 1999).

While a number of CEACAM have been studied on the protein level, this seems not to be the case for the proteins identified herein. The highest similarity to a CEACAM which has actually been studied on the protein level is to biliary glycoprotein precursor (CEACAM1) and is only maximal 44%. Furthermore, the CEACAM1 sequence does not contain the identified peptide.

The present disclosure surprisingly identifies the peptide SDPLKLTVK and, thus, proves the existence of a protein with a sequence predicted in the database entries Q6UY47, Q3KPI0, and 075296. So far, there are no antibodies available which specifically recognize this protein.

In addition, the present disclosure identifies the above-mentioned peptide in tumors but not in normal kidney, thus, indicating said protein as a novel marker overexpressed in human tumors, more preferably as a human kidney tumor marker, most preferably as a human kidney tumor marker readily accessible from the bloodstream and, thus, useful as a target for ligand-based tumor targeting applications.

### Protein #6

The peptide YLPFVPSR was identified as part of the protein fibromodulin (Q8IV47). Fibromodulin was first described as a 59-kDa protein (Heinegard et al., J Biol Chem, 261, 13866-72, 1986) that interacts with collagen types I and II (Hedbom and Heinegard, J Biol Chem, 264, 6898-905, 1989) and is present on collagen fibers in cartilage (Hedlund et al., Matrix Biol., 14, 227-32, 1994). Fibromodulin is thought to play an important role in collagen fiber formation as shown by the observation that FM null mice form abnormal collagen fibrils in tendons (Svensson et al., J. Biol. Chem., 274, 9636-47, 1999). The protein has been associated with a number of tumors.

Surprisingly, this disclosure reveals overexpression of the fibromodulin protein in kidney cancer, thus indicating said protein as a novel human kidney tumor marker, more preferably as a human kidney tumor marker readily accessible from the bloodstream and, thus, useful as a target for ligand-based tumor targeting applications.

### Protein #7

The peroxidasin homolog [fragment] (also designated melanoma-associated antigen MG50) (Q92626) was originally identified by a cDNA subtraction approach, in which cDNA clones were isolated with a subtracted melanoma cDNA probe (melanoma cell line minus lung carcinoma cell line) after screening a melanoma expression library by in situ plaque hybridization (Hutchins et al., Cancer Res, 51, 1418-25, 1991).

Surprisingly, the present disclosure demonstrates that peroxidasin homolog [fragment] was only identified in tumor specimen but not in normal tissue of kidney and indicates a use of this protein as a tumor marker, more preferably as a kidney tumor marker, more preferably as a tumor marker readily accessible from the bloodstream.

It is noted that the peroxidasin homolog [fragment] has so far only been postulated from the demonstration of certain mRNA sequences but the existence of the protein has not yet been demonstrated in nature.

### Protein # 8

The peptide MRAPGALLAR, surprisingly identified in kidney tumors only, matches the protein sequence of Probable G-protein coupled receptor 37 [precursor] (015354).

The orphan G protein-coupled receptor GPR37 and related genes encode a subfamily of putative G protein-coupled receptors that are highly expressed in the mammalian central nervous system.

Toyota and coworkers found that GPR37 is one of the genes exhibiting hypermethylation of promoter-associated CpG-rich regions, termed CpG islands, in acute myeloid leukemia (AML) (Toyota et al., Blood, 97, 2823-9, 2001). Such hypermethylation can result in gene silencing that is clonally propagated through mitosis by the action of DNA-methyltransferase enzymes. Such methylation-associated silencing plays a pathological role in silencing tumor-suppressor genes in neoplasia.

Surprisingly, the present disclosure identifies GPR37 in tumor but not in normal kidney and demonstrates an overexpression of this protein in tumors, thus indicating this protein as a novel marker overexpressed in human tumors, more preferably as a human kidney tumor marker, most preferably as a human kidney tumor marker readily accessible from the bloodstream and, thus, useful as a target for ligand-based tumor targeting applications.

### Protein #9

The existence of the protein sidekick-1 [precursor] (Q8TEN9) has been postulated based on sequencing full-length cDNAs (Nagase, T et al., Kazusa DNA Research Institute, direct submission to the NCBI database), but was never experimentally proven.

Surprisingly, it was demonstrated that such a protein exists (by the identification of the peptide AELTDLK, which is specific for this protein), and that it is over-expressed in and/or around tumor neo-vasculature structures, thus opening vascular targeting biomedical applications.

### Protein # 10

The protein Alpha1A-voltage-dependent calcium channel was shown to be mutated in a disease called spinocerebellar ataxia type 6 (SCA6), which is a autosomal dominant neurodegenerative disease (Toru, S et al., J. Biol. Chem. 275, 10893-8, 2000). Until now, no data concerning expression in normal tissue or tumor tissue is available.

Surprisingly, by the identification of the tryptic peptide RGALVGAPR the present disclosure demonstrates the over-expression in and/or around tumor neo-vasculature structures, thus indicating vascular targeting biomedical applications.

### Protein # 11

The EMILIN2 protein [fragment] (elastin microfibril interfase located Protein 2, Q8N5L1) is an elastic fiber-associated glycoprotein. The mRNA expression of EMILIN2 protein has been shown by the group of Colombatti (Doliana, R et al., J Biol Chem. 276, 12003-11, 2001). The protein has an expression pattern restricted to spinal cord, peripheral leukocytes, lung, placenta and fetal heart. In addition, the group presented an immuno-histochemistry of human leiomyosarcoma cells showing the partial co-localization of EMILIN1 and EMILIN2. The group of Forrest (Amma, LL et al., Mol Cell Neurosci. 23, 460-72, 2003) confirmed with a northern blot analysis the restricted expression pattern of the EMILIN2 protein to heart, lung and cochlea.

By the identification of the protein specific tryptic peptide RGALVGAPR the over-expression in and/or around tumor neo-vasculature structures was surprisingly demonstrated, thus, indicating vascular targeting biomedical applications.

### Protein # 12

The Down syndrome critical region protein 8 (also designated malignant melanoma-associated protein 1) (Q96T75) has 6 different splice-isoforms. The tryptic peptide LFMPRPK is specific for 5 of these 6 splice-isoforms (see table 1).

Surprisingly, the over-expression of one or more of the 5 splice-isoforms Q96T75, Q6EXA9, Q684H4, Q96T75-2, and Q96T75-3 of the Down syndrome critical region protein 8 (of which in total 6 isoforms are published), in and/or around tumor neo-vasculature structures was demonstrated, thus indicating vascular targeting biomedical applications.

### Protein # 13

The protein probable G-protein coupled receptor 113 [precursor] (Q8IZF5) was identified in the course of a large scale BLAST study focused on novel human G-protein coupled receptors (Fredriksson, R et al., FEBS Lett. 531, 407-14, 2002).

Surprisingly, this disclosure identifies the probable G-protein coupled receptor 113 [precursor] in tumors. By the identification of the protein specific tryptic peptide NKISYFR the over-expression in and/or around tumor neo-vasculature structures was demonstrated, thus indicating vascular targeting biomedical applications.

### Proteins # 14

The protein database entries annexin A4 (P09525) and protein ANXA4 [fragment] (Q6LES2) have the same amino acid sequence except for the first 2 amino acids of Q6LES2. Thus, the difference in these two database entries may either be a consequence of a sequencing error or there are two isoforms of this protein existing. Zimmermann et al. (Zimmermann, U et al., Cancer Lett. 209, 111-8, 2004) showed in their paper concerning clear cell renal cell carcinoma that in normal cells annexin A4 is concentrated around the nucleus, whereas the protein is localized to the basolateral membrane in tumor cells. This suggests that the subcellular distribution of annexin IV correlates with the nature of the attachment of the cell to its neighbourhood. These results indicate the possibility that annexin IV plays an important role in the morphological diversification and dissemination of the clear cell renal cell carcinoma.

Surprisingly, the present disclosure demonstrates the over-expression in and/or around tumor neo-vasculature structures by the identification of the protein specific tryptic peptide ISQTYQQQYGR, thus indicating vascular targeting biomedical applications.

### Protein # 15 (4 x)

The uromodulin-like 1 [precursor] (also designated olfactorin) (Q5DID0, Q5DID0-2, Q5DID0-3, and Q5DID0-3) was identified as a novel membrane bound protein specifically expressed by olfactory and vomeronasal sensory neurons (Di Schiavi, E. et al., Eur. J. Neurosci. 21, 3291-300, 2005). The group transfected HEK-cells with olfactorin fused to a flag-tag and identified this fusion-protein with an anti-flag antibody.

Surprisingly, the present disclosure demonstrates the over-expression in and/or around tumor neo-vasculature structures by the identification of the protein-specific tryptic peptide IVNHNLTEKLLNR, thus indicating vascular targeting biomedical applications. In the prior art the protein olfactorin is also known as uromodulin-like protein, also having four different splice isoforms (see table 1).

### Protein # 16

The protein scavenger receptor class F member 2 [precursor] (Q96GP6) has been demonstrated to be expressed in the mouse during embryogenesis in the hair follicle, skin and nasal epithelium as well as in the tongue and oral epithelia, rib bone undergoing ossification and in the medullar region of thymus (Hwang, M et al., Gene Expr Patterns. 5, 801-8, 2005).

Surprisingly, the over-expression in and/or around tumor neo-vasculature structures was demonstrated by the identification of the protein specific tryptic peptide GAGPARRR, thus indicating vascular targeting biomedical applications.

### Protein # 17

The sushi domain-containing protein 2 [precursor] (Q9UGT4) was unambiguously identified by 2D-PAGE and MALDI mass spectrometry by the group of Lubec (Lubec, G. et al., J. Chem. Neuroanat. 26, 171-8, 2003) in the human cortical neuronal cell line HCN-2.

Surprisingly, the over-expression in and/or around tumor neo-vasculature structures was demonstrated by the identification of the protein specific tryptic peptide VAHQLHQR, thus indicating vascular targeting biomedical applications.

### Protein # 18

The Tumor protein, translationally controlled 1 (TCTP) has been known for more than 20 years. In the review of Bommer and Thiele (Int. J. Biochem. Cell Biol. 36, 379-85, 2004) the importance of TCTP for cell growth and its anti-apoptotic activity are highlighted.

Surprisingly, the over-expression in and/or around tumor neo-vasculature structures was demonstrated by the identification of the protein specific tryptic peptide KWVKINNVK, thus indicating vascular targeting biomedical applications.

### Protein # 19

The existence of the putative G-protein coupled receptor (Q8TDU0) was postulated based on sequence homology searches in the human genome (Takeda, S et al., FEBS Lett. 520, 97-101, 2002), but was never experimentally proven.

The present disclosure surprisingly demonstrates that such a protein actually exists (by the identification of the peptide LSVVEAPCR, which is specific for this protein), and also shows that it is over-expressed in and/or around tumor neo-vasculature structures, thus indicating vascular targeting biomedical applications.

### Protein # 20

The existence of the hypothetical protein DKFZp686K0275 (Q7Z3A1) was postulated based on sequencing of full-length cDNAs (Wiemann, S et al., Molecular Genome Analysis, German Cancer Research Center (DKFZ), direct submission to the NCBI homepage), but was never experimentally proven.

The present disclosure surprisingly demonstrates that such a protein actually exists (by the identification of the peptide AGQGFGLR, which is specific for this protein), and also demonstrates that it is over-expressed in and/or around tumor neo-vasculature structures, thus indicating vascular targeting biomedical applications.

### Protein # 21

The existence of the transmembrane protein TMEM55A (Q8N4L2) was postulated based on sequencing of full-length cDNAs (Strausberg, RL. et al., Proc. Natl. Acad. Sci. USA. 99, 16899-903, 2002), but was never experimentally proven.
The present disclosure surprisingly demonstrates that such a protein actually exists (by the identification of the peptide KISSVGSALPR, which is specific for this protein), and also demonstrates that it is over-expressed in and/or around tumor neo-vasculature structures, thus indicating vascular targeting biomedical applications.

### Protein # 22

The existence of the hypothetical protein (Q8WYY4) was postulated based on sequencing of full-length cDNAs (Gu, JR. et al., National Laboratory For Oncogenes & Related Genes, Shanghai Cancer Institute, direct submission to the NCBI homepage), but was never experimentally proven.
The present disclosure surprisingly demonstrates that such a protein actually exists (by the identification of the peptide VLTAMVGK, which is specific for this protein), also demonstrates that it is over-expressed in and/or around tumor neo-vasculature structures, thus indicating vascular targeting biomedical applications.

### Protein # 23

The existence of the family with sequence similarity 116, member A (Q8IWF6) was postulated based on sequencing of full-length cDNAs (Strausberg, RL. et al., Proc. Natl. Acad. Sci. USA. 99, 16899-903, 2002), but was never experimentally proven.

The present disclosure surprisingly demonstrates that such a protein actually exists (by the identification of the peptide GPAGLGPGSR, which is specific for this protein), and also demonstrates that it is over-expressed in and/or around tumor neo-vasculature structures, thus indicating vascular targeting biomedical applications.

### Protein # 24

The protein UPF0260 protein C6orf66 (Q9P032), which is also known as HRPAP20 (hormone-regulated proliferation-associated protein 20 kDa), had been demonstrated to have an increased proliferation in the absence of hormone stimulation and augmented survival in the absence of serum in stable transfected MCF-7 (human breast carcinoma) cells. Karp et al. (Karp, CM et al., Cancer Res. 64, 1016-25, 2004) conclude that HRPAP20 is a phospho-protein that is required for proliferation and survival of hormone dependent tumor cells.
The present disclosure demonstrates for the first time the over-expression of the protein (by identification of the protein specific tryptic peptide MGALVIR) in a human tumor and more preferably in human tumor neo-vasculature structures, thus indicating vascular targeting biomedical applications.

### Protein # 25

The existence of the protein CDNA FLJ45811 fis, clone NT2RP7014778 (Q6ZS59) was postulated based on sequencing of full-length cDNAs (Isogai, T et al., NEDO human cDNA sequencing project, direct submission to the NCBI homepage), but was never experimentally proven.
The present disclosure surprisingly demonstrates that such a protein actually exists (by the identification of the peptide QFWLGGVAR, which is specific for this protein), and also demonstrates that it is over-expressed in and/or around tumor neo-vasculature structures, thus indicating vascular targeting biomedical applications.

### Proteins # 26 & 27

The identified peptide (EAFEAASR) does not allow for distinguishing between the two proteins hypothetical proteins DKFZp779O1248 (Q6AHZ8) and beta-ureidopropionase (Q9UBR1). The RZPD homepage (http://www.rzpd.de) links the hypothetical protein DKFZp779O1248 to beta-ureidopropionase. Beta-ureidopropionase is a protein whose deficiency leads to inborn errors of the pyrimidine degradation pathway (van Kuilenburg, AB et al., Hum Mol Genet. 13, 2793-801, 2004). There are no data published implicating beta-ureidopropionase and cancer. Surprisingly, the present disclosure demonstrates the over-expression in and/or around tumor neo-vasculature structures, thus indicating vascular targeting biomedical applications.

### Protein # 28

The existence of the hypothetical protein DKFZp434F1919 (Q9GZU6) and its isoform MDS011 (Q9GZT6) (the two proteins have 99.6% amino acid sequence identity) was postulated based on sequencing of full-length cDNAs (Ota, T et al., Nature Genetics 36, 40 - 45, 2004), but was never experimentally proven.

The present disclosure surprisingly demonstrates that such a protein actually exists (by the identification of the peptide IDAEIASLK, which is specific for this protein), and also demonstrats that it is over-expressed in and/or around tumor neo-vasculature structures, thus indicating vascular targeting biomedical applications.

### Protein # 29

The expression of the protein cysteine-rich with EGF-like domain protein 2 [precursor] (six isoforms, see table 1) has been studied for different normal human tissues (northern blot) and brain (immuno-histochemistry) (Ortiz, JA et al., J Neurochem. 95, 1585-96, 2005), but no data about tumor tissue is available.

Surprisingly, the present disclosure demonstrates the over-expression in and/or around tumor neo-vasculature structures, thus indicating vascular targeting biomedical applications.

### Protein # 30

The existence of the UPF0378 family protein KIAA0100 [precursor] (Q5H9T4) was postulated based on sequencing cDNAs (Ottenwaelder, B. et al., The German cDNA Consortium, direct submission to the NCBI database), but was never experimentally proven.

The present disclosure surprisingly demonstrates that such a protein actually exists (by the identification of the peptide KLQAELK, which is specific for this protein), and also demonstrate that it is over-expressed in and/or around tumor neo-vasculature structures, thus indicating vascular targeting biomedical applications.

### Protein # 31

In the present disclosure the peptide SPILAEVK was identified as part of the protein potassium voltage-gated channel subfamily H member 1 (095259). Two isoforms of this protein exist, both of which contain this peptide (095259 & 095259-2 (hEAG)).

Pardo and colleagues (Pardo et al., EMBO J., 18, 5540-5547, 1999) showed that the inhibition of this protein expression causes a significant reduction of cell proliferation. They showed expression of KCNH1 in breast and brain tumor cells. In addition, expression was detected by immunohistochemistry in cervix cancer (Farias et al., Cancer Res., 64, 6996-7001, 2004).

Surprisingly, this disclosure reveals overexpression of the protein potassium voltage-gated channel subfamily H member 1 in kidney cancer, thus, indicating said protein as a novel human kidney tumor marker, more preferably as a human kidney tumor marker readily accessible from the bloodstream and, thus, useful as a target for ligand-based tumor targeting applications.

### Brief description of the figures

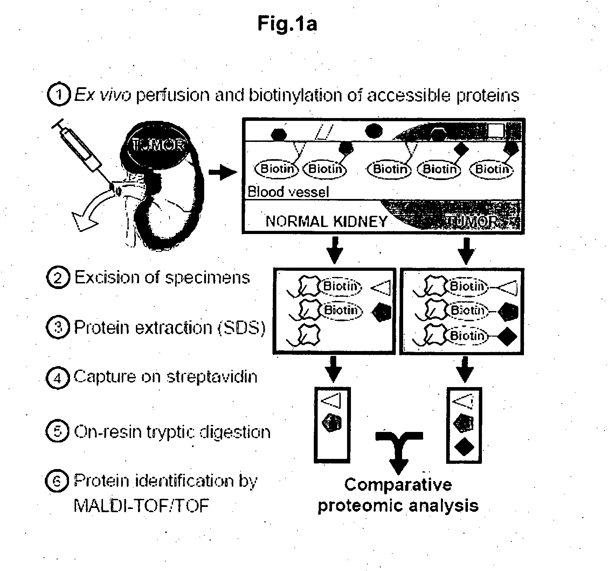

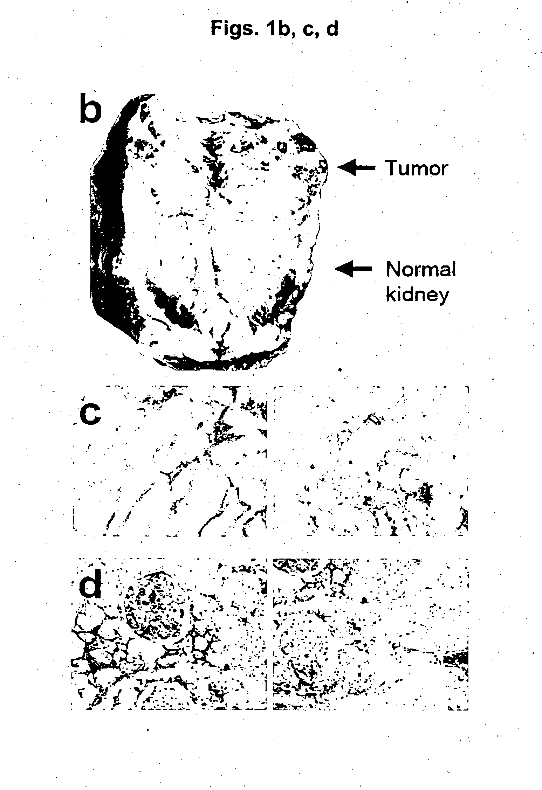

**Fig. 1****. (A)** is a schematic representation of the *ex vivo* kidney perfusion procedure employed for identifying tumor markers in the kidney's vasculature. Within two minutes after nephrectomy, tumor bearing kidney is perfused with a reactive ester derivative of biotin, thus washing away blood components and biotinylating accessible proteins. Biotinylated tissue specimens can be cut, processed separately for the purification of biotinylated proteins, yielding tryptic peptides which are separated by nano-HPLC and analyzed by matrix-assisted laser desorption ionization-tandem time-of-flight (MALDI-TOF/TOF) mass spectrometry. **(B)** Tumor-bearing kidney, cut in half, after *ex vivo* perfusion. Biotinylated structures in tissue sections are detected in the tumor portion **(C)** and in the normal kidney portion **(D)** using avidin-horseradish peroxidase-based staining protocols. Vascular structures are preferentially biotinylated in the neoplastic mass.

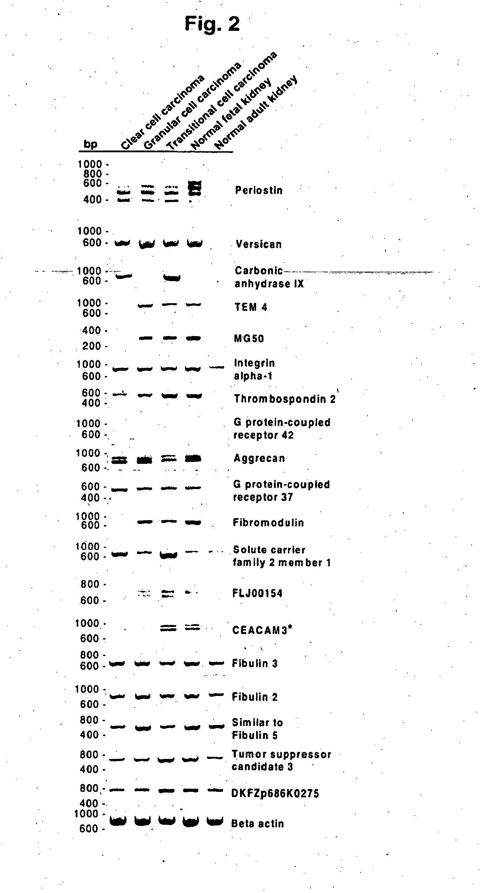

**Fig. 2** shows the target validation by semi-quantititative PCR analysis of cDNA libraries [clear cell carcinoma, lane 1; granular cell carcinoma, lane 2; transitional cell carcinoma, lane 3; normal fetal kidney, lane 4; normal adult kidney, lane 5]. (*) Unlike other proteins, the confidence of the assignment of CEACAM3 with the MASCOT software was below 95% for the best peptide ion and is not unambiguous even after visual inspection of the MS-MS spectra.

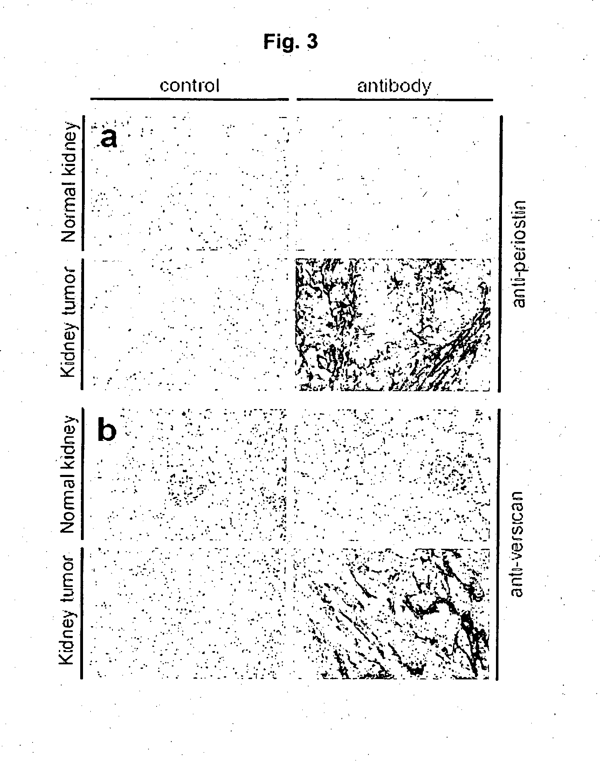

**Fig. 3** is the result of the immunohistochemical analysis of normal kidney and tumor sections with antibodies specific to periostin **(A)** and versican **(B).** Staining with anti-periostin antibody exhibited a low background staining in the normal kidney samples, but revealed a strong over-expression in 8 out of 8 tumors investigated. Versican was strongly over-expressed in 6 out of 8 tumors, but did not stain normal kidneys and other normal tissues. The staining reactions were absent in negative control experiments, omitting the primary antibodies. Scale bars = XXX.

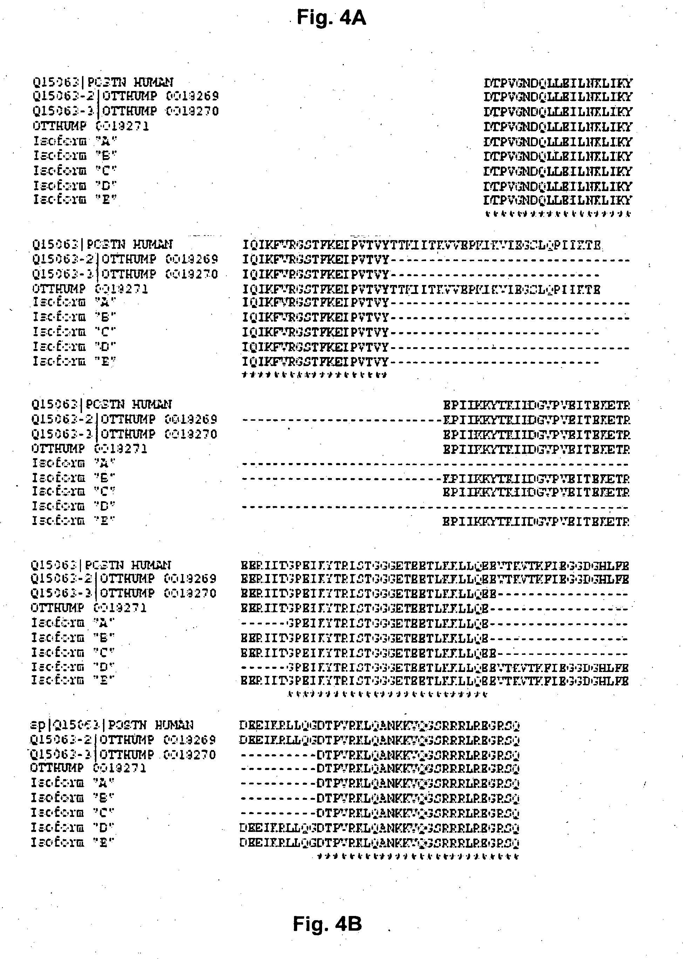

**Fig. 4 (A)** shows the alignment of protein sequences of the various isoforms of periostin limited to the carboxy-terminal region of the protein where the alternative splicing occurs.

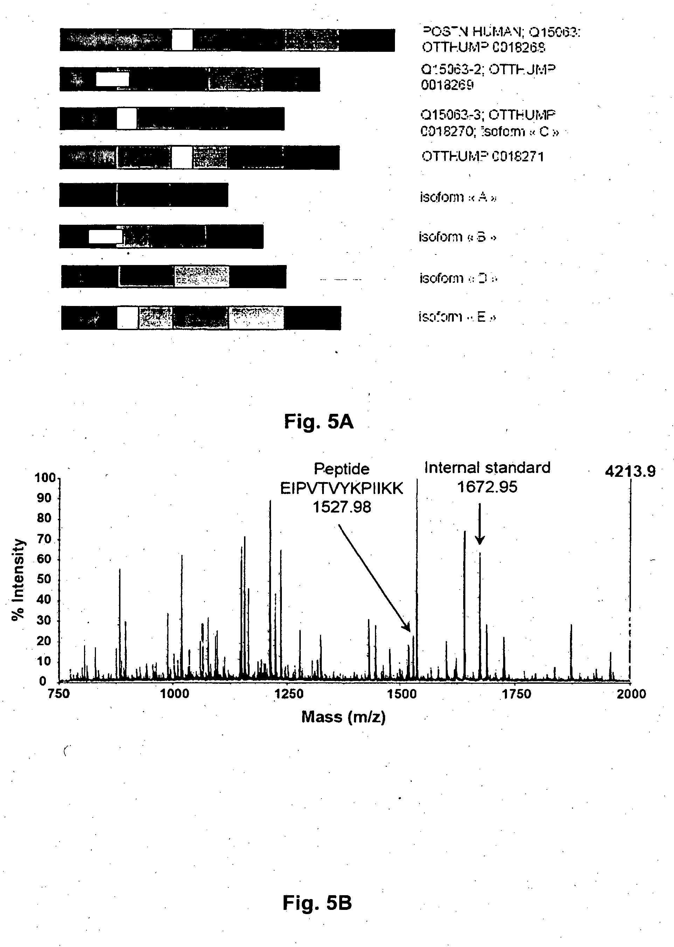

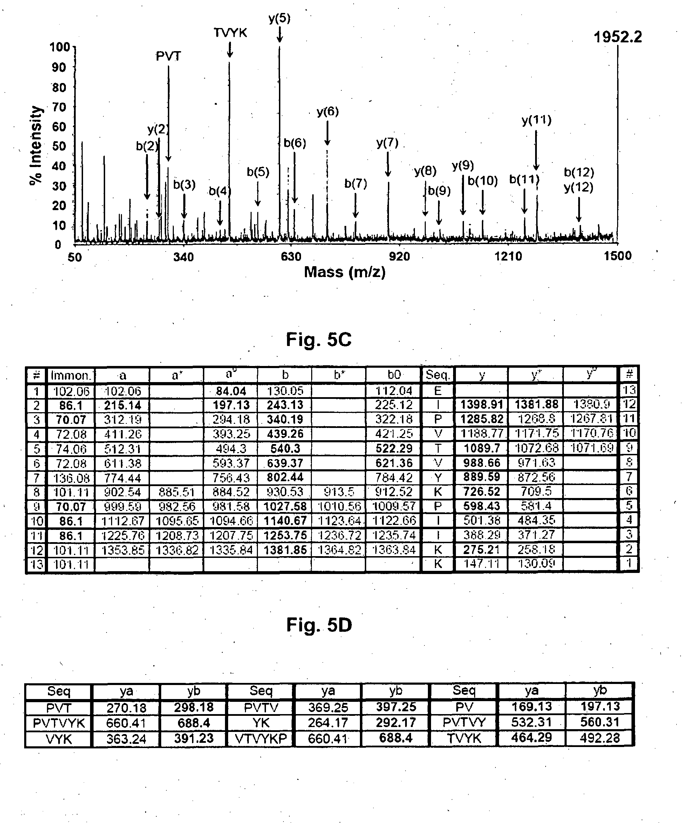

**Fig. 4 (B)** is a graphic representation of the different combinations of exons that correspond to the regions shown in Panel A. The isoforms identified in this study are named « A » to « E ». The pale blue rectangle in corresponds to the isoform-specific peptide EIPVTVYKPIIKK we identified (see Fig. 5).
**Fig. 5** Identification of isoform-specific peptides of periostin. **(A)** MALDI-TOF spectrum of an HPLC fraction containing a peptide with a mass-to-charge ratio of 1527.98. **(B)** The sequence of this peptide (EIPVTVYKPIIKK) was determined by MALDI-TOF/TOF. In the table of theoretical **(C)** peptide fragment ions and (**D**) internal fragment ions, the identified ions are marked in bold. The peptide EIPVTVYKPIIKK (pale blue rectangle in Figure S1, panel B) encompasses the junction between two exons which are only present in isoforms Q15063-2 and « B ».

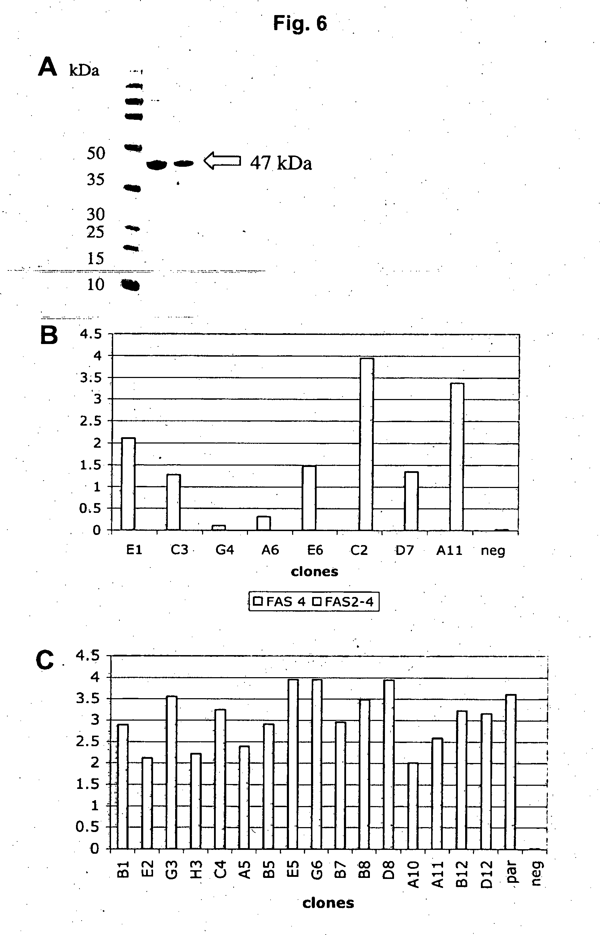

**Fig. 6****. (A)** Polyacrylamide gel electrophoresis (SDS-PAGE) analysis of the purified recombinant fragment (47 kDa) of periostin that was used as antigen in the antibody phage display selections. **(B)** Graphic representation of the ELISA results with selected-scFv clones after 2 rounds panning against FAS2-FAS4 or FAS4. Positive ELISA signals show binding affinity of the particular antibody clone to the antigen. The best clones were selected for further characterization. **(C)** Graphic representation of the results of the ELISA screening after the first round of affinity maturation of clone C2 against FAS2-FAS4.
**Fig**. **7****.** Immunohistochemical staining on tissue sections of a human kidney tumor. **(A)** Staining with the selected human monoclonal scFv antibody (clone C2) against periostin domains FAS2-FAS4 showed strong positive staining of extracellular structures mainly around tumor blood vessels (white arrow points to positive staining around a tumor blood vessel). **(B)** A negative control using the same staining protocol but omitting the scFv resulted in no specific staining.

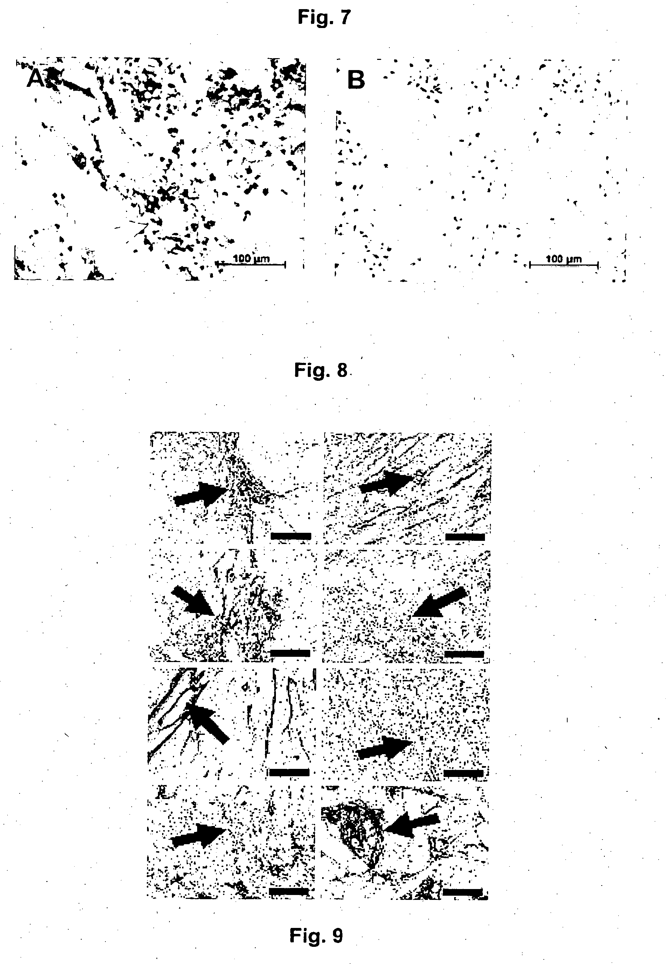

**Fig. 8****.** Immunohistochemical detection of periostin in several patients with renal clear cell carcinoma. Immunohistochemical staining revealed a strong over-expression of periostin in 8/8 tumors investigated. Black arrows point to selected areas with positive staining. Scale bars, 100 µm.
**Fig. 9****.** Immunohistochemical detection of versican in several patients with renal clear cell carcinoma. Immunohistochemical staining revealed a strong over-expression of versican in 6/8 tumors investigated. Staining is located in the extracellular matrix, also around tumor blood vessels. Black arrows point to selected areas with positive staining. Scale bars, 25 µm.

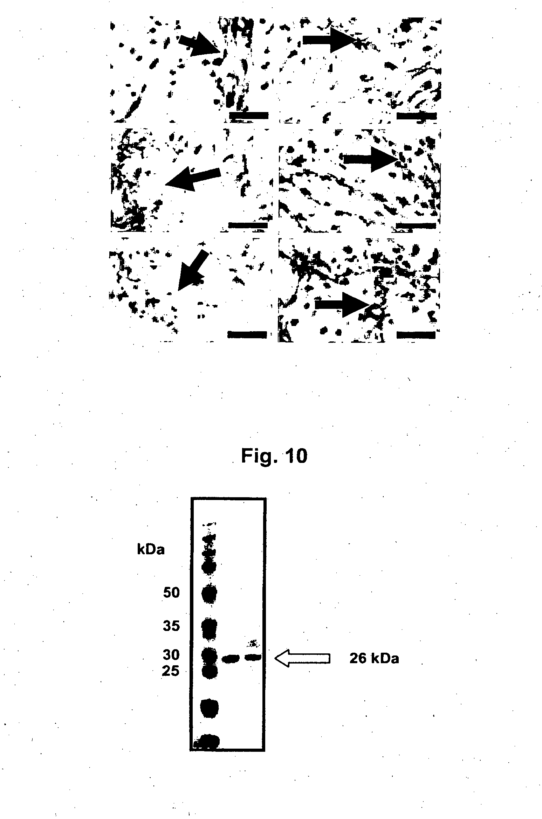

**Fig. 10****.** Polyacrylamide gel electrophoresis (SDS-PAGE) analysis of the purified recombinant fragment (26 kDa) of CEACAM3.

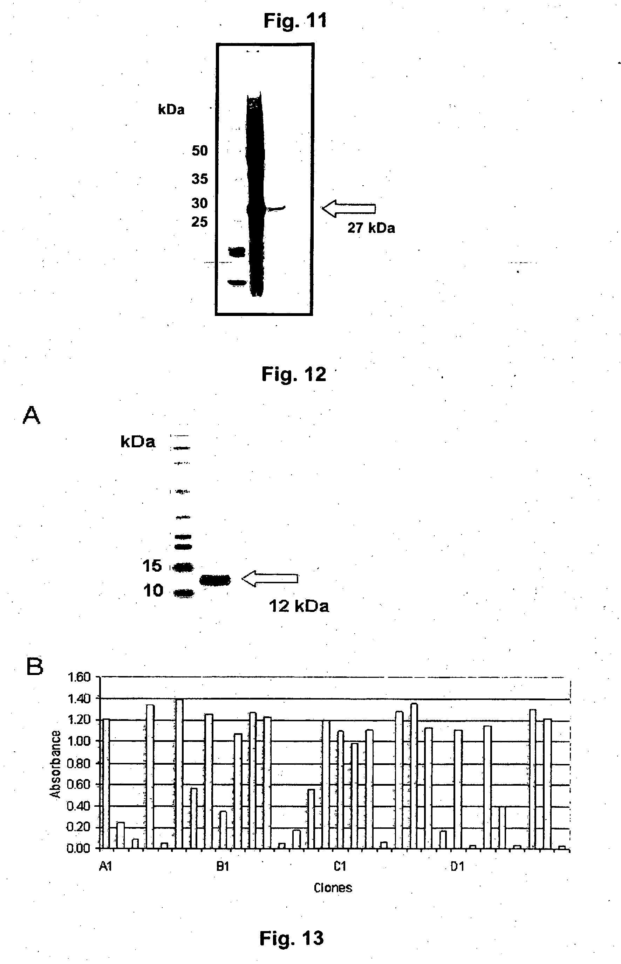

**Fig. 11****.** Polyacrylamide gel electrophoresis (SDS-PAGE) analysis of the purified recombinant fragment (27 kDa) of fibromodulin.
**Fig. 12. (A)** Polyacrylamide gel electrophoresis (SDS-PAGE) analysis of the purified recombinant fragment (12 kDa) of melanoma-associated antigen MG50 that was used as antigen in the antibody phage display selections. **(B)** Graphic representation of the ELISA results with selected scFv clones after 3 rounds of panning against against the melanoma-associated antigen MG50 fragment. Positive ELISA signals show binding affinity of the particular antibody clone to the antigen. The best clones were selected for further characterization.

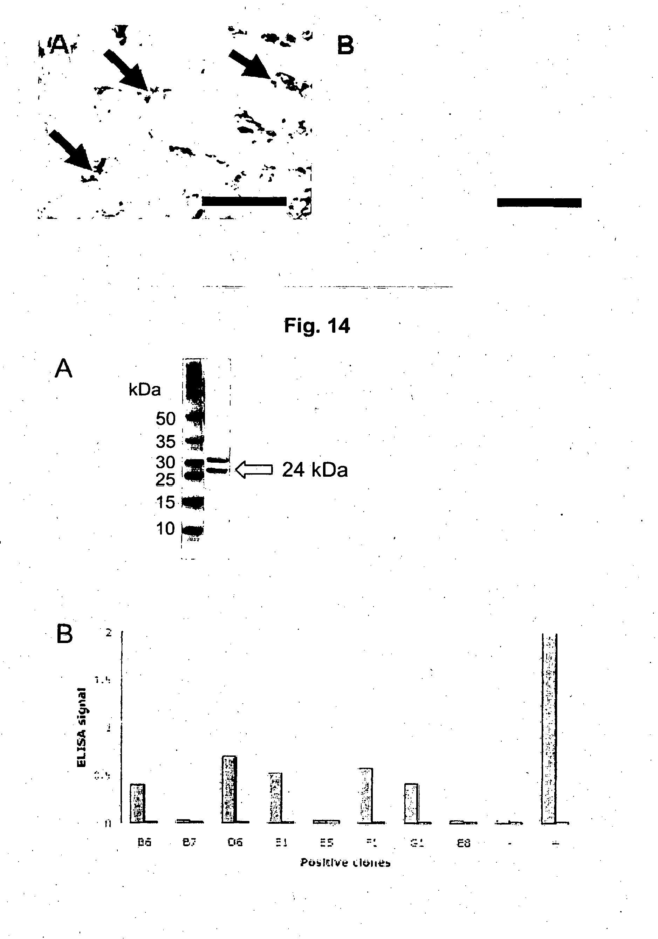

**Fig. 13****.** Immunohistochemical staining on tissue sections of a human kidney tumor. **(A)** Staining with the selected human monoclonal scFv antibody (clone F6) against the melanoma-associated antigen MG50 domains fragment showed strong positive staining mainly around tumor blood vessels (black arrows point to some of the spots of positive staining around tumor blood vessels). **(B)** A negative control using the same staining protocol but omitting the scFv resulted in no specific staining.
**Fig. 14. (A)** Polyacrylamide gel electrophoresis (SDS-PAGE) analysis of the purified recombinant fragment (24 kDa) of Protein sidekick-1 that was used as antigen in the antibody phage display selections. **(B)** Graphic representation of the ELISA results with selected scFv clones after 2 rounds of panning against against the Protein sidekick-1 fragment. Positive ELISA signals show binding affinity of the particular antibody clone to the antigen. The best clones were selected for further characterization.

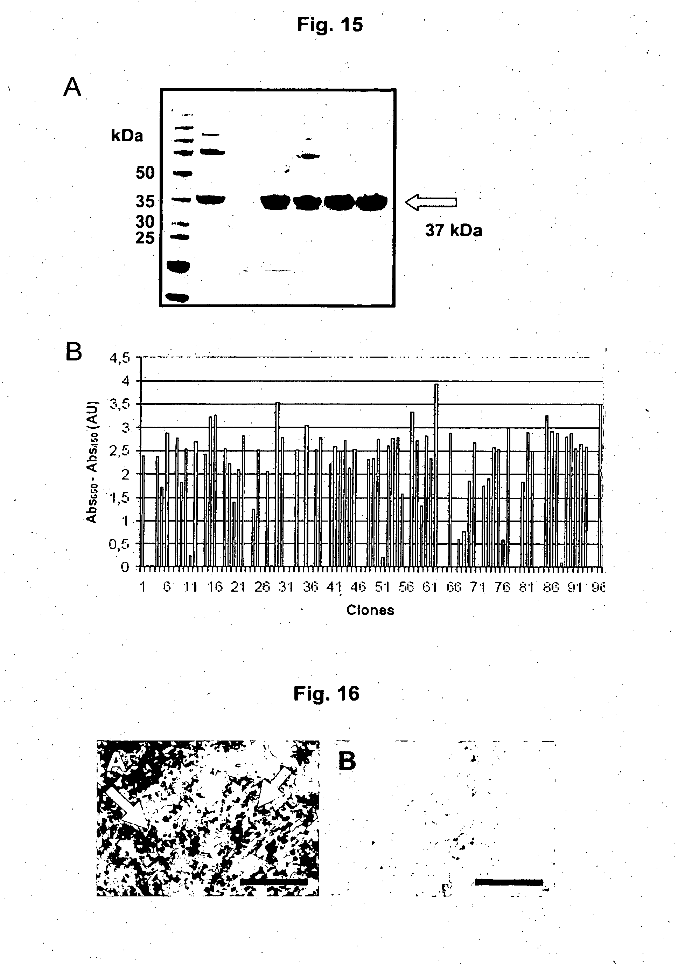

**Fig. 15****. (A)** Polyacrylamide gel electrophoresis (SDS-PAGE) analysis of the purified recombinant fragment (37 kDa) of ANXA4 protein that was used as antigen in the antibody phage display selections. **(B)** Graphic representation of the ELISA results with selected scFv clones after 2 rounds panning against the recombinant ANXA4 protein. Positive ELISA signals show binding affinity of the particular antibody clone to the antigen. The best clones were selected for further characterization.
**Fig. 16****.** Immunohistochemical staining on tissue sections of a human kidney tumor. **(A)** Staining with the selected human monoclonal scFv antibody (clone E11) against recombinant ANXA4 protein showed strong positive staining of tumor cells, including those around tumor blood vessels (white arrows point to some of the spots with positive staining; in fact, most of the tissue is positive). (**B**) A negative control using the same staining protocol but omitting the scFv resulted in only few areas with weak staining. Scale bars, 100 µm.

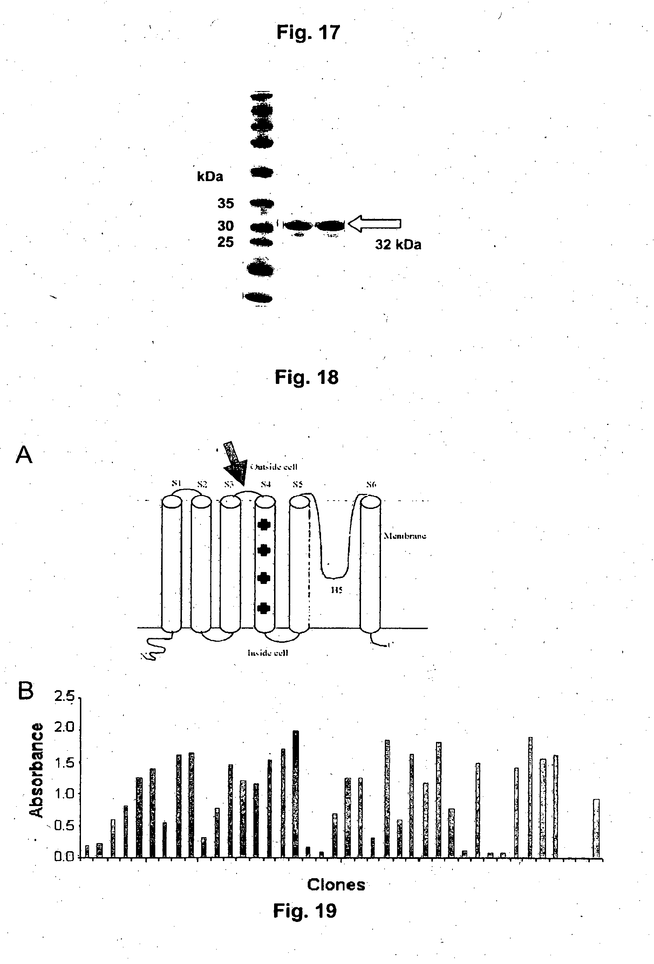

**Fig. 17****.** Polyacrylamide gel electrophoresis (SDS-PAGE) analysis of the purified recombinant fragment (27 kDa) of UPF0378 family protein KIAA0100.
**Fig. 18****. (A)** Schematic representation of the Potassium voltage-gated channel subfamily H member 1. The peptide used as antigen in the phage display selections corresponded to a the second extracellular loop of this membrane protein (see red arrow). **(B)** Graphic representation of the results of the ELISA screening after the first round of affinity maturation of clone H9 against the Potassium voltage-gated channel subfamily H member 1 peptide. Positive ELISA signals show binding affinity of the particular antibody clone to the antigen.

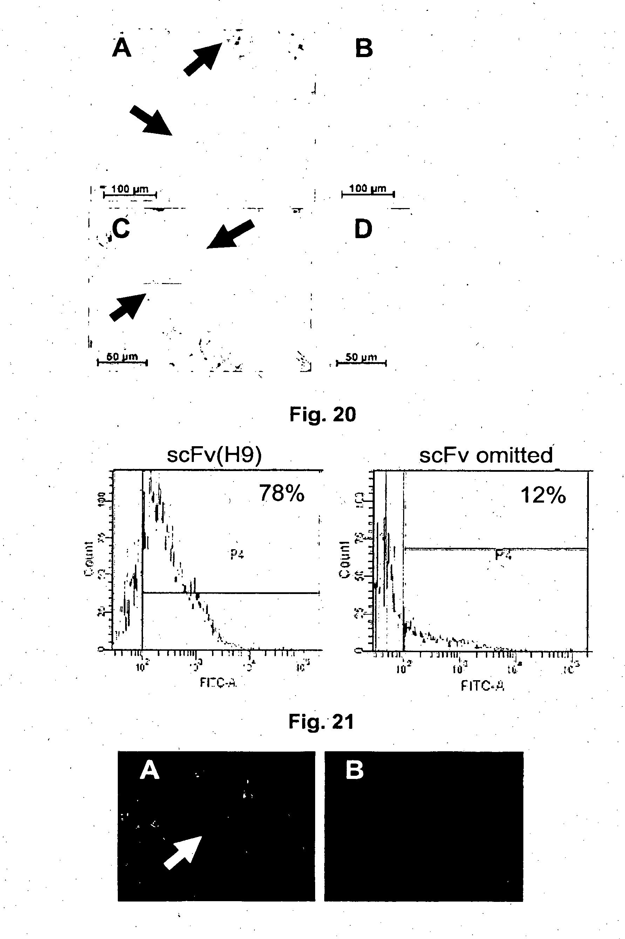

**Fig. 19****.** Immunohistochemical staining on tissue sections of human tumors. Staining with the selected human monoclonal scFv antibody (clone H9) against Potassium voltage-gated channel subfamily H member 1 showed strong positive staining of tumor cell membranes in tissue section of human kidney tumors **(A)** and of human lung tumors **(C)** (black arrows point to positive staining of cell membranes). Negative controls using the same staining protocol but omitting the scFv resulted in no specific staining both in the kidney tumor tissue **(B)** and the lung tumor tissue **(D).**
**Fig. 20****.** FACS experiment with HeLa cells. Treatment of the cells with scFv(H9) selected against Potassium voltage-gated channel subfamily H member 1 as primary antibody, mouse anti-c-myc (clone 9E10) as secondary and FITC-labeled anti-mouse Ig as tertiary antibody revealed a shift in the FACS (left panel) compared to cells treated with the same procedure only omitting the scFv (right panel), indicating a successful binding of scFv(H9) to the HeLa cells.
**Fig. 21****.** Immunocytochemistry experiment with HeLa cells. Staining of cultured HeLa cells with the selected human monoclonal scFv antibody (clone H9) against Potassium voltage-gated channel subfamily H member 1 showed strong positive staining of tumor cell membranes **(A)** (the white arrow points to positive staining of cell membranes). Negative controls using the same staining protocol but omitting the scFv resulted in no specific staining **(B).**

The following examples are provided to better illustrate the present invention in more detail. They are not to be construed as limiting to the scope of the claims in any way.

Example 1 below demonstrates the overexpression of the marker proteins identified in neovascular structures, in particular their overexpression in tumors, more specifically in kidney tumors.

Furthermore, examples 2 to 10 below demonstrate the recombinant production of selected vascular marker proteins (or fragments thereof) and their utility as antigens for producing antibodies against these vascular marker proteins. Such antibodies (or other ligands with the same selective affinity) are useful for further characterizations and biomedical applications of these marker proteins. Furthermore, some of the examples prove the practical utility of selected marker proteins for identifying neovascular structures, in particular neovascular structures in tumors.

### Examples

### Example 1

### Introduction

A chemical proteomic approach based on the ex *vivo* perfusion and biotinylation of accessible structures within surgically-resected human kidneys with tumor was used to gain information about accessible and abundant antigens which are over-expressed in human cancer. Biotinylated proteins were purified on streptavidin resin and identified using mass spectrometric methodologies, revealing 637 proteins, of which 184 were found in tumor specimens only and 223 in portions of normal kidneys only. Thirty of the accessible tumor-associated antigens identified with this methodology are suitable targets for antibody-based anti-cancer therapies.

The specific method used for the identification of accessible antigens in normal organs and in tumors is based on the terminal perfusion of tumor-bearing mice with reactive ester derivatives of biotin that was recently published by the present inventors (Rybak et al. 2005 *supra).* This methodology allows the efficient biotinylation of accessible proteins on the membrane of endothelial cells and on other structures (e.g. extracellular matrix components) which are readily accessible from the bloodstream. The purification of biotinylated proteins from organ lysates on streptavidin resin, followed by a comparative proteomic analysis based on mass spectrometry, allowed the identification of hundreds of accessible proteins, some of which were found to be differentially expressed in organs and in tumors.

The above biotinylation procedure was applied to the ex *vivo* perfusion of three surgically-resected kidneys from patients with renal cell carcinoma (Fig. 1 and Table 2). This procedure lasted 7-9 minutes and allowed the efficient and selective labeling of vascular structures in the tumor portions (Fig. 1 C), while both vascular and tubular structures were labeled in the normal kidney portions (Fig. 1D).

After biotinylation and quenching of excess biotinylation reagent with a primary amine-containing solution (Tris), specimens were excised, homogenized in the presence of SDS and loaded onto streptavidin resin, thus enriching for biotinylated proteins. A subsequent proteolytic digestion, followed by nanoHPLC peptide separation and mass spectrometric analysis by MALDI-TOF/TOF allowed for the identification of a total of 637 proteins in all specimens.

As expected, abundant proteins observed in both normal and neoplastic specimens included components of the extracellular matrix, such as collagens, laminin, perlecan, lumican, vitronectin, fibronectin, and tenascin. Proteins found exclusively in the normal kidney portions included the kidney-specific cadherin 16, several transporters, apolipoprotein E and uromodulin. A number of proteins were found exclusively in the tumor specimens. Some of these had previously been-reported to be overexpressed in certain neoplastic structures [e.g., carbonic anhydrase IX, TEM4, Peroxidasin homolog [fragment], Down syndrome critical region protein 8, integrin alpha-1, ectonucleotide pyrophosphatase/- phosphodiesterase 3]. Only a small portion of the tumor antigens identified in this analysis (e.g., netrin receptor DCC, solute carrier family 2, facilitated glucose transporter member 1, neural cell adhesion molecule 1) have so far been reported in the "Human Protein Atlas": a genome-wide initiative for the characterization of protein expression patterns in normal tissues and cancer.

Since the detection of a protein in the tumor specimens might in principle reflect not only a preferential pattern of expression, but also a differential accessibility to the biotinylation reagent, selected protein candidates were characterized both by immunohistochemistry and by PCR analysis of cDNA libraries. Periostin was the most abundant tumor-associated antigen in this analysis and represents a particularly interesting marker, having been found to be up-regulated in epithelial ovarian tumors, breast cancer, at the periphery of lung carcinomas, and in colorectal cancers and their liver metastases. The existence of five different splice isoforms of periostin in human has been reported, but sequences of only three isoforms are published (Swiss-Prot/TrEMBL and NCBI Protein). However, six isoform sequences were identified in the PCR analysis of cDNA libraries, with different relative abundance in normal, fetal and tumor kidney (Fig. 2 and Fig. 4). An immunohistochemical analysis of normal kidney and clear cell carcinoma specimens revealed a striking over-expression of periostin in the tumors, with a prominent vascular and stromal pattern of staining (Fig. 3). Similarly, versican was found to be more abundant in fetal and tumor specimens, both by PCR (Fig. 2) and by immunohistochemical analysis (Fig. 3).

Some of the putative tumor-associated antigens were found to be present also in cDNA libraries of normal kidney (Fig. 2), including fibulins, tumor suppressor candidate 3 (N33 protein) and the hypothetical protein DKFZp686K0275 [fragment] (Fig. 2). By contrast, a number of interesting markers yielded substantially stronger PCR bands in fetal and tumor specimens including carbonic anhydrase IX, TEM-4, Peroxidasin homolog [fragment], integrin alpha-1, thrombospondin 2, putative G-protein-coupled receptor 42, aggrecan, probable G-protein-coupled receptor 37 [precursor], fibromodulin, solute carrier family 2, facilitated glucose transporter member 1, and Protein sidekick-1 [precursor] (Fig. 2).

Among the 637 proteins identified in this analysis, approx. 20% corresponded to intracellular proteins. While some more abundant intracellular proteins (e.g. actin, tubulin, keratin, histones) could be recovered either by stickiness to the streptavidin resin or as a consequence of biotinylating necrotic structures *ex vivo,* some intracellular proteins have been reported to become accessible on the surface of proliferating endothelial cells.

### Materials and Methods

### Patients

This study was started upon approval by the ethical committee of the University Hospital of Liège (Belgium). Criteria adopted for patient selection were as follows: 1) diagnosis of a tumor highly compatible with a clear cell carcinoma of the kidney, as assessed by routine ultrasound and abdomen CT scan; 2) a therapeutic indication for a total nephrectomy; 3) a tumor size and localization that allowed to clearly distinguish healthy portions of the kidney to be used as normal controls. Immunohistochemical procedures compatible with the detection of specific proteins without biotin interference were adopted for the diagnostic histopathological analysis. Patient's informed consent was obtained and serology for negativity to HIV and Hepatitis A, B, and C was performed. For specific information about the patients see Table S1.

### Ex vivo vascular perfusion

Surgery was performed according to a standard procedure, which includes the ligation and section of renal artery, vein, and ureter, and subsequent nephrectomy. The renal artery carried a longer suture for immediate identification in the perfusion step. Within 2 min after nephrectomy, the renal artery was cannulated, the renal vein was opened (by removing the suture) to allow outflow of the perfusate, and perfusion via the renal artery was started. Kidneys were first perfused 7-9 min with 500 ml of a 1 mg/ml solution of sulfo-NHS-LC-biotin in PBS, washing away blood components and labelling accessible primary amine-containing structures with biotin. Immediately afterwards, a second perfusion step with 450 ml PBS containing a 50 mM solution of the primary amine Tris-(hydroxymethyl)-aminomethane (Tris) was performed for 8-9 min to quench unreacted biotinylation reagent. All perfusion solutions contained 10% dextran-40 as a plasma expander and were pre-warmed to 40°C. Both perfusion steps were performed with a pressure of 100-150 mm Hg. Successful perfusion was indicated by the wash out of blood during the first minutes of perfusion and subsequent flow of clear perfusate out of the renal vein. After perfusion, the organs were washed with 50 mM Tris in PBS, dried, rubbed with black ink to allow the later pathologic investigation of surgical margins, and cut in half along the sagittal axis (starting at the external medial edge) with a blade. Successful perfusion resulted in a whitish color of the tissue. Specimens from the tumor and from the normal kidney tissue (unaffected by the tumor) were excised (from the well perfused, whitish parts) and immediately snap-frozen for proteomic and histochemical analyses, or paraformaldehyde-fixed and paraffin-embedded for histochemical analyses. As a negative control, unperfused organs after nephrectomy were cut in half and specimens were taken as described above from the tumor and from normal kidney tissue. For specific information about the examined organs see Table 2.

### Histochemical staining of tissue sections with avidin-biotinylated peroxidase complex

Sections from paraformaldehyde-fixed, paraffin-embbeded tissue specimens were stained with avidin-biotin-peroxidase complex (Vectastain Elite ABC kit (Vector Laboratories, Burlingame, CA, USA)) according to standard procedures.

### Preparation of protein extracts for proteomic analysis

Specimens from healthy kidney and clear cell carcinoma tissue of human cancer patients were resuspended in 40 µl per mg tissue of a lysis buffer containing 2% SDS, 50 mM Tris, 10 mM EDTA, CompleteE proteinase inhibitor cocktail (Roche Diagnostics, Mannheim, Germany) in PBS, pH 7.4 and homogenized using an Ultra-Turrax T8 disperser (IKA-Werke, Staufen, Germany) applying six intervals of 2 min full power and 2 min standby at moderate cooling. Homogenates were sonicated (6 intervals of 30 s and 1 min standby at moderate cooling) using a Vibra-cell (Sonics, New Town, CT, USA), followed by 15 min incubation at 99°C and 20 min centrifugation at 15000 x g. The supernatant was used as total protein- extract. Protein concentration was determined using the BCA Protein Assay Reagent Kit (Pierce).

### Purification of biotinylated proteins

SA-sepharose slurry (64 µl/mg total protein) was washed three times in buffer A (NP40 1%, SDS 0.1 % in PBS), pelleted and the supernatant removed. Fifteen milligrams of total protein extract from the different specimens were mixed to the pellet of SA-Sepharose. Capture of biotinylated proteins was allowed to proceed for 2 h at RT in a revolving mixer. The supernatant was removed and the resin washed three times with buffer A, two times with buffer B (NP40 0.1%, NaCl 1 M in PBS), and once with 50 mM ammonium bicarbonate. Finally, the resin was resuspended in 400 µl of a 50 mM solution of ammonium bicarbonate and 20 µl of sequencing grade modified porcine trypsin (stock solution of 40 ng/µl in 50 mM ammonium bicarbonate) (Promega, Madison, WI, USA) were added. Protease digestion was carried out overnight at 37°C under constant agitation. The supernatants were collected and trifluoracetic acid was added to a final concentration of 0.1%. Peptides were desalted, purified and concentrated with C18 microcolumns (ZipTip C18, Millipore, Billerica, MA, USA). After lyophilization peptides were stored at -20°C.

### Nano capillary-HPLC with automated online fraction spotting onto MALDI target plates

Tryptic peptides were separated by reverse phase high performance liquid chromatography (RP-HPLC) using an UltiMate nanoscale LC system and a FAMOS microautosampler (LC Packings, Amsterdam, The Netherlands) controlled by the Chromeleon software (Dionex, Sunnyvale, CA, USA). Mobile phase A consisted of 2% acetonitrile and 0.1 % trifluoroacetic acid (TFA) in water, mobile phase B was 80% acetonitrile and 0.1% TFA in water. The flow rate was 300 nl/min leading to a pressure of mobile phase A of ∼170 bar. Lyophilized peptides derived from the digestion of biotinylated proteins affinity purified from 1.5 mg of total protein were dissolved in 5 µl of buffer A and loaded on the column (inner diameter: 75 µm, length 15 cm, filled with C18 PepMap 100, 3 µm, 100 A beads; LC Packings). The peptides were eluted with a gradient of 0 - 30% B for 7 min, 30 - 80% B for 67 min, 80 - 100% B for 3 min and 100% B for 5 min; the column was then equilibrated with 100% A for 20 min before analyzing the next sample. Eluting fractions were mixed with a solution of 3 mg/ml α-cyano-4-hydroxy cinnamic acid, 277 pmol/ml neurotensin (internal standard), 0.1% TFA, and 70% acetonitrile in water and deposed on a 192-well MALDI target plate using an on-line Probot system (Dionex). The flow of the MALDI-matrix solution was set to 1.083 µl/min. Thus, each fraction collected during 20 s contained 361 nl MALDI-matrix solution and 100 nl sample. The end-concentration of neurotensin was 100 fmol per sample well.

### MALDI-TOF/TOF mass spectrometry

Matrix-assisted laser desorption ionization tandem time-of-flight (MALDI-TOF/TOF) mass spectrometric analysis was carried out with the 4700 Proteomics Analyzer (Applied Biosystems, Framingham, MA). All spectra were acquired with an Nd:YAG laser working at a laser frequency of 200 Hz. For precursor ion selection, all fractions were measured in MS mode before MS/MS was performed. A maximum of 15 precursors per sample spot were selected for subsequent fragmentation by collision induced dissociation. Criteria for precursor selection were a minimum S/N of 60 and shot-to-shot precursor mass tolerance of 120 ppm. Spectra were processed and analyzed by the Global Protein Server Workstation (Applied Biosystems), which uses internal MASCOT (Matrix Science, UK) software for matching MS and MS/MS data against databases of in silico digested proteins. The data obtained were screened against a human database downloaded from the NCBI homepage (http://www.ncbi.nlm.nih.gov/). The number of missed cleavages was set to 2. Protein identifications, performed by means of the MASCOT software, were considered to be correct calls within the 95% confidence interval for the best peptide ion. Selected hits within the confidence interval between 90% and 95% were verified by manual inspection of the spectra.

### Immunohistochemical staining

Sections from paraformaldehyde-fixed, paraffin-embbeded tissue specimens were stained by the immunoperoxidase technique (Vectastain Elite ABC kit (Vector Laboratories, Burlingame, CA, USA)) according to standard procedures. The immunoaffinity-purified rabbit polyclonal anti-periostin antibody (Biovendor, Heidelberg, Germany) and the monoclonal anti-versican antibody (clone 12C5; Developmental Studies Hybridoma Bank, University of Iowa, Ames, IA, USA) were used in a dilution of 1:500.

### PCR analysis

A human kidney tumor cDNA panel containing cDNAs from clear cell carcinoma, granular cell carcinoma, transitional cell carcinoma, normal adult and fetal kidney were purchased from BioChain (Hayward, CA, USA). Polymerase chain reaction (PCR) was performed using the Hot Start Taq Polymerase kit (Qiagen, Hilden, Germany). PCR conditions were as follows: denaturation at 95°C for 15 min, followed by 35 cycles of denaturation at 94°C for 1 minute, annealing at 54°C for 1 minute and elongation at 72°C for 1 minute. A final step of elongation at 72°C for 10 min was performed. Primer sequences are available upon request. The products of the PCR reaction were analyzed by 2% agarose gel electrophoresis, stained by ethidium bromide, and imaged using the BioDoc-It imaging system (UVP, Upland, CA, USA). For the analysis of periostin splice isoforms, bands were cut out from the agarose gel and sequenced (Big Dye Terminator v1.1 Cycle Sequencing kit; ABI PRISM 310 Genetic Analyzer; Applied Biosystems, Foster City, CA, USA).

### Identification of new splice variants of periostin

For human periostin, Takeshita and colleagues have reported that five alternative spliced transcripts can be produced, and that all splicing events of periostin occur within the C-terminal region. The possibility that some particular isoform might be selectively expressed in tumors and not expressed in normal tissue (in analogy to the expression pattern of the well-characterized ED-B containing isoform of fibronectin and the C domain-containing large tenascin C isoform) lead to the design of primers for the amplification of the alternative spliced domains by PCR on human cDNA libraries. The PCR amplification of the C-terminal region of periostin (Fig. 5) revealed at least five different splice variants. This would be consistent with the findings of Takeshita and coworkers (*supra*), who, however, have not published the sequences of the isoforms which they have found. An analysis of the existing databases reveals the existence of three isoforms (SwissProt numbers Q15063, Q15063-2 and Q15063-3) and of an EST clone (OTTHUMP 0018271), corresponding to a fourth different periostin isoform. However, excision of the amplified bands shown in Fig. 2 and their sequencing yielded six different isoforms, four of which did not correspond to any of the known ones. The full lenght periostin and an isoform corresponding to isoform Q15063-3 were also identified by sequencing in the analysis.

### Identification of isoform-specific peptide of periostin

It is important to note that the above proteomic analysis was able to identify, peptides which are isoform specific that are specifically present in tumor samples only, meaning that they encompass junctions of exons (or exon portions) which only exist in a particular isoform and not in others. (see Fig. 5 (A) MALDI-TOF spectrum of an HPLC fraction containing a peptide of 1527.5797 m/z. (B and C)) The sequence (EIPVTVYKPIIKK) was determined by collision-induced dissociation in a MALDI-TOF/TOF experiment. The peptide EIPVTVYKPIIKK (pale blue rectangle in Figure 4, panel B) encompasses the junction between two exons which are only present in isoforms Q15063-2 and « B ». Another example of an isoform-specific peptide is represented by the peptide of sequence AELTDLK that encompasses a domain junction only present in the hypothetical protein FLJ00154, a protein specifically detected only in the tumor portion of human kidneys, and which represents a minor transcript of the human homologue of Sidekick-like protein 1 (data not shown). The identification of tumor-specific isoforms of proteins otherwise present in other normal tissues deserves allows for producing isoform-specific antibodies that are selective in that they are able to discriminate among the various isoforms of the same protein, and these antibodies will provide potential targets for tumor therapy.

### Results

**Table 2. Specification of cases involved in this study. The kidneys of five patients of different age and sex affected by tumors of the specified size and stage were analyzed. Three organs were biotinylated ex vivo as described above whereas the other two unperfused kidneys specimens were collected and analyzed as negative controls.**

| # | Age | Sex | Tumor size (cm) | Stage | Experiment |
|---|---|---|---|---|---|
| 1 | 68 | male | 10 | pT2 | *ex vivo* biotinylation |
| 2 | 52 | female | 3 | pT1A | *ex vivo* biotinylation |
| 3 | 63 | | 12 | pT3A | *ex vivo* biotinylation |
| 4 | 46 | | 15.5 | pT3B | unperfused negative control |
| 5 | 66 | | 3 | pt1 | unperfused negative control |

**Table 3. Selection of putative membrane proteins and extracellular matrix proteins identified in normal kidney, in the tumor portion, or both. Numbers indicate in how many of the three patients, whose kidneys were biotinylated ex vivo, the protein was identified. # Patients (total =3)**

| # | Swiss Prot. Acc. No. | normal kidney | tumor |
|---|---|---|---|
| 1 | Q15063 / Q5VSY8 / Q5VSY7 / Q5VSY6 | 0 | 3 |
| 2 | O15529 / O14843 | 0 | 2 |
| 3 | P11166 | 0 | 2 |
| 4 | P13611 | 0 | 2 |
| 5 | Q6UY47 / Q3KPI0 / 075296 | 0 | 1 |
| 6 | Q8IV47 | 0 | 1 |
| 7 | Q92626 | 0 | 1 |
| 8 | O15354 | 0 | 1 |
| 9 | Q8TEN9 | 0 | 1 |
| 10 | Q9NS88 | 0 | 2 |
| 11 | Q8N5L1 | 0 | 2 |
| 12 | Q96T75 / Q6EXA9 / Q684H4 / Q96T75-2 / Q96T75-3 | 0 | 1 |
| 13 | Q8IZF5 | 0 | 1 |
| 14 | Q6LES2 / P09525 | 0 | 1 |
| 15 | Q5DID0 / Q5DID0-2 / Q5DID0-3 / Q5DID0-4 | 0 | 1 |
| 16 | Q96GP6 | 0 | 1 |
| 17 | Q9UGT4 | 0 | 1 |
| 18 | Q5W0H4 | 0 | 2 |
| 19 | Q8TDU0 | 0 | 2 |
| 20 | Q7Z3A1 | 0 | 1 |
| 21 | Q8N4L2 | 0 | 1 |
| 22 | Q8WYY4 | 0 | 1 |
| 23 | Q8IWF6 | 0 | 2 |
| 24 | Q9P032 | 0 | 1 |
| 25 | Q6ZS59 | 0 | 1 |
| 26 and/or 27 | Q6AHZ8 and/or Q9UBR1 | 0 | 1 |
| 28 | Q9GZU6 / Q9GZT6 | 0 | 1 |
| 29 | Q6UXH1 / Q6UXH1-2 / Q6UXH1-3 / Q6UXH1-4 / Q6UXH1-5 / Q6UXH1-6 | 0 | 1 |
| 30 | Q5H9T4 | 0 | 1 |
| 31 | O95259 / 095259-2 | 0 | 1 |

### Example 2

### Introduction

For assessing periostin's utility as tumor marker recombinant fragments of this protein were cloned and expressed. Human monoclonal antibodies against the recombinant fragments were produced and tested in ELISA and immunohistochemistry experiments.

### Materials and Methods

Recombinant protein fragments corresponding to the amino acid sequence positions 232-632 (FAS2-FAS4) and 496-632 (FAS4) of periostin (SEQ ID NO: 1) were cloned for use as antigen for biopanning experiments. The fragments were expressed in *E. coli* strain TG1 using pQE12 vector (Qiagen, Hilden, Germany). Proteins were purified from *E. coli* lysates using Ni-NTA columns (Qiagen). Antibodies in single chain Fv format (scFv) against the periostin fragments were selected from the ETH-2-Gold phage display library according to the procedure reported in Silacci et al., Proteomics. 2005 Jun; 5(9):2340-50. ELISA screening for clones expressing scFv antibody binding the antigen and immunohistochemical staining using single chain Fv preparations on sections of freshly frozen tissue samples were performed as described previously in Silacci et al., Proteomics. 2005 June; 5(9):2340-50 and Brack et al., Clin. Cancer Res. 2006 May 15;12(10):3200-8. Affinity maturation of selected antibodies was done as described in Brack et al., Clin. Cancer Res. 2006 May 15; 12(10):3200-8. Immunohistochemical stainings with the commercial immunoaffinity-purified rabbit polyclonal anti-periostin antibody (Biovendor, Heidelberg, Germany) were performed as described in Example 1.

### Results

The periostin domains FAS2 - FAS4 or the domain FAS4 were cloned and expressed. Fig 6A shows an SDS-PAGE analysis of the purified recombinant protein FAS2 - FAS4 (band at 47 kDa). Phage display selections against this protein resulted in numerous clones expressing scFv antibodies specific to FAS2-4 and to FAS4 as shown by ELISA on the coated recombinant protein (see Fig. 6B). The best clone C2 against FAS2-4 was further affinity matured to obtain an antibody with higher affinity (see ELISA results in Fig. 6C). The selected anti-periostin antibody C2 was used in an immunohistochemical analysis, which showed positive staining of vascular and extracellular matrix structures on tissue sections of human kidney tumors (see Fig. 7A). The negative control, for which sections from the same tissue were submitted to the same staining protocol only omitting the scFv, showed no positive staining (see Fig. 7B). Furthermore, a commercial antibody against periostin (see also Example 1) was used to evaluate the periostin expression in kidney tumors of different patients (see Fig 8). Eight out of eight patients tested showed positive anti-periostin staining in the kidney tumor. These results demonstrate that human monoclonal antibodies against periostin can be produced and used for the detection of this antigen in neovascular structures of human cancer tissues. The immunohistochemical results indicate that periostin is expressed at high levels in human kidney tumors of most if not all patients.

### Example 3

### Introduction

To further study the versican antigen, we have performed an immunohistochemical analysis on kidney tumor sections of different patients.

### Materials and Methods

Immunohistochemical stainings with the monoclonal anti-versican antibody (clone 12C5) on paraformaldehyde-fixed, paraffin-embedded sections of human kidney tumors were performed as described in Example 1.

### Results

Furthermore, a commercial antibody against versican (see also Example 1) was used to evaluate the periostin expression in kidney tumors of different patients (see Fig 9). Six out of eight patients tested showed positive anti-versican staining in the kidney tumor, either more diffuse in the extracellular matrix or more restricted to areas around tumor blood vessels. These results indicate that versican is expressed at high levels in human kidney tumors of most patients and is likely to be accessible from the vasculature.

### Example 4

### Introduction

To assess the CEACAM3 antigen a recombinant fragment of this protein was cloned and expressed for use as an antigen for the selection of antibodies by phage display.

### Materials and Methods

A recombinant protein fragment corresponding to the amino acid sequence positions 36 - 236 of CEACAM3 (SEQ ID NO: 25) was cloned and expressed as described in example 2 for use as antigen for biopanning experiments.

### Results

The CEACAM3 domains corresponding to the amino acid sequence positions 36 - 236 was cloned and expressed. Fig 10 shows an SDS-PAGE analysis of the purified recombinant protein (band at 26 kDa) These results indicates that CEACAM3 fragments can be prepared in order to use them as antigen in antibody selection. Such antibodies could be used for a further validation of CEACAM3 as a vascular tumor marker similar to example 2.

### Example 5

### Introduction

To assess the utility of fibromodulin as antigen a recombinant fragment of this protein was cloned and expressed for the selection of antibodies by phage display.

### Materials and Methods

A recombinant protein fragment corresponding to the amino acid sequence positions 94 - 315 of CEACAM3 (SEQ ID NO: 25) was cloned and expressed as described in example 2 for use as antigen for biopanning experiments.

### Results

The fibromodulin fragment corresponding to the amino acid sequence positions 94 - 315 was cloned and expressed. Fig 11 shows an SDS-PAGE analysis of the purified recombinant protein (band at 27 kDa). This result indicates that fibromodulin fragments can be prepared in order to use them as antigen in antibody selections. Such antibodies could be used for a further validation of fibromodulin as a vascular tumor marker similar to example 2.

### Example 6

### Introduction

For assessing peroxidasin homolog [fragment] for use as tumor marker, a recombinant fragment of this protein was cloned and expressed. Human monoclonal antibodies against the recombinant fragment were produced and tested in ELISA and immunohistochemistry experiments.

### Materials and Methods

A recombinant protein fragment corresponding to the amino acid sequence positions 539-632 of peroxidasin homolog [fragment] (SEQ ID NO: 33) was cloned and expressed, and antibody phage display selections, ELISA screening and immunohistochemistry performed as described in example 2.

### Results

The peroxidasin homolog fragment was cloned and expressed. Fig 12A shows an SDS-PAGE analysis of the purified recombinant protein fragment (band at 12 kDa). Phage display selections against this protein resulted in numerous clones expressing scFv antibodies specific to the recombinant peroxidasin homolog fragment as shown by ELISA on the coated recombinant protein (see Fig. 12B). A selected anti- peroxidasin homolog [fragment] antibody was used in an immunohistochemical analysis, which showed positive staining mainly around tumor blood vessels on tissue sections of human kidney tumors (see Fig. 13A). The negative control, omitting the scFv, showed no positive staining (see Fig. 13B). These results demonstrate that human monoclonal antibodies against peroxidasin homolog [fragment] can be produced and used for the detection of this antigen in neovascular structures of human cancer tissues. The immunohistochemical results indicate that peroxidasin homolog [fragment] is expressed at high levels in human tumor neovasculature.

### Example 7

### Introduction

For assessing the potential of Protein sidekick-1 as tumor marker a recombinant fragment of this protein was cloned and expressed. Human monoclonal antibodies against the recombinant fragment were produced and tested in ELISA.

### Materials and Methods

A recombinant protein fragment corresponding to the amino acid sequence positions 851-1052 of Protein sidekick-1 (SEQ ID NO: 37) was cloned and expressed, and antibody phage display selections and ELISA screening performed as described in example 2.

### Results

The Protein sidekick-1 fragment was cloned and expressed. Fig 14A shows an SDS-PAGE analysis of the purified recombinant protein fragment (band at 24 kDa). Phage display selections against this protein resulted in several clones expressing scFv antibodies specific to the recombinant Protein sidekick-1 fragment as shown by ELISA on the coated recombinant protein (see Fig. 14B). These results demonstrate that human monoclonal antibodies against Protein sidekick-1 can be produced against recombinant antigen fragments. These antibodies could be used for a further validation of Protein sidekick-1 as a tumor marker, similar to the process described in example 2.

### Example 8

### Introduction

For assessing the ANXA4 protein for use as tumor marker a recombinant fragment of this protein was cloned and expressed. Human monoclonal antibodies against the recombinant fragments were produced and tested in ELISA and immuno-histochemistry experiments.

### Materials and Methods

A recombinant protein fragment corresponding to the amino acid sequence positions 3-321 of ANXA4 protein (SEQ ID NO: 52) was cloned and expressed, and antibody phage display selections ELISA screening and immunohistochemistry performed as described in example 2.

### Results

Almost the complete ANXA4 protein was cloned and expressed. Fig 15A shows an SDS-PAGE analysis of the purified recombinant ANXA4 protein (band at 37 kDa). Phage display selections against this protein resulted in numerous clones expressing scFv antibodies specific to ANXA4 protein as shown by ELISA on the coated recombinant protein (see Fig. 15B). The best clone E11 against ANXA4 protein was used in an immunohistochemical analysis, which showed positive staining of tumor cells (also those cells located around the tumor blood vessels) on tissue sections of human kidney tumors (see Fig. 16A). The negative control, for which sections from the same tissue were submitted to the same staining protocol only omitting the scFv, showed no positive staining (see Fig. 16B). These results demonstrate that human monoclonal antibodies against ANXA4 protein can be produced and used for the detection of this antigen in human cancer tissues. The immunohistochemical results indicate that this antigen is expressed at high levels in human tumors (also around the neovasculature).

### Example 9

### Introduction

To further study the antigen UPF0378 family protein KIAA0100, we have cloned and expressed a recombinant fragment of this protein in order to use it as antigen for the selection of antibodies by phage display.

### Materials and Methods

A recombinant protein fragment corresponding to the amino acid sequence positions 755 - 968 of UPF0378 family protein KIAA0100 (SEQ ID NO: 96) was cloned and expressed as described in example 2 for use as antigen for biopanning experiments.

### Results and discussion

The UPF0378 family protein KIAA0100 fragment corresponding to the amino acid sequence positions 755 - 968 was cloned and expressed. Fig. 17 shows an SDS-PAGE analysis of the purified recombinant protein (band at 32 kDa). This result indicates that UPF0378 family protein KIAA0100 fragments can be prepared in order to use them as antigen in antibody selections. Such antibodies could be used for a further validation of this antigen as a vascular tumor marker similar to example 2.

### Example 10

### Introduction

For evaluating the usefulness of Potassium voltage-gated channel subfamily H member 1 as tumor marker, human monoclonal antibodies were produced against a synthetic peptide corresponding to the amino acid sequence of an extracellular loop of this membrane protein. These antibodies were tested in ELISA, immunohistochemistry, FACS and immunocytochemistry experiments.

### Materials and Methods

A synthetic peptide corresponding to the second extracellular loop (amino acid sequence positions 316-349; see Fig. 18A for a graphic representation) of Potassium voltage-gated channel subfamily H member 1 (SEQ ID NO: 98) was used as antigen for biopanning experiments. Antibodies in single chain Fv format (scFv) were selected from the ETH-2-Gold phage display library against the biotinylated peptide according to the procedure described in Silacci et al., Proteomics. 2005 Jun;5(9):2340-50. ELISA screening, immunohistochemical staining and affinity maturation of selected antibodies were performed as described in Example 2. FACS and immunocytochemical staining of cultured cells were performed according to standard procedures using the selected scFv as primary, mouse anti-c-myc (clone 9E10) as secondary and FITC-labeled antimouse Ig as tertiary antibody.

### Results

Phage display selections against the biotinylated Potassium voltage-gated channel subfamily H member 1 peptide (see above and Fig. 18A) resulted in numerous clones expressing binding scFv antibodies as tested by ELISA on the coated peptide (data not shown). The best clone H9 against was further affinity matured to obtain an antibody with higher affinity (see ELISA results in Fig. 18B). The selected anti- Potassium voltage-gated channel subfamily H member 1 antibody H9 was used in an immunohistochemical analysis, which showed positive staining of tumor cell membranes on tissue sections of human kidney tumors (see Fig. 19A) and of human lung tumors (see Fig. 19C). The negative controls, for which sections from the same tissues were submitted to the same staining protocol only omitting the scFv, showed no positive staining (see Figs. 19B and 19D). Further studies on HeLa cells (a human cervix carcinoma cell line) showed that the scFv antibody H9 can recognize these cells in FACS (see Fig. 20) and gives a positive staining of the cell membranes in immunocytochemistry (see Fig. 21). These results demonstrate that human monoclonal antibodies against Potassium voltage-gated channel subfamily H member 1 can be produced and that the antigen is expressed on the tumor cell membrane of human cancer tissues (also those tumor cells which are in close proximity to the neovasculature) and on the membranes of human tumor cell lines. Thus, it is likely that this antigen is a suitable target for vascular tumor targeting approaches.

The following Table is a list of the amino acid sequences of the vascular tumor markers identified. The partial amino acid sequences identified in Example 1 are illustrated in bold letters. The SEQ ID NOs therein correspond to the appended sequence listing which is part of the disclosure.

| **#** | **Name** | **Swiss Prot. Acc. No.** |
|---|---|---|
| 1 | Periostin [precursor] (+ isoforms) | Q15063 |
| **SEQ ID NO: 1** | | |
| | | |
| **SEQ ID NO: 3** | | |
| | | |
| **SEQ ID NO: 5** | | |
| | | |
| | | |
| **SEQ ID NO: 7** | | |
| | | |
| **SEQ ID NO: 9** | | |
| | | |
| **SEQ ID NO: 11** | | |
| | | |
| **SEQ ID NO: 13** | | |
| | | |
| **SEQ ID NO: 15** | | |
| | | |
| 2 | Putative G-protein coupled receptor 42 (+ isoforms) | O15529 |
| **SEQ ID NO: 17** | | |
| | | |
| **SEQ ID NO: 19** | | |
| | | |
| 3 | Solute carrier family 2, facilitated glucose transporter member 1 | P11166 |
| **SEQ ID NO: 21** | | |
| | | |
| 4 | Versican core protein [precursor] | P13611 |
| **SEQ ID NO: 23** | | |
| | | |
| | | |
| 5 | CEACAM3 (+ isoforms) | Q6UY47 |
| **SEQ ID NO: 25** | | |
| | | |
| **SEQ ID NO: 27** | | |
| | | |
| **SEQ ID NO: 29** | | |
| | | |
| 6 | Fibromodulin | Q8IV47 |
| **SEQ ID NO: 31** | | |
| | | |
| 7 | Peroxidasin homolog [Fragment] | Q92626 |
| **SEQ ID NO: 33** | | |
| | | |
| 8 | Probable G-protein coupled receptor 37 [precursor] | O15354 |
| **SEQ ID NO: 35** | | |
| | | |
| 9 | Protein sidekick-1 [precursor] | Q8TEN9 |
| **SEQ ID NO: 37** | | |
| | | |
| | | |
| 10 | Alpha1A-voltage-dependent calcium channel | Q9NS88 |
| **SEQ ID NO: 39** | | |
| | | |
| 11 | EMILIN2 protein [fragment] | Q8N5L1 |
| **SEQ ID NO: 41** | | |
| | | |
| 12 | Down syndrome critical region protein 8 (+ isoforms) | Q96T75 |
| **SEQ ID NO: 43** | | |
| | | |
| **SEQ ID NO: 45** | | |
| MHNIMMVKLKKKKSTTNLGNQESSGMMK**LFMPRPK**VLAQYESIQFMP | | |
| **SEQ ID NO: 46** | | |
| MSQPPEQLGLQTNLGNQESSGMMK**LFMPRPK**VLAQYESIQFMP | | |
| **SEQ ID NO: 47** | | |
| | | |
| **SEQ ID NO: 48** | | |
| | | |
| **SEQ ID NO: 49** | | |
| MKEPGPNFVTVRKGLHSFKMAFVKHLLQTLEIKKVLE | | |
| 13 | Probable G-protein coupled receptor 113 [precursor] | Q8IZF5 |
| **SEQ ID NO: 50** | | |
| | | |
| 14 | ANXA4 protein [fragment] (+ isoforms) | Q6LES2 |
| **SEQ ID NO: 52** | | |
| | | |
| **SEQ ID NO: 54** | | |
| | | |
| | | |
| 15 | Uromodulin-like 1 [precursor] (+ isoforms) | Q5DID0 |
| **SEQ ID NO: 56** | | |
| | | |
| **SEQ ID NO: 58** | | |
| | | |
| **SEQ ID NO: 59** | | |
| | | |
| **SEQ ID NO: 60** | | |
| | | |
| 16 | Scavenger receptor class F member 2 [precursor] | Q96GP6 |
| **SEQ ID NO: 61** | | |
| | | |
| | | |
| 17 | Sushi domain-containing protein 2 [precursor] | Q9UGT4 |
| **SEQ ID NO: 63** | | |
| | | |
| 18 | Tumor protein, translationally controlled 1 | Q5W0H4 |
| **SEQ ID NO: 65** | | |
| | | |
| 19 | Putative G-protein coupled receptor | Q8TDU0 |
| **SEQ ID NO: 67** | | |
| | | |
| 20 | Hypothetical protein DKFZp686K0275 [fragment] | Q7Z3A1 |
| **SEQ ID NO: 69** | | |
| | | |
| 21 | Transmembrane protein TMEM55A | Q8N4L2 |
| **SEQ ID NO: 71** | | |
| | | |
| 22 | Hypothetical protein Q8WYY4 | Q8WYY4 |
| **SEQ ID NO: 73** | | |
| | | |
| 23 | Family with sequence similarity 116, member A | Q8IWF6 |
| **SEQ ID NO: 75** | | |
| | | |
| | | |
| 24 | UPF0240 protein C6orf66 | Q9P032 |
| **SEQ ID NO: 77** | | |
| | | |
| 25 | CDNA FLJ45811 fis, clone NT2RP7014778 | Q6ZS59 |
| **SEQ ID NO: 79** | | |
| | | |
| 26 | Hypothetical protein DKFZp779O1248 | Q6AHZ8 |
| **SEQ ID NO: 81** | | |
| | | |
| 27 | Beta-ureidopropionase | Q9UBR1 |
| **SEQ ID NO: 83** | | |
| | | |
| 28 | Hypothetical protein DKFZp434F1919 (+ isoforms) | Q9GZU6 |
| **SEQ ID NO: 85** | | |
| | | |
| **SEQ ID NO: 87** | | |
| | | |
| 29 | Cysteine-rich with EGF-like domain protein 2 [precursor] (+ isoforms) | Q6UXH1 |
| **SEQ ID NO: 89** | | |
| | | |
| **SEQ ID NO: 91** | | |
| | | |
| | | |
| **SEQ ID NO: 92** | | |
| | | |
| **SEQ ID NO: 93** | | |
| | | |
| **SEQ ID NO: 94** | | |
| | | |
| **SEQ ID NO: 95** | | |
| | | |
| 30 | UPF0378 family protein KIAA0100 [precursor] | Q5H9T4 |
| **SEQ ID NO: 96** | | |
| | | |
| | | |
| 31 | Potassium voltage-gated channel subfamily H member 1 (+ isoforms) | O95259 |
| **SEQ ID NO: 98** | | |
| | | |
| **SEQ ID NO: 100** | | |
| | | |

## Claims

1. An *in vitro* method for identifying neovascular structures in mammalian tissue, wherein said neovascular structures are identified by the detection of at least one surface protein in said tissue, the at least one protein being selected from the group consisting of Periostin including isoforms thereof and new splice variants A, B, D, E, wherein the mammalian tissue is mature mammalian kidney tissue, preferably human mature kidney tissue, and wherein positive identification is determined relative to mature mammalian kidney control tissue.

2. An *in vitro* method for identifying a kidney tumor, preferably a human kidney tumor, wherein said tumor is identified by the detection of at least one surface protein within mammalian kidney tissue of interest, said at least one protein being selected from the group consisting of Periostin including isoforms thereof and new splice variants A, B, D, E, and wherein positive identification is determined relative to mature mammalian kidney control tissue.

3. An *in vitro* method for targeting and/or imaging neovascular structures in mammalian tissue, wherein said neovascular structures are targeted and/or imaged by a ligand having specific binding affinity to at least one surface protein in said neovascular structure, the at least one protein being selected from the group consisting of Periostin including isoforms thereof and new splice variants A, B, D, E, wherein the mammalian tissue is mature mammalian kidney tissue, preferably human mature kidney tissue.

4. An *in vitro* method for targeting and/or imaging a tissue affected by a kidney tumor, preferably a human kidney tumor, wherein said disease or condition is targeted and/or imaged by a ligand having a specific binding affinity to at least one surface protein within the mammalian tissue of interest, said at least one protein being selected from the group consisting of Periostin including isoforms thereof and new splice variants A, B, D, E.

5. *In vitro* use of at least one antibody having specific binding affinity to a protein selected from the group consisting of Periostin splice variants A, B, D, E for identifying tumors or neovascular structures in mammalian kidney tissue, preferably human kidney tissue, according to the methods of any of claims 1 to 4.

6. Use of an antibody having specific binding affinity to a protein selected from the group consisting of Periostin splice variants A, B, D, E in a method of any of claims 1 to 4.

7. Use of a fusion protein having specific binding affinity to a protein selected from the group consisting of Periostin splice variants A, B, D, E and comprising a component having diagnostic activity, preferably selected from the group consisting of intact antibodies, Fc-containing antibody fragments or Fc-functional derivatives thereof, radionucleotides, photosensitizers, liposomes, drugs, pro-coagulatory agents, cytokines, chemokines, toxins as well as bispecific antibodies in a method according to any of claims 1 to 4.

8. An *in vitro* method according to claim 2 for identifying tumors in mammalian kidney, preferably human kidney tissue, more preferably human vascular kidney tissue comprising:
(i) contacting an antibody and/or a fusion protein comprising an antibody having specific binding affinity to at least one surface protein selected from the group consisting of Periostin including isoforms thereof and new splice variants A, B, D, E, preferably new splice variants A, B, D, E, with mammalian tissue of interest, preferably human tissue of interest, under conditions that allow for the specific binding of said antibody and/or fusion protein to at least one of said proteins selected from the group consisting of Periostin including isoforms thereof and new splice variants A, B, D, E, preferably new splice variants A, B, D, E and
(ii) identifying specifically bound antibody and/or fusion protein, and
wherein positive kidney tumor identification is determined relative to mature mammalian kidney control tissue.

## Patentansprüche

1. Ein in vitro Verfahren zur Identifizierung neovaskulärer Strukturen in Säugetiergewebe, wobei die Identifizierung dieser neovaskulären Strukturen durch den Nachweis von wenigstens einem Oberflächenprotein geschieht, dieses wenigstens eine Protein ausgewählt ist aus der Gruppe bestehend aus Periostin, einschliesslich dessen Isoformen und neuer Splice-Varianten A, B, D, E, wobei das Säugetiergewebe ausdifferenziertes Säugetier-Nierengewebe ist, vorzugsweise menschliches, ausdifferenziertes Nierengewebe, und wobei der positive Nachweis relativ zu ausdifferenziertem Säugetier-Nierenkontrollgewebe erfolgt.

2. Ein in vitro Verfahren zur Identifizierung eines Nierentumors, vorzugsweise eines menschlichen Nierentumors, wobei die Identifizierung dieses Tumors durch den Nachweis von wenigstens einem Oberflächenprotein innerhalb des zu untersuchenden Säugetier-Nierengewebes geschieht, dieses wenigstens eine Protein ausgewählt ist aus der Gruppe bestehend aus Periostin, einschliesslich dessen Isoformen und neuer Splice-Varianten A, B, D, E, und wobei der positive Nachweis relativ zu ausdifferenziertem Säugetier-Nierenkontrollgewebe erfolgt.

3. Ein in vitro Verfahren zum Anzielen und/oder Visualisieren neovaskulärer Strukturen in Säugetiergewebe, wobei diese neovaskulären Strukturen angezielt und/oder visualisiert werden durch einen Liganden, der eine spezifische Affinität für wenigstens ein Oberflächenprotein in diesem neovaskulären Gewebe besitzt, das wenigstens eine Protein ausgewählt ist aus der Gruppe bestehend aus Periostin, einschliesslich dessen Isoformen und neuer Splice-Varianten A, B, D, E, und wobei das Säugetiergewebe ausdifferenziertes Säugetier-Nierengewebe ist, vorzugsweise ausdifferenziertes menschliches Nierengewebe.

4. Ein in vitro Verfahren zum Anzielen und/oder Visualisieren eines Gewebes, das von einem Nierentumor betroffen ist, vorzugsweise von einem menschlichen Nierentumor, wobei diese Erkrankung oder dieser Zustand durch einen Liganden angezielt und/oder visualisiert wird, der eine spezifische Bindung an wenigstens ein Oberflächenprotein innerhalb des zu untersuchenden Säugetiergewebes besitzt, und dieses wenigstens eine Protein ausgewählt ist aus der Gruppe bestehend aus Periostin, einschliesslich dessen Isoformen und neuer Splice-Varianten A, B, D, E.

5. Die in vitro Verwendung von wenigstens einem Antikörper, der eine spezifische Bindungsaffinität für ein Protein besitzt ausgewählt aus der Gruppe bestehend aus Periostin, einschliesslich dessen Isoformen und neuer Splice-Varianten A, B, D, E, zur Identifizierung von Tumoren oder neovaskulären Strukturen in Säugetier-Nierengewebe, bevorzugt menschlichem Nierengewebe.

6. Die Verwendung eines Antikörpers, der eine spezifische Bindungsaffinität besitzt für ein Protein ausgewählt aus der Gruppe bestehend aus Periostin-Splice-Varianten A, B, D, E.

7. Die Verwendung eines Fusionsproteins, das eine spezifische Bindungsaffinität für ein Protein besitzt, ausgewählt aus der Gruppe bestehend aus Periostin-Splice-Varianten A, B, D, E, und eine Komponente umfasst, die eine diagnostische Aktivität besitzt, vorzugsweise ausgewählt aus der Gruppe bestehend aus intakten Antikörpern, Fc-enthaltenden Antikörperfragmenten oder Fcfunktionalen Derivaten davon, Radionukleotiden, Photosensitizern, Liposomen, Medikamenten, Prokoagulantien, Zytokinen, Chemokinen, Toxinen, sowie bispezifischen Antikörpern in einem Verfahren gemäss einem der Ansprüche 1 bis 4.

8. Ein in vitro Verfahren gemäss Anspruch 2 zur Identifizierung von Tumoren in Säugetier-Nierengewebe, vorzugsweise menschlichem Nierengewebe, mehr bevorzugt menschlichem vaskulärem Nierengewebe umfassend:
(i) Zusammenführen eines Antikörpers und/oder eines Fusionsproteins, umfassend einen Antikörper mit einer spezifischen Affinität für wenigstens ein Oberflächenprotein ausgewählt aus der Gruppe bestehend aus Periostin, einschliesslich dessen Isoformen und neuer Splice-Varianten A, B, D, E, vorzugsweise neuer Splice-Varianten A, B, D, E, mit zu untersuchendem Säugetiergewebe, vorzugsweise zu untersuchendem menschlichen Gewebe, unter Bedingungen die es erlauben, dass dieser Antikörper und/oder dieses Fusionsprotein spezifisch an wenigstens eines dieser Proteine bindet, ausgewählt aus der Gruppe bestehend aus Periostin einschliesslich dessen Isoformen und neuer Splice-Varianten A, B, D, E, vorzugsweise neuer Splice-Varianten A, B, D, E, und
(ii) Identifizieren spezifisch gebundener Antikörper und/oder Fusionsproteine, und
wobei der positive Nachweis von Nierentumoren relativ zu ausdifferenziertem Säugetier-Nierenkontrollgewebe erfolgt

## Revendications

1. Procédé *in vitro* d'identification de structures néovasculaires dans un tissu mammalien, dans lequel lesdites structures néovasculaires sont identifiées par la détection d'au moins une protéine de surface dans ledit tissu, la au moins une protéine étant sélectionnée parmi le groupe consistant en périostine y compris des isoformes de celle-ci et de nouveaux variants d'épissage A, B, D, E, dans lequel le tissu mammalien est un tissu mammalien rénal mature, de préférence un tissu rénal mature humain, et dans lequel l'identification positive est déterminée par rapport à un tissu mammalien rénal mature témoin.

2. *Procédé in vitro* d'identification d'une tumeur rénale, de préférence une tumeur rénale humaine, dans lequel ladite tumeur est identifiée par la détection d'au moins une protéine de surface dans un tissu mammalien rénal d'intérêt, ladite au moins une protéine étant sélectionnée parmi le groupe consistant en périostine y compris des isoformes de celle-ci et de nouveaux variants d'épissage A, B, D, E, et dans lequel l'identification positive est déterminée par rapport à un tissu mammalien rénal mature témoin.

3. *Procédé in vitro* de ciblage et/ou d'imagerie de structures néovasculaires dans un tissu mammalien, dans lequel lesdites structures néovasculaires sont ciblées et/ou imagées par un ligand ayant une affinité de liaison spécifique à au moins une protéine de surface dans ladite structure néovasculaire, la au moins une protéine étant sélectionnée parmi le groupe consistant en périostine y compris des isoformes de celle-ci et de nouveaux variants d'épissage A, B, D, E, dans lequel le tissu mammalien est un tissu mammalien rénal mature, de préférence un tissu rénal mature humain.

4. *Procédé in vitro* de ciblage et/ou d'imagerie d'un tissu affecté par une tumeur rénale, de préférence une tumeur rénale humaine, dans lequel ladite maladie ou condition est ciblée et/ou imagée par un ligand ayant une affinité de liaison spécifique à au moins une protéine de surface dans ledit tissu mammalien d'intérêt, ladite au moins une protéine étant sélectionnée parmi le groupe consistant en périostine y compris des isoformes de celle-ci et de nouveaux variants d'épissage A, B, D, E.

5. Utilisation *in vitro* d'un anticorps ayant une affinité de liaison spécifique à une protéine sélectionnée parmi le groupe consistant en des variants d'épissage A, B, D, E de périostine, pour identifier des tumeurs ou des structures néovasculaires dans un tissu mammalien rénal, de préférence un tissu rénal humain, conformément aux procédés selon l'une quelconque des revendications 1 à 4.

6. Utilisation d'un anticorps ayant une affinité de liaison spécifique à une protéine sélectionnée parmi le groupe consistant en des variants d'épissage A, B, D, E de périostine, dans un procédé selon l'une quelconque des revendications 1 à 4.

7. Utilisation d'une protéine de fusion ayant une affinité de liaison spécifique à une protéine sélectionnée parmi le groupe consistant en des variants d'épissage A, B, D, E de périostine et comprenant un composant ayant une activité diagnostique, sélectionné de préférence parmi le groupe consistant en anticorps intacts, fragments d'anticorps contenant Fc ou des dérivés Fc fonctionnels de ceux-ci, radionucléotides, photosensibilisateurs, liposomes, médicaments, agents pro-coagulants, cytokines, chimiokines, toxines, ainsi que des anticorps bispécifiques dans un procédé selon l'une quelconque des revendications 1 à 4.

8. *Procédé in vitro* selon la revendication 2, d'identification de tumeurs dans un rein mammalien, de préférence dans un tissu rénal humain, plus préférentiellement dans un tissu rénal vasculaire humain, comprenant:
(i) mettre un anticorps et/ou une protéine de fusion comprenant un anticorps ayant une affinité de liaison spécifique à au moins une protéine de surface sélectionnée parmi le groupe consistant en périostine y compris des isoformes de celle-ci et de nouveaux variants d'épissage A, B, D, E, de préférence de nouveaux variants d'épissage A, B, D, E, en contact avec un tissu mammalien d'intérêt, de préférence un tissu humain d'intérêt, dans des conditions qui permettent la liaison spécifique dudit anticorps et/ou protéine de fusion à au moins l'une desdites protéines sélectionnées parmi le groupe consistant en périostine y compris des isoformes de celle-ci et de nouveaux variants d'épissage A, B, D, E, de préférence de nouveaux variants d'épissage A, B, D, E, et
(ii) identifier l'anticorps et/ou la protéine de fusion lié(s) spécifiquement, et
dans lequel l'identification positive d'une tumeur rénale est déterminée par rapport à un tissu mammalien rénal mature témoin.


### SEQUENCE LISTING

<110> Philogen S.p.A.
<120> Vascular Tumor Markers
<130> 50019PCT
<150> EP 06 003 644.9
   <151> 2006-02-22
<160> 100
<170> PatentIn version 3.3
<210> 1
   <211> 836
   <212> PRT
   <213> Homo sapiens
<400> 1
   Met Ile Pro Phe Leu Pro Met Phe Ser Leu Leu Leu Leu Leu Ile Val
   1               5                   10                  15
   Asn Pro Ile Asn Ala Asn Asn His Tyr Asp Lys Ile Leu Ala His Ser
               20                  25                  30
   Arg Ile Arg Gly Arg Asp Gln Gly Pro Asn Val Cys Ala Leu Gln Gln
           35                  40                  45
   Ile Leu Gly Thr Lys Lys Lys Tyr Phe Ser Thr Cys Lys Asn Trp Tyr
       50                  55                  60
   Lys Lys Ser Ile Cys Gly Gln Lys Thr Thr Val Leu Tyr Glu Cys Cys
   65                  70                  75                  80
   Pro Gly Tyr Met Arg Met Glu Gly Met Lys Gly Cys Pro Ala Val Leu
                   85                  90                  95
   Pro Ile Asp His Val Tyr Gly Thr Leu Gly Ile Val Gly Ala Thr Thr
               100                 105                 110
   Thr Gln Arg Tyr Ser Asp Ala Ser Lys Leu Arg Glu Glu Ile Glu Gly
           115                 120                 125
   Lys Gly Ser Phe Thr Tyr Phe Ala Pro Ser Asn Glu Ala Trp Asp Asn
       130                 135                 140
   Leu Asp Ser Asp Ile Arg Arg Gly Leu Glu Ser Asn Val Asn Val Glu
   145                 150                 155                 160
   Leu Leu Asn Ala Leu His Ser His Met Ile Asn Lys Arg Met Leu Thr
                   165                 170                 175
   Lys Asp Leu Lys Asn Gly Met Ile Ile Pro Ser Met Tyr Asn Asn Leu
               180                 185                 190
   Gly Leu Phe Ile Asn His Tyr Pro Asn Gly Val Val Thr Val Asn Cys
           195                 200                 205
   Ala Arg Ile Ile His Gly Asn Gln Ile Ala Thr Asn Gly Val Val His
       210                 215                 220
   Val Ile Asp Arg Val Leu Thr Gln Ile Gly Thr Ser Ile Gln Asp Phe
   225                 230                 235                 240
   Ile Glu Ala Glu Asp Asp Leu Ser Ser Phe Arg Ala Ala Ala Ile Thr
                   245                 250                 255
   Ser Asp Ile Leu Glu Ala Leu Gly Arg Asp Gly His Phe Thr Leu Phe
               260                 265                 270
   Ala Pro Thr Asn Glu Ala Phe Glu Lys Leu Pro Arg Gly Val Leu Glu
           275                 280                 285
   Arg Phe Met Gly Asp Lys Val Ala Ser Glu Ala Leu Met Lys Tyr His
       290                 295                 300
   Ile Leu Asn Thr Leu Gln Cys Ser Glu Ser Ile Met Gly Gly Ala Val
   305                 310                 315                 320
   Phe Glu Thr Leu Glu Gly Asn Thr Ile Glu Ile Gly Cys Asp Gly Asp
                   325                 330                 335
   Ser Ile Thr Val Asn Gly Ile Lys Met Val Asn Lys Lys Asp Ile Val
               340                 345                 350
   Thr Asn Asn Gly Val Ile His Leu Ile Asp Gln Val Leu Ile Pro Asp
           355                 360                 365
   Ser Ala Lys Gln Val Ile Glu Leu Ala Gly Lys Gln Gln Thr Thr Phe
       370                 375                 380
   Thr Asp Leu Val Ala Gln Leu Gly Leu Ala Ser Ala Leu Arg Pro Asp
   385                 390                 395                 400
   Gly Glu Tyr Thr Leu Leu Ala Pro Val Asn Asn Ala Phe Ser Asp Asp
                   405                 410                 415
   Thr Leu Ser Met Val Gln Arg Leu Leu Lys Leu Ile Leu Gln Asn His
               420                 425                 430
   Ile Leu Lys Val Lys Val Gly Leu Asn Glu Leu Tyr Asn Gly Gln Ile
           435                 440                 445
   Leu Glu Thr Ile Gly Gly Lys Gln Leu Arg Val Phe Val Tyr Arg Thr
       450                 455                 460
   Ala Val Cys Ile Glu Asn Ser Cys Met Glu Lys Gly Ser Lys Gln Gly
   465 470 475 480
   Arg Asn Gly Ala Ile His Ile Phe Arg Glu Ile Ile Lys Pro Ala Glu
   485 490 495
   Lys Ser Leu His Glu Lys Leu Lys Gln Asp Lys Arg Phe Ser Thr Phe
   500 505 510
   Leu Ser Leu Leu Glu Ala Ala Asp Leu Lys Glu Leu Leu Thr Gln Pro
   515 520 525
   Gly Asp Trp Thr Leu Phe Val Pro Thr Asn Asp Ala Phe Lys Gly Met
   530 535 540
   Thr Ser Glu Glu Lys Glu Ile Leu Ile Arg Asp Lys Asn Ala Leu Gln
   545 550 555 560
   Asn Ile Ile Leu Tyr His Leu Thr Pro Gly Val Phe Ile Gly Lys Gly
   565 570 575
   Phe Glu Pro Gly Val Thr Asn Ile Leu Lys Thr Thr Gln Gly Ser Lys
   580 585 590
   Ile Phe Leu Lys Glu Val Asn Asp Thr Leu Leu Val Asn Glu Leu Lys
   595 600 605
   Ser Lys Glu Ser Asp Ile Met Thr Thr Asn Gly Val Ile His Val Val
   610 615 620
   Asp Lys Leu Leu Tyr Pro Ala Asp Thr Pro Val Gly Asn Asp Gln Leu
   625 630 635 640
   Leu Glu Ile Leu Asn Lys Leu Ile Lys Tyr Ile Gln Ile Lys Phe Val
   645 650 655
   Arg Gly Ser Thr Phe Lys Glu Ile Pro Val Thr Val Tyr Thr Thr Lys
   660 665 670
   Ile Ile Thr Lys Val Val Glu Pro Lys Ile Lys Val Ile Glu Gly Ser
   675 680 685
   Leu Gln Pro Ile Ile Lys Thr Glu Gly Pro Thr Leu Thr Lys Val Lys
   690 695 700
   Ile Glu Gly Glu Pro Glu Phe Arg Leu Ile Lys Glu Gly Glu Thr Ile
   705 710 715 720
   Thr Glu Val Ile His Gly Glu Pro Ile Ile Lys Lys Tyr Thr Lys Ile
   725 730 735
   Ile Asp Gly Val Pro Val Glu Ile Thr Glu Lys Glu Thr Arg Glu Glu
   740 745 750
   Arg Ile Ile Thr Gly Pro Glu Ile Lys Tyr Thr Arg Ile Ser Thr Gly
   755 760 765
   Gly Gly Glu Thr Glu Glu Thr Leu Lys Lys Leu Leu Gln Glu Glu Val
   770 775 780
   Thr Lys Val Thr Lys Phe Ile Glu Gly Gly Asp Gly His Leu Phe Glu
   785 790 795 800
   Asp Glu Glu Ile Lys Arg Leu Leu Gln Gly Asp Thr Pro Val Arg Lys
   805 810 815
   Leu Gln Ala Asn Lys Lys Val Gln Gly Ser Arg Arg Arg Leu Arg Glu
   820 825 830
   Gly Arg Ser Gln
   835
<210> 2
   <211> 2511
   <212> DNA
   <213> Homo sapiens
<400> 2
   atgattccct ttttacccat gttttctcta ctattgctgc ttattgttaa ccctataaac 60
   gccaacaatc attatgacaa gatcttggct catagtcgta tcaggggtcg ggaccaaggc 120
   ccaaatgtct gtgcccttca acagattttg ggcaccaaaa agaaatactt cagcacttgt 180
   aagaactggt ataaaaagtc catctgtgga cagaaaacga ctgttttata tgaatgttgc 240
   cctggttata tgagaatgga aggaatgaaa ggctgcccag cagttttgcc cattgaccat 300
   gtttatggca ctctgggcat cgtgggagcc accacaacgc agcgctattc tgacgcctca 360
   aaactgaggg aggagatcga gggaaaggga tccttcactt actttgcacc gagtaatgag 420
   gcttgggaca acttggattc tgatatccgt agaggtttgg agagcaacgt gaatgttgaa 480
   ttactgaatg ctttacatag tcacatgatt aataagagaa tgttgaccaa ggacttaaaa 540
   aatggcatga ttattccttc aatgtataac aatttggggc ttttcattaa ccattatcct 600
   aatggggttg tcactgttaa ttgtgctcga atcatccatg ggaaccagat tgcaacaaat 660
   ggtgttgtcc atgtcattga ccgtgtgctt acacaaattg gtacctcaat tcaagacttc 720
   attgaagcag aagatgacct ttcatctttt agagcagctg ccatcacatc ggacatattg 780
   gaggcccttg gaagagacgg tcacttcaca ctctttgctc ccaccaatga ggcttttgag 840
   aaacttccac gaggtgtcct agaaaggttc atgggagaca aagtggcttc cgaagctctt 900
   atgaagtacc acatcttaaa tactctccag tgttctgagt ctattatggg aggagcagtc 960
   tttgagacgc tggaaggaaa tacaattgag ataggatgtg acggtgacag tataacagta 1020
   aatggaatca aaatggtgaa caaaaaggat attgtgacaa ataatggtgt gatccatttg 1080
   attgatcagg tcctaattcc tgattctgcc aaacaagtta ttgagctggc tggaaaacag 1140
   caaaccacct tcacggatct tgtggcccaa ttaggcttgg catctgctct gaggccagat 1200
   ggagaataca ctttgctggc acctgtgaat aatgcatttt ctgatgatac tctcagcatg 1260
   gttcagcgcc tccttaaatt aattctgcag aatcacatat tgaaagtaaa agttggcctt 1320
   aatgagcttt acaacgggca aatactggaa accatcggag gcaaacagct cagagtcttc 1380
   gtatatcgta cagctgtctg cattgaaaat tcatgcatgg agaaagggag taagcaaggg 1440
   agaaacggtg cgattcacat attccgcgag atcatcaagc cagcagagaa atccctccat 1500
   gaaaagttaa aacaagataa gcgctttagc accttcctca gcctacttga agctgcagac 1560
   ttgaaagagc tcctgacaca acctggagac tggacattat ttgtgccaac caatgatgct 1620
   tttaagggaa tgactagtga agaaaaagaa attctgatac gggacaaaaa tgctcttcaa 1680
   aacatcattc tttatcacct gacaccagga gttttcattg gaaaaggatt tgaacctggt 1740
   gttactaaca ttttaaagac cacacaagga agcaaaatct ttctgaaaga agtaaatgat 1800
   acacttctgg tgaatgaatt gaaatcaaaa gaatctgaca tcatgacaac aaatggtgta 1860
   attcatgttg tagataaact cctctatcca gcagacacac ctgttggaaa tgatcaactg 1920
   ctggaaatac ttaataaatt aatcaaatac atccaaatta agtttgttcg tggtagcacc 1980
   ttcaaagaaa tccccgtgac tgtctataca actaaaatta taaccaaagt tgtggaacca 2040
   aaaattaaag tgattgaagg cagtcttcag cctattatca aaactgaagg acccacacta 2100
   acaaaagtca aaattgaagg tgaacctgaa ttcagactga ttaaagaagg tgaaacaata 2160
   actgaagtga tccatggaga gccaattatt aaaaaataca ccaaaatcat tgatggagtg 2220
   cctgtggaaa taactgaaaa agagacacga gaagaacgaa tcattacagg tcctgaaata 2280
   aaatacacta ggatttctac tggaggtgga gaaacagaag aaactctgaa gaaattgtta 2340
   caagaagagg tcaccaaggt caccaaattc attgaaggtg gtgatggtca tttatttgaa 2400
   gatgaagaaa ttaaaagact gcttcaggga gacacacccg tgaggaagtt gcaagccaac 2460
   aaaaaagttc aaggttctag aagacgatta agggaaggtc gttctcagtg a 2511
<210> 3
   <211> 779
   <212> PRT
   <213> Homo sapiens
<400> 3 <210> 4
   <211> 2340
   <212> DNA
   <213> Homo sapiens
<400> 4
<210> 5
   <211> 809
   <212> PRT
   <213> Homo sapiens
<400> 5
   Met Ile Pro Phe Leu Pro Met Phe Ser Leu Leu Leu Leu Leu Ile Val
   1               5                   10                  15
   Asn Pro Ile Asn Ala Asn Asn His Tyr Asp Lys Ile Leu Ala His Ser
               20                  25                  30
   Arg Ile Arg Gly Arg Asp Gln Gly Pro Asn Val Cys Ala Leu Gln Gln
           35                  40                  45
   Ile Leu Gly Thr Lys Lys Lys Tyr Phe Ser Thr Cys Lys Asn Trp Tyr
       50                  55                  60
   Lys Lys Ser Ile Cys Gly Gln Lys Thr Thr Val Leu Tyr Glu Cys Cys
   65                  70                  75                  80
   Pro Gly Tyr Met Arg Met Glu Gly Met Lys Gly Cys Pro Ala Val Leu
                   85                  90                  95
   Pro Ile Asp His Val Tyr Gly Thr Leu Gly Ile Val Gly Ala Thr Thr
               100                 105                 110
   Thr Gln Arg Tyr Ser Asp Ala Ser Lys Leu Arg Glu Glu Ile Glu Gly
           115                 120                 125
   Lys Gly Ser Phe Thr Tyr Phe Ala Pro Ser Asn Glu Ala Trp Asp Asn
       130                 135                 140
   Leu Asp Ser Asp Ile Arg Arg Gly Leu Glu Ser Asn Val Asn Val Glu
   145                 150                 155                 160
   Leu Leu Asn Ala Leu His Ser His Met Ile Asn Lys Arg Met Leu Thr
                   165                 170                 175
   Lys Asp Leu Lys Asn Gly Met Ile Ile Pro Ser Met Tyr Asn Asn Leu
               180                 185                 190
   Gly Leu Phe Ile Asn His Tyr Pro Asn Gly Val Val Thr Val Asn Cys
           195                 200                 205
   Ala Arg Ile Ile His Gly Asn Gln Ile Ala Thr Asn Gly Val Val His
       210                 215                 220
   Val Ile Asp Arg Val Leu Thr Gln Ile Gly Thr Ser Ile Gln Asp Phe
   225                 230                 235                 240
   Ile Glu Ala Glu Asp Asp Leu Ser Ser Phe Arg Ala Ala Ala Ile Thr
   245 250 255
   Ser Asp Ile Leu Glu Ala Leu Gly Arg Asp Gly His Phe Thr Leu Phe
   260 265 270
   Ala Pro Thr Asn Glu Ala Phe Glu Lys Leu Pro Arg Gly Val Leu Glu
   275 280 285
   Arg Phe Met Gly Asp Lys Val Ala Ser Glu Ala Leu Met Lys Tyr His
   290 295 300
   Ile Leu Asn Thr Leu Gln Cys Ser Glu Ser Ile Met Gly Gly Ala Val
   305 310 315 320
   Phe Glu Thr Leu Glu Gly Asn Thr Ile Glu Ile Gly Cys Asp Gly Asp
   325 330 335
   Ser Ile Thr Val Asn Gly Ile Lys Met Val Asn Lys Lys Asp Ile Val
   340 345 350
   Thr Asn Asn Gly Val Ile His Leu Ile Asp Gln Val Leu Ile Pro Asp
   355 360 365
   Ser Ala Lys Gln Val Ile Glu Leu Ala Gly Lys Gln Gln Thr Thr Phe
   370 375 380
   Thr Asp Leu Val Ala Gln Leu Gly Leu Ala Ser Ala Leu Arg Pro Asp
   385 390 395 400
   Gly Glu Tyr Thr Leu Leu Ala Pro Val Asn Asn Ala Phe Ser Asp Asp
   405 410 415
   Thr Leu Ser Met Asp Gln Arg Leu Leu Lys Leu Ile Leu Gln Asn His
   420 425 430
   Ile Leu Lys Val Lys Val Gly Leu Asn Glu Leu Tyr Asn Gly Gln Ile
   435 440 445
   Leu Glu Thr Ile Gly Gly Lys Gln Leu Arg Val Phe Val Tyr Arg Thr
   450 455 460
   Ala Val Cys Ile Glu Asn Ser Cys Met Glu Lys Gly Ser Lys Gln Gly
   465 470 475 480
   Arg Asn Gly Ala Ile His Ile Phe Arg Glu Ile Ile Lys Pro Ala Glu
   485 490 495
   Lys Ser Leu His Glu Lys Leu Lys Gln Asp Lys Arg Phe Ser Thr Phe
   500 505 510
   Leu Ser Leu Leu Glu Ala Ala Asp Leu Lys Glu Leu Leu Thr Gln Pro
   515 520 525
   Gly Asp Trp Thr Leu Phe Val Pro Thr Asn Asp Ala Phe Lys Gly Met
   530 535 540
   Thr Ser Glu Glu Lys Glu Ile Leu Ile Arg Asp Lys Asn Ala Leu Gln
   545 550 555 560
   Asn Ile Ile Leu Tyr His Leu Thr Pro Gly Val Phe Ile Gly Lys Gly
   565 570 575
   Phe Glu Pro Gly Val Thr Asn Ile Leu Lys Thr Thr Gln Gly Ser Lys
   580 585 590
   Ile Phe Leu Lys Glu Val Asn Asp Thr Leu Leu Val Asn Glu Leu Lys
   595 600 605
   Ser Lys Glu Ser Asp Ile Met Thr Thr Asn Gly Val Ile His Val Val
   610 615 620
   Asp Lys Leu Leu Tyr Pro Ala Asp Thr Pro Val Gly Asn Asp Gln Leu
   625 630 635 640
   Leu Glu Ile Leu Asn Lys Leu Ile Lys Tyr Ile Gln Ile Lys Phe Val
   645 650 655
   Arg Gly Ser Thr Phe Lys Glu Ile Pro Val Thr Val Tyr Arg Pro Thr
   660 665 670
   Leu Thr Lys Val Lys Ile Glu Gly Glu Pro Glu Phe Arg Leu Ile Lys
   675 680 685
   Glu Gly Glu Thr Ile Thr Glu Val Ile His Gly Glu Pro Ile Ile Lys
   690 695 700
   Lys Tyr Thr Lys Ile Ile Asp Gly Val Pro Val Glu Ile Thr Glu Lys
   705 710 715 720
   Glu Thr Arg Glu Glu Arg Ile Ile Thr Gly Pro Glu Ile Lys Tyr Thr
   725 730 735
   Arg Ile Ser Thr Gly Gly Gly Glu Thr Glu Glu Thr Leu Lys Lys Leu
   740 745 750
   Leu Gln Glu Glu Val Thr Lys Val Thr Lys Phe Ile Glu Gly Gly Asp
   755 760 765
   Gly His Leu Phe Glu Asp Glu Glu Ile Lys Arg Leu Leu Gln Gly Asp
   770 775 780
   Thr Pro Val Arg Lys Leu Gln Ala Asn Lys Lys Val Gln Gly Ser Arg
   785 790 795 800
   Arg Arg Leu Arg Glu Gly Arg Ser Gln
   805
<210> 6
   <211> 2430
   <212> DNA
   <213> Homo sapiens
<400> 6
   atgattccct ttttacccat gttttctcta ctattgctgc ttattgttaa ccctataaac      60
   gccaacaatc attatgacaa gatcttggct catagtcgta tcaggggtcg ggaccaaggc     120
   ccaaatgtct gtgcccttca acagattttg ggcaccaaaa agaaatactt cagcacttgt     180
   aagaactggt ataaaaagtc catctgtgga cagaaaacga ctgtgttata tgaatgttgc     240
   cctggttata tgagaatgga aggaatgaaa ggctgcccag cagttttgcc cattgaccat     300
   gtttatggca ctctgggcat cgtgggagcc accacaacgc agcgctattc tgacgcctca     360
   aaactgaggg aggagatcga gggaaaggga tccttcactt actttgcacc gagtaatgag     420
   gcttgggaca acttggattc tgatatccgt agaggtttgg agagcaacgt gaatgttgaa     480
   ttactgaatg ctttacatag tcacatgatt aataagagaa tgttgaccaa ggacttaaaa     540
   aatggcatga ttattccttc aatgtataac aatttggggc ttttcattaa ccattatcct     600
   aatggggttg tcactgttaa ttgtgctcga atcatccatg ggaaccagat tgcaacaaat     660
   ggtgttgtcc atgtcattga ccgtgtgctt acacaaattg gtacctcaat tcaagacttc     720
   attgaagcag aagatgacct ttcatctttt agagcagctg ccatcacatc ggacatattg     780
   gaggcccttg gaagagacgg tcacttcaca ctctttgctc ccaccaatga ggcttttgag     840
   aaacttccac gaggtgtcct agaaaggatc atgggagaca aagtggcttc cgaagctctt     900
   atgaagtacc acatcttaaa tactctccag tgttctgagt ctattatggg aggagcagtc     960
   tttgagacgc tggaaggaaa tacaattgag ataggatgtg acggtgacag tataacagta    1020
   aatggaatca aaatggtgaa caaaaaggat attgtgacaa ataatggtgt gatccatttg    1080
   attgatcagg tcctaattcc tgattctgcc aaacaagtta ttgagctggc tggaaaacag    1140
   caaaccacct tcacggatct tgtggcccaa ttaggcttgg catctgctct gaggccagat    1200
   ggagaataca ctttgctggc acctgtgaat aatgcatttt ctgatgatac tctcagcatg    1260
   gatcagcgcc tccttaaatt aattctgcag aatcacatat tgaaagtaaa agttggcctt    1320
   aatgagcttt acaacgggca aatactggaa accatcggag gcaaacagct cagagtcttc    1380
   gtatatcgta cagctgtctg cattgaaaat tcatgcatgg agaaagggag taagcaaggg    1440
   agaaacggtg cgattcacat attccgcgag atcatcaagc cagcagagaa atccctccat    1500
   gaaaagttaa aacaagataa gcgctttagc accttcctca gcctacttga agctgcagac    1560
   ttgaaagagc tcctgacaca acctggagac tggacattat ttgtgccaac caatgatgct    1620
   tttaagggaa tgactagtga agaaaaagaa attctgatac gggacaaaaa tgctcttcaa    1680
   aacatcattc tttatcacct gacaccagga gttttcattg gaaaaggatt tgaacctggt    1740
   gttactaaca ttttaaagac cacacaagga agcaaaatct ttctgaaaga agtaaatgat    1800
   acacttctgg tgaatgaatt gaaatcaaaa gaatctgaca tcatgacaac aaatggtgta    1860
   attcatgttg tagataaact cctctatcca gcagacacac ctgttggaaa tgatcaactg    1920
   ctggaaatac ttaataaatt aatcaaatac atccaaatta agtttgttcg tggtagcacc    1980
   ttcaaagaaa tccccgtgac tgtctataga cccacactaa caaaagtcaa aattgaaggt    2040
   gaacctgaat tcagactgat taaagaaggt gaaacaataa ctgaagtgat ccatggagag    2100
   ccaattatta aaaaatacac caaaatcatt gatggagtgc ctgtggaaat aactgaaaaa    2160
   gagacacgag aagaacgaat cattacaggt cctgaaataa aatacactag gatttctact    2220
   ggaggtggag aaacagaaga aactctgaag aaattgttac aagaagaggt caccaaggtc    2280
   accaaattca ttgaaggtgg tgatggtcat ttatttgaag atgaagaaat taaaagactg    2340
   cttcagggag acacacccgt gaggaagttg caagccaaca aaaaagttca aggatctaga    2400
   agacgattaa gggaaggtcg ttctcagtga                                     2430
<210> 7
   <211> 808
   <212> PRT
   <213> Homo sapiens
<400> 7
   Met Ile Pro Phe Leu Pro Met Phe Ser Leu Leu Leu Leu Leu Ile Val
   1               5                   10                  15
   Asn Pro Ile Asn Ala Asn Asn His Tyr Asp Lys Ile Leu Ala His Ser
               20                  25                  30
   Arg Ile Arg Gly Arg Asp Gln Gly Pro Asn Val Cys Ala Leu Gln Gln
           35                  40                  45
   Ile Leu Gly Thr Lys Lys Lys Tyr Phe Ser Thr Cys Lys Asn Trp Tyr
       50                  55                  60
   Lys Lys Ser Ile Cys Gly Gln Lys Thr Thr Val Leu Tyr Glu Cys Cys
   65                  70                  75                  80
   Pro Gly Tyr Met Arg Met Glu Gly Met Lys Gly Cys Pro Ala Val Leu
                   85                  90                  95
   Pro Ile Asp His Val Tyr Gly Thr Leu Gly Ile Val Gly Ala Thr Thr
               100                 105                 110
   Thr Gln Arg Tyr Ser Asp Ala Ser Lys Leu Arg Glu Glu Ile Glu Gly
           115                 120                 125
   Lys Gly Ser Phe Thr Tyr Phe Ala Pro Ser Asn Glu Ala Trp Asp Asn
       130                 135                 140
   Leu Asp Ser Asp Ile Arg Arg Gly Leu Glu Ser Asn Val Asn Val Glu
   145                 150                 155                 160
   Leu Leu Asn Ala Leu His Ser His Met Ile Asn Lys Arg Met Leu Thr
                   165                 170                 175
   Lys Asp Leu Lys Asn Gly Met Ile Ile Pro Ser Met Tyr Asn Asn Leu
               180                 185                 190
   Gly Leu Phe Ile Asn His Tyr Pro Asn Gly Val Val Thr Val Asn Cys
           195                 200                 205
   Ala Arg Ile Ile His Gly Asn Gln Ile Ala Thr Asn Gly Val Val His
       210                 215                 220
   Val Ile Asp Arg Val Leu Thr Gln Ile Gly Thr Ser Ile Gln Asp Phe
   225                 230                 235                 240
   Ile Glu Ala Glu Asp Asp Leu Ser Ser Phe Arg Ala Ala Ala Ile Thr
                   245                 250                 255
   Ser Asp Ile Leu Glu Ala Leu Gly Arg Asp Gly His Phe Thr Leu Phe
               260                 265                 270
   Ala Pro Thr Asn Glu Ala Phe Glu Lys Leu Pro Arg Gly Val Leu Glu
           275                 280                 285
   Arg Phe Met Gly Asp Lys Val Ala Ser Glu Ala Leu Met Lys Tyr His
       290                 295                 300
   Ile Leu Asn Thr Leu Gln Cys Ser Glu Ser Ile Met Gly Gly Ala Val
   305                 310                 315                 320
   Phe Glu Thr Leu Glu Gly Asn Thr Ile Glu Ile Gly Cys Asp Gly Asp
                   325                 330                 335
   Ser Ile Thr Val Asn Gly Ile Lys Met Val Asn Lys Lys Asp Ile Val
               340                 345                 350
   Thr Asn Asn Gly Val Ile His Leu Ile Asp Gln Val Leu Ile Pro Asp
           355                 360                 365
   Ser Ala Lys Gln Val Ile Glu Leu Ala Gly Lys Gln Gln Thr Thr Phe
       370                 375                 380
   Thr Asp Leu Val Ala Gln Leu Gly Leu Ala Ser Ala Leu Arg Pro Asp
   385                 390                 395                 400
   Gly Glu Tyr Thr Leu Leu Ala Pro Val Asn Asn Ala Phe Ser Asp Asp
   405 410 415
   Thr Leu Ser Met Asp Gln Arg Leu Leu Lys Leu Ile Leu Gln Asn His
   420 425 430
   Ile Leu Lys Val Lys Val Gly Leu Asn Glu Leu Tyr Asn Gly Gln Ile
   435 440 445
   Leu Glu Thr Ile Gly Gly Lys Gln Leu Arg Val Phe Val Tyr Arg Thr
   450 455 460
   Ala Val Cys Ile Glu Asn Ser Cys Met Glu Lys Gly Ser Lys Gln Gly
   465 470 475 480
   Arg Asn Gly Ala Ile His Ile Phe Arg Glu Ile Ile Lys Pro Ala Glu
   485 490 495
   Lys Ser Leu His Glu Lys Leu Lys Gln Asp Lys Arg Phe Ser Thr Phe
   500 505 510
   Leu Ser Leu Leu Glu Ala Ala Asp Leu Lys Glu Leu Leu Thr Gln Pro
   515 520 525
   Gly Asp Trp Thr Leu Phe Val Pro Thr Asn Asp Ala Phe Lys Gly Met
   530 535 540
   Thr Ser Glu Glu Lys Glu Ile Leu Ile Arg Asp Lys Asn Ala Leu Gln
   545 550 555 560
   Asn Ile Ile Leu Tyr His Leu Thr Pro Gly Val Phe Ile Gly Lys Gly
   565 570 575
   Phe Glu Pro Gly Val Thr Asn Ile Leu Lys Thr Thr Gln Gly Ser Lys
   580 585 590
   Ile Phe Leu Lys Glu Val Asn Asp Thr Leu Leu Val Asn Glu Leu Lys
   595 600 605
   Ser Lys Glu Ser Asp Ile Met Thr Thr Asn Gly Val Ile His Val Val
   610 615 620
   Asp Lys Leu Leu Tyr Pro Ala Asp Thr Pro Val Gly Asn Asp Gln Leu
   625 630 635 640
   Leu Glu Ile Leu Asn Lys Leu Ile Lys Tyr Ile Gln Ile Lys Phe Val
   645 650 655
   Arg Gly Ser Thr Phe Lys Glu Ile Pro Val Thr Val Tyr Thr Thr Lys
   660 665 670
   Ile Ile Thr Lys Val Val Glu Pro Lys Ile Lys Val Ile Glu Gly Ser
           675                 680                 685
   Leu Gln Pro Ile Ile Lys Thr Glu Gly Pro Thr Leu Thr Lys Val Lys
       690                 695                 700
   Ile Glu Gly Glu Pro Glu Phe Arg Leu Ile Lys Glu Gly Glu Thr Ile
   705                 710                 715                 720
   Thr Glu Val Ile His Gly Glu Pro Ile Ile Lys Lys Tyr Thr Lys Ile
                   725                 730                 735
   Ile Asp Gly Val Pro Val Glu Ile Thr Glu Lys Glu Thr Arg Glu Glu
               740                 745                 750
   Arg Ile Ile Thr Gly Pro Glu Ile Lys Tyr Thr Arg Ile Ser Thr Gly
           755                 760                 765
   Gly Gly Glu Thr Glu Glu Thr Leu Lys Lys Leu Leu Gln Glu Asp Thr
       770                 775                 780
   Pro Val Arg Lys Leu Gln Ala Asn Lys Lys Val Gln Gly Ser Arg Arg
   785                 790                 795                 800
   Arg Leu Arg Glu Gly Arg Ser Gln
                   805
<210> 8
   <211> 2427
   <212> DNA
   <213> Homo sapiens
<400> 8
   atgattccct ttttacccat gttttctcta ctattgctgc ttattgttaa ccctataaac 60
   gccaacaatc attatgacaa gatcttggct catagtcgta tcaggggtcg ggaccaaggc 120
   ccaaatgtct gtgcccttca acagattttg ggcaccaaaa agaaatactt cagcacttgt 180
   aagaactggt ataaaaagtc catctgtgga cagaaaacga ctgtgttata tgaatgttgc 240
   cctggttata tgagaatgga aggaatgaaa ggctgcccag cagttttgcc cattgaccat 300
   gtttatggca ctctgggcat cgtgggagcc accacaacgc agcgctattc tgacgcctca 360
   aaactgaggg aggagatcga gggaaaggga tccttcactt actttgcacc gagtaatgag 420
   gcttgggaca acttggattc tgatatccgt agaggtttgg agagcaacgt gaatgttgaa 480
   ttactgaatg ctttacatag tcacatgatt aataagagaa tgttgaccaa ggacttaaaa 540
   aatggcatga ttattccttc aatgtataac aatttggggc ttttcattaa ccattatcct 600
   aatggggttg tcactgttaa ttgtgctcga atcatccatg ggaaccagat tgcaacaaat 660
   ggtgttgtcc atgtcattga ccgtgtgctt acacaaattg gtacctcaat tcaagacttc 720
   attgaagcag aagatgacct ttcatctttt agagcagctg ccatcacatc ggacatattg 780
   gaggcccttg gaagagacgg tcacttcaca ctctttgctc ccaccaatga ggcttttgag     840
   aaacttccac gaggtgtcct agaaaggatc atgggagaca aagtggcttc cgaagctctt     900
   atgaagtacc acatcttaaa tactctccag tgttctgagt ctattatggg aggagcagtc     960
   tttgagacgc tggaaggaaa tacaattgag ataggatgtg acggtgacag tataacagta    1020
   aatggaatca aaatggtgaa caaaaaggat attgtgacaa ataatggtgt gatccatttg    1080
   attgatcagg tcctaattcc tgattctgcc aaacaagtta ttgagctggc tggaaaacag    1140
   caaaccacct tcacggatct tgtggcccaa ttaggcttgg catctgctct gaggccagat    1200
   ggagaataca ctttgctggc acctgtgaat aatgcatttt ctgatgatac tctcagcatg    1260
   gatcagcgcc tccttaaatt aattctgcag aatcacatat tgaaagtaaa agttggcctt    1320
   aatgagcttt acaacgggca aatactggaa accatcggag gcaaacagct cagagtcttc    1380
   gtatatcgta cagctgtctg cattgaaaat tcatgcatgg agaaagggag taagcaaggg    1440
   agaaacggtg cgattcacat attccgcgag atcatcaagc cagcagagaa atccctccat    1500
   gaaaagttaa aacaagataa gcgctttagc accttcctca gcctacttga agctgcagac    1560
   ttgaaagagc tcctgacaca acctggagac tggacattat ttgtgccaac caatgatgct    1620
   tttaagggaa tgactagtga agaaaaagaa attctgatac gggacaaaaa tgctcttcaa    1680
   aacatcattc tttatcacct gacaccagga gttttcattg gaaaaggatt tgaacctggt    1740
   gttactaaca ttttaaagac cacacaagga agcaaaatct ttctgaaaga agtaaatgat    1800
   acacttctgg tgaatgaatt gaaatcaaaa gaatctgaca tcatgacaac aaatggtgta    1860
   attcatgttg tagataaact cctctatcca gcagacacac ctgttggaaa tgatcaactg    1920
   ctggaaatac ttaataaatt aatcaaatac atccaaatta agtttgttcg tggtagcacc    1980
   ttcaaagaaa tccccgtgac tgtctataca actaaaatta taaccaaagt tgtggaacca    2040
   aaaattaaag tgattgaagg cagtcttcag cctattatca aaactgaagg acccacacta    2100
   acaaaagtca aaattgaagg tgaacctgaa ttcagactga ttaaagaagg tgaaacaata    2160
   actgaagtga tccatggaga gccaattatt aaaaaataca ccaaaatcat tgatggagtg    2220
   cctgtggaaa taactgaaaa agagacacga gaagaacgaa tcattacagg tcctgaaata    2280
   aaatacacta ggatttctac tggaggtgga gaaacagaag aaactctgaa gaaattgtta    2340
   caagaagaca cacccgtgag gaagttgcaa gccaacaaaa aagttcaagg atctagaaga    2400
   cgattaaggg aaggtcgttc tcagtga                                        2427
<210> 9
   <211> 721
   <212> PRT
   <213> Homo sapiens
<400> 9
   Met Ile Pro Phe Leu Pro Met Phe Ser Leu Leu Leu Leu Leu Ile Val
   1               5                   10                  15
   Asn Pro Ile Asn Ala Asn Asn His Tyr Asp Lys Ile Leu Ala His Ser
               20                  25                  30
   Arg Ile Arg Gly Arg Asp Gln Gly Pro Asn Val Cys Ala Leu Gln Gln
           35                  40                  45
   Ile Leu Gly Thr Lys Lys Lys Tyr Phe Ser Thr Cys Lys Asn Trp Tyr
       50                  55                  60
   Lys Lys Ser Ile Cys Gly Gln Lys Thr Thr Val Leu Tyr Glu Cys Cys
   65                  70                  75                  80
   Pro Gly Tyr Met Arg Met Glu Gly Met Lys Gly Cys Pro Ala Val Leu
                   85                  90                  95
   Pro Ile Asp His Val Tyr Gly Thr Leu Gly Ile Val Gly Ala Thr Thr
               100                 105                 110
   Thr Gln Arg Tyr Ser Asp Ala Ser Lys Leu Arg Glu Glu Ile Glu Gly
           115                 120                 125
   Lys Gly Ser Phe Thr Tyr Phe Ala Pro Ser Asn Glu Ala Trp Asp Asn
       130                 135                 140
   Leu Asp Ser Asp Ile Arg Arg Gly Leu Glu Ser Asn Val Asn Val Glu
   145                 150                 155                 160
   Leu Leu Asn Ala Leu His Ser His Met Ile Asn Lys Arg Met Leu Thr
                   165                 170                 175
   Lys Asp Leu Lys Asn Gly Met Ile Ile Pro Ser Met Tyr Asn Asn Leu
               180                 185                 190
   Gly Leu Phe Ile Asn His Tyr Pro Asn Gly Val Val Thr Val Asn Cys
           195                 200                 205
   Ala Arg Ile Ile His Gly Asn Gln Ile Ala Thr Asn Gly Val Val His
       210                 215                 220
   Val Ile Asp Arg Val Leu Thr Gln Ile Gly Thr Ser Ile Gln Asp Phe
   225                 230                 235                 240
   Ile Glu Ala Glu Asp Asp Leu Ser Ser Phe Arg Ala Ala Ala Ile Thr
                   245                 250                 255
   Ser Asp Ile Leu Glu Ala Leu Gly Arg Asp Gly His Phe Thr Leu Phe
               260                 265                 270
   Ala Pro Thr Asn Glu Ala Phe Glu Lys Leu Pro Arg Gly Val Leu Glu
           275                 280                 285
   Arg Phe Met Gly Asp Lys Val Ala Ser Glu Ala Leu Met Lys Tyr His
   290 295 300
   Ile Leu Asn Thr Leu Gln Cys Ser Glu Ser Ile Met Gly Gly Ala Val
   305 310 315 320
   Phe Glu Thr Leu Glu Gly Asn Thr Ile Glu Ile Gly Cys Asp Gly Asp
   325 330 335
   Ser Ile Thr Val Asn Gly Ile Lys Met Val Asn Lys Lys Asp Ile Val
   340 345 350
   Thr Asn Asn Gly Val Ile His Leu Ile Asp Gln Val Leu Ile Pro Asp
   355 360 365
   Ser Ala Lys Gln Val Ile Glu Leu Ala Gly Lys Gln Gln Thr Thr Phe
   370 375 380
   Thr Asp Leu Val Ala Gln Leu Gly Leu Ala Ser Ala Leu Arg Pro Asp
   385 390 395 400
   Gly Glu Tyr Thr Leu Leu Ala Pro Val Asn Asn Ala Phe Ser Asp Asp
   405 410 415
   Thr Leu Ser Met Val Gln Arg Leu Leu Lys Leu Ile Leu Gln Asn His
   420 425 430
   Ile Leu Lys Val Lys Val Gly Leu Asn Glu Leu Tyr Asn Gly Gln Ile
   435 440 445
   Leu Glu Thr Ile Gly Gly Lys Gln Leu Arg Val Phe Val Tyr Arg Thr
   450 455 460
   Ala Val Cys Ile Glu Asn Ser Cys Met Glu Lys Gly Ser Lys Gln Gly
   465 470 475 480
   Arg Asn Gly Ala Ile His Ile Phe Arg Glu Ile Ile Lys Pro Ala Glu
   485 490 495
   Lys Ser Leu His Glu Lys Leu Lys Gln Asp Lys Arg Phe Ser Thr Phe
   500 505 510
   Leu Ser Leu Leu Glu Ala Ala Asp Leu Lys Glu Leu Leu Thr Gln Pro
   515 520 525
   Gly Asp Trp Thr Leu Phe Val Pro Thr Asn Asp Ala Phe Lys Gly Met
   530 535 540
   Thr Ser Glu Glu Lys Glu Ile Leu Ile Arg Asp Lys Asn Ala Leu Gln
   545 550 555 560
   Asn Ile Ile Leu Tyr His Leu Thr Pro Gly Val Phe Ile Gly Lys Gly
   565 570 575
   Phe Glu Pro Gly Val Thr Asn Ile Leu Lys Thr Thr Gln Gly Ser Lys
   580 585 590
   Ile Phe Leu Lys Glu Val Asn Asp Thr Leu Leu Val Asn Glu Leu Lys
   595 600 605
   Ser Lys Glu Ser Asp Ile Met Thr Thr Asn Gly Val Ile His Val Val
   610 615 620
   Asp Lys Leu Leu Tyr Pro Ala Asp Thr Pro Val Gly Asn Asp Gln Leu
   625 630 635 640
   Leu Glu Ile Leu Asn Lys Leu Ile Lys Tyr Ile Gln Ile Lys Phe Val
   645 650 655
   Arg Gly Ser Thr Phe Lys Glu Ile Pro Val Thr Val Tyr Gly Pro Glu
   660 665 670
   Ile Lys Tyr Thr Arg Ile Ser Thr Gly Gly Gly Glu Thr Glu Glu Thr
   675 680 685
   Leu Lys Lys Leu Leu Gln Glu Asp Thr Pro Val Arg Lys Leu Gln Ala
   690 695 700
   Asn Lys Lys Val Gln Gly Ser Arg Arg Arg Leu Arg Glu Gly Arg Ser
   705 710 715 720
   Gln
<210> 10
   <211> 2166
   <212> DNA
   <213> Homo sapiens
<400> 10
   atgattccct ttttacccat gttttctcta ctattgctgc ttattgttaa ccctataaac 60
   gccaacaatc attatgacaa gatcttggct catagtcgta tcaggggtcg ggaccaaggc 120
   ccaaatgtct gtgcccttca acagattttg ggcaccaaaa agaaatactt cagcacttgt 180
   aagaactggt ataaaaagtc catctgtgga cagaaaacga ctgttttata tgaatgttgc 240
   cctggttata tgagaatgga aggaatgaaa ggctgcccag cagttttgcc cattgaccat 300
   gtttatggca ctctgggcat cgtgggagcc accacaacgc agcgctattc tgacgcctca 360
   aaactgaggg aggagatcga gggaaaggga tccttcactt actttgcacc gagtaatgag 420
   gcttgggaca acttggattc tgatatccgt agaggtttgg agagcaacgt gaatgttgaa 480
   ttactgaatg ctttacatag tcacatgatt aataagagaa tgttgaccaa ggacttaaaa 540
   aatggcatga ttattccttc aatgtataac aatttggggc ttttcattaa ccattatcct 600
   aatggggttg tcactgttaa ttgtgctcga atcatccatg ggaaccagat tgcaacaaat 660
   ggtgttgtcc atgtcattga ccgtgtgctt acacaaattg gtacctcaat tcaagacttc 720
   attgaagcag aagatgacct ttcatctttt agagcagctg ccatcacatc ggacatattg 780
   gaggcccttg gaagagacgg tcacttcaca ctctttgctc ccaccaatga ggcttttgag 840
   aaacttccac gaggtgtcct agaaaggttc atgggagaca aagtggcttc cgaagctctt 900
   atgaagtacc acatcttaaa tactctccag tgttctgagt ctattatggg aggagcagtc 960
   tttgagacgc tggaaggaaa tacaattgag ataggatgtg acggtgacag tataacagta 1020
   aatggaatca aaatggtgaa caaaaaggat attgtgacaa ataatggtgt gatccatttg 1080
   attgatcagg tcctaattcc tgattctgcc aaacaagtta ttgagctggc tggaaaacag 1140
   caaaccacct tcacggatct tgtggcccaa ttaggcttgg catctgctct gaggccagat 1200
   ggagaataca ctttgctggc acctgtgaat aatgcatttt ctgatgatac tctcagcatg 1260
   gttcagcgcc tccttaaatt aattctgcag aatcacatat tgaaagtaaa agttggcctt 1320
   aatgagcttt acaacgggca aatactggaa accatcggag gcaaacagct cagagtcttc 1380
   gtatatcgta cagctgtctg cattgaaaat tcatgcatgg agaaagggag taagcaaggg 1440
   agaaacggtg cgattcacat attccgcgag atcatcaagc cagcagagaa atccctccat 1500
   gaaaagttaa aacaagataa gcgctttagc accttcctca gcctacttga agctgcagac 1560
   ttgaaagagc tcctgacaca acctggagac tggacattat ttgtgccaac caatgatgct 1620
   tttaagggaa tgactagtga agaaaaagaa attctgatac gggacaaaaa tgctcttcaa 1680
   aacatcattc tttatcacct gacaccagga gttttcattg gaaaaggatt tgaacctggt 1740
   gttactaaca ttttaaagac cacacaagga agcaaaatct ttctgaaaga agtaaatgat 1800
   acacttctgg tgaatgaatt gaaatcaaaa gaatctgaca tcatgacaac aaatggtgta 1860
   attcatgttg tagataaact cctctatcca gcagacacac ctgttggaaa tgatcaactg 1920
   ctggaaatac ttaataaatt aatcaaatac atccaaatta agtttgttcg tggtagcacc 1980
   ttcaaagaaa tccccgtgac tgtctatggt cctgaaataa aatacactag gatttctact 2040
   ggaggtggag aaacagaaga aactctgaag aaattgttac aagaagacac acccgtgagg 2100
   aagttgcaag ccaacaaaaa agttcaaggt tctagaagac gattaaggga aggtcgttct 2160
   cagtga 2166
<210> 11
   <211> 752
   <212> PRT
   <213> Homo sapiens
<400> 11
   Met Ile Pro Phe Leu Pro Met Phe Ser Leu Leu Leu Leu Leu Ile Val
   1               5                   10                  15
   Asn Pro Ile Asn Ala Asn Asn His Tyr Asp Lys Ile Leu Ala His Ser
   20 25 30
   Arg Ile Arg Gly Arg Asp Gln Gly Pro Asn Val Cys Ala Leu Gln Gln
   35 40 45
   Ile Leu Gly Thr Lys Lys Lys Tyr Phe Ser Thr Cys Lys Asn Trp Tyr
   50 55 60
   Lys Lys Ser Ile Cys Gly Gln Lys Thr Thr Val Leu Tyr Glu Cys Cys
   65 70 75 80
   Pro Gly Tyr Met Arg Met Glu Gly Met Lys Gly Cys Pro Ala Val Leu
   85 90 95
   Pro Ile Asp His Val Tyr Gly Thr Leu Gly Ile Val Gly Ala Thr Thr
   100 105 110
   Thr Gln Arg Tyr Ser Asp Ala Ser Lys Leu Arg Glu Glu Ile Glu Gly
   115 120 125
   Lys Gly Ser Phe Thr Tyr Phe Ala Pro Ser Asn Glu Ala Trp Asp Asn
   130 135 140
   Leu Asp Ser Asp Ile Arg Arg Gly Leu Glu Ser Asn Val Asn Val Glu
   145 150 155 160
   Leu Leu Asn Ala Leu His Ser His Met Ile Asn Lys Arg Met Leu Thr
   165 170 175
   Lys Asp Leu Lys Asn Gly Met Ile Ile Pro Ser Met Tyr Asn Asn Leu
   180 185 190
   Gly Leu Phe Ile Asn His Tyr Pro Asn Gly Val Val Thr Val Asn Cys
   195 200 205
   Ala Arg Ile Ile His Gly Asn Gln Ile Ala Thr Asn Gly Val Val His
   210 215 220
   Val Ile Asp Arg Val Leu Thr Gln Ile Gly Thr Ser Ile Gln Asp Phe
   225 230 235 240
   Ile Glu Ala Glu Asp Asp Leu Ser Ser Phe Arg Ala Ala Ala Ile Thr
   245 250 255
   Ser Asp Ile Leu Glu Ala Leu Gly Arg Asp Gly His Phe Thr Leu Phe
   260 265 270
   Ala Pro Thr Asn Glu Ala Phe Glu Lys Leu Pro Arg Gly Val Leu Glu
   275 280 285
   Arg Phe Met Gly Asp Lys Val Ala Ser Glu Ala Leu Met Lys Tyr His
       290                 295                 300
   Ile Leu Asn Thr Leu Gln Cys Ser Glu Ser Ile Met Gly Gly Ala Val
   305                 310                 315                 320
   Phe Glu Thr Leu Glu Gly Asn Thr Ile Glu Ile Gly Cys Asp Gly Asp
                   325                 330                 335
   Ser Ile Thr Val Asn Gly Ile Lys Met Val Asn Lys Lys Asp Ile Val
               340                 345                 350
   Thr Asn Asn Gly Val Ile His Leu Ile Asp Gln Val Leu Ile Pro Asp
           355                 360                 365
   Ser Ala Lys Gln Val Ile Glu Leu Ala Gly Lys Gln Gln Thr Thr Phe
       370                 375                 380
   Thr Asp Leu Val Ala Gln Leu Gly Leu Ala Ser Ala Leu Arg Pro Asp
   385                 390                 395                 400
   Gly Glu Tyr Thr Leu Leu Ala Pro Val Asn Asn Ala Phe Ser Asp Asp
                   405                 410                 415
   Thr Leu Ser Met Val Gln Arg Leu Leu Lys Leu Ile Leu Gln Asn His
               420                 425                 430
   Ile Leu Lys Val Lys Val Gly Leu Asn Glu Leu Tyr Asn Gly Gln Ile
           435                 440                 445
   Leu Glu Thr Ile Gly Gly Lys Gln Leu Arg Val Phe Val Tyr Arg Thr
       450                 455                 460
   Ala Val Cys Ile Glu Asn Ser Cys Met Glu Lys Gly Ser Lys Gln Gly
   465                 470                 475                 480
   Arg Asn Gly Ala Ile His Ile Phe Arg Glu Ile Ile Lys Pro Ala Glu
                   485                 490                 495
   Lys Ser Leu His Glu Lys Leu Lys Gln Asp Lys Arg Phe Ser Thr Phe
               500                 505                 510
   Leu Ser Leu Leu Glu Ala Ala Asp Leu Lys Glu Leu Leu Thr Gln Pro
           515                 520                 525
   Gly Asp Trp Thr Leu Phe Val Pro Thr Asn Asp Ala Phe Lys Gly Met
       530                 535                 540
   Thr Ser Glu Glu Lys Glu Ile Leu Ile Arg Asp Lys Asn Ala Leu Gln
   545                 550                 555                 560
   Asn Ile Ile Leu Tyr His Leu Thr Pro Gly Val Phe Ile Gly Lys Gly
                   565                 570                 575
   Phe Glu Pro Gly Val Thr Asn Ile Leu Lys Thr Thr Gln Gly Ser Lys
               580                 585                 590
   Ile Phe Leu Lys Glu Val Asn Asp Thr Leu Leu Val Asn Glu Leu Lys
           595                 600                 605
   Ser Lys Glu Ser Asp Ile Met Thr Thr Asn Gly Val Ile His Val Val
       610                 615                 620
   Asp Lys Leu Leu Tyr Pro Ala Asp Thr Pro Val Gly Asn Asp Gln Leu
   625                 630                 635                 640
   Leu Glu Ile Leu Asn Lys Leu Ile Lys Tyr Ile Gln Ile Lys Phe Val
                   645                 650                 655
   Arg Gly Ser Thr Phe Lys Glu Ile Pro Val Thr Val Tyr Lys Pro Ile
               660                 665                 670
   Ile Lys Lys Tyr Thr Lys Ile Ile Asp Gly Val Pro Val Glu Ile Thr
           675                 680                 685
   Glu Lys Glu Thr Arg Glu Glu Arg Ile Ile Thr Gly Pro Glu Ile Lys
       690                 695                 700
   Tyr Thr Arg Ile Ser Thr Gly Gly Gly Glu Thr Glu Glu Thr Leu Lys
   705                 710                 715                 720
   Lys Leu Leu Gln Glu Glu Asp Thr Pro Val Arg Lys Leu Gln Ala Asn
                   725                 730                 735
   Lys Lys Val Gln Gly Ser Arg Arg Arg Leu Arg Glu Gly Arg Ser Gln
               740                 745                 750
<210> 12
   <211> 2259
   <212> DNA
   <213> Homo sapiens
<400> 12
   atgattccct ttttacccat gttttctcta ctattgctgc ttattgttaa ccctataaac 60
   gccaacaatc attatgacaa gatcttggct catagtcgta tcaggggtcg ggaccaaggc 120
   ccaaatgtct gtgcccttca acagattttg ggcaccaaaa agaaatactt cagcacttgt 180
   aagaactggt ataaaaagtc catctgtgga cagaaaacga ctgttttata tgaatgttgc 240
   cctggttata tgagaatgga aggaatgaaa ggctgcccag cagttttgcc cattgaccat 300
   gtttatggca ctctgggcat cgtgggagcc accacaacgc agcgctattc tgacgcctca 360
   aaactgaggg aggagatcga gggaaaggga tccttcactt actttgcacc gagtaatgag 420
   gcttgggaca acttggattc tgatatccgt agaggtttgg agagcaacgt gaatgttgaa 480
   ttactgaatg ctttacatag tcacatgatt aataagagaa tgttgaccaa ggacttaaaa 540
   aatggcatga ttattccttc aatgtataac aatttggggc ttttcattaa ccattatcct 600
   aatggggttg tcactgttaa ttgtgctcga atcatccatg ggaaccagat tgcaacaaat 660
   ggtgttgtcc atgtcattga ccgtgtgctt acacaaattg gtacctcaat tcaagacttc 720
   attgaagcag aagatgacct ttcatctttt agagcagctg ccatcacatc ggacatattg 780
   gaggcccttg gaagagacgg tcacttcaca ctctttgctc ccaccaatga ggcttttgag 840
   aaacttccac gaggtgtcct agaaaggttc atgggagaca aagtggcttc cgaagctctt 900
   atgaagtacc acatcttaaa tactctccag tgttctgagt ctattatggg aggagcagtc 960
   tttgagacgc tggaaggaaa tacaattgag ataggatgtg acggtgacag tataacagta 1020
   aatggaatca aaatggtgaa caaaaaggat attgtgacaa ataatggtgt gatccatttg 1080
   attgatcagg tcctaattcc tgattctgcc aaacaagtta ttgagctggc tggaaaacag 1140
   caaaccacct tcacggatct tgtggcccaa ttaggcttgg catctgctct gaggccagat 1200
   ggagaataca ctttgctggc acctgtgaat aatgcatttt ctgatgatac tctcagcatg 1260
   gttcagcgcc tccttaaatt aattctgcag aatcacatat tgaaagtaaa agttggcctt 1320
   aatgagcttt acaacgggca aatactggaa accatcggag gcaaacagct cagagtcttc 1380
   gtatatcgta cagctgtctg cattgaaaat tcatgcatgg agaaagggag taagcaaggg 1440
   agaaacggtg cgattcacat attccgcgag atcatcaagc cagcagagaa atccctccat 1500
   gaaaagttaa aacaagataa gcgctttagc accttcctca gcctacttga agctgcagac 1560
   ttgaaagagc tcctgacaca acctggagac tggacattat ttgtgccaac caatgatgct 1620
   tttaagggaa tgactagtga agaaaaagaa attctgatac gggacaaaaa tgctcttcaa 1680
   aacatcattc tttatcacct gacaccagga gttttcattg gaaaaggatt tgaacctggt 1740
   gttactaaca ttttaaagac cacacaagga agcaaaatct ttctgaaaga agtaaatgat 1800
   acacttctgg tgaatgaatt gaaatcaaaa gaatctgaca tcatgacaac aaatggtgta 1860
   attcatgttg tagataaact cctctatcca gcagacacac ctgttggaaa tgatcaactg 1920
   ctggaaatac ttaataaatt aatcaaatac atccaaatta agtttgttcg tggtagcacc 1980
   ttcaaagaaa tccccgtgac tgtctataaa ccaattatta aaaaatacac caaaatcatt 2040
   gatggagtgc ctgtggaaat aactgaaaaa gagacacgag aagaacgaat cattacaggt 2100
   cctgaaataa aatacactag gatttctact ggaggtggag aaacagaaga aactctgaag 2160
   aaattgttac aagaagagga cacacccgtg aggaagttgc aagccaacaa aaaagttcaa 2220
   ggttctagaa gacgattaag ggaaggtcgt tctcagtga 2259
<210> 13
   <211> 749
   <212> PRT
   <213> Homo sapiens
<400> 13
   Met Ile Pro Phe Leu Pro Met Phe Ser Leu Leu Leu Leu Leu Ile Val
   1               5                   10                  15
   Asn Pro Ile Asn Ala Asn Asn His Tyr Asp Lys Ile Leu Ala His Ser
               20                  25                  30
   Arg Ile Arg Gly Arg Asp Gln Gly Pro Asn Val Cys Ala Leu Gln Gln
           35                  40                  45
   Ile Leu Gly Thr Lys Lys Lys Tyr Phe Ser Thr Cys Lys Asn Trp Tyr
       50                  55                  60
   Lys Lys Ser Ile Cys Gly Gln Lys Thr Thr Val Leu Tyr Glu Cys Cys
   65                  70                  75                  80
   Pro Gly Tyr Met Arg Met Glu Gly Met Lys Gly Cys Pro Ala Val Leu
                   85                  90                  95
   Pro Ile Asp His Val Tyr Gly Thr Leu Gly Ile Val Gly Ala Thr Thr
               100                 105                 110
   Thr Gln Arg Tyr Ser Asp Ala Ser Lys Leu Arg Glu Glu Ile Glu Gly
           115                 120                 125
   Lys Gly Ser Phe Thr Tyr Phe Ala Pro Ser Asn Glu Ala Trp Asp Asn
       130                 135                 140
   Leu Asp Ser Asp Ile Arg Arg Gly Leu Glu Ser Asn Val Asn Val Glu
   145                 150                 155                 160
   Leu Leu Asn Ala Leu His Ser His Met Ile Asn Lys Arg Met Leu Thr
                   165                 170                 175
   Lys Asp Leu Lys Asn Gly Met Ile Ile Pro Ser Met Tyr Asn Asn Leu
               180                 185                 190
   Gly Leu Phe Ile Asn His Tyr Pro Asn Gly Val Val Thr Val Asn Cys
           195                 200                 205
   Ala Arg Ile Ile His Gly Asn Gln Ile Ala Thr Asn Gly Val Val His
       210                 215                 220
   Val Ile Asp Arg Val Leu Thr Gln Ile Gly Thr Ser Ile Gln Asp Phe
   225                 230                 235                 240
   Ile Glu Ala Glu Asp Asp Leu Ser Ser Phe Arg Ala Ala Ala Ile Thr
                   245                 250                 255
   Ser Asp Ile Leu Glu Ala Leu Gly Arg Asp Gly His Phe Thr Leu Phe
               260                 265                 270
   Ala Pro Thr Asn Glu Ala Phe Glu Lys Leu Pro Arg Gly Val Leu Glu
   275 280 285
   Arg Phe Met Gly Asp Lys Val Ala Ser Glu Ala Leu Met Lys Tyr His
   290 295 300
   Ile Leu Asn Thr Leu Gln Cys Ser Glu Ser Ile Met Gly Gly Ala Val
   305 310 315 320
   Phe Glu Thr Leu Glu Gly Asn Thr Ile Glu Ile Gly Cys Asp Gly Asp
   325 330 335
   Ser Ile Thr Val Asn Gly Ile Lys Met Val Asn Lys Lys Asp Ile Val
   340 345 350
   Thr Asn Asn Gly Val Ile His Leu Ile Asp Gln Val Leu Ile Pro Asp
   355 360 365
   Ser Ala Lys Gln Val Ile Glu Leu Ala Gly Lys Gln Gln Thr Thr Phe
   370 375 380
   Thr Asp Leu Val Ala Gln Leu Gly Leu Ala Ser Ala Leu Arg Pro Asp
   385 390 395 400
   Gly Glu Tyr Thr Leu Leu Ala Pro Val Asn Asn Ala Phe Ser Asp Asp
   405 410 415
   Thr Leu Ser Met Val Gln Arg Leu Leu Lys Leu Ile Leu Gln Asn His
   420 425 430
   Ile Leu Lys Val Lys Val Gly Leu Asn Glu Leu Tyr Asn Gly Gln Ile
   435 440 445
   Leu Glu Thr Ile Gly Gly Lys Gln Leu Arg Val Phe Val Tyr Arg Thr
   450 455 460
   Ala Val Cys Ile Glu Asn Ser Cys Met Glu Lys Gly Ser Lys Gln Gly
   465 470 475 480
   Arg Asn Gly Ala Ile His Ile Phe Arg Glu Ile Ile Lys Pro Ala Glu
   485 490 495
   Lys Ser Leu His Glu Lys Leu Lys Gln Asp Lys Arg Phe Ser Thr Phe
   500 505 510
   Leu Ser Leu Leu Glu Ala Ala Asp Leu Lys Glu Leu Leu Thr Gln Pro
   515 520 525
   Gly Asp Trp Thr Leu Phe Val Pro Thr Asn Asp Ala Phe Lys Gly Met
   530 535 540
   Thr Ser Glu Glu Lys Glu Ile Leu Ile Arg Asp Lys Asn Ala Leu Gln
   545                 550                 555                 560
   Asn Ile Ile Leu Tyr His Leu Thr Pro Gly Val Phe Ile Gly Lys Gly
                   565                 570                 575
   Phe Glu Pro Gly Val Thr Asn Ile Leu Lys Thr Thr Gln Gly Ser Lys
               580                 585                 590
   Ile Phe Leu Lys Glu Val Asn Asp Thr Leu Leu Val Asn Glu Leu Lys
           595                 600                 605
   Ser Lys Glu Ser Asp Ile Met Thr Thr Asn Gly Val Ile His Val Val
       610                 615                 620
   Asp Lys Leu Leu Tyr Pro Ala Asp Thr Pro Val Gly Asn Asp Gln Leu
   625                 630                 635                 640
   Leu Glu Ile Leu Asn Lys Leu Ile Lys Tyr Ile Gln Ile Lys Phe Val
                   645                 650                 655
   Arg Gly Ser Thr Phe Lys Glu Ile Pro Val Thr Val Tyr Gly Pro Glu
               660                 665                 670
   Ile Lys Tyr Thr Arg Ile Ser Thr Gly Gly Gly Glu Thr Glu Glu Thr
           675                 680                 685
   Leu Lys Lys Leu Leu Gln Glu Glu Val Thr Lys Val Thr Lys Phe Ile
       690                 695                 700
   Glu Gly Gly Asp Gly His Leu Phe Glu Asp Glu Glu Ile Lys Arg Leu
   705                 710                 715                 720
   Leu Gln Gly Asp Thr Pro Val Arg Lys Leu Gln Ala Asn Lys Lys Val
                   725                 730                 735
   Gln Gly Ser Arg Arg Arg Leu Arg Glu Gly Arg Ser Gln
               740                 745
<210> 14
   <211> 2250
   <212> DNA
   <213> Homo sapiens
<400> 14
   atgattccct ttttacccat gttttctcta ctattgctgc ttattgttaa ccctataaac      60
   gccaacaatc attatgacaa gatcttggct catagtcgta tcaggggtcg ggaccaaggc     120
   ccaaatgtct gtgcccttca acagattttg ggcaccaaaa agaaatactt cagcacttgt     180
   aagaactggt ataaaaagtc catctgtgga cagaaaacga ctgttttata tgaatgttgc     240
   cctggttata tgagaatgga aggaatgaaa ggctgcccag cagttttgcc cattgaccat 300
   gtttatggca ctctgggcat cgtgggagcc accacaacgc agcgctattc tgacgcctca 360
   aaactgaggg aggagatcga gggaaaggga tccttcactt actttgcacc gagtaatgag 420
   gcttgggaca acttggattc tgatatccgt agaggtttgg agagcaacgt gaatgttgaa 480
   ttactgaatg ctttacatag tcacatgatt aataagagaa tgttgaccaa ggacttaaaa 540
   aatggcatga ttattccttc aatgtataac aatttggggc ttttcattaa ccattatcct 600
   aatggggttg tcactgttaa ttgtgctcga atcatccatg ggaaccagat tgcaacaaat 660
   ggtgttgtcc atgtcattga ccgtgtgctt acacaaattg gtacctcaat tcaagacttc 720
   attgaagcag aagatgacct ttcatctttt agagcagctg ccatcacatc ggacatattg 780
   gaggcccttg gaagagacgg tcacttcaca ctctttgctc ccaccaatga ggcttttgag 840
   aaacttccac gaggtgtcct agaaaggttc atgggagaca aagtggcttc cgaagctctt 900
   atgaagtacc acatcttaaa tactctccag tgttctgagt ctattatggg aggagcagtc 960
   tttgagacgc tggaaggaaa tacaattgag ataggatgtg acggtgacag tataacagta 1020
   aatggaatca aaatggtgaa caaaaaggat attgtgacaa ataatggtgt gatccatttg 1080
   attgatcagg tcctaattcc tgattctgcc aaacaagtta ttgagctggc tggaaaacag 1140
   caaaccacct tcacggatct tgtggcccaa ttaggcttgg catctgctct gaggccagat 1200
   ggagaataca ctttgctggc acctgtgaat aatgcatttt ctgatgatac tctcagcatg 1260
   gttcagcgcc tccttaaatt aattctgcag aatcacatat tgaaagtaaa agttggcctt 1320
   aatgagcttt acaacgggca aatactggaa accatcggag gcaaacagct cagagtcttc 1380
   gtatatcgta cagctgtctg cattgaaaat tcatgcatgg agaaagggag taagcaaggg 1440
   agaaacggtg cgattcacat attccgcgag atcatcaagc cagcagagaa atccctccat 1500
   gaaaagttaa aacaagataa gcgctttagc accttcctca gcctacttga agctgcagac 1560
   ttgaaagagc tcctgacaca acctggagac tggacattat ttgtgccaac caatgatgct 1620
   tttaagggaa tgactagtga agaaaaagaa attctgatac gggacaaaaa tgctcttcaa 1680
   aacatcattc tttatcacct gacaccagga gttttcattg gaaaaggatt tgaacctggt 1740
   gttactaaca ttttaaagac cacacaagga agcaaaatct ttctgaaaga agtaaatgat 1800
   acacttctgg tgaatgaatt gaaatcaaaa gaatctgaca tcatgacaac aaatggtgta 1860
   attcatgttg tagataaact cctctatcca gcagacacac ctgttggaaa tgatcaactg 1920
   ctggaaatac ttaataaatt aatcaaatac atccaaatta agtttgttcg tggtagcacc 1980
   ttcaaagaaa tccccgtgac tgtctatggt cctgaaataa aatacactag gatttctact 2040
   ggaggtggag aaacagaaga aactctgaag aaattgttac aagaagaggt caccaaggtc 2100
   accaaattca ttgaaggtgg tgatggtcat ttatttgaag atgaagaaat taaaagactg 2160
   cttcagggag acacacccgt gaggaagttg caagccaaca aaaaagttca aggttctaga 2220
   agacgattaa gggaaggtcg ttctcagtga 2250
<210> 15
   <211> 809
   <212> PRT
   <213> Homo sapiens
<400> 15
   Met Ile Pro Phe Leu Pro Met Phe Ser Leu Leu Leu Leu Leu Ile Val
   1               5                   10                  15
   Asn Pro Ile Asn Ala Asn Asn His Tyr Asp Lys Ile Leu Ala His Ser
               20                  25                  30
   Arg Ile Arg Gly Arg Asp Gln Gly Pro Asn Val Cys Ala Leu Gln Gln
           35                  40                  45
   Ile Leu Gly Thr Lys Lys Lys Tyr Phe Ser Thr Cys Lys Asn Trp Tyr
       50                  55                  60
   Lys Lys Ser Ile Cys Gly Gln Lys Thr Thr Val Leu Tyr Glu Cys Cys
   65                  70                  75                  80
   Pro Gly Tyr Met Arg Met Glu Gly Met Lys Gly Cys Pro Ala Val Leu
                   85                  90                  95
   Pro Ile Asp His Val Tyr Gly Thr Leu Gly Ile Val Gly Ala Thr Thr
               100                 105                 110
   Thr Gln Arg Tyr Ser Asp Ala Ser Lys Leu Arg Glu Glu Ile Glu Gly
           115                 120                 125
   Lys Gly Ser Phe Thr Tyr Phe Ala Pro Ser Asn Glu Ala Trp Asp Asn
       130                 135                 140
   Leu Asp Ser Asp Ile Arg Arg Gly Leu Glu Ser Asn Val Asn Val Glu
   145                 150                 155                 160
   Leu Leu Asn Ala Leu His Ser His Met Ile Asn Lys Arg Met Leu Thr
                   165                 170                 175
   Lys Asp Leu Lys Asn Gly Met Ile Ile Pro Ser Met Tyr Asn Asn Leu
               180                 185                 190
   Gly Leu Phe Ile Asn His Tyr Pro Asn Gly Val Val Thr Val Asn Cys
           195                 200                 205
   Ala Arg Ile Ile His Gly Asn Gln Ile Ala Thr Asn Gly Val Val His
       210                 215                 220
   Val Ile Asp Arg Val Leu Thr Gln Ile Gly Thr Ser Ile Gln Asp Phe
   225                 230                 235                 240
   Ile Glu Ala Glu Asp Asp Leu Ser Ser Phe Arg Ala Ala Ala Ile Thr
   245 250 255
   Ser Asp Ile Leu Glu Ala Leu Gly Arg Asp Gly His Phe Thr Leu Phe
   260 265 270
   Ala Pro Thr Asn Glu Ala Phe Glu Lys Leu Pro Arg Gly Val Leu Glu
   275 280 285
   Arg Phe Met Gly Asp Lys Val Ala Ser Glu Ala Leu Met Lys Tyr His
   290 295 300
   Ile Leu Asn Thr Leu Gln Cys Ser Glu Ser Ile Met Gly Gly Ala Val
   305 310 315 320
   Phe Glu Thr Leu Glu Gly Asn Thr Ile Glu Ile Gly Cys Asp Gly Asp
   325 330 335
   Ser Ile Thr Val Asn Gly Ile Lys Met Val Asn Lys Lys Asp Ile Val
   340 345 350
   Thr Asn Asn Gly Val Ile His Leu Ile Asp Gln Val Leu Ile Pro Asp
   355 360 365
   Ser Ala Lys Gln Val Ile Glu Leu Ala Gly Lys Gln Gln Thr Thr Phe
   370 375 380
   Thr Asp Leu Val Ala Gln Leu Gly Leu Ala Ser Ala Leu Arg Pro Asp
   385 390 395 400
   Gly Glu Tyr Thr Leu Leu Ala Pro Val Asn Asn Ala Phe Ser Asp Asp
   405 410 415
   Thr Leu Ser Met Val Gln Arg Leu Leu Lys Leu Ile Leu Gln Asn His
   420 425 430
   Ile Leu Lys Val Lys Val Gly Leu Asn Glu Leu Tyr Asn Gly Gln Ile
   435 440 445
   Leu Glu Thr Ile Gly Gly Lys Gln Leu Arg Val Phe Val Tyr Arg Thr
   450 455 460
   Ala Val Cys Ile Glu Asn Ser Cys Met Glu Lys Gly Ser Lys Gln Gly
   465 470 475 480
   Arg Asn Gly Ala Ile His Ile Phe Arg Glu Ile Ile Lys Pro Ala Glu
   485 490 495
   Lys Ser Leu His Glu Lys Leu Lys Gln Asp Lys Arg Phe Ser Thr Phe
   500 505 510
   Leu Ser Leu Leu Glu Ala Ala Asp Leu Lys Glu Leu Leu Thr Gln Pro
   515 520 525
   Gly Asp Trp Thr Leu Phe Val Pro Thr Asn Asp Ala Phe Lys Gly Met
   530 535 540
   Thr Ser Glu Glu Lys Glu Ile Leu Ile Arg Asp Lys Asn Ala Leu Gln
   545 550 555 560
   Asn Ile Ile Leu Tyr His Leu Thr Pro Gly Val Phe Ile Gly Lys Gly
   565 570 575
   Phe Glu Pro Gly Val Thr Asn Ile Leu Lys Thr Thr Gln Gly Ser Lys
   580 585 590
   Ile Phe Leu Lys Glu Val Asn Asp Thr Leu Leu Val Asn Glu Leu Lys
   595 600 605
   Ser Lys Glu Ser Asp Ile Met Thr Thr Asn Gly Val Ile His Val Val
   610 615 620
   Asp Lys Leu Leu Tyr Pro Ala Asp Thr Pro Val Gly Asn Asp Gln Leu
   625 630 635 640
   Leu Glu Ile Leu Asn Lys Leu Ile Lys Tyr Ile Gln Ile Lys Phe Val
   645 650 655
   Arg Gly Ser Thr Phe Lys Glu Ile Pro Val Thr Val Tyr Arg Pro Thr
   660 665 670
   Leu Thr Lys Val Lys Ile Glu Gly Glu Pro Glu Phe Arg Leu Ile Lys
   675 680 685
   Glu Gly Glu Thr Ile Thr Glu Val Ile His Gly Glu Pro Ile Ile Lys
   690 695 700
   Lys Tyr Thr Lys Ile Ile Asp Gly Val Pro Val Glu Ile Thr Glu Lys
   705 710 715 720
   Glu Thr Arg Glu Glu Arg Ile Ile Thr Gly Pro Glu Ile Lys Tyr Thr
   725 730 735
   Arg Ile Ser Thr Gly Gly Gly Glu Thr Glu Glu Thr Leu Lys Lys Leu
   740 745 750
   Leu Gln Glu Glu Val Thr Lys Val Thr Lys Phe Ile Glu Gly Gly Asp
   755 760 765
   Gly His Leu Phe Glu Asp Glu Glu Ile Lys Arg Leu Leu Gln Gly Asp
   770 775 780
   Thr Pro Val Arg Lys Leu Gln Ala Asn Lys Lys Val Gln Gly Ser Arg
   785 790 795 800
   Arg Arg Leu Arg Glu Gly Arg Ser Gln
   805
<210> 16
   <211> 2430
   <212> DNA
   <213> Homo sapiens
<400> 16
   atgattccct ttttacccat gttttctcta ctattgctgc ttattgttaa ccctataaac     60
   gccaacaatc attatgacaa gatcttggct catagtcgta tcaggggtcg ggaccaaggc    120
   ccaaatgtct gtgcccttca acagattttg ggcaccaaaa agaaatactt cagcacttgt    180
   aagaactggt ataaaaagtc catctgtgga cagaaaacga ctgttttata tgaatgttgc    240
   cctggttata tgagaatgga aggaatgaaa ggctgcccag cagttttgcc cattgaccat    300
   gtttatggca ctctgggcat cgtgggagcc accacaacgc agcgctattc tgacgcctca    360
   aaactgaggg aggagatcga gggaaaggga tccttcactt actttgcacc gagtaatgag    420
   gcttgggaca acttggattc tgatatccgt agaggtttgg agagcaacgt gaatgttgaa    480
   ttactgaatg ctttacatag tcacatgatt aataagagaa tgttgaccaa ggacttaaaa    540
   aatggcatga ttattccttc aatgtataac aatttggggc ttttcattaa ccattatcct    600
   aatggggttg tcactgttaa ttgtgctcga atcatccatg ggaaccagat tgcaacaaat    660
   ggtgttgtcc atgtcattga ccgtgtgctt acacaaattg gtacctcaat tcaagacttc    720
   attgaagcag aagatgacct ttcatctttt agagcagctg ccatcacatc ggacatattg    780
   gaggcccttg gaagagacgg tcacttcaca ctctttgctc ccaccaatga ggcttttgag    840
   aaacttccac gaggtgtcct agaaaggttc atgggagaca aagtggcttc cgaagctctt    900
   atgaagtacc acatcttaaa tactctccag tgttctgagt ctattatggg aggagcagtc    960
   tttgagacgc tggaaggaaa tacaattgag ataggatgtg acggtgacag tataacagta   1020
   aatggaatca aaatggtgaa caaaaaggat attgtgacaa ataatggtgt gatccatttg   1080
   attgatcagg tcctaattcc tgattctgcc aaacaagtta ttgagctggc tggaaaacag   1140
   caaaccacct tcacggatct tgtggcccaa ttaggcttgg catctgctct gaggccagat   1200
   ggagaataca ctttgctggc acctgtgaat aatgcatttt ctgatgatac tctcagcatg   1260
   gttcagcgcc tccttaaatt aattctgcag aatcacatat tgaaagtaaa agttggcctt   1320
   aatgagcttt acaacgggca aatactggaa accatcggag gcaaacagct cagagtcttc   1380
   gtatatcgta cagctgtctg cattgaaaat tcatgcatgg agaaagggag taagcaaggg   1440
   agaaacggtg cgattcacat attccgcgag atcatcaagc cagcagagaa atccctccat   1500
   gaaaagttaa aacaagataa gcgctttagc accttcctca gcctacttga agctgcagac   1560
   ttgaaagagc tcctgacaca acctggagac tggacattat ttgtgccaac caatgatgct   1620
   tttaagggaa tgactagtga agaaaaagaa attctgatac gggacaaaaa tgctcttcaa 1680
   aacatcattc tttatcacct gacaccagga gttttcattg gaaaaggatt tgaacctggt 1740
   gttactaaca ttttaaagac cacacaagga agcaaaatct ttctgaaaga agtaaatgat 1800
   acacttctgg tgaatgaatt gaaatcaaaa gaatctgaca tcatgacaac aaatggtgta 1860
   attcatgttg tagataaact cctctatcca gcagacacac ctgttggaaa tgatcaactg 1920
   ctggaaatac ttaataaatt aatcaaatac atccaaatta agtttgttcg tggtagcacc 1980
   ttcaaagaaa tccccgtgac tgtctataga cccacactaa caaaagtcaa aattgaaggt 2040
   gaacctgaat tcagactgat taaagaaggt gaaacaataa ctgaagtgat ccatggagag 2100
   ccaattatta aaaaatacac caaaatcatt gatggagtgc ctgtggaaat aactgaaaaa 2160
   gagacacgag aagaacgaat cattacaggt cctgaaataa aatacactag gatttctact 2220
   ggaggtggag aaacagaaga aactctgaag aaattgttac aagaagaggt caccaaggtc 2280
   accaaattca ttgaaggtgg tgatggtcat ttatttgaag atgaagaaat taaaagactg 2340
   cttcagggag acacacccgt gaggaagttg caagccaaca aaaaagttca aggttctaga 2400
   agacgattaa gggaaggtcg ttctcagtga 2430
<210> 17
   <211> 346
   <212> PRT
   <213> Homo sapiens
<400> 17
   Met Asp Thr Gly Pro Asp Gln Ser Tyr Phe Ser Gly Asn His Trp Phe
   1               5                   10                  15
   Val Phe Ser Val Tyr Leu Leu Thr Phe Leu Val Gly Leu Pro Leu Asn
               20                  25                  30
   Leu Leu Ala Leu Val Val Phe Val Gly Lys Leu Arg Cys Arg Pro Val
           35                  40                  45
   Ala Val Asp Val Leu Leu Leu Asn Leu Thr Ala Ser Asp Leu Leu Leu
       50                  55                  60
   Leu Leu Phe Leu Pro Phe Arg Met Val Glu Ala Ala Asn Gly Met His
   65                  70                  75                  80
   Trp Pro Leu Pro Phe Ile Leu Cys Pro Leu Ser Gly Phe Ile Phe Phe
                   85                  90                  95
   Thr Thr Ile Tyr Leu Thr Ala Leu Phe Leu Ala Ala Val Ser Ile Glu
               100                 105                 110
   Arg Phe Leu Ser Val Ala His Pro Leu Trp Tyr Lys Thr Arg Pro Arg
           115                 120                 125
   Leu Gly Gln Ala Gly Leu Val Ser Val Ala Cys Trp Leu Leu Ala Ser
   130 135 140
   Ala His Cys Ser Val Val Tyr Val Ile Glu Phe Ser Gly Asp Ile Ser
   145 150 155 160
   His Ser Gln Gly Thr Asn Gly Thr Cys Tyr Leu Glu Phe Trp Lys Asp
   165 170 175
   Gln Leu Ala Ile Leu Leu Pro Val Arg Leu Glu Met Ala Val Val Leu
   180 185 190
   Phe Val Val Pro Leu Ile Ile Thr Ser Tyr Cys Tyr Ser Arg Leu Val
   195 200 205
   Trp Ile Leu Gly Arg Gly Gly Ser His Arg Arg Gln Arg Arg Val Ala
   210 215 220
   Gly Leu Val Ala Ala Thr Leu Leu Asn Phe Leu Val Cys Phe Gly Pro
   225 230 235 240
   Tyr Asn Val Ser His Val Val Gly Tyr Ile Cys Gly Glu Ser Pro Val
   245 250 255
   Trp Arg Ile Tyr Val Thr Leu Leu Ser Thr Leu Asn Ser Cys Val Asp
   260 265 270
   Pro Phe Val Tyr Tyr Phe Ser Ser Ser Gly Phe Gln Ala Asp Phe His
   275 280 285
   Glu Leu Leu Arg Arg Leu Cys Gly Leu Trp Gly Gln Trp Gln Gln Glu
   290 295 300
   Ser Ser Met Glu Leu Lys Glu Gln Lys Gly Gly Glu Glu Gln Arg Ala
   305 310 315 320
   Asp Arg Pro Ala Glu Arg Lys Thr Ser Glu His Ser Gln Gly Cys Gly
   325 330 335
   Thr Gly Gly Gln Val Ala Cys Ala Glu Asn
   340 345
<210> 18
   <211> 1041
   <212> DNA
   <213> Homo sapiens
<400> 18
   atggatacag gccccgacca gtcctacttc tccggcaatc actggttcgt cttctcggtg 60
   taccttctca ctttcctggt ggggctcccc ctcaacctgc tggccctggt ggtcttcgtg 120
   ggcaagctgc ggtgccgccc ggtggccgtg gacgtgctcc tgctcaacct gaccgcctcg 180
   gacctgctcc tgctgctgtt cctgcctttc cgcatggtgg aggcagccaa tggcatgcac 240
   tggcccctgc ccttcatcct ctgcccactc tctggattca tcttcttcac caccatctat 300
   ctcaccgccc tcttcctggc agctgtgagc attgaacgct tcctgagtgt ggcccaccca 360
   ctgtggtaca agacccggcc gaggctgggg caggcaggtc tggtgagtgt ggcctgctgg 420
   ctgttggcct ctgctcactg cagcgtggtc tacgtcatag aattctcagg ggacatctcc 480
   cacagccagg gcaccaatgg gacctgctac ctggagttct ggaaggacca gctagccatc 540
   ctcctgcccg tgcggctgga gatggctgtg gtcctctttg tggtcccgct gatcatcacc 600
   agctactgct acagccgcct ggtgtggatc ctcggcagag ggggcagcca ccgccggcag 660
   aggagggtgg cggggctggt ggcggccacg ctgctcaact tccttgtctg ctttgggccc 720
   tacaacgtgt cccatgtcgt gggctatatc tgcggtgaaa gcccggtgtg gaggatctac 780
   gtgacgcttc tcagcaccct gaactcctgt gtcgacccct ttgtctacta cttctcctcc 840
   tccgggttcc aagccgactt tcatgagctg ctgaggaggt tgtgtgggct ctggggccag 900
   tggcagcagg agagcagcat ggagctgaag gagcagaagg gaggggagga gcagagagcg 960
   gaccgaccag ctgaaagaaa gaccagtgaa cactcacagg gctgtggaac tggtggccag 1020
   gtggcctgtg ctgaaaacta g 1041
<210> 19
   <211> 346
   <212> PRT
   <213> Homo sapiens
<400> 19
   Met Asp Thr Gly Pro Asp Gln Ser Tyr Phe Ser Gly Asn His Trp Phe
   1               5                   10                  15
   Val Phe Ser Val Tyr Leu Leu Thr Phe Leu Val Gly Leu Pro Leu Asn
               20                  25                  30
   Leu Leu Ala Leu Val Val Phe Val Gly Lys Leu Gln Arg Arg Pro Val
           35                  40                  45
   Ala Val Asp Val Leu Leu Leu Asn Leu Thr Ala Ser Asp Leu Leu Leu
       50                  55                  60
   Leu Leu Phe Leu Pro Phe Arg Met Val Glu Ala Ala Asn Gly Met His
   65                  70                  75                  80
   Trp Pro Leu Pro Phe Ile Leu Cys Pro Leu Ser Gly Phe Ile Phe Phe
                   85                  90                  95
   Thr Thr Ile Tyr Leu Thr Ala Leu Phe Leu Ala Ala Val Ser Ile Glu
               100                 105                 110
   Arg Phe Leu Ser Val Ala His Pro Leu Trp Tyr Lys Thr Arg Pro Arg
   115 120 125
   Leu Gly Gln Ala Gly Leu Val Ser Val Ala Cys Trp Leu Leu Ala Ser
   130 135 140
   Ala His Cys Ser Val Val Tyr Val Ile Glu Phe Ser Gly Asp Ile Ser
   145 150 155 160
   His Ser Gln Gly Thr Asn Gly Thr Cys Tyr Leu Glu Phe Arg Lys Asp
   165 170 175
   Gln Leu Ala Ile Leu Leu Pro Val Arg Leu Glu Met Ala Val Val Leu
   180 185 190
   Phe Val Val Pro Leu Ile Ile Thr Ser Tyr Cys Tyr Ser Arg Leu Val
   195 200 205
   Trp Ile Leu Gly Arg Gly Gly Ser His Arg Arg Gln Arg Arg Val Ala
   210 215 220
   Gly Leu Leu Ala Ala Thr Leu Leu Asn Phe Leu Val Cys Phe Gly Pro
   225 230 235 240
   Tyr Asn Val Ser His Val Val Gly Tyr Ile Cys Gly Glu Ser Pro Ala
   245 250 255
   Trp Arg Ile Tyr Val Thr Leu Leu Ser Thr Leu Asn Ser Cys Val Asp
   260 265 270
   Pro Phe Val Tyr Tyr Phe Ser Ser Ser Gly Phe Gln Ala Asp Phe His
   275 280 285
   Glu Leu Leu Arg Arg Leu Cys Gly Leu Trp Gly Gln Trp Gln Gln Glu
   290 295 300
   Ser Ser Met Glu Leu Lys Glu Gln Lys Gly Gly Glu Glu Gln Arg Ala
   305 310 315 320
   Asp Arg Pro Ala Glu Arg Lys Thr Ser Glu His Ser Gln Gly Cys Gly
   325 330 335
   Thr Gly Gly Gln Val Ala Cys Ala Glu Ser
   340 345
<210> 20
   <211> 1041
   <212> DNA
   <213> Homo sapiens
<400> 20
   atggatacag gccccgacca gtcctacttc tccggcaatc actggttcgt cttctcggtg 60
   taccttctca ctttcctggt ggggctcccc ctcaacctgc tggccctggt ggtcttcgtg 120
   ggcaagctgc agcgccgccc ggtggccgtg gacgtgctcc tgctcaacct gaccgcctcg 180
   gacctgctcc tgctgctgtt cctgcctttc cgcatggtgg aggcagccaa tggcatgcac 240
   tggcccctgc ccttcatcct ctgcccactc tctggattca tcttcttcac caccatctat 300
   ctcaccgccc tcttcctggc agctgtgagc attgaacgct tcctgagtgt ggcccaccca 360
   ctgtggtaca agacccggcc gaggctgggg caggcaggtc tggtgagtgt ggcctgctgg 420
   ctgttggcct ctgctcactg cagcgtggtc tacgtcatag aattctcagg ggacatctcc 480
   cacagccagg gcaccaatgg gacctgctac ctggagttcc ggaaggacca gctagccatc 540
   ctcctgcccg tgcggctgga gatggctgtg gtcctctttg tggtcccgct gatcatcacc 600
   agctactgct acagccgcct ggtgtggatc ctcggcagag ggggcagcca ccgccggcag 660
   aggagggtgg cggggctgtt ggcggccacg ctgctcaact tccttgtctg ctttgggccc 720
   tacaacgtgt cccatgtcgt gggctatatc tgcggtgaaa gcccggcatg gaggatctac 780
   gtgacgcttc tcagcaccct gaactcctgt gtcgacccct ttgtctacta cttctcctcc 840
   tccgggttcc aagccgactt tcatgagctg ctgaggaggt tgtgtgggct ctggggccag 900
   tggcagcagg agagcagcat ggagctgaag gagcagaagg gaggggagga gcagagagcg 960
   gaccgaccag ctgaaagaaa gaccagtgaa cactcacagg gctgtggaac tggtggccag 1020
   gtggcctgtg ctgaaagcta g 1041
<210> 21
   <211> 492
   <212> PRT
   <213> Homo sapiens
<400> 21
   Met Glu Pro Ser Ser Lys Lys Leu Thr Gly Arg Leu Met Leu Ala Val
   1 5 10 15
   Gly Gly Ala Val Leu Gly Ser Leu Gln Phe Gly Tyr Asn Thr Gly Val
   20 25 30
   Ile Asn Ala Pro Gln Lys Val Ile Glu Glu Phe Tyr Asn Gln Thr Trp
   35 40 45
   Val His Arg Tyr Gly Glu Ser Ile Leu Pro Thr Thr Leu Thr Thr Leu
   50 55 60
   Trp Ser Leu Ser Val Ala Ile Phe Ser Val Gly Gly Met Ile Gly Ser
   65 70 75 80
   Phe Ser Val Gly Leu Phe Val Asn Arg Phe Gly Arg Arg Asn Ser Met
   85 90 95
   Leu Met Met Asn Leu Leu Ala Phe Val Ser Ala Val Leu Met Gly Phe
   100 105 110
   Ser Lys Leu Gly Lys Ser Phe Glu Met Leu Ile Leu Gly Arg Phe Ile
           115                 120                 125
   Ile Gly Val Tyr Cys Gly Leu Thr Thr Gly Phe Val Pro Met Tyr Val
       130                 135                 140
   Gly Glu Val Ser Pro Thr Ala Phe Arg Gly Ala Leu Gly Thr Leu His
   145                 150                 155                 160
   Gln Leu Gly Ile Val Val Gly Ile Leu Ile Ala Gln Val Phe Gly Leu
                   165                 170                 175
   Asp Ser Ile Met Gly Asn Lys Asp Leu Trp Pro Leu Leu Leu Ser Ile
               180                 185                 190
   Ile Phe Ile Pro Ala Leu Leu Gln Cys Ile Val Leu Pro Phe Cys Pro
           195                 200                 205
   Glu Ser Pro Arg Phe Leu Leu Ile Asn Arg Asn Glu Glu Asn Arg Ala
       210                 215                 220
   Lys Ser Val Leu Lys Lys Leu Arg Gly Thr Ala Asp Val Thr His Asp
   225                 230                 235                 240
   Leu Gln Glu Met Lys Glu Glu Ser Arg Gln Met Met Arg Glu Lys Lys
                   245                 250                 255
   Val Thr Ile Leu Glu Leu Phe Arg Ser Pro Ala Tyr Arg Gln Pro Ile
               260                 265                 270
   Leu Ile Ala Val Val Leu Gln Leu Ser Gln Gln Leu Ser Gly Ile Asn
           275                 280                 285
   Ala Val Phe Tyr Tyr Ser Thr Ser Ile Phe Glu Lys Ala Gly Val Gln
       290                 295                 300
   Gln Pro Val Tyr Ala Thr Ile Gly Ser Gly Ile Val Asn Thr Ala Phe
   305                 310                 315                 320
   Thr Val Val Ser Leu Phe Val Val Glu Arg Ala Gly Arg Arg Thr Leu
                   325                 330                 335
   His Leu Ile Gly Leu Ala Gly Met Ala Gly Cys Ala Ile Leu Met Thr
               340                 345                 350
   Ile Ala Leu Ala Leu Leu Glu Gln Leu Pro Trp Met Ser Tyr Leu Ser
           355                 360                 365
   Ile Val Ala Ile Phe Gly Phe Val Ala Phe Phe Glu Val Gly Pro Gly
       370                 375                 380
   Pro Ile Pro Trp Phe Ile Val Ala Glu Leu Phe Ser Gln Gly Pro Arg
   385                 390                 395                 400
   Pro Ala Ala Ile Ala Val Ala Gly Phe Ser Asn Trp Thr Ser Asn Phe
                   405                 410                 415
   Ile Val Gly Met Cys Phe Gln Tyr Val Glu Gln Leu Cys Gly Pro Tyr
               420                 425                 430
   Val Phe Ile Ile Phe Thr Val Leu Leu Val Leu Phe Phe Ile Phe Thr
           435                 440                 445
   Tyr Phe Lys Val Pro Glu Thr Lys Gly Arg Thr Phe Asp Glu Ile Ala
       450                 455                 460
   Ser Gly Phe Arg Gln Gly Gly Ala Ser Gln Ser Asp Lys Thr Pro Glu
   465                 470                 475                 480
   Glu Leu Phe His Pro Leu Gly Ala Asp Ser Gln Val
                   485                 490
<210> 22
   <211> 1479
   <212> DNA
   <213> Homo sapiens
<400> 22
   atggagccca gcagcaagaa gctgacgggt cgcctcatgc tggctgtggg aggagcagtg     60
   cttggctccc tgcagtttgg ctacaacact ggagtcatca atgcccccca gaaggtgatc    120
   gaggagttct acaaccagac atgggtccac cgctatgggg agagcatcct gcccaccacg    180
   ctcaccacgc tctggtccct ctcagtggcc atcttttctg ttgggggcat gattggctcc    240
   ttctctgtgg gccttttcgt taaccgcttt ggccggcgga attcaatgct gatgatgaac    300
   ctgctggcct tcgtgtccgc cgtgctcatg ggcttctcga aactgggcaa gtcctttgag    360
   atgctgatcc tgggccgctt catcatcggt gtgtactgcg gcctgaccac aggcttcgtg    420
   cccatgtatg tgggtgaagt gtcacccaca gcctttcgtg ggccctgggg caccctgcac    480
   cagctgggca tcgtcgtcgg catcctcatc gcccaggtgt tcggcctgga ctccatcatg    540
   ggcaacaagg acctgtggcc cctgctgctg agcatcatct tcatcccggc cctgctgcag    600
   tgcatcgtgc tgcccttctg ccccgagagt ccccgcttcc tgctcatcaa ccgcaacgag    660
   gagaaccggg ccaagagtgt gctaaagaag ctgcgcggga cagctgacgt gacccatgac    720
   ctgcaggaga tgaaggaaga gagtcggcag atgatgcggg agaagaaggt caccatcctg    780
   gagctgttcc gctcccccgc ctaccgccag cccatcctca tcgctgtggt gctgcagctg    840
   tcccagcagc tgtctggcat caacgctgtc ttctattact ccacgagcat cttcgagaag    900
   gcgggggtgc agcagcctgt gtatgccacc attggctccg gtatcgtcaa cacggccttc    960
   actgtcgtgt cgctgtttgt ggtggagcga gcaggccggc ggaccctgca cctcataggc 1020
   ctcgctggca tggcgggttg tgccatactc atgaccatcg cgctagcact gctggagcag 1080
   ctaccctgga tgtcctatct gagcatcgtg gccatctttg gctttgtggc cttctttgaa 1140
   gtgggtcctg gccccatccc atggttcatc gtggctgaac tcttcagcca gggtccacgt 1200
   ccagctgcca ttgccgttgc aggcttctcc aactggacct caaatttcat tgtgggcatg 1260
   tgcttccagt atgtggagca actgtgtggt ccctacgtct tcatcatctt cactgtgctc 1320
   ctggttctgt tcttcatctt cacctacttc aaagttcctg agactaaagg ccggaccttc 1380
   gatgagatcg cttccggctt ccggcagggg ggagccagcc aaagtgataa gacacccgag 1440
   gagctgttcc atcccctggg ggctgattcc caagtgtga 1479
<210> 23
   <211> 3396
   <212> PRT
   <213> Homo sapiens
<400> 23
   Met Phe Ile Asn Ile Lys Ser Ile Leu Trp Met Cys Ser Thr Leu Ile
   1 5 10 15
   Val Thr His Ala Leu His Lys Val Lys Val Gly Lys Ser Pro Pro Val
   20 25 30
   Arg Gly Ser Leu Ser Gly Lys Val Ser Leu Pro Cys His Phe Ser Thr
   35 40 45
   Met Pro Thr Leu Pro Pro Ser Tyr Asn Thr Ser Glu Phe Leu Arg Ile
   50 55 60
   Lys Trp Ser Lys Ile Glu Val Asp Lys Asn Gly Lys Asp Leu Lys Glu
   65 70 75 80
   Thr Thr Val Leu Val Ala Gln Asn Gly Asn Ile Lys Ile Gly Gln Asp
   85 90 95
   Tyr Lys Gly Arg Val Ser Val Pro Thr His Pro Glu Ala Val Gly Asp
   100 105 110
   Ala Ser Leu Thr Val Val Lys Leu Leu Ala Ser Asp Ala Gly Leu Tyr
   115 120 125
   Arg Cys Asp Val Met Tyr Gly Ile Glu Asp Thr Gln Asp Thr Val Ser
   130 135 140
   Leu Thr Val Asp Gly Val Val Phe His Tyr Arg Ala Ala Thr Ser Arg
   145 150 155 160
   Tyr Thr Leu Asn Phe Glu Ala Ala Gln Lys Ala Cys Leu Asp Val Gly
   165 170 175
   Ala Val Ile Ala Thr Pro Glu Gln Leu Phe Ala Ala Tyr Glu Asp Gly
   180 185 190
   Phe Glu Gln Cys Asp Ala Gly Trp Leu Ala Asp Gln Thr Val Arg Tyr
   195 200 205
   Pro Ile Arg Ala Pro Arg Val Gly Cys Tyr Gly Asp Lys Met Gly Lys
   210 215 220
   Ala Gly Val Arg Thr Tyr Gly Phe Arg Ser Pro Gln Glu Thr Tyr Asp
   225 230 235 240
   Val Tyr Cys Tyr Val Asp His Leu Asp Gly Asp Val Phe His Leu Thr
   245 250 255
   Val Pro Ser Lys Phe Thr Phe Glu Glu Ala Ala Lys Glu Cys Glu Asn
   260 265 270
   Gln Asp Ala Arg Leu Ala Thr Val Gly Glu Leu Gln Ala Ala Trp Arg
   275 280 285
   Asn Gly Phe Asp Gln Cys Asp Tyr Gly Trp Leu Ser Asp Ala Ser Val
   290 295 300
   Arg His Pro Val Thr Val Ala Arg Ala Gln Cys Gly Gly Gly Leu Leu
   305 310 315 320
   Gly Val Arg Thr Leu Tyr Arg Phe Glu Asn Gln Thr Gly Phe Pro Pro
   325 330 335
   Pro Asp Ser Arg Phe Asp Ala Tyr Cys Phe Lys Pro Lys Glu Ala Thr
   340 345 350
   Thr Ile Asp Leu Ser Ile Leu Ala Glu Thr Ala Ser Pro Ser Leu Ser
   355 360 365
   Lys Glu Pro Gln Met Val Ser Asp Arg Thr Thr Pro Ile Ile Pro Leu
   370 375 380
   Val Asp Glu Leu Pro Val Ile Pro Thr Glu Phe Pro Pro Val Gly Asn
   385 390 395 400
   Ile Val Ser Phe Glu Gln Lys Ala Thr Val Gln Pro Gln Ala Ile Thr
   405 410 415
   Asp Ser Leu Ala Thr Lys Leu Pro Thr Pro Thr Gly Ser Thr Lys Lys
   420 425 430
   Pro Trp Asp Met Asp Asp Tyr Ser Pro Ser Ala Ser Gly Pro Leu Gly
   435 440 445
   Lys Leu Asp Ile Ser Glu Ile Lys Glu Glu Val Leu Gln Ser Thr Thr
   450 455 460
   Gly Val Ser His Tyr Ala Thr Asp Ser Trp Asp Gly Val Val Glu Asp
   465 470 475 480
   Lys Gln Thr Gln Glu Ser Val Thr Gln Ile Glu Gln Ile Glu Val Gly
   485 490 495
   Pro Leu Val Thr Ser Met Glu Ile Leu Lys His Ile Pro Ser Lys Glu
   500 505 510
   Phe Pro Val Thr Glu Thr Pro Leu Val Thr Ala Arg Met Ile Leu Glu
   515 520 525
   Ser Lys Thr Glu Lys Lys Met Val Ser Thr Val Ser Glu Leu Val Thr
   530 535 540
   Thr Gly His Tyr Gly Phe Thr Leu Gly Glu Glu Asp Asp Glu Asp Arg
   545 550 555 560
   Thr Leu Thr Val Gly Ser Asp Glu Ser Thr Leu Ile Phe Asp Gln Ile
   565 570 575
   Pro Glu Val Ile Thr Val Ser Lys Thr Ser Glu Asp Thr Ile His Thr
   580 585 590
   His Leu Glu Asp Leu Glu Ser Val Ser Ala Ser Thr Thr Val Ser Pro
   595 600 605
   Leu Ile Met Pro Asp Asn Asn Gly Ser Ser Met Asp Asp Trp Glu Glu
   610 615 620
   Arg Gln Thr Ser Gly Arg Ile Thr Glu Glu Phe Leu Gly Lys Tyr Leu
   625 630 635 640
   Ser Thr Thr Pro Phe Pro Ser Gln His Arg Thr Glu Ile Glu Leu Phe
   645 650 655
   Pro Tyr Ser Gly Asp Lys Ile Leu Val Glu Gly Ile Ser Thr Val Ile
   660 665 670
   Tyr Pro Ser Leu Gln Thr Glu Met Thr His Arg Arg Glu Arg Thr Glu
   675 680 685
   Thr Leu Ile Pro Glu Met Arg Thr Asp Thr Tyr Thr Asp Glu Ile Gln
   690 695 700
   Glu Glu Ile Thr Lys Ser Pro Phe Met Gly Lys Thr Glu Glu Glu Val
   705 710 715 720
   Phe Ser Gly Met Lys Leu Ser Thr Ser Leu Ser Glu Pro Ile His Val
   725 730 735
   Thr Glu Ser Ser Val Glu Met Thr Lys Ser Phe Asp Phe Pro Thr Leu
   740 745 750
   Ile Thr Lys Leu Ser Ala Glu Pro Thr Glu Val Arg Asp Met Glu Glu
   755 760 765
   Asp Phe Thr Ala Thr Pro Gly Thr Thr Lys Tyr Asp Glu Asn Ile Thr
   770 775 780
   Thr Val Leu Leu Ala His Gly Thr Leu Ser Val Glu Ala Ala Thr Val
   785 790 795 800
   Ser Lys Trp Ser Trp Asp Glu Asp Asn Thr Thr Ser Lys Pro Leu Glu
   805 810 815
   Ser Thr Glu Pro Ser Ala Ser Ser Lys Leu Pro Pro Ala Leu Leu Thr
   820 825 830
   Thr Val Gly Met Asn Gly Lys Asp Lys Asp Ile Pro Ser Phe Thr Glu
   835 840 845
   Asp Gly Ala Asp Glu Phe Thr Leu Ile Pro Asp Ser Thr Gln Lys Gln
   850 855 860
   Leu Glu Glu Val Thr Asp Glu Asp Ile Ala Ala His Gly Lys Phe Thr
   865 870 875 880
   Ile Arg Phe Gln Pro Thr Thr Ser Thr Gly Ile Ala Glu Lys Ser Thr
   885 890 895
   Leu Arg Asp Ser Thr Thr Glu Glu Lys Val Pro Pro Ile Thr Ser Thr
   900 905 910
   Glu Gly Gln Val Tyr Ala Thr Met Glu Gly Ser Ala Leu Gly Glu Val
   915 920 925
   Glu Asp Val Asp Leu Ser Lys Pro Val Ser Thr Val Pro Gln Phe Ala
   930 935 940
   His Thr Ser Glu Val Glu Gly Leu Ala Phe Val Ser Tyr Ser Ser Thr
   945 950 955 960
   Gln Glu Pro Thr Thr Tyr Val Asp Ser Ser His Thr Ile Pro Leu Ser
   965 970 975
   Val Ile Pro Lys Thr Asp Trp Gly Val Leu Val Pro Ser Val Pro Ser
   980 985 990
   Glu Asp Glu Val Leu Gly Glu Pro Ser Gln Asp Ile Leu Val Ile Asp
   995 1000 1005
   Gln Thr Arg Leu Glu Ala Thr Ile Ser Pro Glu Thr Met Arg Thr
   1010 1015 1020
   Thr Lys Ile Thr Glu Gly Thr Thr Gln Glu Glu Phe Pro Trp Lys
   1025 1030 1035
   Glu Gln Thr Ala Glu Lys Pro Val Pro Ala Leu Ser Ser Thr Ala
   1040 1045 1050
   Trp Thr Pro Lys Glu Ala Val Thr Pro Leu Asp Glu Gln Glu Gly
   1055 1060 1065
   Asp Gly Ser Ala Tyr Thr Val Ser Glu Asp Glu Leu Leu Thr Gly
   1070 1075 1080
   Ser Glu Arg Val Pro Val Leu Glu Thr Thr Pro Val Gly Lys Ile
   1085 1090 1095
   Asp His Ser Val Ser Tyr Pro Pro Gly Ala Val Thr Glu His Lys
   1100 1105 1110
   Val Lys Thr Asp Glu Val Val Thr Leu Thr Pro Arg Ile Gly Pro
   1115 1120 1125
   Lys Val Ser Leu Ser Pro Gly Pro Glu Gln Lys Tyr Glu Thr Glu
   1130 1135 1140
   Gly Ser Ser Thr Thr Gly Phe Thr Ser Ser Leu Ser Pro Phe Ser
   1145 1150 1155
   Thr His Ile Thr Gln Leu Met Glu Glu Thr Thr Thr Glu Lys Thr
   1160 1165 1170
   Ser Leu Glu Asp Ile Asp Leu Gly Ser Gly Leu Phe Glu Lys Pro
   1175 1180 1185
   Lys Ala Thr Glu Leu Ile Glu Phe Ser Thr Ile Lys Val Thr Val
   1190 1195 1200
   Pro Ser Asp Ile Thr Thr Ala Phe Ser Ser Val Asp Arg Leu His
   1205 1210 1215
   Thr Thr Ser Ala Phe Lys Pro Ser Ser Ala Ile Thr Lys Lys Pro
   1220 1225 1230
   Pro Leu Ile Asp Arg Glu Pro Gly Glu Glu Thr Thr Ser Asp Met
   1235 1240 1245
   Val Ile Ile Gly Glu Ser Thr Ser His Val Pro Pro Thr Thr Leu
   1250 1255 1260
   Glu Asp Ile Val Ala Lys Glu Thr Glu Thr Asp Ile Asp Arg Glu
   1265 1270 1275
   Tyr Phe Thr Thr Ser Ser Pro Pro Ala Thr Gln Pro Thr Arg Pro
   1280 1285 1290
   Pro Thr Val Glu Asp Lys Glu Ala Phe Gly Pro Gln Ala Leu Ser
   1295 1300 1305
   Thr Pro Gln Pro Pro Ala Ser Thr Lys Phe His Pro Asp Ile Asn
   1310 1315 1320
   Val Tyr Ile Ile Glu Val Arg Glu Asn Lys Thr Gly Arg Met Ser
   1325 1330 1335
   Asp Leu Ser Val Ile Gly His Pro Ile Asp Ser Glu Ser Lys Glu
   1340 1345 1350
   Asp Glu Pro Cys Ser Glu Glu Thr Asp Pro Val His Asp Leu Met
   1355 1360 1365
   Ala Glu Ile Leu Pro Glu Phe Pro Asp Ile Ile Glu Ile Asp Leu
   1370 1375 1380
   Tyr His Ser Glu Glu Asn Glu Glu Glu Glu Glu Glu Cys Ala Asn
   1385 1390 1395
   Ala Thr Asp Val Thr Thr Thr Pro Ser Val Gln Tyr Ile Asn Gly
   1400 1405 1410
   Lys His Leu Val Thr Thr Val Pro Lys Asp Pro Glu Ala Ala Glu
   1415 1420 1425
   Ala Arg Arg Gly Gln Phe Glu Ser Val Ala Pro Ser Gln Asn Phe
   1430 1435 1440
   Ser Asp Ser Ser Glu Ser Asp Thr His Pro Phe Val Ile Ala Lys
   1445 1450 1455
   Thr Glu Leu Ser Thr Ala Val Gln Pro Asn Glu Ser Thr Glu Thr
   1460 1465 1470
   Thr Glu Ser Leu Glu Val Thr Trp Lys Pro Glu Thr Tyr Pro Glu
   1475 1480 1485
   Thr Ser Glu His Phe Ser Gly Gly Glu Pro Asp Val Phe Pro Thr
       1490                 1495                 1500
   Val Pro  Phe His Glu Glu Phe  Glu Ser Gly Thr Ala  Lys Lys Gly
       1505                 1510                 1515
   Ala Glu  Ser Val Thr Glu Arg  Asp Thr Glu Val Gly  His Gln Ala
       1520                 1525                 1530
   His Glu  His Thr Glu Pro Val  Ser Leu Phe Pro Glu  Glu Ser Ser
       1535                 1540                 1545
   Gly Glu  Ile Ala Ile Asp Gln  Glu Ser Gln Lys Ile  Ala Phe Ala
       1550                 1555                 1560
   Arg Ala  Thr Glu Val Thr Phe  Gly Glu Glu Val Glu  Lys Ser Thr
       1565                 1570                 1575
   Ser Val  Thr Tyr Thr Pro Thr  Ile Val Pro Ser Ser  Ala Ser Ala
       1580                 1585                 1590
   Tyr Val  Ser Glu Glu Glu Ala  Val Thr Leu Ile Gly  Asn Pro Trp
       1595                 1600                 1605
   Pro Asp  Asp Leu Leu Ser Thr  Lys Glu Ser Trp Val  Glu Ala Thr
       1610                 1615                 1620
   Pro Arg  Gln Val Val Glu Leu  Ser Gly Ser Ser Ser  Ile Pro Ile
       1625                 1630                 1635
   Thr Glu  Gly Ser Gly Glu Ala  Glu Glu Asp Glu Asp  Thr Met Phe
       1640                 1645                 1650
   Thr Met  Val Thr Asp Leu Ser  Gln Arg Asn Thr Thr  Asp Thr Leu
       1655                 1660                 1665
   Ile Thr  Leu Asp Thr Ser Arg  Ile Ile Thr Glu Ser  Phe Phe Glu
       1670                 1675                 1680
   Val Pro  Ala Thr Thr Ile Tyr  Pro Val Ser Glu Gln  Pro Ser Ala
       1685                 1690                 1695
   Lys Val  Val Pro Thr Lys Phe  Val Ser Glu Thr Asp  Thr Ser Glu
       1700                 1705                 1710
   Trp Ile  Ser Ser Thr Thr Val  Glu Glu Lys Lys Arg  Lys Glu Glu
       1715                 1720                 1725
   Glu Gly  Thr Thr Gly Thr Ala  Ser Thr Phe Glu Val  Tyr Ser Ser
       1730                 1735                 1740
   Thr Gln  Arg Ser Asp Gln Leu  Ile Leu Pro Phe Glu  Leu Glu Ser
   1745 1750 1755
   Pro Asn Val Ala Thr Ser Ser Asp Ser Gly Thr Arg Lys Ser Phe
   1760 1765 1770
   Met Ser Leu Thr Thr Pro Thr Gln Ser Glu Arg Glu Met Thr Asp
   1775 1780 1785
   Ser Thr Pro Val Phe Thr Glu Thr Asn Thr Leu Glu Asn Leu Gly
   1790 1795 1800
   Ala Gln Thr Thr Glu His Ser Ser Ile His Gln Pro Gly Val Gln
   1805 1810 1815
   Glu Gly Leu Thr Thr Leu Pro Arg Ser Pro Ala Ser Val Phe Met
   1820 1825 1830
   Glu Gln Gly Ser Gly Glu Ala Ala Ala Asp Pro Glu Thr Thr Thr
   1835 1840 1845
   Val Ser Ser Phe Ser Leu Asn Val Glu Tyr Ala Ile Gln Ala Glu
   1850 1855 1860
   Lys Glu Val Ala Gly Thr Leu Ser Pro His Val Glu Thr Thr Phe
   1865 1870 1875
   Ser Thr Glu Pro Thr Gly Leu Val Leu Ser Thr Val Met Asp Arg
   1880 1885 1890
   Val Val Ala Glu Asn Ile Thr Gln Thr Ser Arg Glu Ile Val Ile
   1895 1900 1905
   Ser Glu Arg Leu Gly Glu Pro Asn Tyr Gly Ala Glu Ile Arg Gly
   1910 1915 1920
   Phe Ser Thr Gly Phe Pro Leu Glu Glu Asp Phe Ser Gly Asp Phe
   1925 1930 1935
   Arg Glu Tyr Ser Thr Val Ser His Pro Ile Ala Lys Glu Glu Thr
   1940 1945 1950
   Val Met Met Glu Gly Ser Gly Asp Ala Ala Phe Arg Asp Thr Gln
   1955 1960 1965
   Thr Ser Pro Ser Thr Val Pro Thr Ser Val His Ile Ser His Ile
   1970 1975 1980
   Ser Asp Ser Glu Gly Pro Ser Ser Thr Met Val Ser Thr Ser Ala
   1985 1990 1995
   Phe Pro Trp Glu Glu Phe Thr Ser Ser Ala Glu Gly Ser Gly Glu
   2000 2005 2010
   Gln Leu Val Thr Val Ser Ser Ser Val Val Pro Val Leu Pro Ser
   2015 2020 2025
   Ala Val Gln Lys Phe Ser Gly Thr Ala Ser Ser Ile Ile Asp Glu
   2030 2035 2040
   Gly Leu Gly Glu Val Gly Thr Val Asn Glu Ile Asp Arg Arg Ser
   2045 2050 2055
   Thr Ile Leu Pro Thr Ala Glu Val Glu Gly Thr Lys Ala Pro Val
   2060 2065 2070
   Glu Lys Glu Glu Val Lys Val Ser Gly Thr Val Ser Thr Asn Phe
   2075 2080 2085
   Pro Gln Thr Ile Glu Pro Ala Lys Leu Trp Ser Arg Gln Glu Val
   2090 2095 2100
   Asn Pro Val Arg Gln Glu Ile Glu Ser Glu Thr Thr Ser Glu Glu
   2105 2110 2115
   Gln Ile Gln Glu Glu Lys Ser Phe Glu Ser Pro Gln Asn Ser Pro
   2120 2125 2130
   Ala Thr Glu Gln Thr Ile Phe Asp Ser Gln Thr Phe Thr Glu Thr
   2135 2140 2145
   Glu Leu Lys Thr Thr Asp Tyr Ser Val Leu Thr Thr Lys Lys Thr
   2150 2155 2160
   Tyr Ser Asp Asp Lys Glu Met Lys Glu Glu Asp Thr Ser Leu Val
   2165 2170 2175
   Asn Met Ser Thr Pro Asp Pro Asp Ala Asn Gly Leu Glu Ser Tyr
   2180 2185 2190
   Thr Thr Leu Pro Glu Ala Thr Glu Lys Ser His Phe Phe Leu Ala
   2195 2200 2205
   Thr Ala Leu Val Thr Glu Ser Ile Pro Ala Glu His Val Val Thr
   2210 2215 2220
   Asp Ser Pro Ile Lys Lys Glu Glu Ser Thr Lys His Phe Pro Lys
   2225 2230 2235
   Gly Met Arg Pro Thr Ile Gln Glu Ser Asp Thr Glu Leu Leu Phe
   2240 2245 2250
   Ser Gly Leu Gly Ser Gly Glu Glu Val Leu Pro Thr Leu Pro Thr
   2255 2260 2265
   Glu Ser Val Asn Phe Thr Glu Val Glu Gln Ile Asn Asn Thr Leu
   2270 2275 2280
   Tyr Pro His Thr Ser Gln Val Glu Ser Thr Ser Ser Asp Lys Ile
   2285 2290 2295
   Glu Asp Phe Asn Arg Met Glu Asn Val Ala Lys Glu Val Gly Pro
   2300 2305 2310
   Leu Val Ser Gln Thr Asp Ile Phe Glu Gly Ser Gly Ser Val Thr
   2315 2320 2325
   Ser Thr Thr Leu Ile Glu Ile Leu Ser Asp Thr Gly Ala Glu Gly
   2330 2335 2340
   Pro Thr Val Ala Pro Leu Pro Phe Ser Thr Asp Ile Gly His Pro
   2345 2350 2355
   Gln Asn Gln Thr Val Arg Trp Ala Glu Glu Ile Gln Thr Ser Arg
   2360 2365 2370
   Pro Gln Thr Ile Thr Glu Gln Asp Ser Asn Lys Asn Ser Ser Thr
   2375 2380 2385
   Ala Glu Ile Asn Glu Thr Thr Thr Ser Ser Thr Asp Phe Leu Ala
   2390 2395 2400
   Arg Ala Tyr Gly Phe Glu Met Ala Lys Glu Phe Val Thr Ser Ala
   2405 2410 2415
   Pro Lys Pro Ser Asp Leu Tyr Tyr Glu Pro Ser Gly Glu Gly Ser
   2420 2425 2430
   Gly Glu Val Asp Ile Val Asp Ser Phe His Thr Ser Ala Thr Thr
   2435 2440 2445
   Gln Ala Thr Arg Gln Glu Ser Ser Thr Thr Phe Val Ser Asp Gly
   2450 2455 2460
   Ser Leu Glu Lys His Pro Glu Val Pro Ser Ala Lys Ala Val Thr
   2465 2470 2475
   Ala Asp Gly Phe Pro Thr Val Ser Val Met Leu Pro Leu His Ser
   2480 2485 2490
   Glu Gln Asn Lys Ser Ser Pro Asp Pro Thr Ser Thr Leu Ser Asn
   2495 2500 2505
   Thr Val Ser Tyr Glu Arg Ser Thr Asp Gly Ser Phe Gln Asp Arg
   2510 2515 2520
   Phe Arg Glu Phe Glu Asp Ser Thr Leu Lys Pro Asn Arg Lys Lys
   2525 2530 2535
   Pro Thr Glu Asn Ile Ile Ile Asp Leu Asp Lys Glu Asp Lys Asp
   2540 2545 2550
   Leu Ile Leu Thr Ile Thr Glu Ser Thr Ile Leu Glu Ile Leu Pro
   2555 2560 2565
   Glu Leu Thr Ser Asp Lys Asn Thr Ile Ile Asp Ile Asp His Thr
   2570 2575 2580
   Lys Pro Val Tyr Glu Asp Ile Leu Gly Met Gln Thr Asp Ile Asp
   2585 2590 2595
   Thr Glu Val Pro Ser Glu Pro His Asp Ser Asn Asp Glu Ser Asn
   2600 2605 2610
   Asp Asp Ser Thr Gln Val Gln Glu Ile Tyr Glu Ala Ala Val Asn
   2615 2620 2625
   Leu Ser Leu Thr Glu Glu Thr Phe Glu Gly Ser Ala Asp Val Leu
   2630 2635 2640
   Ala Ser Tyr Thr Gln Ala Thr His Asp Glu Ser Met Thr Tyr Glu
   2645 2650 2655
   Asp Arg Ser Gln Leu Asp His Met Gly Phe His Phe Thr Thr Gly
   2660 2665 2670
   Ile Pro Ala Pro Ser Thr Glu Thr Glu Leu Asp Val Leu Leu Pro
   2675 2680 2685
   Thr Ala Thr Ser Leu Pro Ile Pro Arg Lys Ser Ala Thr Val Ile
   2690 2695 2700
   Pro Glu Ile Glu Gly Ile Lys Ala Glu Ala Lys Ala Leu Asp Asp
   2705 2710 2715
   Met Phe Glu Ser Ser Thr Leu Ser Asp Gly Gln Ala Ile Ala Asp
   2720 2725 2730
   Gln Ser Glu Ile Ile Pro Thr Leu Gly Gln Phe Glu Arg Thr Gln
   2735 2740 2745
   Glu Glu Tyr Glu Asp Lys Lys His Ala Gly Pro Ser Phe Gln Pro
   2750 2755 2760
   Glu Phe Ser Ser Gly Ala Glu Glu Ala Leu Val Asp His Thr Pro
   2765 2770 2775
   Tyr Leu Ser Ile Ala Thr Thr His Leu Met Asp Gln Ser Val Thr
   2780 2785 2790
   Glu Val Pro Asp Val Met Glu Gly Ser Asn Pro Pro Tyr Tyr Thr
   2795 2800 2805
   Asp Thr Thr Leu Ala Val Ser Thr Phe Ala Lys Leu Ser Ser Gln
   2810 2815 2820
   Thr Pro Ser Ser Pro Leu Thr Ile Tyr Ser Gly Ser Glu Ala Ser
   2825 2830 2835
   Gly His Thr Glu Ile Pro Gln Pro Ser Ala Leu Pro Gly Ile Asp
   2840 2845 2850
   Val Gly Ser Ser Val Met Ser Pro Gln Asp Ser Phe Lys Glu Ile
   2855 2860 2865
   His Val Asn Ile Glu Ala Thr Phe Lys Pro Ser Ser Glu Glu Tyr
   2870 2875 2880
   Leu His Ile Thr Glu Pro Pro Ser Leu Ser Pro Asp Thr Lys Leu
   2885 2890 2895
   Glu Pro Ser Glu Asp Asp Gly Lys Pro Glu Leu Leu Glu Glu Met
   2900 2905 2910
   Glu Ala Ser Pro Thr Glu Leu Ile Ala Val Glu Gly Thr Glu Ile
   2915 2920 2925
   Leu Gln Asp Phe Gln Asn Lys Thr Asp Gly Gln Val Ser Gly Glu
   2930 2935 2940
   Ala Ile Lys Met Phe Pro Thr Ile Lys Thr Pro Glu Ala Gly Thr
   2945 2950 2955
   Val Ile Thr Thr Ala Asp Glu Ile Glu Leu Glu Gly Ala Thr Gln
   2960 2965 2970
   Trp Pro His Ser Thr Ser Ala Ser Ala Thr Tyr Gly Val Glu Ala
   2975 2980 2985
   Gly Val Val Pro Trp Leu Ser Pro Gln Thr Ser Glu Arg Pro Thr
   2990 2995 3000
   Leu Ser Ser Ser Pro Glu Ile Asn Pro Glu Thr Gln Ala Ala Leu
   3005 3010 3015
   Ile Arg Gly Gln Asp Ser Thr Ile Ala Ala Ser Glu Gln Gln Val
   3020 3025 3030
   Ala Ala Arg Ile Leu Asp Ser Asn Asp Gln Ala Thr Val Asn Pro
   3035 3040 3045
   Val Glu Phe Asn Thr Glu Val Ala Thr Pro Pro Phe Ser Leu Leu
   3050 3055 3060
   Glu Thr Ser Asn Glu Thr Asp Phe Leu Ile Gly Ile Asn Glu Glu
   3065 3070 3075
   Ser Val Glu Gly Thr Ala Ile Tyr Leu Pro Gly Pro Asp Arg Cys
   3080 3085 3090
   Lys Met Asn Pro Cys Leu Asn Gly Gly Thr Cys Tyr Pro Thr Glu
   3095 3100 3105
   Thr Ser Tyr Val Cys Thr Cys Val Pro Gly Tyr Ser Gly Asp Gln
   3110 3115 3120
   Cys Glu Leu Asp Phe Asp Glu Cys His Ser Asn Pro Cys Arg Asn
   3125 3130 3135
   Gly Ala Thr Cys Val Asp Gly Phe Asn Thr Phe Arg Cys Leu Cys
   3140 3145 3150
   Leu Pro Ser Tyr Val Gly Ala Leu Cys Glu Gln Asp Thr Glu Thr
   3155 3160 3165
   Cys Asp Tyr Gly Trp His Lys Phe Gln Gly Gln Cys Tyr Lys Tyr
   3170 3175 3180
   Phe Ala His Arg Arg Thr Trp Asp Ala Ala Glu Arg Glu Cys Arg
   3185 3190 3195
   Leu Gln Gly Ala His Leu Thr Ser Ile Leu Ser His Glu Glu Gln
   3200 3205 3210
   Met Phe Val Asn Arg Val Gly His Asp Tyr Gln Trp Ile Gly Leu
   3215 3220 3225
   Asn Asp Lys Met Phe Glu His Asp Phe Arg Trp Thr Asp Gly Ser
   3230 3235 3240
   Thr Leu Gln Tyr Glu Asn Trp Arg Pro Asn Gln Pro Asp Ser Phe
   3245 3250 3255
   Phe Ser Ala Gly Glu Asp Cys Val Val Ile Ile Trp His Glu Asn
   3260 3265 3270
   Gly Gln Trp Asn Asp Val Pro Cys Asn Tyr His Leu Thr Tyr Thr
   3275 3280 3285
   Cys Lys Lys Gly Thr Val Ala Cys Gly Gln Pro Pro Val Val Glu
   3290 3295 3300
   Asn Ala Lys Thr Phe Gly Lys Met Lys Pro Arg Tyr Glu Ile Asn
   3305 3310 3315
   Ser Leu Ile Arg Tyr His Cys Lys Asp Gly Phe Ile Gln Arg His
   3320 3325 3330
   Leu Pro Thr Ile Arg Cys Leu Gly Asn Gly Arg Trp Ala Ile Pro
   3335 3340 3345
   Lys Ile Thr Cys Met Asn Pro Ser Ala Tyr Gln Arg Thr Tyr Ser
   3350 3355 3360
   Met Lys Tyr Phe Lys Asn Ser Ser Ser Ala Lys Asp Asn Ser Ile
   3365 3370 3375
   Asn Thr Ser Lys His Asp His Arg Trp Ser Arg Arg Trp Gln Glu
   3380 3385 3390
   Ser Arg Arg
   3395
<210> 24
   <211> 7230
   <212> DNA
   <213> Homo sapiens
<400> 24
   atgttcataa atataaagag catcttatgg atgtgttcaa ccttaatagt aacccatgcg 60
   ctacataaag tcaaagtggg aaaaagccca ccggtgaggg gctccctctc tggaaaagtc 120
   agcctacctt gtcatttttc aacgatgcct actttgccac ccagttacaa caccagtgaa 180
   tttctccgca tcaaatggtc taagattgaa gtggacaaaa atggaaaaga tttgaaagag 240
   actactgtcc ttgtggccca aaatggaaat atcaagattg gtcaggacta caaagggaga 300
   gtgtctgtgc ccacacatcc cgaggctgtg ggcgatgcct ccctcactgt ggtcaagctg 360
   ctggcaagtg atgcgggtct ttaccgctgt gacgtcatgt acgggattga agacacacaa 420
   gacacggtgt cactgactgt ggatggggtt gtgtttcact acagggcggc aaccagcagg 480
   tacacactga attttgaggc tgctcagaag gcttgtttgg acgttggggc agtcatagca 540
   actccagagc agctctttgc tgcctatgaa gatggatttg agcagtgtga cgcaggctgg 600
   ctggctgatc agactgtcag atatcccatc cgggctccca gagtaggctg ttatggagat 660
   aagatgggaa aggcaggagt caggacttat ggattccgtt ctccccagga aacttacgat 720
   gtgtattgtt atgtggatca tctggatggt gatgtgttcc acctcactgt ccccagtaaa 780
   ttcaccttcg aggaggctgc aaaagagtgt gaaaaccagg atgccaggct ggcaacagtg 840
   ggggaactcc aggcggcatg gaggaacggc tttgaccagt gcgattacgg gtggctgtcg 900
   gatgccagcg tgcgccaccc tgtgactgtg gccagggccc agtgtggagg tggtctactt 960
   ggggtgagaa ccctgtatcg ttttgagaac cagacaggct tccctccccc tgatagcaga 1020
   tttgatgcct actgctttaa acgtcgaatg agtgatttga gtgtaattgg tcatccaata 1080
   gattcagaat ctaaagaaga tgaaccttgt agtgaagaaa cagatccagt gcatgatcta 1140
   atggctgaaa ttttacctga attccctgac ataattgaaa tagacctata ccacagtgaa 1200
   gaaaatgaag aagaagaaga agagtgtgca aatgctactg atgtgacaac caccccatct 1260
   gtgcagtaca taaatgggaa gcatctcgtt accactgtgc ccaaggaccc agaagctgca 1320
   gaagctaggc gtggccagtt tgaaagtgtt gcaccttctc agaatttctc ggacagctct 1380
   gaaagtgata ctcatccatt tgtaatagcc aaaacggaat tgtctactgc tgtgcaacct 1440
   aatgaatcta cagaaacaac tgagtctctt gaagttacat ggaagcctga gacttaccct 1500
   gaaacatcag aacatttttc aggtggtgag cctgatgttt tccccacagt cccattccat 1560
   gaggaatttg aaagtggaac agccaaaaaa ggggcagaat cagtcacaga gagagatact 1620
   gaagttggtc atcaggcaca tgaacatact gaacctgtat ctctgtttcc tgaagagtct 1680
   tcaggagaga ttgccattga ccaagaatct cagaaaatag cctttgcaag ggctacagaa 1740
   gtaacatttg gtgaagaggt agaaaaaagt acttctgtca catacactcc cactatagtt 1800
   ccaagttctg catcagcata tgtttcagag gaagaagcag ttaccctaat aggaaatcct 1860
   tggccagatg acctgttgtc taccaaagaa agctgggtag aagcaactcc tagacaagtt 1920
   gtagagctct cagggagttc ttcgattcca attacagaag gctctggaga agcagaagaa 1980
   gatgaagata caatgttcac catggtaact gatttatcac agagaaatac tactgataca 2040
   ctcattactt tagacactag caggataatc acagaaagct tttttgaggt tcctgcaacc 2100
   accatttatc cagtttctga acaaccttct gcaaaagtgg tgcctaccaa gtttgtaagt 2160
   gaaacagaca cttctgagtg gatttccagt accactgttg aggaaaagaa aaggaaggag 2220
   gaggagggaa ctacaggtac ggcttctaca tttgaggtat attcatctac acagagatcg 2280
   gatcaattaa ttttaccctt tgaattagaa agtccaaatg tagctacatc tagtgattca 2340
   ggtaccagga aaagttttat gtccttgaca acaccaacac agtctgaaag ggaaatgaca 2400
   gattctactc ctgtctttac agaaacaaat acattagaaa atttgggggc acagaccact 2460
   gagcacagca gtatccatca acctggggtt caggaagggc tgaccactct cccacgtagt 2520
   cctgcctctg tctttatgga gcagggctct ggagaagctg ctgccgaccc agaaaccacc 2580
   actgtttctt cattttcatt aaacgtagag tatgcaattc aagccgaaaa ggaagtagct 2640
   ggcactttgt ctccgcatgt ggaaactaca ttctccactg agccaacagg actggttttg 2700
   agtacagtaa tggacagagt agttgctgaa aatataaccc aaacatccag ggaaatagtg 2760
   atttcagagc gattaggaga accaaattat ggggcagaaa taaggggctt ttccacaggt 2820
   tttcctttgg aggaagattt cagtggtgac tttagagaat actcaacagt gtctcatccc 2880
   atagcaaaag aagaaacggt aatgatggaa ggctctggag atgcagcatt tagggacacc 2940
   cagacttcac catctacagt acctacttca gttcacatca gtcacatatc tgactcagaa 3000
   ggacccagta gcaccatggt cagcacttca gccttcccct gggaagagtt tacatcctca 3060
   gctgagggct caggtgagca actggtcaca gtcagcagct ctgttgttcc agtgcttccc 3120
   agtgctgtgc aaaagttttc tggtacagct tcctccatta tcgacgaagg attgggagaa 3180
   gtgggtactg tcaatgaaat tgatagaaga tccaccattt taccaacagc agaagtggaa 3240
   ggtacgaaag ctccagtaga gaaggaggaa gtaaaggtca gtggcacagt ttcaacaaac 3300
   tttccccaaa ctatagagcc agccaaatta tggtctaggc aagaagtcaa ccctgtaaga 3360
   caagaaattg aaagtgaaac aacatcagag gaacaaattc aagaagaaaa gtcatttgaa 3420
   tcccctcaaa actctcctgc aacagaacaa acaatctttg attcacagac atttactgaa 3480
   actgaactca aaaccacaga ttattctgta ctaacaacaa agaaaactta cagtgatgat 3540
   aaagaaatga aggaggaaga cacttcttta gttaacatgt ctactccaga tccagatgca 3600
   aatggcttgg aatcttacac aactctccct gaagctactg aaaagtcaca tttttcttta 3660
   gctactgcat tagtaactga atctatacca gctgaacatg tagtcacaga ttcaccaatc 3720
   aaaaaggaag aaagtacaaa acattttccg aaaggcatga gaccaacaat tcaagagtca 3780
   gatactgagc tcttattctc tggactggga tcaggagaag aagttttacc tactctacca 3840
   acagagtcag tgaattttac tgaagtggaa caaatcaata acacattata tccccacact 3900
   tctcaagtgg aaagtacctc aagtgacaaa attgaagact ttaacagaat ggaaaatgtg 3960
   gcaaaagaag ttggaccact cgtatctcaa acagacatct ttgaaggtag tgggtcagta 4020
   accagcacaa cattaataga aattttaagt gacactggag cagaaggacc cacggtggca 4080
   cctctccctt tctccacgga catcggacat cctcaaaatc agactgtcag gtgggcagaa 4140
   gaaatccaga ctagtagacc acaaaccata actgaacaag actctaacaa gaattcttca 4200
   acagcagaaa ttaacgaaac aacaacctca tctactgatt ttctggctag agcttatggt 4260
   tttgaaatgg ccaaagaatt tgttacatca gcaccaaaac catctgactt gtattatgaa 4320
   ccttctggag aaggatctgg agaagtggat attgttgatt catttcacac ttctgcaact 4380
   actcaggcaa ccagacaaga aagcagcacc acatttgttt ctgatgggtc cctggaaaaa 4440
   catcctgagg tgccaagcgc taaagctgtt actgctgatg gattcccaac agtttcagtg 4500
   atgctgcctc ttcattcaga gcagaacaaa agctcccctg atccaactag cacactgtca 4560
   aatacagtgt catatgagag gtccacagac ggtagtttcc aagaccgttt cagggaattc 4620
   gaggattcca ccttaaaacc taacagaaaa aaacccactg aaaatattat catagacctg 4680
   gacaaagagg acaaggattt aatattgaca attacagaga gtaccatcct tgaaattcta 4740
   cctgagctga catcggataa aaatactatc atagatattg atcatactaa acctgtgtat 4800
   gaagacattc ttggaatgca aacagatata gatacagagg taccatcaga accacatgac 4860
   agtaatgatg aaagtaatga tgacagcact caagttcaag agatctatga ggcagctgtc 4920
   aacctttctt taactgagga aacatttgag ggctctgctg atgttctggc tagctacact 4980
   caggcaacac atgatgaatc aatgacttat gaagatagaa gccaactaga tcacatgggc 5040
   tttcacttca caactgggat ccctgctcct agcacagaaa cagaattaga cgttttactt 5100
   cccacggcaa catccctgcc aattcctcgt aagtctgcca cagttattcc agagattgaa 5160
   ggaataaaag ctgaagcaaa agccctggat gacatgtttg aatcaagcac tttgtctgat 5220
   ggtcaagcta ttgcagacca aagtgaaata ataccaacat tgggccaatt tgaaaggact 5280
   caggaggagt atgaagacaa aaaacatgct ggtccttctt ttcagccaga attctcttca 5340
   ggagctgagg aggcattagt agaccatact ccctatctaa gtattgctac tacccacctt 5400
   atggatcaga gtgtaacaga ggtgcctgat gtgatggaag gatccaatcc cccatattac 5460
   actgatacaa cattagcagt ttcaacattt gcgaagttgt cttctcagac accatcatct 5520
   cccctcacta tctactcagg cagtgaagcc tctggacaca cagagatccc ccagcccagt 5580
   gctctgccag gaatagacgt cggctcatct gtaatgtccc cacaggattc ttttaaggaa 5640
   attcatgtaa atattgaagc aactttcaaa ccatcaagtg aggaatacct tcacataact 5700
   gagcctccct ctttatctcc tgacacaaaa ttagaacctt cagaagatga tggtaaacct 5760
   gagttattag aagaaatgga agcttctccc acagaactta ttgctgtgga aggaactgag 5820
   attctccaag atttccaaaa caaaaccgat ggtcaagttt ctggagaagc aatcaagatg 5880
   tttcccacca ttaaaacacc tgaggctgga actgttatta caactgccga tgaaattgaa 5940
   ttagaaggtg ctacacagtg gccacactct acttctgctt ctgccaccta tggggtcgag 6000
   gcaggtgtgg tgccttggct aagtccacag acttctgaga ggcccacgct ttcttcttct 6060
   ccagaaataa accctgaaac tcaagcagct ttaatcagag ggcaggattc cacgatagca 6120
   gcatcagaac agcaagtggc agcgagaatt cttgattcca atgatcaggc aacagtaaac 6180
   cctgtggaat ttaatactga ggttgcaaca ccaccatttt cccttctgga gacttctaat 6240
   gaaacagatt tcctgattgg cattaatgaa gagtcagtgg aaggcacggc aatctattta 6300
   ccaggacctg atcgctgcaa aatgaacccg tgccttaacg gaggcacctg ttatcctact 6360
   gaaacttcct acgtatgcac ctgtgtgcca ggatacagcg gagaccagtg tgaacttgat 6420
   tttgatgaat gtcactctaa tccctgtcgt aatggagcca cttgtgttga tggttttaac 6480
   acattcaggt gcctctgcct tccaagttat gttggtgcac tttgtgagca agataccgag 6540
   acatgtgact atggctggca caaattccaa gggcagtgct acaaatactt tgcccatcga 6600
   cgcacatggg atgcagctga acgggaatgc cgtctgcagg gtgcccatct cacaagcatc 6660
   ctgtctcacg aagaacaaat gtttgttaat cgtgtgggcc atgattatca gtggataggc 6720
   ctcaatgaca agatgtttga gcatgacttc cgttggactg atggcagcac actgcaatac 6780
   gagaattgga gacccaacca gccagacagc ttcttttctg ctggagaaga ctgtgttgta 6840
   atcatttggc atgagaatgg ccagtggaat gatgttccct gcaattacca tctcacctat 6900
   acgtgcaaga aaggaacagt tgcttgcggc cagccccctg ttgtagaaaa tgccaagacc 6960
   tttggaaaga tgaaacctcg ttatgaaatc aactccctga ttagatacca ctgcaaagat   7020
   ggtttcattc aacgtcacct tccaactatc cggtgcttag gaaatggaag atgggctata   7080
   cctaaaatta cctgcatgaa cccatctgca taccaaagga cttattctat gaaatacttt   7140
   aaaaattcct catcagcaaa ggacaattca ataaatacat ccaaacatga tcatcgttgg   7200
   agccggaggt ggcaggagtc gaggcgctga                                    7230
<210> 25
   <211> 292
   <212> PRT
   <213> Homo sapiens
<400> 25
   Met Gly Pro Pro Ser Ala Cys Pro His Arg Glu Cys Ile Pro Trp Gln
   1 5 10 15
   Gly Leu Leu Leu Thr Ala Ser Leu Leu Thr Phe Trp Asn Ala Pro Thr
   20 25 30
   Thr Ala Trp Leu Phe Ile Ala Ser Ala Pro Phe Glu Val Ala Glu Gly
   35 40 45
   Glu Asn Val His Leu Ser Val Val Tyr Leu Pro Glu Asn Leu Tyr Ser
   50 55 60
   Tyr Gly Trp Tyr Lys Gly Lys Thr Val Glu Pro Asn Gln Leu Ile Ala
   65 70 75 80
   Ala Tyr Val Ile Asp Thr His Val Arg Thr Pro Gly Pro Ala Tyr Ser
   85 90 95
   Gly Arg Glu Thr Ile Ser Pro Ser Gly Asp Leu His Phe Gln Asn Val
   100 105 110
   Thr Leu Glu Asp Thr Gly Tyr Tyr Asn Leu Gln Val Thr Tyr Arg Asn
   115 120 125
   Ser Gln Ile Glu Gln Ala Ser His His Leu Arg Val Tyr Glu Ser Val
   130 135 140
   Ala Gln Pro Ser Ile Gln Ala Ser Ser Thr Thr Val Thr Glu Lys Gly
   145 150 155 160
   Ser Val Val Leu Thr Cys His Thr Asn Asn Thr Gly Thr Ser Phe Gln
   165 170 175
   Trp Ile Phe Asn Asn Gln Arg Leu Gln Val Thr Lys Arg Met Lys Leu
   180 185 190
   Ser Trp Phe Asn His Val Leu Thr Ile Asp Pro Ile Arg Gln Glu Asp
               195                 200                 205
   Ala Gly Glu Tyr Gln Cys Glu Val Ser Asn Pro Val Ser Ser Asn Arg
       210                 215                 220
   Ser Asp Pro Leu Lys Leu Thr Val Lys Tyr Asp Asn Thr Leu Gly Ile
   225                 230                 235                 240
   Leu Ile Gly Val Leu Val Gly Ser Leu Leu Val Ala Ala Leu Val Cys
                   245                 250                 255
   Phe Leu Leu Leu Arg Lys Thr Gly Arg Ala Ser Asp Gln Ser Asp Phe
               260                 265                 270
   Arg Glu Gln Gln Pro Pro Ala Ser Thr Pro Gly His Gly Pro Ser Asp
           275                 280                 285
   Ser Ser Ile Ser
       290
<210> 26
   <211> 879
   <212> DNA
   <213> Homo sapiens
<400> 26
   atggggcccc cctcagcttg tccccacaga gaatgcatcc cctggcaggg gctcttgctc     60
   acagcctcac ttttaacttt ctggaacgca cccaccactg cctggctctt tattgcatca    120
   gcgccctttg aagttgctga aggggagaat gttcatctct ctgtggttta tctgcccgag    180
   aatctttaca gctatggctg gtacaaaggg aaaacggtgg agcccaacca gctaatcgca    240
   gcatatgtaa tagacactca cgttaggact ccagggcctg catacagcgg tcgagagaca    300
   atatcaccca gtggagatct gcatttccag aacgtcaccc tagaggacac gggatactac    360
   aacctacaag tcacatacag aaattctcag attgaacagg catctcacca tctccgtgta    420
   tacgagtcag tggctcagcc ctccatccaa gccagcagca ccacagtcac agagaagggc    480
   tccgtggtcc tgacctgcca cacaaataac actggaacct ctttccagtg gattttcaac    540
   aaccagcgtc tgcaggtcac gaagaggatg aagctgtcct ggtttaacca tgtgctcacc    600
   atagacccca tcaggcagga ggacgctggg gagtatcagt gtgaggtctc caacccagtc    660
   agctccaaca ggagcgaccc cctcaagctg actgtaaaat atgacaacac tctaggcatc    720
   ctgatcgggg tcctggttgg gagtcttctg gtggctgcac ttgtgtgttt cctgctcctc    780
   cgaaaaactg gcagggccag cgatcagagt gacttcaggg agcagcagcc cccagcctcc    840
   acccccggcc atggaccctc tgacagctcc atctcctag                           879
<210> 27
   <211> 293
   <212> PRT
   <213> Homo sapiens
<400> 27
   Met Gly Pro Pro Ser Ala Cys Pro His Arg Glu Cys Ile Pro Trp Gln
   1 5 10 15
   Gly Leu Leu Leu Thr Ala Ser Leu Leu Thr Phe Trp Asn Ala Pro Thr
   20 25 30
   Thr Ala Trp Leu Phe Ile Ala Ser Ala Pro Phe Glu Val Ala Glu Gly
   35 40 45
   Glu Asn Val His Leu Ser Val Val Tyr Leu Pro Glu Asn Leu Tyr Ser
   50 55 60
   Tyr Gly Trp Tyr Lys Gly Lys Thr Val Glu Pro Asn Gln Leu Ile Ala
   65 70 75 80
   Ala Tyr Val Ile Asp Thr His Val Arg Thr Pro Gly Pro Ala Tyr Ser
   85 90 95
   Gly Arg Glu Thr Ile Ser Pro Ser Gly Asp Leu His Phe Gln Asn Val
   100 105 110
   Thr Leu Glu Asp Thr Gly Tyr Tyr Asn Leu Gln Val Thr Tyr Arg Asn
   115 120 125
   Ser Gln Ile Glu Gln Ala Ser His His Leu Arg Val Tyr Glu Ser Val
   130 135 140
   Ala Gln Pro Ser Ile Gln Ala Ser Ser Thr Thr Val Thr Glu Lys Gly
   145 150 155 160
   Ser Val Val Leu Thr Cys His Thr Asn Asn Thr Gly Thr Ser Phe Gln
   165 170 175
   Trp Ile Phe Asn Asn Gln Arg Leu Gln Val Thr Lys Arg Met Lys Leu
   180 185 190
   Ser Trp Phe Asn His Val Leu Thr Ile Asp Pro Ile Arg Gln Glu Asp
   195 200 205
   Ala Gly Glu Tyr Gln Cys Glu Val Ser Asn Pro Val Ser Ser Asn Arg
   210 215 220
   Ser Asp Pro Leu Lys Leu Thr Val Lys Ser Asp Asp Asn Thr Leu Gly
   225 230 235 240
   Ile Leu Ile Gly Val Leu Val Gly Ser Leu Leu Val Ala Ala Leu Val
   245 250 255
   Cys Phe Leu Leu Leu Arg Lys Thr Gly Arg Ala Ser Asp Gln Ser Asp
   260 265 270
   Phe Arg Glu Gln Gln Pro Pro Ala Ser Thr Pro Gly His Gly Pro Ser
   275 280 285
   Asp Ser Ser Ile Ser
   290
<210> 28
   <211> 882
   <212> DNA
   <213> Homo sapiens
<400> 28
   atggggcccc cctcagcttg tccccacaga gaatgcatcc cctggcaggg gctcttgctc 60
   acagcctcac ttttaacttt ctggaacgca cccaccactg cctggctctt tattgcatca 120
   gcgccctttg aagttgctga aggggagaat gttcatctct ctgtggttta tctgcccgag 180
   aatctttaca gctatggctg gtacaaaggg aaaacggtgg agcccaacca gctaatcgca 240
   gcatatgtaa tagacactca cgttaggact ccagggcctg catacagcgg tcgagagaca 300
   atatcaccca gtggagatct gcatttccag aacgtcaccc tagaggacac gggatactac 360
   aacctacaag tcacatacag aaattctcag attgaacagg catctcacca tctccgtgta 420
   tacgagtcag tggctcagcc ctccatccaa gccagcagca ccacagtcac agagaagggc 480
   tccgtggtcc tgacctgcca cacaaataac actggaacct ctttccagtg gattttcaac 540
   aaccagcgtc tgcaggtcac gaagaggatg aagctgtcct ggtttaacca tgtgctcacc 600
   atagacccca tcaggcagga ggacgctggg gagtatcagt gtgaggtctc caacccagtc 660
   agctccaaca ggagcgaccc cctcaagctg actgtaaaat cagatgacaa cactctaggc 720
   atcctgatcg gggtcctggt tgggagtctt ctggtggctg cacttgtgtg tttcctgctc 780
   ctccgaaaaa ctggcagggc cagcgatcag agtgacttca gggagcagca gcccccagcc 840
   tccacccccg gccatggacc ctctgacagc tccatctcct ag 882
<210> 29
   <211> 235
   <212> PRT
   <213> Homo sapiens
<400> 29
   Met Gly Pro Pro Ser Ala Cys Pro His Arg Glu Cys Ile Pro Trp Gln
   1               5                   10                  15
   Gly Leu Leu Leu Thr Ala Ser Leu Leu Thr Phe Trp Asn Ala Pro Thr
               20                  25                  30
   Thr Ala Trp Leu Phe Ile Ala Ser Ala Pro Phe Glu Val Ala Glu Gly
           35                  40                  45
   Glu Asn Val His Leu Ser Val Val Tyr Leu Pro Glu Asn Leu Tyr Ser
       50                  55                  60
   Tyr Gly Trp Tyr Lys Gly Lys Thr Val Glu Pro Asn Gln Leu Ile Ala
   65                  70                  75                  80
   Ala Tyr Val Ile Asp Thr His Val Arg Thr Pro Gly Pro Ala Tyr Ser
                   85                  90                  95
   Gly Arg Glu Thr Ile Ser Pro Ser Gly Asp Leu His Phe Gln Asn Val
               100                 105                 110
   Thr Leu Glu Asp Thr Gly Tyr Tyr Thr Leu Gln Val Thr Tyr Arg Asn
           115                 120                 125
   Ser Gln Ile Glu Gln Ala Ser His His Leu Arg Val Tyr Glu Ser Val
       130                 135                 140
   Ala Gln Pro Ser Ile Gln Ala Ser Ser Thr Thr Val Thr Glu Lys Gly
   145                 150                 155                 160
   Ser Val Val Leu Thr Cys His Thr Asn Asn Thr Gly Thr Ser Phe Gln
                   165                 170                 175
   Trp Ile Phe Asn Asn Gln Arg Leu Gln Val Thr Lys Arg Met Lys Leu
               180                 185                 190
   Ser Trp Phe Asn His Met Leu Thr Ile Asp Pro Ile Arg Gln Glu Asp
           195                 200                 205
   Ala Gly Glu Tyr Gln Cys Glu Val Ser Asn Pro Val Ser Ser Asn Arg
       210                 215                 220
   Ser Asp Pro Leu Lys Leu Thr Val Lys Cys Glu
   225                 230                 235
<210> 30
   <211> 708
   <212> DNA
   <213> Homo sapiens
<400> 30
   atggggcccc cctcagcttg tccccacaga gaatgcatcc cctggcaggg gctcttgctc     60
   acagcctcac ttttaacttt ctggaacgca cccaccactg cctggctctt tattgcatca    120
   gcgccctttg aagttgctga aggggagaat gttcatctct ctgtggttta tctgcccgag    180
   aatctttaca gctatggctg gtacaaaggg aaaacggtgg agcccaacca gctaatcgca    240
   gcatatgtaa tagacactca cgttaggact ccagggcctg catacagcgg tcgagagaca    300
   atatcaccca gtggagatct gcatttccag aacgtcaccc tagaggacac gggatactac    360
   accctacaag tcacatacag aaattctcag attgaacagg catctcacca tctccgtgta    420
   tacgagtcag tggctcagcc ctccatccaa gccagcagca ccacagtcac agagaagggc    480
   tccgtggtcc tgacctgcca cacaaataac actggaacct ctttccagtg gattttcaac    540
   aaccagcgtc tgcaggtcac gaagaggatg aagctgtcct ggtttaacca tatgctcacc    600
   atagacccca tcaggcagga ggacgctggg gagtatcagt gtgaggtctc caacccagtc    660
   agctccaaca ggagcgaccc cctcaagctg actgtaaaat gtgagtga                 708
<210> 31
   <211> 376
   <212> PRT
   <213> Homo sapiens
<400> 31
   Met Gln Trp Thr Ser Leu Leu Leu Leu Ala Gly Leu Phe Ser Leu Ser
   1               5                   10                  15
   Gln Ala Gln Tyr Glu Asp Asp Pro His Trp Trp Phe His Tyr Leu Arg
               20                  25                  30
   Ser Gln Gln Ser Thr Tyr Tyr Asp Pro Tyr Asp Pro Tyr Pro Tyr Glu
           35                  40                  45
   Thr Tyr Glu Pro Tyr Pro Tyr Gly Val Asp Glu Gly Pro Ala Tyr Thr
       50                  55                  60
   Tyr Gly Ser Pro Ser Pro Pro Asp Pro Arg Asp Cys Pro Gln Glu Cys
   65                  70                  75                  80
   Asp Cys Pro Pro Asn Phe Pro Thr Ala Met Tyr Cys Asp Asn Arg Asn
                   85                  90                  95
   Leu Lys Tyr Leu Pro Phe Val Pro Ser Arg Met Lys Tyr Val Tyr Phe
               100                 105                 110
   Gln Asn Asn Gln Ile Thr Ser Ile Gln Glu Gly Val Phe Asp Asn Ala
           115                 120                 125
   Thr Gly Leu Leu Trp Ile Ala Leu His Gly Asn Gln Ile Thr Ser Asp
       130                 135                 140
   Lys Val Gly Arg Lys Val Phe Ser Lys Leu Arg His Leu Glu Arg Leu
   145                 150                 155                 160
   Tyr Leu Asp His Asn Asn Leu Thr Arg Met Pro Gly Pro Leu Pro Arg
                   165                 170                 175
   Ser Leu Arg Glu Leu His Leu Asp His Asn Gln Ile Ser Arg Val Pro
               180                 185                 190
   Asn Asn Ala Leu Glu Gly Leu Glu Asn Leu Thr Ala Leu Tyr Leu Gln
           195                 200                 205
   His Asn Glu Ile Gln Glu Val Gly Ser Ser Met Arg Gly Leu Arg Ser
       210                 215                 220
   Leu Ile Leu Leu Asp Leu Ser Tyr Asn His Leu Arg Lys Val Pro Asp
   225                 230                 235                 240
   Gly Leu Pro Ser Ala Leu Glu Gln Leu Tyr Met Glu His Asn Asn Val
                   245                 250                 255
   Tyr Thr Val Pro Asp Ser Tyr Phe Arg Gly Ala Pro Lys Leu Leu Tyr
               260                 265                 270
   Val Arg Leu Ser His Asn Ser Leu Thr Asn Asn Gly Leu Ala Ser Asn
           275                 280                 285
   Thr Phe Asn Ser Ser Ser Leu Leu Glu Leu Asp Leu Ser Tyr Asn Gln
       290                 295                 300
   Leu Gln Lys Ile Pro Pro Val Asn Thr Asn Leu Glu Asn Leu Tyr Leu
   305                 310                 315                 320
   Gln Gly Asn Arg Ile Asn Glu Phe Ser Ile Ser Ser Phe Cys Thr Val
                   325                 330                 335
   Val Asp Val Val Asn Phe Ser Lys Leu Gln Val Leu Arg Leu Asp Gly
               340                 345                 350
   Asn Glu Ile Lys Arg Ser Ala Met Pro Ala Asp Ala Pro Leu Cys Leu
           355                 360                 365
   Arg Leu Ala Ser Leu Ile Glu Ile
       370                 375
<210> 32
   <211> 1131
   <212> DNA
   <213> Homo sapiens
<400> 32
   atgcagtgga cctccctcct gctgctggca gggctcttct ccctctccca ggcccagtat     60
   gaagatgacc ctcattggtg gttccactac ctccgcagcc agcagtccac ctactacgat    120
   ccctatgacc cttacccgta tgagacctac gagccttacc cctatggggt ggatgaaggg    180
   ccagcctaca cctacggctc tccatcccct ccagatcccc gcgactgccc ccaggagtgc    240
   gactgcccac ccaacttccc cacggccatg tactgtgaca atcgcaacct caagtacctg    300
   cccttcgttc cctcccgcat gaagtatgtg tacttccaga acaaccagat cacctccatc    360
   caggaaggcg tctttgacaa tgccacaggg ctgctctgga ttgctctcca cggcaaccag    420
   atcaccagtg ataaggtggg caggaaggtc ttctccaagc tgaggcacct ggagaggctg    480
   tacctggacc acaacaacct gacccggatg cccggtcccc tgcctcgatc cctgagagag    540
   ctccatctcg accacaacca gatctcacgg gtccccaaca atgctctgga ggggctggag    600
   aacctcacgg ccttgtacct ccaacacaat gagatccagg aagtgggcag ttccatgagg    660
   ggcctccggt cactgatctt gctggacctg agttataacc accttcggaa ggtgcctgat    720
   gggctgccct cagctcttga gcagctgtac atggagcaca acaatgtcta caccgtcccc    780
   gatagctact tccggggggc gcccaagctg ctgtatgtgc ggctgtccca caacagtcta     840
   accaacaatg gcctggcctc caacaccttc aattccagca gcctccttga gctagacctc    900
   tcctacaacc agctgcagaa gatcccccca gtcaacacca acctggagaa cctctacctc    960
   caaggcaata ggatcaatga gttctccatc agcagcttct gcaccgtggt ggacgtcgtg   1020
   aacttctcca agctgcaggt gctgcgcctg gacgggaacg agatcaagcg cagcgccatg   1080
   cctgccgacg cgcccctctg cctgcgcctt gccagcctca tcgagatctg a            1131
<210> 33
   <211> 1496
   <212> PRT
   <213> Homo sapiens
<400> 33
   Ser Arg Pro Trp Trp Leu Arg Ala Ser Glu Arg Pro Ser Ala Pro Ser
   1               5                   10                  15
   Ala Met Ala Lys Arg Ser Arg Gly Pro Gly Arg Arg Cys Leu Leu Ala
               20                  25                  30
   Leu Val Leu Phe Cys Ala Trp Gly Thr Leu Ala Val Val Ala Gln Lys
           35                  40                  45
   Pro Gly Ala Gly Cys Pro Ser Arg Cys Leu Cys Phe Arg Thr Thr Val
       50                  55                  60
   Arg Cys Met His Leu Leu Leu Glu Ala Val Pro Ala Val Ala Pro Gln
   65                  70                  75                  80
   Thr Ser Ile Leu Asp Leu Arg Phe Asn Arg Ile Arg Glu Ile Gln Pro
                   85                  90                  95
   Gly Ala Phe Arg Arg Leu Arg Asn Leu Asn Thr Leu Leu Leu Asn Asn
               100                 105                 110
   Asn Gln Ile Lys Arg Ile Pro Ser Gly Ala Phe Glu Asp Leu Glu Asn
           115                 120                 125
   Leu Lys Tyr Leu Tyr Leu Tyr Lys Asn Glu Ile Gln Ser Ile Asp Arg
       130                 135                 140
   Gln Ala Phe Lys Gly Leu Ala Ser Leu Glu Gln Leu Tyr Leu His Phe
   145                 150                 155                 160
   Asn Gln Ile Glu Thr Leu Asp Pro Asp Ser Phe Gln His Leu Pro Lys
   165 170 175
   Leu Glu Arg Leu Phe Leu His Asn Asn Arg Ile Thr His Leu Val Pro
   180 185 190
   Gly Thr Phe Asn His Leu Glu Ser Met Lys Arg Leu Arg Leu Asp Ser
   195 200 205
   Asn Thr Leu His Cys Asp Cys Glu Ile Leu Trp Leu Ala Asp Leu Leu
   210 215 220
   Lys Thr Tyr Ala Glu Ser Gly Asn Ala Gln Ala Ala Ala Ile Cys Glu
   225 230 235 240
   Tyr Pro Arg Arg Ile Gln Gly Arg Ser Val Ala Thr Ile Thr Pro Glu
   245 250 255
   Glu Leu Asn Cys Glu Arg Pro Arg Ile Thr Ser Glu Pro Gln Asp Ala
   260 265 270
   Asp Val Thr Ser Gly Asn Thr Val Tyr Phe Thr Cys Arg Ala Glu Gly
   275 280 285
   Asn Pro Lys Pro Glu Ile Ile Trp Leu Arg Asn Asn Asn Glu Leu Ser
   290 295 300
   Met Lys Thr Asp Ser Arg Leu Asn Leu Leu Asp Asp Gly Thr Leu Met
   305 310 315 320
   Ile Gln Asn Thr Gln Glu Thr Asp Gln Gly Ile Tyr Gln Cys Met Ala
   325 330 335
   Lys Asn Val Ala Gly Glu Val Lys Thr Gln Glu Val Thr Leu Arg Tyr
   340 345 350
   Phe Gly Ser Pro Ala Arg Pro Thr Phe Val Ile Gln Pro Gln Asn Thr
   355 360 365
   Glu Val Leu Val Gly Glu Ser Val Thr Leu Glu Cys Ser Ala Thr Gly
   370 375 380
   His Pro Pro Pro Arg Ile Ser Trp Thr Arg Gly Asp Arg Thr Pro Leu
   385 390 395 400
   Pro Val Asp Pro Arg Val Asn Ile Thr Pro Ser Gly Gly Leu Tyr Ile
   405 410 415
   Gln Asn Val Val Gln Gly Asp Ser Gly Glu Tyr Ala Cys Ser Ala Thr
   420 425 430
   Asn Asn Ile Asp Ser Val His Ala Thr Ala Phe Ile Ile Val Gln Ala
   435 440 445
   Leu Pro Gln Phe Thr Val Thr Pro Gln Asp Arg Val Val Ile Glu Gly
   450 455 460
   Gln Thr Val Asp Phe Gln Cys Glu Ala Lys Gly Asn Pro Pro Pro Val
   465 470 475 480
   Ile Ala Trp Thr Lys Gly Gly Ser Gln Leu Ser Val Asp Arg Arg His
   485 490 495
   Leu Val Leu Ser Ser Gly Thr Leu Arg Ile Ser Gly Val Ala Leu His
   500 505 510
   Asp Gln Gly Gln Tyr Glu Cys Gln Ala Val Asn Ile Ile Gly Ser Gln
   515 520 525
   Lys Val Val Ala His Leu Thr Val Gln Pro Arg Val Thr Pro Val Phe
   530 535 540
   Ala Ser Ile Pro Ser Asp Thr Thr Val Glu Val Gly Ala Asn Val Gln
   545 550 555 560
   Leu Pro Cys Ser Ser Gln Gly Glu Pro Glu Pro Ala Ile Thr Trp Asn
   565 570 575
   Lys Asp Gly Val Gln Val Thr Glu Ser Gly Lys Phe His Ile Ser Pro
   580 585 590
   Glu Gly Phe Leu Thr Ile Asn Asp Val Gly Pro Ala Asp Ala Gly Arg
   595 600 605
   Tyr Glu Cys Val Ala Arg Asn Thr Ile Gly Ser Ala Ser Val Ser Met
   610 615 620
   Val Leu Ser Val Asn Val Pro Asp Val Ser Arg Asn Gly Asp Pro Phe
   625 630 635 640
   Val Ala Thr Ser Ile Val Glu Ala Ile Ala Thr Val Asp Arg Ala Ile
   645 650 655
   Asn Ser Thr Arg Thr His Leu Phe Asp Ser Arg Pro Arg Ser Pro Asn
   660 665 670
   Asp Leu Leu Ala Leu Phe Arg Tyr Pro Arg Asp Pro Tyr Thr Val Glu
   675 680 685
   Gln Ala Arg Ala Gly Glu Ile Phe Glu Arg Thr Leu Gln Leu Ile Gln
   690 695 700
   Glu His Val Gln His Gly Leu Met Val Asp Leu Asn Gly Thr Ser Tyr
   705 710 715 720
   His Tyr Asn Asp Leu Val Ser Pro Gln Tyr Leu Asn Leu Ile Ala Asn
   725 730 735
   Leu Ser Gly Cys Thr Ala His Arg Arg Val Asn Asn Cys Ser Asp Met
   740 745 750
   Cys Phe His Gln Lys Tyr Arg Thr His Asp Gly Thr Cys Asn Asn Leu
   755 760 765
   Gln His Pro Met Trp Gly Ala Ser Leu Thr Ala Phe Glu Arg Leu Leu
   770 775 780
   Lys Ser Val Tyr Glu Asn Gly Phe Asn Thr Pro Arg Gly Ile Asn Pro
   785 790 795 800
   His Arg Leu Tyr Asn Gly His Ala Leu Pro Met Pro Arg Leu Val Ser
   805 810 815
   Thr Thr Leu Ile Gly Thr Glu Thr Val Thr Pro Asp Glu Gln Phe Thr
   820 825 830
   His Met Leu Met Gln Trp Gly Gln Phe Leu Asp His Asp Leu Asp Ser
   835 840 845
   Thr Val Val Ala Leu Ser Gln Ala Arg Phe Ser Asp Gly Gln His Cys
   850 855 860
   Ser Asn Val Cys Ser Asn Asp Pro Pro Cys Phe Ser Val Met Ile Pro
   865 870 875 880
   Pro Asn Asp Ser Arg Ala Arg Ser Gly Ala Arg Cys Met Phe Phe Val
   885 890 895
   Arg Ser Ser Pro Val Cys Gly Ser Gly Met Thr Ser Leu Leu Met Asn
   900 905 910
   Ser Val Tyr Pro Arg Glu Gln Ile Asn Gln Leu Thr Ser Tyr Ile Asp
   915 920 925
   Ala Ser Asn Val Tyr Gly Ser Thr Glu His Glu Ala Arg Ser Ile Arg
   930 935 940
   Asp Leu Ala Ser His Arg Gly Leu Leu Arg Gln Gly Ile Val Gln Arg
   945 950 955 960
   Ser Gly Lys Pro Leu Leu Pro Phe Ala Thr Gly Pro Pro Thr Glu Cys
   965 970 975
   Met Arg Asp Glu Asn Glu Ser Pro Ile Pro Cys Phe Leu Ala Gly Asp
   980 985 990
   His Arg Ala Asn Glu Gln Leu Gly Leu Thr Ser Met His Thr Leu Trp
   995 1000 1005
   Phe Arg Glu His Asn Arg Ile Ala Thr Glu Leu Leu Lys Leu Asn
   1010 1015 1020
   Pro His Trp Asp Gly Asp Thr Ile Tyr Tyr Glu Thr Arg Lys Ile
   1025 1030 1035
   Val Gly Ala Glu Ile Gln His Ile Thr Tyr Gln His Trp Leu Pro
   1040 1045 1050
   Lys Ile Leu Gly Glu Val Gly Met Arg Thr Leu Gly Glu Tyr His
   1055 1060 1065
   Gly Tyr Asp Pro Gly Ile Asn Ala Gly Ile Phe Asn Ala Phe Ala
   1070 1075 1080
   Thr Ala Ala Phe Arg Phe Gly His Thr Leu Val Asn Pro Leu Leu
   1085 1090 1095
   Tyr Arg Leu Asp Glu Asn Phe Gln Pro Ile Ala Gln Asp His Leu
   1100 1105 1110
   Pro Leu His Lys Ala Phe Phe Ser Pro Phe Arg Ile Val Asn Glu
   1115 1120 1125
   Gly Gly Ile Asp Pro Leu Leu Arg Gly Leu Phe Gly Val Ala Gly
   1130 1135 1140
   Lys Met Arg Val Pro Ser Gln Leu Leu Asn Thr Glu Leu Thr Glu
   1145 1150 1155
   Arg Leu Phe Ser Met Ala His Thr Val Ala Leu Asp Leu Ala Ala
   1160 1165 1170
   Ile Asn Ile Gln Arg Gly Arg Asp His Gly Ile Pro Pro Tyr His
   1175 1180 1185
   Asp Tyr Arg Val Tyr Cys Asn Leu Ser Ala Ala His Thr Phe Glu
   1190 1195 1200
   Asp Leu Lys Asn Glu Ile Lys Asn Pro Glu Ile Arg Glu Lys Leu
   1205 1210 1215
   Lys Arg Leu Tyr Gly Ser Thr Leu Asn Ile Asp Leu Phe Pro Ala
   1220 1225 1230
   Leu Val Val Glu Asp Leu Val Pro Gly Ser Arg Leu Gly Pro Thr
   1235 1240 1245
   Leu Met Cys Leu Leu Ser Thr Gln Phe Lys Arg Leu Arg Asp Gly
   1250 1255 1260
   Asp Arg Leu Trp Tyr Glu Asn Pro Gly Val Phe Ser Pro Ala Gln
   1265 1270 1275
   Leu Thr Gln Ile Lys Gln Thr Ser Leu Ala Arg Ile Leu Cys Asp
   1280 1285 1290
   Asn Ala Asp Asn Ile Thr Arg Val Gln Ser Asp Val Phe Arg Val
   1295 1300 1305
   Ala Glu Phe Pro His Gly Tyr Gly Ser Cys Asp Glu Ile Pro Arg
   1310 1315 1320
   Val Asp Leu Arg Val Trp Gln Asp Cys Cys Glu Asp Cys Arg Thr
   1325 1330 1335
   Arg Gly Gln Phe Asn Ala Phe Ser Tyr His Phe Arg Gly Arg Arg
   1340 1345 1350
   Ser Leu Glu Phe Ser Tyr Gln Glu Asp Lys Pro Thr Lys Lys Thr
   1355 1360 1365
   Arg Pro Arg Lys Ile Pro Ser Val Gly Arg Gln Gly Glu His Leu
   1370 1375 1380
   Ser Asn Ser Thr Ser Ala Phe Ser Thr Arg Ser Asp Ala Ser Gly
   1385 1390 1395
   Thr Asn Asp Phe Arg Glu Phe Val Leu Glu Met Gln Lys Thr Ile
   1400 1405 1410
   Thr Asp Leu Arg Thr Gln Ile Lys Lys Leu Glu Ser Arg Leu Ser
   1415 1420 1425
   Thr Thr Glu Cys Val Asp Ala Gly Gly Glu Ser His Ala Asn Asn
   1430 1435 1440
   Thr Lys Trp Lys Lys Asp Ala Cys Thr Ile Cys Glu Cys Lys Asp
   1445 1450 1455
   Gly Gln Val Thr Cys Phe Val Glu Ala Cys Pro Pro Ala Thr Cys
   1460 1465 1470
   Ala Val Pro Val Asn Ile Pro Gly Ala Cys Cys Pro Val Cys Leu
   1475 1480 1485
   Gln Lys Arg Ala Glu Glu Lys Pro
   1490 1495
<210> 34
   <211> 4491
   <212> DNA
   <213> Homo sapiens
<400> 34
   agccggccgt ggtggctccg tgcgtccgag cgtccgtccg cgccgtcggc catggccaag 60
   cgctccaggg gccccgggcg ccgctgcctg ttggcgctcg tgctgttctg cgcctggggg 120
   acgctggccg tggtggccca gaagccgggc gcagggtgtc cgagccgctg cctgtgcttc 180
   cgcaccaccg tgcgctgcat gcatctgctg ctggaggccg tgcccgccgt ggcgccgcag 240
   acctccatcc tagatcttcg ctttaacaga atcagagaga tccaacctgg ggcattcagg 300
   cggctgagga acttgaacac attgcttctc aataataatc agatcaagag gatacctagt 360
   ggagcatttg aagacttgga aaatttaaaa tatctctatc tgtacaagaa tgagatccag 420
   tcaattgaca ggcaagcatt taagggactt gcctctctag agcaactata cctgcacttt 480
   aatcagatag aaactttgga cccagattcg ttccagcatc tcccgaagct cgagaggcta 540
   tttttgcata acaaccggat tacacattta gttccaggga catttaatca cttggaatct 600
   atgaagagat tgcgactgga ctcaaacaca cttcactgcg actgtgaaat cctgtggttg 660
   gcggatttgc tgaaaaccta cgcggagtcg gggaacgcgc aggcagcggc catctgtgaa 720
   tatcccagac gcatccaggg acgctcagtg gcaaccatca ccccggaaga gctgaactgt 780
   gaaaggcccc ggatcacctc cgagccccag gacgcagatg tgacctcggg gaacaccgtg 840
   tacttcacct gcagagccga aggcaacccc aagcctgaga tcatctggct gcgaaacaat 900
   aatgagctga gcatgaagac agattcccgc ctaaacttgc tggacgatgg gaccctgatg 960
   atccagaaca cacaggagac agaccagggt atctaccagt gcatggcaaa gaacgtggcc 1020
   ggagaggtga agacgcaaga ggtgaccctc aggtacttcg ggtctccagc tcgacccact 1080
   tttgtaatcc agccacagaa tacagaggtg ctggttgggg agagcgtcac gctggagtgc 1140
   agcgccacag gccacccccc gccgcggatc tcctggacga gaggtgaccg cacacccttg 1200
   ccagttgacc cgcgggtgaa catcacgcct tctggcgggc tttacataca gaacgtcgta 1260
   cagggggaca gcggagagta tgcgtgctct gcgaccaaca acattgacag cgtccatgcc 1320
   accgctttca tcatcgtcca ggctcttcct cagttcactg tgacgcctca ggacagagtc 1380
   gttattgagg gccagaccgt ggatttccag tgtgaagcca agggcaaccc gccgcccgtc 1440
   atcgcctgga ccaagggagg gagccagctc tccgtggacc ggcggcacct ggtcctgtca 1500
   tcgggaacac ttagaatctc tggtgttgcc ctccacgacc agggccagta cgaatgccag 1560
   gctgtcaaca tcatcggctc ccagaaggtc gtggcccacc tgactgtgca gcccagagtc 1620
   accccagtgt ttgccagcat tcccagcgac acaacagtgg aggtgggcgc caatgtgcag 1680
   ctcccgtgca gctcccaggg cgagcccgag ccagccatca cctggaacaa ggatggggtt 1740
   caggtgacag aaagtggaaa atttcacatc agccctgaag gattcttgac catcaatgac 1800
   gttggccctg cagacgcagg tcgctatgag tgtgtggccc ggaacaccat tgggtcggcc 1860
   tcggtgagca tggtgctcag tgtgaacgtt cctgacgtca gtcgaaatgg agatccgttt 1920
   gtagctacct ccatcgtgga agcgattgcg actgttgaca gagctataaa ctcaacccga 1980
   acacatttgt ttgacagccg tcctcgttct ccaaatgatt tgctggcctt gttccggtat 2040
   ccgagggatc cttacacagt tgaacaggca cgggcgggag aaatctttga acggacattg 2100
   cagctcattc aggagcatgt acagcatggc ttgatggtcg acctcaacgg aacaagttac 2160
   cactacaacg acctggtgtc tccacagtac ctgaacctca tcgcaaacct gtcgggctgt 2220
   accgcccacc ggcgcgtgaa caactgctcg gacatgtgct tccaccagaa gtaccggacg 2280
   cacgacggca cctgtaacaa cctgcagcac cccatgtggg gcgcctcgct gaccgccttc 2340
   gagcgcctgc tgaaatccgt gtacgagaat ggcttcaaca cccctcgggg catcaacccc 2400
   caccgactgt acaacgggca cgcccttccc atgccgcgcc tggtgtccac caccctgatc 2460
   gggacggaga ccgtcacacc cgacgagcag ttcacccaca tgctgatgca gtggggccag 2520
   ttcctggacc acgacctcga ctccacggtg gtggccctga gccaggcacg cttctccgac 2580
   ggacagcact gcagcaacgt gtgcagcaac gaccccccct gcttctctgt catgatcccc 2640
   cccaatgact cccgggccag gagcggggcc cgctgcatgt tcttcgtgcg ctccagccct 2700
   gtgtgcggca gcggcatgac ttcgctgctc atgaactccg tgtacccgcg ggagcagatc 2760
   aaccagctca cctcctacat cgacgcatcc aacgtgtacg ggagcacgga gcatgaggcc 2820
   cgcagcatcc gcgacctggc cagccaccgc ggcctgctgc ggcagggcat cgtgcagcgg 2880
   tccgggaagc cgctgctccc cttcgccacc gggccgccca cggagtgcat gcgggacgag 2940
   aacgagagcc ccatcccctg cttcctggcc ggggaccacc gcgccaacga gcagctgggc 3000
   ctgaccagca tgcacacgct gtggttccgc gagcacaacc gcattgccac ggagctgctc 3060
   aagctgaacc cgcactggga cggcgacacc atctactatg agaccaggaa gatcgtgggt 3120
   gcggagatcc agcacatcac ctaccagcac tggctcccga agatcctggg ggaggtgggc 3180
   atgaggacgc tgggagagta ccacggctac gaccccggca tcaatgctgg catcttcaac 3240
   gccttcgcca ccgcggcctt caggtttggc cacacgcttg tcaacccact gctttaccgg 3300
   ctggacgaga acttccagcc cattgcacaa gatcacctcc cccttcacaa agctttcttc 3360
   tctcccttcc ggattgtgaa tgagggcggc atcgatccgc ttctcagggg gctgttcggg 3420
   gtggcgggga aaatgcgtgt gccctcgcag ctgctgaaca cggagctcac ggagcggctg 3480
   ttctccatgg cacacacggt ggctctggac ctggcggcca tcaacatcca gcggggccgg 3540
   gaccacggga tcccacccta ccacgactac agggtctact gcaatctatc ggcggcacac 3600
   acgttcgagg acctgaaaaa tgagattaaa aaccctgaga tccgggagaa actgaaaagg 3660
   ttgtatggct cgacactcaa catcgacctg tttccggcgc tcgtggtgga ggacctggtg 3720
   cctggcagcc ggctgggccc caccctgatg tgtcttctca gcacacagtt caagcgcctg 3780
   cgagatgggg acaggttgtg gtatgagaac cctggggtgt tctccccggc ccagctgact 3840
   cagatcaagc agacgtcgct ggccaggatc ctatgcgaca acgcggacaa catcacccgg 3900
   gtgcagagcg acgtgttcag ggtggcggag ttccctcacg gctacggcag ctgtgacgag 3960
   atccccaggg tggacctccg ggtgtggcag gactgctgtg aagactgtag gaccaggggg 4020
   cagttcaatg ccttttccta tcatttccga ggcagacggt ctcttgagtt cagctaccag 4080
   gaggacaagc cgaccaagaa aacaagacca cggaaaatac ccagtgttgg gagacagggg 4140
   gaacatctca gcaacagcac ctcagccttc agcacacgct cagatgcatc tgggacaaat 4200
   gacttcagag agtttgttct ggaaatgcag aagaccatca cagacctcag aacacagata 4260
   aagaaacttg aatcacggct cagtaccaca gagtgcgtgg atgccggggg cgaatctcac 4320
   gccaacaaca ccaagtggaa aaaagatgca tgcaccattt gtgaatgcaa agacgggcag 4380
   gtcacctgct tcgtggaagc ttgcccccct gccacctgtg ctgtccccgt gaacatccca 4440
   ggggcctgct gtccagtctg cttacagaag agggcggagg aaaagcccta g 4491
<210> 35
   <211> 613
   <212> PRT
   <213> Homo sapiens
<400> 35
   Met Arg Ala Pro Gly Ala Leu Leu Ala Arg Met Ser Arg Leu Leu Leu
   1               5                   10                  15
   Leu Leu Leu Leu Lys Val Ser Ala Ser Ser Ala Leu Gly Val Ala Pro
               20                  25                  30
   Ala Ser Arg Asn Glu Thr Cys Leu Gly Glu Ser Cys Ala Pro Thr Val
           35                  40                  45
   Ile Gln Arg Arg Gly Arg Asp Ala Trp Gly Pro Gly Asn Ser Ala Arg
       50                  55                  60
   Asp Val Leu Arg Ala Arg Ala Pro Arg Glu Glu Gln Gly Ala Ala Phe
   65                  70                  75                  80
   Leu Ala Gly Pro Ser Trp Asp Leu Pro Ala Ala Pro Gly Arg Asp Pro
                   85                  90                  95
   Ala Ala Gly Arg Gly Ala Glu Ala Ser Ala Ala Gly Pro Pro Gly Pro
               100                 105                 110
   Pro Thr Arg Pro Pro Gly Pro Trp Arg Trp Lys Gly Ala Arg Gly Gln
           115                 120                 125
   Glu Pro Ser Glu Thr Leu Gly Arg Gly Asn Pro Thr Ala Leu Gln Leu
       130                 135                 140
   Phe Leu Gln Ile Ser Glu Glu Glu Glu Lys Gly Pro Arg Gly Ala Gly
   145 150 155 160
   Ile Ser Gly Arg Ser Gln Glu Gln Ser Val Lys Thr Val Pro Gly Ala
   165 170 175
   Ser Asp Leu Phe Tyr Trp Pro Arg Arg Ala Gly Lys Leu Gln Gly Ser
   180 185 190
   His His Lys Pro Leu Ser Lys Thr Ala Asn Gly Leu Ala Gly His Glu
   195 200 205
   Gly Trp Thr Ile Ala Leu Pro Gly Arg Ala Leu Ala Gln Asn Gly Ser
   210 215 220
   Leu Gly Glu Gly Ile His Glu Pro Gly Gly Pro Arg Arg Gly Asn Ser
   225 230 235 240
   Thr Asn Arg Arg Val Arg Leu Lys Asn Pro Phe Tyr Pro Leu Thr Gln
   245 250 255
   Glu Ser Tyr Gly Ala Tyr Ala Val Met Cys Leu Ser Val Val Ile Phe
   260 265 270
   Gly Thr Gly Ile Ile Gly Asn Leu Ala Val Met Cys Ile Val Cys His
   275 280 285
   Asn Tyr Tyr Met Arg Ser Ile Ser Asn Ser Leu Leu Ala Asn Leu Ala
   290 295 300
   Phe Trp Asp Phe Leu Ile Ile Phe Phe Cys Leu Pro Leu Val Ile Phe
   305 310 315 320
   His Glu Leu Thr Lys Lys Trp Leu Leu Glu Asp Phe Ser Cys Lys Ile
   325 330 335
   Val Pro Tyr Ile Glu Val Ala Ser Leu Gly Val Thr Thr Phe Thr Leu
   340 345 350
   Cys Ala Leu Cys Ile Asp Arg Phe Arg Ala Ala Thr Asn Val Gln Met
   355 360 365
   Tyr Tyr Glu Met Ile Glu Asn Cys Ser Ser Thr Thr Ala Lys Leu Ala
   370 375 380
   Val Ile Trp Val Gly Ala Leu Leu Leu Ala Leu Pro Glu Val Val Leu
   385 390 395 400
   Arg Gln Leu Ser Lys Glu Asp Leu Gly Phe Ser Gly Arg Ala Pro Ala
   405 410 415
   Glu Arg Cys Ile Ile Lys Ile Ser Pro Asp Leu Pro Asp Thr Ile Tyr
   420 425 430
   Val Leu Ala Leu Thr Tyr Asp Ser Ala Arg Leu Trp Trp Tyr Phe Gly
   435 440 445
   Cys Tyr Phe Cys Leu Pro Thr Leu Phe Thr Ile Thr Cys Ser Leu Val
   450 455 460
   Thr Ala Arg Lys Ile Arg Lys Ala Glu Lys Ala Cys Thr Arg Gly Asn
   465 470 475 480
   Lys Arg Gln Ile Gln Leu Glu Ser Gln Met Asn Cys Thr Val Val Ala
   485 490 495
   Leu Thr Ile Leu Tyr Gly Phe Cys Ile Ile Pro Glu Asn Ile Cys Asn
   500 505 510
   Ile Val Thr Ala Tyr Met Ala Thr Gly Val Ser Gln Gln Thr Met Asp
   515 520 525
   Leu Leu Asn Ile Ile Ser Gln Phe Leu Leu Phe Phe Lys Ser Cys Val
   530 535 540
   Thr Pro Val Leu Leu Phe Cys Leu Cys Lys Pro Phe Ser Arg Ala Phe
   545 550 555 560
   Met Glu Cys Cys Cys Cys Cys Cys Glu Glu Cys Ile Gln Lys Ser Ser
   565 570 575
   Thr Val Thr Ser Asp Asp Asn Asp Asn Glu Tyr Thr Thr Glu Leu Glu
   580 585 590
   Leu Ser Pro Phe Ser Thr Ile Arg Arg Glu Met Ser Thr Phe Ala Ser
   595 600 605
   Val Gly Thr His Cys
   610
<210> 36
   <211> 1842
   <212> DNA
   <213> Homo sapiens
<400> 36
   atgcgagccc cgggcgcgct tctcgcccgc atgtcgcggc tactgcttct gctactgctc 60
   aaggtgtctg cctcttctgc cctcggggtc gcccctgcgt ccagaaacga aacttgtctg 120
   ggggagagct gtgcacctac agtgatccag cgccgcggca gggacgcctg gggaccggga 180
   aattctgcaa gagacgttct gcgagcccga gcacccaggg aggagcaggg ggcagcgttt 240
   cttgcgggac cctcctggga cctgccggcg gccccgggcc gtgacccggc tgcaggcaga 300
   ggggcggagg cgtcggcagc cggacccccg ggacctccaa ccaggccacc tggcccctgg 360
   aggtggaaag gtgctcgggg tcaggagcct tctgaaactt tggggagagg gaaccccacg 420
   gccctccagc tcttccttca gatctcagag gaggaagaga agggtcccag aggcgctggc 480
   atttccgggc gtagccagga gcagagtgtg aagacagtcc ccggagccag cgatcttttt 540
   tactggccaa ggagagccgg gaaactccag ggttcccacc acaagcccct gtccaagacg 600
   gccaatggac tggcggggca cgaagggtgg acaattgcac tcccgggccg ggcgctggcc 660
   cagaatggat ccttgggtga aggaatccat gagcctgggg gtccccgccg gggaaacagc 720
   acgaaccggc gtgtgagact gaagaacccc ttctacccgc tgacccagga gtcctatgga 780
   gcctacgcgg tcatgtgtct gtccgtggtg atcttcggga ccggcatcat tggcaacctg 840
   gcggtgatgt gcatcgtgtg ccacaactac tacatgcgga gcatctccaa ctccctcttg 900
   gccaacctgg ccttctggga ctttctcatc atcttcttct gccttccgct ggtcatcttc 960
   cacgagctga ccaagaagtg gctgctggag gacttctcct gcaagatcgt gccctatata 1020
   gaggtcgctt ctctgggagt caccactttc accttatgtg ctctgtgcat agaccgcttc 1080
   cgtgctgcca ccaacgtaca gatgtactac gaaatgatcg aaaactgttc ctcaacaact 1140
   gccaaacttg ctgttatatg ggtgggagct ctattgttag cacttccaga agttgttctc 1200
   cgccagctga gcaaggagga tttggggttt agtggccgag ctccggcaga aaggtgcatt 1260
   attaagatct ctcctgattt accagacacc atctatgttc tagccctcac ctacgacagt 1320
   gcgagactgt ggtggtattt tggctgttac ttttgtttgc ccacgctttt caccatcacc 1380
   tgctctctag tgactgcgag gaaaatccgc aaagcagaga aagcctgtac ccgagggaat 1440
   aaacggcaga ttcaactaga gagtcagatg aactgtacag tagtggcact gaccatttta 1500
   tatggatttt gcattattcc tgaaaatatc tgcaacattg ttactgccta catggctaca 1560
   ggggtttcac agcagacaat ggacctcctt aatatcatca gccagttcct tttgttcttt 1620
   aagtcctgtg tcaccccagt cctccttttc tgtctctgca aacccttcag tcgggccttc 1680
   atggagtgct gctgctgttg ctgtgaggaa tgcattcaga agtcttcaac ggtgaccagt 1740
   gatgacaatg acaacgagta caccacggaa ctcgaactct cgcctttcag taccatacgc 1800
   cgtgaaatgt ccacttttgc ttctgtcgga actcattgct ga 1842
<210> 37
   <211> 1471
   <212> PRT
   <213> Homo sapiens
<400> 37
   Pro Glu Met Thr Gly Val Thr Val Ser Gly Leu Thr Pro Ala Arg Thr
   1               5                   10                  15
   Tyr Gln Phe Arg Val Cys Ala Val Asn Glu Val Gly Arg Gly Gln Tyr
               20                  25                  30
   Ser Ala Glu Thr Ser Arg Leu Met Leu Pro Glu Glu Pro Pro Ser Ala
   35 40 45
   Pro Pro Lys Asn Ile Val Ala Ser Gly Arg Thr Asn Gln Ser Ile Met
   50 55 60
   Val Gln Trp Gln Pro Pro Pro Glu Thr Glu His Asn Gly Val Leu Arg
   65 70 75 80
   Gly Tyr Ile Leu Arg Tyr Arg Leu Ala Gly Leu Pro Gly Glu Tyr Gln
   85 90 95
   Gln Arg Asn Ile Thr Ser Pro Glu Val Asn Tyr Cys Leu Val Thr Asp
   100 105 110
   Leu Ile Ile Trp Thr Gln Tyr Glu Ile Gln Val Ala Ala Tyr Asn Gly
   115 120 125
   Ala Gly Leu Gly Val Phe Ser Arg Ala Val Thr Glu Tyr Thr Leu Gln
   130 135 140
   Gly Val Pro Thr Ala Pro Pro Gln Asn Val Gln Thr Glu Ala Val Asn
   145 150 155 160
   Ser Thr Thr Ile Gln Phe Leu Trp Asn Pro Pro Pro Gln Gln Phe Ile
   165 170 175
   Asn Gly Ile Asn Gln Gly Tyr Lys Leu Leu Ala Trp Pro Ala Asp Ala
   180 185 190
   Pro Glu Ala Val Thr Val Val Thr Ile Ala Pro Asp Phe His Gly Val
   195 200 205
   His His Gly His Ile Thr Asn Leu Lys Lys Phe Thr Ala Tyr Phe Thr
   210 215 220
   Ser Val Leu Cys Phe Thr Thr Pro Gly Asp Gly Pro Pro Ser Thr Pro
   225 230 235 240
   Gln Leu Val Trp Thr Gln Glu Asp Lys Pro Gly Ala Val Gly His Leu
   245 250 255
   Ser Phe Thr Glu Ile Leu Asp Thr Ser Leu Lys Val Ser Trp Gln Glu
   260 265 270
   Pro Leu Glu Lys Asn Gly Ile Ile Thr Gly Tyr Gln Ile Ser Trp Glu
   275 280 285
   Val Tyr Gly Arg Asn Asn Ser Arg Leu Thr His Thr Leu Asn Ser Thr
   290 295 300
   Thr His Glu Tyr Lys Ile Gln Gly Leu Ser Ser Leu Thr Thr Tyr Thr
   305 310 315 320
   Ile Asp Val Ala Ala Val Thr Ala Val Gly Thr Gly Leu Val Thr Ser
   325 330 335
   Ser Thr Ile Ser Ser Gly Val Pro Pro Asp Leu Pro Gly Ala Pro Ser
   340 345 350
   Asn Leu Val Ile Ser Asn Ile Ser Pro Arg Ser Ala Thr Leu Gln Phe
   355 360 365
   Arg Pro Gly Tyr Asp Gly Lys Thr Ser Ile Ser Arg Trp Ile Val Glu
   370 375 380
   Gly Gln Val Gly Ala Ile Gly Asp Glu Glu Glu Trp Val Thr Leu Tyr
   385 390 395 400
   Glu Glu Glu Asn Glu Pro Asp Ala Gln Met Leu Glu Ile Pro Asn Leu
   405 410 415
   Thr Pro Tyr Thr His Tyr Arg Phe Arg Met Lys Gln Val Asn Ile Val
   420 425 430
   Gly Pro Ser Pro Tyr Ser Pro Ser Ser Arg Val Ile Gln Thr Leu Gln
   435 440 445
   Ala Pro Pro Asp Val Ala Pro Thr Ser Val Thr Val Arg Thr Ala Ser
   450 455 460
   Glu Thr Ser Leu Arg Leu Arg Trp Val Pro Leu Pro Asp Ser Gln Tyr
   465 470 475 480
   Asn Gly Asn Pro Glu Ser Val Gly Tyr Arg Ile Lys Tyr Trp Arg Ser
   485 490 495
   Asp Leu Gln Ser Ser Ala Val Ala Gln Val Val Ser Asp Arg Leu Glu
   500 505 510
   Arg Glu Phe Thr Ile Glu Glu Leu Glu Glu Trp Met Glu Tyr Glu Leu
   515 520 525
   Gln Met Gln Ala Phe Asn Ala Val Gly Ala Gly Pro Trp Ser Glu Val
   530 535 540
   Val Arg Gly Arg Thr Arg Glu Ser Val Pro Ser Ala Ala Pro Glu Asn
   545 550 555 560
   Val Ser Ala Glu Ala Val Ser Ser Thr Gln Ile Leu Leu Thr Trp Thr
   565 570 575
   Ser Val Pro Glu Gln Asp Gln Asn Gly Leu Ile Leu Gly Tyr Lys Ile
   580 585 590
   Leu Phe Arg Ala Lys Asp Leu Asp Pro Glu Pro Arg Ser His Ile Val
   595 600 605
   Arg Gly Asn His Thr Gln Ser Ala Leu Leu Ala Gly Leu Arg Lys Phe
   610 615 620
   Val Leu Tyr Glu Leu Gln Val Leu Ala Phe Thr Arg Ile Gly Asn Gly
   625 630 635 640
   Val Pro Ser Thr Pro Leu Ile Leu Glu Arg Thr Lys Asp Asp Ala Pro
   645 650 655
   Gly Pro Pro Val Arg Leu Val Phe Pro Glu Val Arg Leu Thr Ser Val
   660 665 670
   Arg Ile Val Trp Gln Pro Pro Glu Glu Pro Asn Gly Ile Ile Leu Gly
   675 680 685
   Tyr Gln Ile Ala Tyr Arg Leu Ala Ser Ser Ser Pro His Thr Phe Thr
   690 695 700
   Thr Val Glu Val Gly Ala Thr Val Arg Gln Phe Thr Ala Thr Asp Leu
   705 710 715 720
   Ala Pro Glu Ser Ala Tyr Ile Phe Arg Leu Ser Ala Lys Thr Arg Gln
   725 730 735
   Gly Trp Gly Glu Pro Leu Glu Ala Thr Val Ile Thr Thr Glu Lys Arg
   740 745 750
   Glu Arg Pro Ala Pro Pro Arg Glu Leu Leu Val Pro Gln Ala Glu Val
   755 760 765
   Thr Ala Arg Ser Leu Arg Leu Gln Trp Val Pro Gly Ser Asp Gly Ala
   770 775 780
   Ser Pro Ile Arg Tyr Phe Thr Met Gln Val Arg Glu Leu Pro Arg Gly
   785 790 795 800
   Glu Trp Gln Thr Tyr Ser Ser Ser Ile Ser His Glu Ala Thr Ala Cys
   805 810 815
   Val Val Asp Arg Leu Arg Pro Phe Thr Ser Tyr Lys Leu Arg Leu Lys
   820 825 830
   Ala Thr Asn Asp Ile Gly Asp Ser Asp Phe Ser Ser Glu Thr Glu Ala
   835 840 845
   Val Thr Thr Leu Gln Asp Val Pro Gly Glu Pro Pro Gly Ser Val Ser
   850 855 860
   Ala Thr Pro His Thr Thr Ser Ser Val Leu Ile Gln Trp Gln Pro Pro
   865 870 875 880
   Arg Asp Glu Ser Leu Asn Gly Leu Leu Gln Gly Tyr Arg Ile Tyr Tyr
   885 890 895
   Arg Glu Leu Glu Tyr Glu Ala Gly Ser Gly Thr Glu Ala Lys Thr Leu
   900 905 910
   Lys Asn Pro Ile Ala Leu Arg Ala Glu Leu Thr Asp Leu Lys Lys Tyr
   915 920 925
   Arg Arg Tyr Glu Val Ile Met Thr Ala Tyr Asn Ile Ile Gly Glu Ser
   930 935 940
   Pro Ala Ser Ala Pro Val Glu Val Phe Val Gly Glu Ala Ala Pro Ala
   945 950 955 960
   Met Ala Pro Gln Asn Val Gln Val Thr Pro Leu Thr Ala Ser Gln Leu
   965 970 975
   Glu Val Thr Trp Asp Pro Pro Pro Pro Glu Ser Gln Asn Gly Asn Ile
   980 985 990
   Gln Gly Tyr Lys Ile Tyr Tyr Trp Glu Ala Asp Ser Gln Asn Glu Thr
   995 1000 1005
   Glu Lys Met Lys Val Leu Phe Leu Pro Glu Pro Val Val Arg Leu
   1010 1015 1020
   Lys Asn Leu Thr Ser His Thr Lys Tyr Leu Val Ser Ile Ser Ala
   1025 1030 1035
   Phe Asn Ala Ala Gly Asp Gly Pro Lys Ser Asp Pro Gln Gln Gly
   1040 1045 1050
   Arg Thr His Gln Ala Ala Pro Gly Ala Pro Ser Phe Leu Ala Phe
   1055 1060 1065
   Ser Glu Ile Thr Ser Thr Thr Leu Asn Val Ser Trp Gly Glu Pro
   1070 1075 1080
   Ala Ala Ala Asn Gly Ile Leu Gln Gly Tyr Arg Val Val Tyr Glu
   1085 1090 1095
   Pro Leu Ala Pro Val Gln Gly Val Ser Lys Val Val Thr Val Glu
   1100 1105 1110
   Val Arg Gly Asn Trp Gln Arg Trp Leu Lys Val Arg Asp Leu Thr
   1115 1120 1125
   Lys Gly Val Thr Tyr Phe Phe Arg Val Gln Ala Arg Thr Ile Thr
   1130 1135 1140
   Tyr Gly Pro Glu Leu Gln Ala Asn Ile Thr Ala Gly Pro Ala Glu
   1145 1150 1155
   Gly Ser Pro Gly Ser Pro Arg Asp Val Leu Val Thr Lys Ser Ala
   1160 1165 1170
   Ser Glu Leu Thr Leu Gln Trp Thr Glu Gly His Ser Gly Asp Thr
   1175 1180 1185
   Pro Thr Thr Gly Tyr Val Ile Glu Ala Arg Pro Ser Asp Glu Gly
   1190 1195 1200
   Leu Trp Asp Met Phe Val Lys Asp Ile Pro Arg Ser Ala Thr Ser
   1205 1210 1215
   Tyr Thr Leu Ser Leu Asp Lys Leu Arg Gln Gly Val Thr Tyr Glu
   1220 1225 1230
   Phe Arg Val Val Ala Val Asn Glu Ala Gly Tyr Gly Glu Pro Ser
   1235 1240 1245
   Asn Pro Ser Thr Ala Val Ser Ala Gln Val Glu Ala Pro Phe Tyr
   1250 1255 1260
   Glu Glu Trp Trp Phe Leu Leu Val Met Ala Leu Ser Ser Leu Ile
   1265 1270 1275
   Val Ile Leu Leu Val Val Phe Ala Leu Val Leu His Gly Gln Asn
   1280 1285 1290
   Lys Lys Tyr Lys Asn Cys Ser Thr Gly Lys Gly Ile Ser Thr Met
   1295 1300 1305
   Glu Glu Ser Val Thr Leu Asp Asn Gly Gly Phe Ala Ala Leu Glu
   1310 1315 1320
   Leu Ser Ser Arg His Leu Asn Val Lys Ser Thr Phe Ser Lys Lys
   1325 1330 1335
   Asn Gly Thr Arg Ser Pro Pro Arg Pro Ser Pro Gly Gly Leu His
   1340 1345 1350
   Tyr Ser Asp Glu Asp Ile Cys Asn Lys Tyr Asn Gly Ala Val Leu
   1355 1360 1365
   Thr Glu  Ser Val Ser Leu Lys  Glu Lys Ser Ala Asp  Ala Ser Glu
       1370                 1375                 1380
   Ser Glu  Ala Thr Asp Ser Asp  Tyr Glu Asp Ala Leu  Pro Lys His
       1385                 1390                 1395
   Ser Phe  Val Asn His Tyr Met  Ser Asp Pro Thr Tyr  Tyr Asn Ser
       1400                 1405                 1410
   Trp Lys  Arg Arg Ala Gln Gly  Arg Ala Pro Ala Pro  His Arg Tyr
       1415                 1420                 1425
   Glu Ala  Val Ala Gly Ser Glu  Ala Gly Ala Gln Leu  His Pro Val
       1430                 1435                 1440
   Ile Thr  Thr Gln Ser Ala Gly  Gly Val Tyr Thr Pro  Ala Gly Pro
       1445                 1450                 1455
   Gly Ala  Arg Thr Pro Leu Thr  Gly Phe Ser Ser Phe  Val
       1460                 1465                 1470
<210> 38
   <211> 4417
   <212> DNA
   <213> Homo sapiens
<400> 38
   ccctgagatg acaggcgtca ccgtgagtgg cctgactccg gctcgtacct atcaattccg 60
   ggtgtgcgcg gtgaatgaag tgggcagggg ccagtacagt gccgagacaa gcaggttgat 120
   gctacctgaa gaaccaccca gtgctccccc gaaaaatata gtggccagtg ggcggactaa 180
   tcagtccatt atggtccagt ggcagccacc cccagaaaca gagcacaacg gggtgttgcg 240
   tggatacatc ctcaggtacc gcctggctgg ccttcccgga gagtaccagc agcggaacat 300
   caccagcccg gaggtgaact actgcctggt gacagacctg atcatctgga cacagtatga 360
   gatacaggtg gcggcgtaca acggggccgg tctgggcgtc ttcagcaggg cagtgaccga 420
   gtacaccttg cagggagtgc ccaccgcgcc cccgcagaac gtgcagacgg aagccgtgaa 480
   ctccaccacc attcagttcc tgtggaaccc tccgcctcag cagtttatca atggcatcaa 540
   ccagggatac aagcttctgg catggccggc agatgccccc gaggctgtca ctgtggtcac 600
   tattgcccca gatttccacg gagtccacca tggacacata acgaacctga agaagtttac 660
   cgcctacttc acttccgttc tgtgcttcac cacccctggg gacgggcctc ccagcacacc 720
   tcagctggtc tggactcagg aagacaaacc aggagctgtg ggacatctga gtttcacaga 780
   gatcttggac acatctctca aggtcagctg gcaggagccc ctggagaaaa atggcatcat 840
   tactggctat cagatctctt gggaagtgta cggcaggaac aactctcgtc tcacgcacac 900
   cctgaacagc acgacgcacg agtacaagat ccaaggcctc tcatctctca ccacctacac 960
   catcgacgtg gccgctgtga ctgccgtggg cactggcctg gtgacttcat ccaccatttc 1020
   ttctggagtg cccccagacc ttcctggtgc cccatccaac ctggtcattt ccaacatcag 1080
   ccctcgctcc gccacccttc agttccggcc aggctatgac gggaaaacgt ccatctccag 1140
   gtggattgtt gaggggcagg tgggagctat cggcgacgag gaggagtggg tcaccctcta 1200
   tgaagaggag aatgagcctg atgcccagat gctggagatc ccaaacctca caccctacac 1260
   tcactacaga tttcgaatga agcaagtgaa cattgttggg ccgagcccct acagtccgtc 1320
   ttcccgggtc atccagaccc tgcaggcccc acccgacgtg gctccaacca gcgtcacggt 1380
   ccgtactgcc agtgagacca gcctgcggct tcgctgggtg cccctgccgg attctcagta 1440
   caacgggaac cccgagtccg tgggctacag gattaagtac tggcgctcag acctccagtc 1500
   ctcagcagtg gcccaagtcg tcagtgaccg gctggagaga gaattcacca tcgaggagct 1560
   ggaggagtgg atggaatacg agctgcagat gcaggccttc aacgccgtcg ggctgggccc 1620
   gtggagcgag gtggtgcggg gccggacgcg ggagtcagtt ccttcagccg cccctgagaa 1680
   cgtgtcagcc gaggctgtca gctcgaccca gattttactg acatggacat ccgtgccgga 1740
   acaggaccag aatgggctca tactgggcta caagatcctg ttccgggcca aagacctgga 1800
   tcccgagccc aggagccaca tcgtgcgagg gaaccacacg cagtcggccc tgctggcagg 1860
   cctgcgcaag ttcgtgctct acgagctcca ggtgctggcg ttcacccgca tcgggaacgg 1920
   ggtccccagc acgcccctca tcctggagcg caccaaagac gatgccccag gcccaccagt 1980
   gaggctcgtg ttccccgaag tgagactcac ctctgtgcgg atagtgtggc aacctccgga 2040
   ggagcccaac ggcatcatcc tggggtacca gattgcctac cgcctggcca gcagcagccc 2100
   ccacaccttc accaccgtgg aggtcggcgc cacagtgagg cagttcacag ccaccgacct 2160
   ggccccggag tccgcataca tcttcaggct gtccgccaag acgaggcagg gctgggggga 2220
   gccactggag gccaccgtca tcaccaccga gaagagagag cggccggcac cccccagaga 2280
   gctcctggtg ccccaggcag aagtgaccgc acgcagcctc cggctccagt gggtcccggg 2340
   cagcgacggg gcctccccca tccggtactt caccatgcag gtgcgagagc tgcctcgggg 2400
   tgagtggcag acctactcct cgtccatcag ccatgaggcc acagcatgcg tcgttgacag 2460
   actgaggccc ttcacctcct acaagctgcg cctgaaagcc accaacgaca ttggggacag 2520
   tgacttcagt tcagagacag aggcggtgac cacgctgcag gatgttccag gagagccccc 2580
   gggatctgtc tcagcgacgc cacacaccac gtcctctgtc ctgatacagt ggcagcctcc 2640
   gagggacgaa agcctgaacg gccttcttca gggatacagg atctactaca gggagctgga 2700
   gtatgaagcc gggtcaggca ccgaggccaa gacgctcaaa aaccctatag ctttacgtgc 2760
   tgagctcaca gatttaaaga agtaccggcg ctatgaagta ataatgaccg cctataacat 2820
   catcggcgag agcccagcca gcgcgcccgt ggaggtcttt gtcggcgagg ctgccccggc 2880
   catggccccg cagaacgtgc aggtgacccc actcacggcc agccagctgg aggtcacgtg 2940
   ggacccacca cccccggaga gccagaatgg gaacatccaa ggctacaaga tttactactg 3000
   ggaggcagac agccagaacg aaacggagaa aatgaaggtc ctcttcctcc ccgagcccgt 3060
   ggtgaggctg aagaacctga ccagccatac caagtacctg gtcagcatat cagccttcaa 3120
   cgccgccgga gatggaccta agagtgaccc ccagcagggg cgcacccacc aggccgcccc 3180
   tggggccccc agctttctgg cgttctcaga aataacctcc accacgctca acgtgtcctg 3240
   gggcgagcct gcggcggcca acggcatcct gcagggctat cgggtggtgt acgagccctt 3300
   ggcccctgta caaggggtga gcaaggtggt gaccgtggaa gtgagaggga actggcagcg 3360
   ctggctgaag gtgcgggacc tcaccaaggg agtgacctat ttcttccgtg tccaagcgcg 3420
   gaccatcacc tacgggcccg agctccaagc caatatcaca gccgggccag ccgagggatc 3480
   cccgggctcg cctagagatg tcctggtcac caagtccgcc tctgaactga cgctgcagtg 3540
   gactgaggga cactctggcg acacacctac cacgggctat gtgatcgagg cccggccctc 3600
   agatgaaggc ttatgggaca tgtttgtgaa ggacatcccg cggagcgcca catcctacac 3660
   cctcagcctg gataagctcc ggcaaggagt gacttacgag ttccgggtgg tggctgtgaa 3720
   tgaggcgggc tacggggagc ccagcaaccc ctccacggct gtgtcagctc aagtggaagc 3780
   cccattctac gaggagtggt ggttcctcct ggtgatggct ctgtccagcc tgatcgtcat 3840
   cctgctggtg gtgttcgccc tcgtcctgca cgggcagaat aagaagtata agaactgcag 3900
   cacaggaaag gggatctcca ccatggagga gtctgtgacc ctggacaacg gaggatttgc 3960
   tgccctggag ctcagcagcc gccacctcaa tgtcaagagc accttctcca agaagaacgg 4020
   gaccaggtcc ccaccccggc ctagccccgg cggcctgcac tactcagacg aggacatctg 4080
   caacaagtac aacggcgccg tgctgaccga gagcgtgagc ctcaaggaga agtcggcaga 4140
   tgcatcagaa tctgaggcca cggactctga ctacgaggac gcgctgccca agcactcctt 4200
   cgtgaaccac tacatgagcg accccaccta ctacaactca tggaagcgca gggcccaggg 4260
   ccgcgcacct gcgccgcaca ggtacgaggc ggtggcgggc tccgaggcgg gcgcgcagct 4320
   gcacccggtc atcaccacgc agagcgcggg cggcgtctac acccccgctg gccccggcgc 4380
   gcgaactccg ctcaccggct tctcctcctt cgtgtga 4417
<210> 39
   <211> 2506
   <212> PRT
   <213> Homo sapiens
<400> 39
   Met Ala Arg Phe Gly Asp Glu Met Pro Ala Arg Tyr Gly Gly Gly Gly
   1 5 10 15
   Ser Gly Ala Ala Ala Gly Val Val Val Gly Ser Gly Gly Gly Arg Gly
   20 25 30
   Ala Gly Gly Ser Arg Gln Gly Gly Gln Pro Gly Ala Gln Arg Met Tyr
   35 40 45
   Lys Gln Ser Met Ala Gln Arg Ala Arg Thr Met Ala Leu Tyr Asn Pro
   50 55 60
   Ile Pro Val Arg Gln Asn Cys Leu Thr Val Asn Arg Ser Leu Phe Leu
   65 70 75 80
   Phe Ser Glu Asp Asn Val Val Arg Lys Tyr Ala Lys Lys Ile Thr Glu
   85 90 95
   Trp Pro Pro Phe Glu Tyr Met Ile Leu Ala Thr Ile Ile Ala Asn Cys
   100 105 110
   Ile Val Leu Ala Leu Glu Gln His Leu Pro Asp Asp Asp Lys Thr Pro
   115 120 125
   Met Ser Glu Arg Leu Asp Asp Thr Glu Pro Tyr Phe Ile Gly Ile Phe
   130 135 140
   Cys Phe Glu Ala Gly Ile Lys Ile Ile Ala Leu Gly Phe Ala Phe His
   145 150 155 160
   Lys Gly Ser Tyr Leu Arg Asn Gly Trp Asn Val Met Asp Phe Val Val
   165 170 175
   Val Leu Thr Gly Ile Leu Ala Thr Val Gly Thr Glu Phe Asp Leu Arg
   180 185 190
   Thr Leu Arg Ala Val Arg Val Leu Arg Pro Leu Lys Leu Val Ser Gly
   195 200 205
   Ile Pro Ser Leu Gln Val Val Leu Lys Ser Ile Met Lys Ala Met Ile
   210 215 220
   Pro Leu Leu Gln Ile Gly Leu Leu Leu Phe Phe Ala Ile Leu Ile Phe
   225 230 235 240
   Ala Ile Ile Gly Leu Glu Phe Tyr Met Gly Lys Phe His Thr Thr Cys
   245 250 255
   Phe Glu Glu Gly Thr Asp Asp Ile Gln Gly Glu Ser Pro Ala Pro Cys
   260 265 270
   Gly Thr Glu Glu Pro Ala Arg Thr Cys Pro Asn Gly Thr Lys Cys Gln
   275 280 285
   Pro Tyr Trp Glu Gly Pro Asn Asn Gly Ile Thr Gln Phe Asp Asn Ile
   290 295 300
   Leu Phe Ala Val Leu Thr Val Phe Gln Cys Ile Thr Met Glu Gly Trp
   305 310 315 320
   Thr Asp Leu Leu Tyr Asn Ser Asn Asp Ala Ser Gly Asn Thr Trp Asn
   325 330 335
   Trp Leu Tyr Phe Ile Pro Leu Ile Ile Ile Gly Ser Phe Phe Met Leu
   340 345 350
   Asn Leu Val Leu Gly Val Leu Ser Gly Glu Phe Ala Lys Glu Arg Glu
   355 360 365
   Arg Val Glu Asn Arg Arg Ala Phe Leu Lys Leu Arg Arg Gln Gln Gln
   370 375 380
   Ile Glu Arg Glu Leu Asn Gly Tyr Met Glu Trp Ile Ser Lys Ala Glu
   385 390 395 400
   Glu Val Ile Leu Ala Glu Asp Glu Thr Asp Gly Glu Gln Arg His Pro
   405 410 415
   Phe Asp Ala Leu Arg Arg Thr Thr Ile Lys Lys Ser Lys Thr Asp Leu
   420 425 430
   Leu Asn Pro Glu Glu Ala Glu Asp Gln Leu Ala Asp Ile Ala Ser Val
   435 440 445
   Gly Ser Pro Phe Ala Arg Ala Ser Ile Lys Ser Ala Lys Leu Glu Asn
   450 455 460
   Ser Thr Phe Phe His Lys Lys Glu Arg Arg Met Arg Phe Tyr Ile Arg
   465 470 475 480
   Arg Met Val Lys Thr Gln Ala Phe Tyr Trp Thr Val Leu Ser Leu Val
   485 490 495
   Ala Leu Asn Thr Leu Cys Val Ala Ile Val His Tyr Asn Gln Pro Glu
   500 505 510
   Trp Leu Ser Asp Phe Leu Tyr Tyr Ala Glu Phe Ile Phe Leu Gly Leu
   515 520 525
   Phe Met Ser Glu Met Phe Ile Lys Met Tyr Gly Leu Gly Thr Arg Pro
   530 535 540
   Tyr Phe His Ser Ser Phe Asn Cys Phe Asp Cys Gly Val Ile Ile Gly
   545 550 555 560
   Ser Ile Phe Glu Val Ile Trp Ala Val Ile Lys Pro Gly Thr Ser Phe
   565 570 575
   Gly Ile Ser Val Leu Arg Ala Leu Arg Leu Leu Arg Ile Phe Lys Val
   580 585 590
   Thr Lys Tyr Trp Ala Ser Leu Arg Asn Leu Val Val Ser Leu Leu Asn
   595 600 605
   Ser Met Lys Ser Ile Ile Ser Leu Leu Phe Leu Leu Phe Leu Phe Ile
   610 615 620
   Val Val Phe Ala Leu Leu Gly Met Gln Leu Phe Gly Gly Gln Phe Asn
   625 630 635 640
   Phe Asp Glu Gly Thr Pro Pro Thr Asn Phe Asp Thr Phe Pro Ala Ala
   645 650 655
   Ile Met Thr Val Phe Gln Ile Leu Thr Gly Glu Asp Trp Asn Glu Val
   660 665 670
   Met Tyr Asp Gly Ile Lys Ser Gln Gly Gly Val Gln Gly Gly Met Val
   675 680 685
   Phe Ser Ile Tyr Phe Ile Val Leu Thr Leu Phe Gly Asn Tyr Thr Leu
   690 695 700
   Leu Asn Val Phe Leu Ala Ile Ala Val Asp Asn Leu Ala Asn Ala Gln
   705 710 715 720
   Glu Leu Thr Lys Asp Glu Gln Glu Glu Glu Glu Ala Ala Asn Gln Lys
   725 730 735
   Leu Ala Leu Gln Lys Ala Lys Glu Val Ala Glu Val Ser Pro Leu Ser
   740 745 750
   Ala Ala Asn Met Ser Ile Ala Val Lys Glu Gln Gln Lys Asn Gln Lys
   755 760 765
   Pro Ala Lys Ser Val Trp Glu Gln Arg Thr Ser Glu Met Arg Lys Gln
   770 775 780
   Asn Leu Leu Ala Ser Arg Glu Ala Leu Tyr Asn Glu Met Asp Pro Asp
   785 790 795 800
   Glu Arg Trp Lys Ala Ala Tyr Thr Arg His Leu Arg Pro Asp Met Lys
   805 810 815
   Thr His Leu Asp Arg Pro Leu Val Val Asp Pro Gln Glu Asn Arg Asn
   820 825 830
   Asn Asn Thr Asn Lys Ser Arg Ala Ala Glu Pro Thr Val Asp Gln Arg
   835 840 845
   Leu Gly Gln Gln Arg Ala Glu Asp Phe Leu Arg Lys Gln Ala Arg Tyr
   850 855 860
   His Asp Arg Ala Arg Asp Pro Ser Gly Ser Ala Gly Leu Asp Ala Arg
   865 870 875 880
   Arg Pro Trp Ala Gly Ser Gln Glu Ala Glu Leu Ser Arg Glu Asp Pro
   885 890 895
   Tyr Gly Arg Glu Ser Asp His His Ala Arg Glu Gly Ser Leu Glu Gln
   900 905 910
   Pro Gly Phe Trp Glu Gly Glu Ala Glu Arg Gly Lys Ala Gly Asp Pro
   915 920 925
   His Arg Arg His Val His Arg Gln Gly Gly Ser Arg Glu Ser Arg Ser
   930 935 940
   Gly Ser Pro Arg Thr Gly Ala Asn Gly Glu His Arg Arg His Arg Ala
   945 950 955 960
   His Arg Ser Pro Gly Glu Glu Gly Pro Glu Asp Lys Ala Glu Arg Arg
   965 970 975
   Ala Arg His Arg Glu Gly Ser Arg Pro Ala Arg Gly Gly Glu Gly Glu
   980 985 990
   Gly Glu Gly Pro Asp Gly Gly Glu Arg Arg Arg Arg His Arg His Gly
   995 1000 1005
   Ala Pro Ala Thr Tyr Glu Gly Asp Ala Arg Arg Glu Asp Lys Glu
   1010 1015 1020
   Arg Arg His Arg Arg Arg Lys Glu Asn Gln Gly Ser Gly Val Pro
   1025 1030 1035
   Val Ser Gly Pro Asn Leu Ser Thr Thr Arg Pro Ile Gln Gln Asp
   1040 1045 1050
   Leu Gly Arg Gln Asp Pro Pro Leu Ala Glu Asp Ile Asp Asn Met
   1055 1060 1065
   Lys Asn Asn Lys Leu Ala Thr Ala Glu Ser Ala Ala Pro His Gly
   1070 1075 1080
   Ser Leu Gly His Ala Gly Leu Pro Gln Ser Pro Ala Lys Met Gly
   1085 1090 1095
   Asn Ser Thr Asp Pro Ser Pro Met Leu Ala Ile Pro Ala Met Ala
   1100 1105 1110
   Thr Asn Pro Gln Asn Ala Ala Ser Arg Arg Thr Pro Asn Asn Pro
   1115 1120 1125
   Gly Asn Pro Ser Asn Pro Gly Pro Pro Lys Thr Pro Glu Asn Ser
   1130 1135 1140
   Leu Ile Val Thr Asn Pro Ser Gly Thr Gln Thr Asn Ser Ala Lys
   1145 1150 1155
   Thr Ala Arg Lys Pro Asp His Thr Thr Val Asp Ile Pro Pro Ala
   1160 1165 1170
   Cys Pro Pro Pro Leu Asn His Thr Val Val Gln Val Asn Lys Asn
   1175 1180 1185
   Ala Asn Pro Asp Pro Leu Pro Lys Lys Glu Glu Glu Lys Lys Glu
   1190 1195 1200
   Glu Glu Glu Asp Asp Arg Gly Glu Asp Gly Pro Lys Pro Met Pro
   1205 1210 1215
   Pro Tyr Ser Ser Met Phe Ile Leu Ser Thr Thr Asn Pro Leu Arg
   1220 1225 1230
   Arg Leu Cys His Tyr Ile Leu Asn Leu Arg Tyr Phe Glu Met Cys
   1235 1240 1245
   Ile Leu Met Val Ile Ala Met Ser Ser Ile Ala Leu Ala Ala Glu
   1250 1255 1260
   Asp Pro Val Gln Pro Asn Ala Pro Arg Asn Asn Val Leu Arg Tyr
   1265 1270 1275
   Phe Asp Tyr Val Phe Thr Gly Val Phe Thr Phe Glu Met Val Ile
   1280 1285 1290
   Lys Met Ile Asp Leu Gly Leu Val Leu His Gln Gly Ala Tyr Phe
   1295 1300 1305
   Arg Asp Leu Trp Asn Ile Leu Asp Phe Ile Val Val Ser Gly Ala
   1310 1315 1320
   Leu Val Ala Phe Ala Phe Thr Gly Asn Ser Lys Gly Lys Asp Ile
   1325 1330 1335
   Asn Thr Ile Lys Ser Leu Arg Val Leu Arg Val Leu Arg Pro Leu
   1340 1345 1350
   Lys Thr Ile Lys Arg Leu Pro Lys Leu Lys Ala Val Phe Asp Cys
   1355 1360 1365
   Val Val Asn Ser Leu Lys Asn Val Phe Asn Ile Leu Ile Val Tyr
   1370 1375 1380
   Met Leu Phe Met Phe Ile Phe Ala Val Val Ala Val Gln Leu Phe
   1385 1390 1395
   Lys Gly Lys Phe Phe His Cys Thr Asp Glu Ser Lys Glu Phe Glu
   1400 1405 1410
   Lys Asp Cys Arg Gly Lys Tyr Leu Leu Tyr Glu Lys Asn Glu Val
   1415 1420 1425
   Lys Ala Arg Asp Arg Glu Trp Lys Lys Tyr Glu Phe His Tyr Asp
   1430 1435 1440
   Asn Val Leu Trp Ala Leu Leu Thr Leu Phe Thr Val Ser Thr Gly
   1445 1450 1455
   Glu Gly Trp Pro Gln Val Leu Lys His Ser Val Asp Ala Thr Phe
   1460 1465 1470
   Glu Asn Gln Gly Pro Ser Pro Gly Tyr Arg Met Glu Met Ser Ile
   1475 1480 1485
   Phe Tyr Val Val Tyr Phe Val Val Phe Pro Phe Phe Phe Val Asn
   1490 1495 1500
   Ile Phe Val Ala Leu Ile Ile Ile Thr Phe Gln Glu Gln Gly Asp
   1505 1510 1515
   Lys Met Met Glu Glu Tyr Ser Leu Glu Lys Asn Glu Arg Ala Cys
   1520 1525 1530
   Ile Asp Phe Ala Ile Ser Ala Lys Pro Leu Thr Arg His Met Pro
   1535 1540 1545
   Gln Asn Lys Gln Ser Phe Gln Tyr Arg Met Trp Gln Phe Val Val
   1550 1555 1560
   Ser Pro Pro Phe Glu Tyr Thr Ile Met Ala Met Ile Ala Leu Asn
   1565 1570 1575
   Thr Ile Val Leu Met Met Lys Phe Tyr Gly Ala Ser Val Ala Tyr
   1580 1585 1590
   Glu Asn Ala Leu Arg Val Phe Asn Ile Val Phe Thr Ser Leu Phe
   1595 1600 1605
   Ser Leu Glu Cys Val Leu Lys Val Met Ala Phe Gly Ile Leu Asn
   1610 1615 1620
   Tyr Phe Arg Asp Ala Trp Asn Ile Phe Asp Phe Val Thr Val Leu
   1625 1630 1635
   Gly Ser Ile Thr Asp Ile Leu Val Thr Glu Phe Gly Asn Asn Phe
   1640 1645 1650
   Ile Asn Leu Ser Phe Leu Arg Leu Phe Arg Ala Ala Arg Leu Ile
   1655 1660 1665
   Lys Leu Leu Arg Gln Gly Tyr Thr Ile Arg Ile Leu Leu Trp Thr
   1670 1675 1680
   Phe Val Gln Ser Phe Lys Ala Leu Pro Tyr Val Cys Leu Leu Ile
   1685 1690 1695
   Ala Met Leu Phe Phe Ile Tyr Ala Ile Ile Gly Met Gln Val Phe
   1700 1705 1710
   Gly Asn Ile Gly Ile Asp Val Glu Asp Glu Asp Ser Asp Glu Asp
   1715 1720 1725
   Glu Phe Gln Ile Thr Glu His Asn Asn Phe Arg Thr Phe Phe Gln
   1730 1735 1740
   Ala Leu Met Leu Leu Phe Arg Ser Ala Thr Gly Glu Ala Trp His
   1745 1750 1755
   Asn Ile Met Leu Ser Cys Leu Ser Gly Lys Pro Cys Asp Lys Asn
   1760 1765 1770
   Ser Gly Ile Leu Thr Arg Glu Cys Gly Asn Glu Phe Ala Tyr Phe
   1775 1780 1785
   Tyr Phe Val Ser Phe Ile Phe Leu Cys Ser Phe Leu Met Leu Asn
   1790 1795 1800
   Leu Phe Val Ala Val Ile Met Asp Asn Phe Glu Tyr Leu Thr Arg
   1805 1810 1815
   Asp Ser Ser Ile Leu Gly Pro His His Leu Asp Glu Tyr Val Arg
   1820 1825 1830
   Val Trp Ala Glu Tyr Asp Pro Ala Ala Trp Gly Arg Met Pro Tyr
   1835 1840 1845
   Leu Asp Met Tyr Gln Met Leu Arg His Met Ser Pro Pro Leu Gly
   1850 1855 1860
   Leu Gly Lys Lys Cys Pro Ala Arg Val Ala Tyr Lys Arg Leu Leu
   1865 1870 1875
   Arg Met Asp Leu Pro Val Ala Asp Asp Asn Thr Val His Phe Asn
   1880 1885 1890
   Ser Thr Leu Met Ala Leu Ile Arg Thr Ala Leu Asp Ile Lys Ile
   1895 1900 1905
   Ala Lys Gly Gly Ala Asp Lys Gln Gln Met Asp Ala Glu Leu Arg
   1910 1915 1920
   Lys Glu Met Met Ala Ile Trp Pro Asn Leu Ser Gln Lys Thr Leu
   1925 1930 1935
   Asp Leu Leu Val Thr Pro His Lys Ser Thr Asp Leu Thr Val Gly
   1940 1945 1950
   Lys Ile Tyr Ala Ala Met Met Ile Met Glu Tyr Tyr Arg Gln Ser
   1955 1960 1965
   Lys Ala Lys Lys Leu Gln Ala Met Arg Glu Glu Gln Asp Arg Thr
   1970 1975 1980
   Pro Leu Met Phe Gln Arg Met Glu Pro Pro Ser Pro Thr Gln Glu
   1985 1990 1995
   Gly Gly Pro Gly Gln Asn Ala Leu Pro Ser Thr Gln Leu Asp Pro
   2000 2005 2010
   Gly Gly Ala Leu Met Ala His Glu Ser Gly Leu Lys Glu Ser Pro
   2015 2020 2025
   Ser Trp Val Thr Gln Arg Ala Gln Glu Met Phe Gln Lys Thr Gly
   2030 2035 2040
   Thr Trp Ser Pro Glu Gln Gly Pro Pro Thr Asp Met Pro Asn Ser
   2045 2050 2055
   Gln Pro Asn Ser Gln Ser Val Glu Met Arg Glu Met Gly Arg Asp
   2060 2065 2070
   Gly Tyr Ser Asp Ser Glu His Tyr Leu Pro Met Glu Gly Gln Gly
   2075 2080 2085
   Arg Ala Ala Ser Met Pro Arg Leu Pro Ala Glu Asn Gln Arg Arg
   2090 2095 2100
   Arg Gly Arg Pro Arg Gly Asn Asn Leu Ser Thr Ile Ser Asp Thr
   2105 2110 2115
   Ser Pro Met Lys Arg Ser Ala Ser Val Leu Gly Pro Lys Ala Arg
   2120 2125 2130
   Arg Leu Asp Asp Tyr Ser Leu Glu Arg Val Pro Pro Glu Glu Asn
   2135 2140 2145
   Gln Arg His His Gln Arg Arg Arg Asp Arg Ser His Arg Ala Ser
   2150 2155 2160
   Glu Arg Ser Leu Gly Arg Tyr Thr Asp Val Asp Thr Gly Leu Gly
   2165 2170 2175
   Thr Asp Leu Ser Met Thr Thr Gln Ser Gly Asp Leu Pro Ser Lys
   2180 2185 2190
   Glu Arg Asp Gln Glu Arg Gly Arg Pro Lys Asp Arg Lys His Arg
   2195 2200 2205
   Gln His His His His His His His His His His Pro Pro Pro Pro
   2210 2215 2220
   Asp Lys Asp Arg Tyr Ala Gln Glu Arg Pro Asp His Gly Arg Ala
   2225 2230 2235
   Arg Ala Arg Asp Gln Arg Trp Ser Arg Ser Pro Ser Glu Gly Arg
   2240 2245 2250
   Glu His Met Ala His Arg Gln Gly Ser Ser Ser Val Ser Gly Ser
   2255 2260 2265
   Pro Ala Pro Ser Thr Ser Gly Thr Ser Thr Pro Arg Arg Gly Arg
   2270 2275 2280
   Arg Gln Leu Pro Gln Thr Pro Ser Thr Pro Arg Pro His Val Ser
   2285 2290 2295
   Tyr Ser Pro Val Ile Arg Lys Ala Gly Gly Ser Gly Pro Pro Gln
   2300 2305 2310
   Gln Gln Gln Gln Gln Gln Gln Gln Gln Gln Gln Gln Ala Val Ala
   2315 2320 2325
   Arg Pro Gly Arg Ala Ala Thr Ser Gly Pro Arg Arg Tyr Pro Gly
   2330 2335 2340
   Pro Thr Ala Glu Pro Leu Ala Gly Asp Arg Pro Pro Thr Gly Gly
   2345 2350 2355
   His Ser Ser Gly Arg Ser Pro Arg Met Glu Arg Arg Val Pro Gly
   2360 2365 2370
   Pro Ala Arg Ser Glu Ser Pro Arg Ala Cys Arg His Gly Gly Ala
   2375 2380 2385
   Arg Trp Pro Ala Ser Gly Pro His Val Ser Glu Gly Pro Pro Gly
   2390 2395 2400
   Pro Arg His His Gly Tyr Tyr Arg Gly Ser Asp Tyr Asp Glu Ala
           2405                    2410                    2415
   Asp Gly  Pro Gly Ser Gly Gly  Gly Glu Glu Ala Met  Ala Gly Ala
       2420                    2425                    2430
   Tyr Asp  Ala Pro Pro Pro Val  Arg His Ala Ser Ser  Gly Ala Thr
       2435                    2440                    2445
   Gly Arg  Ser Pro Arg Thr Pro  Arg Ala Ser Gly Pro  Ala Cys Ala
       2450                    2455                    2460
   Ser Pro  Ser Arg His Gly Arg  Arg Leu Pro Asn Gly  Tyr Tyr Pro
       2465                    2470                    2475
   Ala His  Gly Leu Ala Arg Pro  Arg Gly Pro Gly Ser  Arg Lys Gly
       2480                    2485                    2490
   Leu His  Glu Pro Tyr Ser Glu  Ser Asp Asp Asp Trp  Cys
       2495                    2500                    2505
<210> 40
   <211> 7521
   <212> DNA
   <213> Homo sapiens
<400> 40
   atggcccgct tcggagacga gatgccggcc cgctacgggg gaggaggctc cggggcagcc 60
   gccggggtgg tcgtgggcag cggaggcggg cgaggagccg ggggcagccg gcagggcggg 120
   cagcccgggg cgcaaaggat gtacaagcag tcaatggcgc agagagcgcg gaccatggca 180
   ctctacaacc ccatccccgt ccgacagaac tgcctcacgg ttaaccggtc tctcttcctc 240
   ttcagcgaag acaacgtggt gagaaaatac gccaaaaaga tcaccgaatg gcctcccttt 300
   gaatatatga ttttagccac catcatagcg aattgcatcg tcctcgcact ggagcagcat 360
   ctgcctgatg atgacaagac cccgatgtct gaacggctgg atgacacaga accatacttc 420
   attggaattt tttgtttcga ggctggaatt aaaatcattg cccttgggtt tgccttccac 480
   aaaggctcct acttgaggaa tggctggaat gtcatggact ttgtggtggt gctaacgggc 540
   atcttggcga cagttgggac ggagtttgac ctacggacgc tgagggcagt tcgagtgctg 600
   cggccgctca agctggtgtc tggaatccca agtttacaag tcgtcctgaa gtcgatcatg 660
   aaggcgatga tccctttgct gcagatcggc ctcctcctat tttttgcaat ccttattttt 720
   gcaatcatag ggttagaatt ttatatggga aaatttcata ccacctgctt tgaagagggg 780
   acagatgaca ttcagggtga gtctccggct ccatgtggga cagaagagcc cgcccgcacc 840
   tgccccaatg ggaccaaatg tcagccctac tgggaggggc ccaacaacgg gatcactcag 900
   ttcgacaaca tcctgtttgc agtgctgact gttttccagt gcataaccat ggaagggtgg 960
   actgatctcc tctacaatag caacgatgcc tcagggaaca cttggaactg gttgtacttc 1020
   atccccctca tcatcatcgg ctcctttttt atgctgaacc ttgtgctggg tgtgctgtca 1080
   ggggagtttg ccaaagaaag ggaacgggtg gagaaccggc gggcttttct gaagctgagg 1140
   cggcaacaac agattgaacg tgagctcaat gggtacatgg aatggatctc aaaagcagaa 1200
   gaggtgatcc tcgccgagga tgaaactgac ggggagcaga ggcatccctt tgatgctctg 1260
   cggagaacca ccataaagaa aagcaagaca gatttgctca accccgaaga ggctgaggat 1320
   cagctggctg atatagcctc tgtgggttct cccttcgccc gagccagcat taaaagtgcc 1380
   aagctggaga actcgacctt ttttcacaaa aaggagagga ggatgcgttt ctacatccgc 1440
   cgcatggtca aaactcaggc cttctactgg actgtactca gtttggtagc tctcaacacg 1500
   ctgtgtgttg ctattgttca ctacaaccag cccgagtggc tctccgactt cctttactat 1560
   gcagaattca ttttcttagg actctttatg tccgaaatgt ttataaaaat gtacgggctt 1620
   gggacgcggc cttacttcca ctcttccttc aactgctttg actgtggggt tatcattggg 1680
   agcatcttcg aggtcatctg ggctgtcata aaacctggca catcctttgg aatcagcgtg 1740
   ttacgagccc tcaggttatt gcgtattttc aaagtcacaa agtactgggc atctctcaga 1800
   aacctggtcg tctctctcct caactccatg aagtccatca tcagcctgtt gtttctcctt 1860
   ttcctgttca ttgtcgtctt cgcccttttg ggaatgcaac tcttcggcgg ccagtttaat 1920
   ttcgatgaag ggactcctcc caccaacttc gatacttttc cagcagcaat aatgacggtg 1980
   tttcagatcc tgacgggcga agactggaac gaggtcatgt acgacgggat caagtctcag 2040
   gggggcgtgc agggcggcat ggtgttctcc atctatttca ttgtactgac actctttggg 2100
   aactacaccc tcctgaatgt gttcttggcc atcgctgtgg acaatctggc caacgcccag 2160
   gagctcacca aggacgagca agaggaagaa gaagcagcga accagaaact tgccctacag 2220
   aaagccaagg aggtggcaga agtgagtcct ctgtccgcgg ccaacatgtc tatagctgtg 2280
   aaagagcaac agaagaatca aaagccagcc aagtccgtgt gggagcagcg gaccagtgag 2340
   atgcgaaagc agaacttgct ggccagccgg gaggccctgt ataacgaaat ggacccggac 2400
   gagcgctgga aggctgccta cacgcggcac ctgcggccag acatgaagac gcacttggac 2460
   cggccgctgg tggtggaccc gcaggagaac cgcaacaaca acaccaacaa gagccgggcg 2520
   gccgagccca ccgtggacca gcgcctcggc cagcagcgcg ccgaggactt cctcaggaaa 2580
   caggcccgct accacgatcg ggcccgggac cccagcggct cggcgggcct ggacgcacgg 2640
   aggccctggg cgggaagcca ggaggccgag ctgagccggg aggaccccta cggccgcgag 2700
   tcggaccacc acgcccggga gggcagcctg gagcaacccg ggttctggga gggcgaggcc 2760
   gagcgaggca aggccgggga cccccaccgg aggcacgtgc accggcaggg gggcagcagg 2820
   gagagccgca gcgggtcccc gcgcacgggc gcgaacgggg agcatcgacg tcatcgcgcg 2880
   caccgcagcc ccggggagga gggtccggag gacaaggcgg agcggagggc gcggcaccgc 2940
   gagggcagcc ggccggcccg gggcggcgag ggcgagggcg agggccccga cgggggcgag 3000
   cgcaggagaa ggcaccggca tggcgctcca gccacgtacg agggggacgc gcggagggag 3060
   gacaaggagc ggaggcatcg gaggaggaaa gagaaccagg gctccggggt ccctgtgtcg 3120
   ggccccaacc tgtcaaccac ccggccaatc cagcaggacc tgggccgcca agacccaccc 3180
   ctggcagagg atattgacaa catgaagaac aacaagctgg ccaccgcgga gtcggccgct 3240
   ccccacggca gccttggcca cgccggcctg ccccagagcc cagccaagat gggaaacagc 3300
   accgacccca gccccatgct ggccatccct gccatggcca ccaaccccca gaacgccgcc 3360
   agccgccgga cgcccaacaa cccggggaac ccatccaatc ccggcccccc caagaccccc 3420
   gagaatagcc ttatcgtcac caaccccagc ggcacccaga ccaattcagc taagactgcc 3480
   aggaaacccg accacaccac agtggacatc cccccagcct gcccaccccc cctcaaccac 3540
   accgtcgtac aagtgaacaa aaacgccaac ccagacccac tgccaaaaaa agaggaagag 3600
   aagaaggagg aggaggaaga cgaccgtggg gaagacggcc ctaagccaat gcctccctat 3660
   agctccatgt tcatcctgtc cacgaccaac ccccttcgcc gcctgtgcca ttacatcctg 3720
   aacctgcgct actttgagat gtgcatcctc atggtcattg ccatgagcag catcgccctg 3780
   gccgccgagg accctgtgca gcccaacgca cctcggaaca acgtgctgcg atactttgac 3840
   tacgttttta caggcgtctt tacctttgag atggtgatca agatgattga cctggggctc 3900
   gtcctgcatc agggtgccta cttccgtgac ctctggaata ttctcgactt catagtggtc 3960
   agtggggccc tggtagcctt tgccttcact ggcaatagca aaggaaaaga catcaacacg 4020
   attaaatccc tccgagtcct ccgggtgcta cgacctctta aaaccatcaa gcggctgcca 4080
   aagctcaagg ctgtgtttga ctgtgtggtg aactcactta aaaacgtctt caacatcctc 4140
   atcgtctaca tgctattcat gttcatcttc gccgtggtgg ctgtgcagct cttcaagggg 4200
   aaattcttcc actgcactga cgagtccaaa gagtttgaga aagattgtcg aggcaaatac 4260
   ctcctctacg agaagaatga ggtgaaggcg cgagaccggg agtggaagaa gtatgaattc 4320
   cattacgaca atgtgctgtg ggctctgctg accctcttca ccgtgtccac gggagaaggc 4380
   tggccacagg tcctcaagca ttcggtggac gccacctttg agaaccaggg ccccagcccc 4440
   gggtaccgca tggagatgtc cattttctac gtcgtctact ttgtggtgtt ccccttcttc 4500
   tttgtcaata tctttgtggc cttgatcatc atcaccttcc aggagcaagg ggacaagatg 4560
   atggaggaat acagcctgga gaaaaatgag agggcctgca ttgatttcgc catcagcgcc 4620
   aagccgctga cccgacacat gccgcagaac aagcagagct tccagtaccg catgtggcag 4680
   ttcgtggtgt ctccgccttt cgagtacacg atcatggcca tgatcgccct caacaccatc 4740
   gtgcttatga tgaagttcta tggggcttct gttgcttatg aaaatgccct gcgggtgttc 4800
   aacatcgtct tcacctccct cttctctctg gaatgtgtgc tgaaagtcat ggcttttggg 4860
   attctgaatt atttccgcga tgcctggaac atcttcgact ttgtgactgt tctgggcagc 4920
   atcaccgata tcctcgtgac tgagtttggg aataacttca tcaacctgag ctttctccgc 4980
   ctcttccgag ctgcccggct catcaaactt ctccgtcagg gttacaccat ccgcattctt 5040
   ctctggacct ttgtgcagtc cttcaaggcc ctgccttatg tctgtctgct gatcgccatg 5100
   ctcttcttca tctatgccat cattgggatg caggtgtttg gtaacattgg catcgacgtg 5160
   gaggacgagg acagtgatga agatgagttc caaatcactg agcacaataa cttccggacc 5220
   ttcttccagg ccctcatgct tctcttccgg agtgccaccg gggaagcttg gcacaacatc 5280
   atgctttcct gcctcagcgg gaaaccgtgt gataagaact ctggcatcct gactcgagag 5340
   tgtggcaatg aatttgctta tttttacttt gtttccttca tcttcctctg ctcgtttctg 5400
   atgctgaatc tctttgtcgc cgtcatcatg gacaactttg agtacctcac ccgagactcc 5460
   tccatcctgg gcccccacca cctggatgag tacgtgcgtg tctgggccga gtatgacccc 5520
   gcagcttggg gccgcatgcc ttacctggac atgtatcaga tgctgagaca catgtctccg 5580
   cccctgggtc tggggaagaa gtgtccggcc agagtggctt acaagcggct tctgcggatg 5640
   gacctgcccg tcgcagatga caacaccgtc cacttcaatt ccaccctcat ggctctgatc 5700
   cgcacagccc tggacatcaa gattgccaag ggaggagccg acaaacagca gatggacgct 5760
   gagctgcgga aggagatgat ggcgatttgg cccaatctgt cccagaagac gctagacctg 5820
   ctggtcacac ctcacaagtc cacggacctc accgtgggga agatctacgc agccatgatg 5880
   atcatggagt actaccggca gagcaaggcc aagaagctgc aggccatgcg cgaggagcag 5940
   gaccggacac ccctcatgtt ccagcgcatg gagcccccgt ccccaacgca ggaaggggga 6000
   cctggccaga acgccctccc ctccacccag ctggacccag gaggagccct gatggctcac 6060
   gaaagcggcc tcaaggagag cccgtcctgg gtgacccagc gtgcccagga gatgttccag 6120
   aagacgggca catggagtcc ggaacaaggc ccccctaccg acatgcccaa cagccagcct 6180
   aactctcagt ccgtggagat gcgagagatg ggcagagatg gctactccga cagcgagcac 6240
   tacctcccca tggaaggcca gggccgggct gcctccatgc cccgcctccc tgcagagaac 6300
   cagaggagaa ggggccggcc acgtgggaat aacctcagta ccatctcaga caccagcccc 6360
   atgaagcgtt cagcctccgt gctgggcccc aaggcccgac gcctggacga ttactcgctg 6420
   gagcgggtcc cgcccgagga gaaccagcgg caccaccagc ggcgccgcga ccgcagccac 6480
   cgcgcctctg agcgctccct gggccgctac accgatgtgg acacaggctt ggggacagac 6540
   ctgagcatga ccacccaatc cggggacctg ccgtcgaagg agcgggacca ggagcggggc 6600
   cggcccaagg atcggaagca tcgacagcac caccaccacc accaccacca ccaccatccc 6660
   ccgccccccg acaaggaccg ctatgcccag gaacggccgg accacggccg ggcacgggct 6720
   cgggaccagc gctggtcccg ctcgcccagc gagggccgag agcacatggc gcaccggcag 6780
   ggcagtagtt ccgtaagtgg aagcccagcc ccctcaacat ctggtaccag cactccgcgg 6840
   cggggccgcc gccagctccc ccagaccccc tccacccccc ggccacacgt gtcctattcc 6900
   cctgtgatcc gtaaggccgg cggctcgggg cccccgcagc agcagcagca gcagcagcag 6960
   cagcagcagc agcaggcggt ggccaggccg ggccgggcgg ccaccagcgg ccctcggagg 7020
   tacccaggcc ccacggccga gcctctggcc ggagatcggc cgcccacggg gggccacagc 7080
   agcggccgct cgcccaggat ggagaggcgg gtcccaggcc cggcccggag cgagtccccc 7140
   agggcctgtc gacacggcgg ggcccggtgg ccggcatctg gcccgcacgt gtccgagggg 7200
   cccccgggtc cccggcacca tggctactac cggggctccg actacgacga ggccgatggc 7260
   ccgggcagcg ggggcggcga ggaggccatg gccggggcct acgacgcgcc accccccgta 7320
   cgacacgcgt cctcgggcgc caccgggcgc tcgcccagga ctccccgggc ctcgggcccg 7380
   gcctgcgcct cgccttctcg gcacggccgg cgactcccca acggctacta cccggcgcac 7440
   ggactggcca ggccccgcgg gccgggctcc aggaagggcc tgcacgaacc ctacagcgag 7500
   agtgacgatg attggtgcta a 7521
<210> 41
   <211> 330
   <212> PRT
   <213> Homo sapiens
<400> 41
   Arg Gly Ala Leu Val Gly Ala Pro Arg Ser Ala Arg Ala Ser Cys Leu
   1 5 10 15
   Arg Gly Arg Arg Pro Gly Arg Arg Gln Pro Cys Gly Arg Cys Pro Asp
   20 25 30
   Pro Pro Arg Ser Gly Pro Gly Gln Ala Gly Arg Ala Arg Cys Ala Arg
   35 40 45
   Asp Val Ala Ala Gln Thr Ala Leu Ala Pro Arg Ala Leu Ala Leu Gly
   50 55 60
   Ala Gly Ala Ala Gly Pro Gly Trp Arg Gly Ala Val Pro Arg Arg Pro
   65 70 75 80
   Ala Ala Arg Val Ser Arg Ala Ala Gln Arg Gln Glu Gln Glu Leu Val
   85 90 95
   Arg Leu His Arg Glu Gln Glu Cys Glu Leu Gln Pro Glu Pro Ala Pro
   100 105 110
   Pro Arg Pro Ser Gly Pro Ala Thr Ala Glu Asp Pro Gly Arg Arg Pro
   115 120 125
   Val Leu Pro Gln Arg Pro Pro Glu Glu Arg Pro Pro Gln Pro Pro Gly
   130 135 140
   Ser Thr Gly Val Ile Ala Glu Thr Gly Gln Ala Gly Pro Pro Ala Gly
   145 150 155 160
   Ala Gly Ala Pro Gly Tyr Pro Lys Ser Pro Pro Val Ala Ser Pro Gly
   165 170 175
   Ala Pro Val Pro Ser Leu Val Ser Phe Ser Ala Gly Leu Thr Gln Lys
   180 185 190
   Pro Phe Pro Ser Asp Gly Gly Val Val Leu Phe Asn Lys Val Leu Val
           195                 200                 205
   Asn Asp Gly Asp Val Tyr Asn Pro Ser Thr Gly Val Phe Thr Ala Pro
       210                 215                 220
   Tyr Asp Gly Arg Tyr Leu Ile Thr Ala Thr Leu Thr Pro Glu Arg Asp
   225                 230                 235                 240
   Ala Tyr Val Glu Ala Val Leu Ser Val Ser Asn Ala Ser Val Ala Gln
                   245                 250                 255
   Leu His Thr Ala Gly Tyr Arg Arg Glu Phe Leu Glu Tyr His Arg Pro
               260                 265                 270
   Pro Gly Ala Leu His Thr Cys Gly Gly Pro Gly Ala Phe His Leu Ile
           275                 280                 285
   Val His Leu Lys Ala Gly Asp Ala Val Asn Val Val Val Thr Gly Gly
       290                 295                 300
   Lys Leu Ala His Thr Asp Phe Asp Glu Met Tyr Ser Thr Phe Ser Gly
   305                 310                 315                 320
   Val Phe Leu Tyr Pro Phe Leu Ser His Leu
                   325                 330
<210> 42
   <211> 994
   <212> DNA
   <213> Homo sapiens
<400> 42
   ccgcggagca ctggttggag cgccgcgaag cgcccgagcc tcttgccttc gcgggcggcg 60
   ccctggccgc cggcagcctt gtggccggtg ccccgatccg ccgcgctccg gacccgggca 120
   ggcggggcgc gcccgctgcg cgcgggatgt ggcagcccag acggccctgg ccccgcgtgc 180
   cctggcgctg ggcgctggcg ctgctggccc tggttggcgc ggggctgtgc cacgccggcc 240
   cgcagcccgg gtatcccgcg cggcccagcg ccaggaacaa gaactggtgc gcctacatcg 300
   tgaacaagaa tgtgagctgc agcccgagcc cgccccgccg aggcccagcg gccccgcaac 360
   cgcagaggac cctgggcgac ggcccgtcct gccccagcgg ccccccgagg agaggccgcc 420
   ccagccgcca ggctccaccg gggtcatcgc ggagacgggc caggccgggc cccccgcagg 480
   cgcaggcgca ccaggatacc cgaagtcacc tcctgtagct tccccaggag ctccggtgcc 540
   ttctctggtg tctttttctg cggggctcac ccagaagcct ttccccagtg atgggggcgt 600
   tgtcctcttt aacaaagtgc tggtgaacga cggggatgtt tacaacccca gcaccggggt 660
   cttcacggct ccttatgatg ggcgctacct gatcacggcc accctcaccc ccgagagaga 720
   cgcctacgtg gaagcagtgc tgtcggtctc caacgccagc gtggcccagc tgcataccgc 780
   tgggtacagg agagagttcc tggaatacca ccgccctcca ggagctttgc atacctgcgg 840
   gggcccgggg gcattccacc tcatcgtgca cctgaaggcg ggagatgcag tcaacgtcgt 900
   ggtgactggg ggcaagctgg ctcacacaga ctttgatgaa atgtactcca catttagtgg 960
   ggttttctta tatcctttcc tttcccacct ctaa 994
<210> 43
   <211> 97
   <212> PRT
   <213> Homo sapiens
<400> 43
   Met Lys Glu Pro Gly Pro Asn Phe Val Thr Val Arg Lys Gly Leu His
   1               5                   10                  15
   Ser Phe Lys Met Ala Phe Val Lys His Leu Leu Leu Phe Leu Ser Pro
               20                  25                  30
   Arg Leu Glu Cys Ser Gly Ser Ile Thr Asp His Cys Ser Leu His Leu
           35                  40                  45
   Pro Val Gln Glu Ile Leu Met Ser Gln Pro Pro Glu Gln Leu Gly Leu
       50                  55                  60
   Gln Thr Asn Leu Gly Asn Gln Glu Ser Ser Gly Met Met Lys Leu Phe
   65                  70                  75                  80
   Met Pro Arg Pro Lys Val Leu Ala Gln Tyr Glu Ser Ile Gln Phe Met
                   85                  90                  95
   Pro
<210> 44
   <211> 294
   <212> DNA
   <213> Homo sapiens
<400> 44
   atgaaggagc ctggacccaa ctttgttact gtgagaaagg gtcttcattc attcaagatg 60
   gcatttgtta agcacctact tctctttctg tcacctaggc tggagtgcag tggttcaatc 120
   acggatcact gcagcctcca cctcccagtt caagaaattc tcatgtctca gcctcctgag 180
   cagctaggat tacagacaaa ccttggaaat caagaaagtt ctggaatgat gaagctgttc 240
   atgccaagac cgaaagtgct ggcccagtat gagtccattc agttcatgcc gtga 294
<210> 45
   <211> 47
   <212> PRT
   <213> Homo sapiens
<400> 45
   Met His Asn Ile Met Met Val Lys Leu Lys Lys Lys Lys Ser Thr Thr
   1               5                   10                  15
   Asn Leu Gly Asn Gln Glu Ser Ser Gly Met Met Lys Leu Phe Met Pro
               20                  25                  30
   Arg Pro Lys Val Leu Ala Gln Tyr Glu Ser Ile Gln Phe Met Pro
           35                  40                  45
<210> 46
   <211> 43
   <212> PRT
   <213> Homo sapiens
<400> 46
   Met Ser Gln Pro Pro Glu Gln Leu Gly Leu Gln Thr Asn Leu Gly Asn
   1               5                   10                  15
   Gln Glu Ser Ser Gly Met Met Lys Leu Phe Met Pro Arg Pro Lys Val
               20                  25                  30
   Leu Ala Gln Tyr Glu Ser Ile Gln Phe Met Pro
           35                  40
<210> 47
   <211> 70
   <212> PRT
   <213> Homo sapiens
<400> 47
   Met Tyr Ser Tyr Trp Arg Leu Glu Cys Ser Gly Ser Ile Thr Asp His
   1               5                   10                  15
   Cys Ser Leu His Leu Pro Val Gln Glu Ile Leu Met Ser Gln Pro Pro
               20                  25                  30
   Glu Gln Leu Gly Leu Gln Thr Asn Leu Gly Asn Gln Glu Ser Ser Gly
           35                  40                  45
   Met Met Lys Leu Phe Met Pro Arg Pro Lys Val Leu Ala Gln Tyr Glu
       50                  55                  60
   Ser Ile Gln Phe Met Pro
   65                  70
<210> 48
   <211> 91
   <212> PRT
   <213> Homo sapiens
<400> 48
   Met Lys Glu Pro Gly Pro Asn Phe Val Thr Val Arg Lys Gly Leu His
   1               5                   10                  15
   Ser Phe Lys Met Ala Phe Val Lys His Leu Leu Leu Glu Cys Ser Gly
   20 25 30
   Ser Ile Thr Asp His Cys Ser Leu His Leu Pro Val Gln Glu Ile Leu
   35 40 45
   Met Ser Gln Pro Pro Glu Gln Leu Gly Leu Gln Thr Asn Leu Gly Asn
   50 55 60
   Gln Glu Ser Ser Gly Met Met Lys Leu Phe Met Pro Arg Pro Lys Val
   65 70 75 80
   Leu Ala Gln Tyr Glu Ser Ile Gln Phe Met Pro
   85 90
<210> 49
   <211> 37
   <212> PRT
   <213> Homo sapiens
<400> 49
   Met Lys Glu Pro Gly Pro Asn Phe Val Thr Val Arg Lys Gly Leu His
   1 5 10 15
   Ser Phe Lys Met Ala Phe Val Lys His Leu Leu Gln Thr Leu Glu Ile
   20 25 30
   Lys Lys Val Leu Glu
   35
<210> 50
   <211> 1079
   <212> PRT
   <213> Homo sapiens
<400> 50
   Met Val Cys Ser Ala Ala Pro Leu Leu Leu Leu Ala Thr Thr Leu Pro
   1 5 10 15
   Leu Leu Gly Ser Pro Val Ala Gln Ala Ser Gln Pro Val Ser Glu Thr
   20 25 30
   Gly Val Arg Pro Arg Glu Gly Leu Gln Arg Arg Gln Trp Gly Pro Leu
   35 40 45
   Ile Gly Arg Asp Lys Ala Trp Asn Glu Arg Ile Asp Arg Pro Phe Pro
   50 55 60
   Ala Cys Pro Ile Pro Leu Ser Ser Ser Phe Gly Arg Trp Pro Lys Gly
   65 70 75 80
   Gln Thr Met Trp Ala Gln Thr Ser Thr Leu Thr Leu Thr Glu Glu Glu
   85 90 95
   Leu Gly Gln Ser Gln Ala Gly Gly Glu Ser Gly Ser Gly Gln Leu Leu
   100 105 110
   Asp Gln Glu Asn Gly Ala Gly Glu Ser Ala Leu Val Ser Val Tyr Val
   115 120 125
   His Leu Asp Phe Pro Asp Lys Thr Trp Pro Pro Glu Leu Ser Arg Thr
   130 135 140
   Leu Thr Leu Pro Ala Ala Ser Ala Ser Ser Ser Pro Arg Pro Leu Leu
   145 150 155 160
   Thr Gly Leu Arg Leu Thr Thr Glu Cys Asn Val Asn His Lys Gly Asn
   165 170 175
   Phe Tyr Cys Ala Cys Leu Ser Gly Tyr Gln Trp Asn Thr Ser Ile Cys
   180 185 190
   Leu His Tyr Pro Pro Cys Gln Ser Leu His Asn His Gln Pro Cys Gly
   195 200 205
   Cys Leu Val Phe Ser His Pro Glu Pro Gly Tyr Cys Gln Leu Leu Pro
   210 215 220
   Pro Gly Ser Pro Val Thr Cys Leu Pro Ala Val Pro Gly Ile Leu Asn
   225 230 235 240
   Leu Asn Ser Gln Leu Gln Met Pro Gly Asp Thr Leu Ser Leu Thr Leu
   245 250 255
   His Leu Ser Gln Glu Ala Thr Asn Leu Ser Trp Phe Leu Arg His Pro
   260 265 270
   Gly Ser Pro Ser Pro Ile Leu Leu Gln Pro Gly Thr Gln Val Ser Val
   275 280 285
   Thr Ser Ser His Gly Gln Ala Ala Leu Ser Val Ser Asn Met Ser His
   290 295 300
   His Trp Ala Gly Glu Tyr Met Ser Cys Phe Glu Ala Gln Gly Phe Lys
   305 310 315 320
   Trp Asn Leu Tyr Glu Val Val Arg Val Pro Leu Lys Ala Thr Asp Val
   325 330 335
   Ala Arg Leu Pro Tyr Gln Leu Ser Ile Ser Cys Ala Thr Ser Pro Gly
   340 345 350
   Phe Gln Leu Ser Cys Cys Ile Pro Ser Thr Asn Leu Ala Tyr Thr Ala
   355 360 365
   Ala Trp Ser Pro Gly Glu Gly Ser Lys Ala Ser Ser Phe Asn Glu Ser
   370 375 380
   Gly Ser Gln Cys Phe Val Leu Ala Val Gln Arg Cys Pro Met Ala Asp
   385 390 395 400
   Thr Thr Tyr Ala Cys Asp Leu Gln Ser Leu Gly Leu Ala Pro Leu Arg
   405 410 415
   Val Pro Ile Ser Ile Thr Ile Ile Gln Asp Gly Asp Ile Thr Cys Pro
   420 425 430
   Glu Asp Ala Ser Val Leu Thr Trp Asn Val Thr Lys Ala Gly His Val
   435 440 445
   Ala Gln Ala Pro Cys Pro Glu Ser Lys Arg Gly Ile Val Arg Arg Leu
   450 455 460
   Cys Gly Ala Asp Gly Val Trp Gly Pro Val His Ser Ser Cys Thr Asp
   465 470 475 480
   Ala Arg Leu Leu Ala Leu Phe Thr Arg Thr Lys Leu Leu Gln Ala Gly
   485 490 495
   Gln Gly Ser Pro Ala Glu Glu Val Pro Gln Ile Leu Ala Gln Leu Pro
   500 505 510
   Gly Gln Ala Ala Glu Ala Ser Ser Pro Ser Asp Leu Leu Thr Leu Leu
   515 520 525
   Ser Thr Met Lys Tyr Val Ala Lys Val Val Ala Glu Ala Arg Ile Gln
   530 535 540
   Leu Asp Arg Arg Ala Leu Lys Asn Leu Leu Ile Ala Thr Asp Lys Val
   545 550 555 560
   Leu Asp Met Asp Thr Arg Ser Leu Trp Thr Leu Ala Gln Ala Arg Lys
   565 570 575
   Pro Trp Ala Gly Ser Thr Leu Leu Leu Ala Val Glu Thr Leu Ala Cys
   580 585 590
   Ser Leu Cys Pro Gln Asp His Pro Phe Ala Phe Ser Leu Pro Asn Val
   595 600 605
   Leu Leu Gln Ser Gln Leu Phe Gly Pro Thr Phe Pro Ala Asp Tyr Ser
   610 615 620
   Ile Ser Phe Pro Thr Arg Pro Pro Leu Gln Ala Gln Ile Pro Arg His
   625                 630                 635                 640
   Ser Leu Ala Pro Leu Val Arg Asn Gly Thr Glu Ile Ser Ile Thr Ser
                   645                 650                 655
   Leu Val Leu Arg Lys Leu Asp His Leu Leu Pro Ser Asn Tyr Gly Gln
               660                 665                 670
   Gly Leu Gly Asp Ser Leu Tyr Ala Thr Pro Gly Leu Val Leu Val Ile
           675                 680                 685
   Ser Ile Met Ala Gly Asp Arg Ala Phe Ser Gln Gly Glu Val Ile Met
       690                 695                 700
   Asp Phe Gly Asn Thr Asp Gly Ser Pro His Cys Val Phe Trp Asp His
   705                 710                 715                 720
   Ser Leu Phe Gln Gly Arg Gly Gly Trp Ser Lys Glu Gly Cys Gln Ala
                   725                 730                 735
   Gln Val Ala Ser Ala Ser Pro Thr Ala Gln Cys Leu Cys Gln His Leu
               740                 745                 750
   Thr Ala Phe Ser Val Leu Met Ser Pro His Thr Val Pro Glu Glu Pro
           755                 760                 765
   Ala Leu Ala Leu Leu Thr Gln Val Gly Leu Gly Ala Ser Ile Leu Ala
       770                 775                 780
   Leu Leu Val Cys Leu Gly Val Tyr Trp Leu Val Trp Arg Val Val Val
   785                 790                 795                 800
   Arg Asn Lys Ile Ser Tyr Phe Arg His Ala Ala Leu Leu Asn Met Val
                   805                 810                 815
   Phe Cys Leu Leu Ala Ala Asp Thr Cys Phe Leu Gly Ala Pro Phe Leu
               820                 825                 830
   Ser Pro Gly Pro Arg Ser Pro Leu Cys Leu Ala Ala Ala Phe Leu Cys
           835                 840                 845
   His Phe Leu Tyr Leu Ala Thr Phe Phe Trp Met Leu Ala Gln Ala Leu
       850                 855                 860
   Val Leu Ala His Gln Leu Leu Phe Val Phe His Gln Leu Ala Lys His
   865                 870                 875                 880
   Arg Val Leu Pro Leu Met Val Leu Leu Gly Tyr Leu Cys Pro Leu Gly
                   885                 890                 895
   Leu Ala Gly Val Thr Leu Gly Leu Tyr Leu Pro Gln Gly Gln Tyr Leu
   900 905 910
   Arg Glu Gly Glu Cys Trp Leu Asp Gly Lys Gly Gly Ala Leu Tyr Thr
   915 920 925
   Phe Val Gly Pro Val Leu Ala Ile Ile Gly Val Asn Gly Leu Val Leu
   930 935 940
   Ala Met Ala Met Leu Lys Leu Leu Arg Pro Ser Leu Ser Glu Gly Pro
   945 950 955 960
   Pro Ala Glu Lys Arg Gln Ala Leu Leu Gly Val Ile Lys Ala Leu Leu
   965 970 975
   Ile Leu Thr Pro Ile Phe Gly Leu Thr Trp Gly Leu Gly Leu Ala Thr
   980 985 990
   Leu Leu Glu Glu Val Ser Thr Val Pro His Tyr Ile Phe Thr Ile Leu
   995 1000 1005
   Asn Thr Leu Gln Gly Val Phe Ile Leu Leu Phe Gly Cys Leu Met
   1010 1015 1020
   Asp Arg Lys Ile Gln Glu Ala Leu Arg Lys Arg Phe Cys Arg Ala
   1025 1030 1035
   Gln Ala Pro Ser Ser Thr Ile Ser Leu Val Ser Cys Cys Leu Gln
   1040 1045 1050
   Ile Leu Ser Cys Ala Ser Lys Ser Met Ser Glu Gly Ile Pro Trp
   1055 1060 1065
   Pro Ser Ser Glu Asp Met Gly Thr Ala Arg Ser
   1070 1075
<210> 51
   <211> 3240
   <212> DNA
   <213> Homo sapiens
<400> 51
   atggtctgtt cggctgcccc actgctgctc ctggccacaa ctcttcccct gctggggtca 60
   ccagttgccc aagcatccca acctgtaagt gagactgggg tgagacccag ggaaggtctg 120
   cagaggcgac aatggggacc cctgattggg agagacaaag catggaatga aaggatagac 180
   agacccttcc ctgcctgccc catcccccta tcttctagct ttggccgatg gcccaagggc 240
   cagacaatgt gggcccagac ctccaccctc accctgacag aggaggagtt gggacagagt 300
   caggctggag gggaatctgg atctgggcag ctcctggacc aagagaatgg agcaggggaa 360
   tcagcgctgg tctccgtcta tgtacatctg gactttccag ataagacctg gccccctgaa 420
   ctctccagga cactgactct ccctgctgcc tcagcttcct cttccccaag gcctcttctc    480
   actggcctca gactcacaac agagtgtaat gtcaaccaca aggggaattt ctattgtgct    540
   tgcctctctg gctaccagtg gaacaccagc atctgcctcc attaccctcc ttgtcaaagc    600
   ctccacaacc accagccttg tggctgcctt gtcttcagcc atcccgaacc cgggtactgc    660
   cagttgctgc cacctgggtc ccctgtcacc tgcctccctg cagtccccgg gatcctcaac    720
   ctgaactccc agctgcagat gcctggtgac acgctgagcc tgactctcca tctgagccag    780
   gaggccacca acctgagctg gttcctgagg cacccaggga gccccagtcc catcctcctg    840
   cagccaggga cacaggtgtc tgtgacttcc agccacggcc aggctgccct cagcgtctcc    900
   aacatgtccc atcactgggc aggtgagtac atgagctgct tcgaggccca gggcttcaag    960
   tggaacctgt atgaggtggt gagggtgccc ttgaaggcga cagatgtggc tcgacttcca   1020
   taccagctgt ccatctcctg tgccacctcc cctggcttcc agctgagctg ctgcatcccc   1080
   agcacaaacc tggcctacac cgcggcctgg agccctggag agggcagcaa agcttcctcc   1140
   ttcaacgagt caggctctca gtgctttgtg ctggctgttc agcgctgccc gatggctgac   1200
   accacgtacg cttgtgacct gcagagcctg ggcctggctc cactcagggt ccccatctcc   1260
   atcaccatca tccaggatgg agacatcacc tgccctgagg acgcctcggt gctcacctgg   1320
   aatgtcacca aggctggcca cgtggcacag gccccatgtc ctgagagcaa gaggggcata   1380
   gtgaggaggc tctgtggggc tgacggagtc tgggggccgg tccacagcag ctgcacagat   1440
   gcgaggctcc tggccttgtt cactagaacc aagctgctgc aggcaggcca gggcagtcct   1500
   gctgaggagg tgccacagat cctggcacag ctgccagggc aggcggcaga ggcaagttca   1560
   ccctccgact tactgaccct gctgagcacc atgaaatacg tggccaaggt ggtggcagag   1620
   gccagaatac agcttgaccg cagagccctg aagaatctcc tgattgccac agacaaggtc   1680
   ctagatatgg acaccaggtc tctgtggacc ctggcccaag cccggaagcc ctgggcaggc   1740
   tcgactctcc tgctggctgt ggagaccctg gcatgcagcc tgtgcccaca ggaccacccc   1800
   ttcgccttca gcttacccaa tgtgctgctg cagagccagc tgtttggacc cacgtttcct   1860
   gctgactaca gcatctcctt ccctactcgg cccccactgc aggctcagat tcccaggcac   1920
   tcactggccc cattggtccg taatggaact gaaataagta ttactagcct ggtgctgcga   1980
   aaactggacc accttctgcc ctcaaactat ggacaagggc tgggggattc cctctatgcc   2040
   actcctggcc tggtccttgt catttccatc atggcaggtg accgggcctt cagccaggga   2100
   gaggtcatca tggactttgg gaacacagat ggttcccctc actgtgtctt ctgggatcac   2160
   agtctcttcc agggcagggg gggttggtcc aaagaagggt gccaggcaca ggtggccagt   2220
   gccagcccca ctgctcagtg cctctgccag cacctcactg ccttctccgt cctcatgtcc   2280
   ccacacactg ttccggaaga acccgctctg gcgctgctga ctcaagtggg cttgggagct   2340
   tccatactgg cgctgcttgt gtgcctgggt gtgtactggc tggtgtggag agtcgtggtg   2400
   cggaacaaga tctcctattt ccgccacgcc gccctgctca acatggtgtt ctgcttgctg   2460
   gccgcagaca cttgcttcct gggcgcccca ttcctctctc cagggccccg aagcccgctc 2520
   tgccttgctg ccgccttcct ctgtcatttc ctctacctgg ccaccttttt ctggatgctg 2580
   gcgcaggccc tggtgttggc ccaccagctg ctctttgtct ttcaccagct ggcaaagcac 2640
   cgagttctcc ccctcatggt gctcctgggc tacctgtgcc cactggggtt ggcaggtgtc 2700
   accctggggc tctacctacc tcaagggcaa tacctgaggg agggggaatg ctggttggat 2760
   gggaagggag gggcgttata caccttcgtg gggccagtgc tggccatcat aggcgtgaat 2820
   gggctggtac tagccatggc catgctgaag ttgctgagac cttcgctgtc agagggaccc 2880
   ccagcagaga agcgccaagc tctgctgggg gtgatcaaag ccctgctcat tcttacaccc 2940
   atctttggcc tcacctgggg gctgggcctg gccactctgt tagaggaagt ctccacggtc 3000
   cctcattaca tcttcaccat tctcaacacc ctccagggcg tcttcatcct attgtttggt 3060
   tgcctcatgg acaggaagat acaagaagct ttgcgcaaac gcttctgccg cgcccaagcc 3120
   cccagctcca ccatctccct ggtgagttgc tgccttcaga tcctcagctg tgcatccaag 3180
   agcatgtcag aaggcattcc atggccctcc tcagaggaca tgggcacagc cagaagctaa 3240
<210> 52
   <211> 321
   <212> PRT
   <213> Homo sapiens
<400> 52
   Met Ala Met Ala Thr Lys Gly Gly Thr Val Lys Ala Ala Ser Gly Phe
   1 5 10 15
   Asn Ala Met Glu Asp Ala Gln Thr Leu Arg Lys Ala Met Lys Gly Leu
   20 25 30
   Gly Thr Asp Glu Asp Ala Ile Ile Ser Val Leu Ala Tyr Arg Asn Thr
   35 40 45
   Ala Gln Arg Gln Glu Ile Arg Thr Ala Tyr Lys Ser Thr Ile Gly Arg
   50 55 60
   Asp Leu Ile Asp Asp Leu Lys Ser Glu Leu Ser Gly Asn Phe Glu Gln
   65 70 75 80
   Val Ile Val Gly Met Met Thr Pro Thr Val Leu Tyr Asp Val Gln Glu
   85 90 95
   Leu Arg Arg Ala Met Lys Gly Ala Gly Thr Asp Glu Gly Cys Leu Ile
   100 105 110
   Glu Ile Leu Ala Ser Arg Thr Pro Glu Glu Ile Arg Arg Ile Ser Gln
   115 120 125
   Thr Tyr Gln Gln Gln Tyr Gly Arg Ser Leu Glu Asp Asp Ile Arg Ser
   130 135 140
   Asp Thr Ser Phe Met Phe Gln Arg Val Leu Val Ser Leu Ser Ala Gly
   145                 150                 155                 160
   Gly Arg Asp Glu Gly Asn Tyr Leu Asp Asp Ala Leu Val Arg Gln Asp
                   165                 170                 175
   Ala Gln Asp Leu Tyr Glu Ala Gly Glu Lys Lys Trp Gly Thr Asp Glu
               180                 185                 190
   Val Lys Phe Leu Thr Val Leu Cys Ser Arg Asn Arg Asn His Leu Leu
           195                 200                 205
   His Val Phe Asp Glu Tyr Lys Arg Ile Ser Gln Lys Asp Ile Glu Gln
       210                 215                 220
   Ser Ile Lys Ser Glu Thr Ser Gly Ser Phe Glu Asp Ala Leu Leu Ala
   225                 230                 235                 240
   Ile Val Lys Cys Met Arg Asn Lys Ser Ala Tyr Phe Ala Glu Lys Leu
                   245                 250                 255
   Tyr Lys Ser Met Lys Gly Leu Gly Thr Asp Asp Asn Thr Leu Ile Arg
               260                 265                 270
   Val Met Val Ser Arg Ala Glu Ile Asp Met Leu Asp Ile Arg Ala His
           275                 280                 285
   Phe Lys Arg Leu Tyr Gly Lys Ser Leu Tyr Ser Phe Ile Lys Gly Asp
       290                 295                 300
   Thr Ser Gly Asp Tyr Arg Lys Val Leu Leu Val Leu Cys Gly Gly Asp
   305                 310                 315                 320
   Asp
<210> 53
   <211> 963
   <212> DNA
   <213> Homo sapiens
<400> 53
   atggccatgg caaccaaagg aggtactgtc aaagctgctt caggattcaa tgccatggaa     60
   gatgcccaga ccctgaggaa ggccatgaaa gggctcggca ccgatgaaga cgccattatt    120
   agcgtccttg cctaccgcaa caccgcccag cgccaggaga tcaggacagc ctacaagagc    180
   accatcggca gggacttgat agacgacctg aagtcagaac tgagtggcaa cttcgagcag    240
   gtgattgtgg ggatgatgac gcccacggtg ctgtatgacg tgcaagagct gcgaagggcc    300
   atgaagggag ccggcactga tgagggctgc ctaattgaga tcctggcctc ccggacccct    360
   gaggagatcc ggcgcataag ccaaacctac cagcagcaat atggacggag ccttgaagat 420
   gacattcgct ctgacacatc gttcatgttc cagcgagtgc tggtgtctct gtcagctggt 480
   gggagggatg aaggaaatta tctggacgat gctctcgtga gacaggatgc ccaggacctg 540
   tatgaggctg gagagaagaa atgggggaca gatgaggtga aatttctaac tgttctctgt 600
   tcccggaacc gaaatcacct gttgcatgtg tttgatgaat acaaaaggat atcacagaag 660
   gatattgaac agagtattaa atctgaaaca tctggtagct ttgaagatgc tctgctggct 720
   atagtaaagt gcatgaggaa caaatctgca tattttgctg aaaagctcta taaatcgatg 780
   aagggcttgg gcaccgatga taacaccctc atcagagtga tggtttctcg agcagaaatt 840
   gacatgttgg atatccgggc acacttcaag agactctatg gaaagtctct gtactcgttc 900
   atcaagggtg acacatctgg agactacagg aaagtactgc ttgttctctg tggaggagat 960
   gat 963
<210> 54
   <211> 318
   <212> PRT
   <213> Homo sapiens
<400> 54
   Ala Thr Lys Gly Gly Thr Val Lys Ala Ala Ser Gly Phe Asn Ala Met
   1 5 10 15
   Glu Asp Ala Gln Thr Leu Arg Lys Ala Met Lys Gly Leu Gly Thr Asp
   20 25 30
   Glu Asp Ala Ile Ile Ser Val Leu Ala Tyr Arg Asn Thr Ala Gln Arg
   35 40 45
   Gln Glu Ile Arg Thr Ala Tyr Lys Ser Thr Ile Gly Arg Asp Leu Ile
   50 55 60
   Asp Asp Leu Lys Ser Glu Leu Ser Gly Asn Phe Glu Gln Val Ile Val
   65 70 75 80
   Gly Met Met Thr Pro Thr Val Leu Tyr Asp Val Gln Glu Leu Arg Arg
   85 90 95
   Ala Met Lys Gly Ala Gly Thr Asp Glu Gly Cys Leu Ile Glu Ile Leu
   100 105 110
   Ala Ser Arg Thr Pro Glu Glu Ile Arg Arg Ile Ser Gln Thr Tyr Gln
   115 120 125
   Gln Gln Tyr Gly Arg Ser Leu Glu Asp Asp Ile Arg Ser Asp Thr Ser
   130 135 140
   Phe Met Phe Gln Arg Val Leu Val Ser Leu Ser Ala Gly Gly Arg Asp
   145 150 155 160
   Glu Gly Asn Tyr Leu Asp Asp Ala Leu Val Arg Gln Asp Ala Gln Asp
   165 170 175
   Leu Tyr Glu Ala Gly Glu Lys Lys Trp Gly Thr Asp Glu Val Lys Phe
   180 185 190
   Leu Thr Val Leu Cys Ser Arg Asn Arg Asn His Leu Leu His Val Phe
   195 200 205
   Asp Glu Tyr Lys Arg Ile Ser Gln Lys Asp Ile Glu Gln Ser Ile Lys
   210 215 220
   Ser Glu Thr Ser Gly Ser Phe Glu Asp Ala Leu Leu Ala Ile Val Lys
   225 230 235 240
   Cys Met Arg Asn Lys Ser Ala Tyr Phe Ala Glu Lys Leu Tyr Lys Ser
   245 250 255
   Met Lys Gly Leu Gly Thr Asp Asp Asn Thr Leu Ile Arg Val Met Val
   260 265 270
   Ser Arg Ala Glu Ile Asp Met Leu Asp Ile Arg Ala His Phe Lys Arg
   275 280 285
   Leu Tyr Gly Lys Ser Leu Tyr Ser Phe Ile Lys Gly Asp Thr Ser Gly
   290 295 300
   Asp Tyr Arg Lys Val Leu Leu Val Leu Cys Gly Gly Asp Asp
   305 310 315
<210> 55
   <211> 966
   <212> DNA
   <213> Homo sapiens
<400> 55
   atggccatgg caaccaaagg aggtactgtc aaagctgctt caggattcaa tgccatggaa 60
   gatgcccaga ccctgaggaa ggccatgaaa gggctcggca ccgatgaaga cgccattatt 120
   agcgtccttg cctaccgcaa caccgcccag cgccaggaga tcaggacagc ctacaagagc 180
   accatcggca gggacttgat agacgacctg aagtcagaac tgagtggcaa cttcgagcag 240
   gtgattgtgg ggatgatgac gcccacggtg ctgtatgacg tgcaagagct gcgaagggcc 300
   atgaagggag ccggcactga tgagggctgc ctaattgaga tcctggcctc ccggacccct 360
   gaggagatcc ggcgcataag ccaaacctac cagcagcaat atggacggag ccttgaagat 420
   gacattcgct ctgacacatc gttcatgttc cagcgagtgc tggtgtctct gtcagctggt 480
   gggagggatg aaggaaatta tctggacgat gctctcgtga gacaggatgc ccaggacctg 540
   tatgaggctg gagagaagaa atgggggaca gatgaggtga aatttctaac tgttctctgt 600
   tcccggaacc gaaatcacct gttgcatgtg tttgatgaat acaaaaggat atcacagaag 660
   gatattgaac agagtattaa atctgaaaca tctggtagct ttgaagatgc tctgctggct 720
   atagtaaagt gcatgaggaa caaatctgca tattttgctg aaaagctcta taaatcgatg 780
   aagggcttgg gcaccgatga taacaccctc atcagagtga tggtttctcg agcagaaatt 840
   gacatgttgg atatccgggc acacttcaag agactctatg gaaagtctct gtactcgttc 900
   atcaagggtg acacatctgg agactacagg aaagtactgc ttgttctctg tggaggagat 960
   gattaa 966
<210> 56
   <211> 1318
   <212> PRT
   <213> Homo sapiens
<400> 56
   Met Leu Arg Thr Ser Gly Leu Ala Leu Leu Ala Leu Val Ser Ala Val
   1               5                   10                  15
   Gly Pro Ser Gln Ala Ser Gly Phe Thr Glu Lys Gly Leu Ser Leu Leu
               20                  25                  30
   Gly Tyr Gln Leu Cys Ser His Arg Val Thr His Thr Val Gln Lys Val
           35                  40                  45
   Glu Ala Val Gln Thr Ser Tyr Thr Ser Tyr Val Ser Cys Gly Gly Trp
       50                  55                  60
   Ile Pro Trp Arg Arg Cys Pro Lys Met Val Tyr Arg Thr Gln Tyr Leu
   65                  70                  75                  80
   Val Val Glu Val Pro Glu Ser Arg Asn Val Thr Asp Cys Cys Glu Gly
                   85                  90                  95
   Tyr Glu Gln Leu Gly Leu Tyr Cys Val Leu Pro Leu Asn Gln Ser Gly
               100                 105                 110
   Gln Phe Thr Ser Arg Pro Gly Ala Cys Pro Ala Glu Gly Pro Glu Pro
           115                 120                 125
   Ser Thr Ser Pro Cys Ser Leu Asp Ile Asp Cys Pro Gly Leu Glu Lys
       130                 135                 140
   Cys Cys Pro Trp Ser Gly Gly Arg Tyr Cys Met Ala Pro Ala Pro Gln
   145                 150                 155                 160
   Ala Pro Glu Arg Asp Pro Val Gly Ser Trp Tyr Asn Val Thr Ile Leu
                   165                 170                 175
   Val Lys Met Asp Phe Lys Glu Leu Gln Gln Val Asp Pro Arg Leu Leu
   180 185 190
   Asn His Met Arg Leu Leu His Ser Leu Val Thr Ser Ala Leu Gln Pro
   195 200 205
   Met Ala Ser Thr Val His His Leu His Ser Ala Pro Gly Asn Ala Ser
   210 215 220
   Thr Thr Val Ser Arg Leu Leu Leu Gly Leu Pro Arg Pro Leu Pro Val
   225 230 235 240
   Ala Asp Val Ser Thr Leu Leu Gly Asp Ile Ala Lys Arg Val Tyr Glu
   245 250 255
   Val Ile Ser Val Gln Val Gln Asp Val Asn Glu Cys Phe Tyr Glu Glu
   260 265 270
   Leu Asn Ala Cys Ser Gly Arg Glu Leu Cys Ala Asn Leu Glu Gly Ser
   275 280 285
   Tyr Trp Cys Val Cys His Gln Glu Ala Pro Ala Thr Ser Pro Arg Lys
   290 295 300
   Leu Asn Leu Glu Trp Glu Asp Cys Pro Pro Val Ser Asp Tyr Val Val
   305 310 315 320
   Leu Asn Val Thr Ser Asp Ser Phe Gln Val Ser Trp Arg Leu Asn Ser
   325 330 335
   Thr Gln Asn His Thr Phe His Val Arg Val Tyr Arg Gly Met Glu Leu
   340 345 350
   Leu Arg Ser Ala Arg Thr Gln Ser Gln Ala Leu Ala Val Ala Gly Leu
   355 360 365
   Glu Ala Gly Val Leu Tyr Arg Val Lys Thr Ser Tyr Gln Gly Cys Gly
   370 375 380
   Ala Asp Val Ser Thr Thr Leu Thr Ile Lys Thr Asn Ala Gln Val Phe
   385 390 395 400
   Glu Val Thr Ile Lys Ile Val Asn His Asn Leu Thr Glu Lys Leu Leu
   405 410 415
   Asn Arg Ser Ser Val Glu Tyr Gln Asp Phe Ser Arg Gln Leu Leu His
   420 425 430
   Glu Val Glu Ser Ser Phe Pro Pro Val Val Ser Asp Leu Tyr Arg Ser
   435 440 445
   Gly Lys Leu Arg Met Gln Ile Val Ser Leu Gln Ala Gly Ser Val Val
   450 455 460
   Val Arg Leu Lys Leu Thr Val Gln Asp Pro Gly Phe Pro Met Gly Ile
   465 470 475 480
   Ser Thr Leu Ala Pro Ile Leu Gln Pro Leu Leu Ala Ser Thr Val Phe
   485 490 495
   Gln Ile Asp Arg Gln Gly Thr Arg Val Gln Asp Trp Asp Glu Cys Val
   500 505 510
   Asp Ser Ala Glu His Asp Cys Ser Pro Ala Ala Trp Cys Ile Asn Leu
   515 520 525
   Glu Gly Ser Tyr Thr Cys Gln Cys Arg Thr Thr Arg Asp Ala Thr Pro
   530 535 540
   Ser Arg Ala Gly Arg Ala Cys Glu Gly Asp Leu Val Ser Pro Met Gly
   545 550 555 560
   Gly Gly Leu Ser Ala Ala Thr Gly Val Thr Val Pro Gly Leu Gly Thr
   565 570 575
   Gly Thr Ala Ala Leu Gly Leu Glu Asn Phe Thr Leu Ser Pro Ser Pro
   580 585 590
   Gly Tyr Pro Gln Gly Thr Pro Ala Ala Gly Gln Ala Trp Thr Pro Glu
   595 600 605
   Pro Ser Pro Arg Arg Gly Gly Ser Asn Val Val Gly Tyr Asp Arg Asn
   610 615 620
   Asn Thr Gly Lys Gly Val Glu Gln Glu Leu Gln Gly Asn Ser Ile Met
   625 630 635 640
   Glu Pro Pro Ser Trp Pro Ser Pro Thr Glu Asp Pro Thr Gly His Phe
   645 650 655
   Leu Trp His Ala Thr Arg Ser Thr Arg Glu Thr Leu Leu Asn Pro Thr
   660 665 670
   Trp Leu Arg Asn Glu Asp Ser Gly Pro Ser Gly Ser Val Asp Leu Pro
   675 680 685
   Leu Thr Ser Thr Leu Thr Ala Leu Lys Thr Pro Ala Cys Val Pro Val
   690 695 700
   Ser Ile Gly Arg Ile Met Val Ser Asn Val Thr Ser Thr Gly Phe His
   705 710 715 720
   Leu Ala Trp Glu Ala Asp Leu Ala Met Asp Ser Thr Phe Gln Leu Thr
   725 730 735
   Leu Thr Ser Met Trp Ser Pro Ala Val Val Leu Glu Thr Trp Asn Thr
   740 745 750
   Ser Val Thr Leu Ser Gly Leu Glu Pro Gly Val Leu His Leu Val Glu
   755 760 765
   Ile Met Ala Lys Ala Cys Gly Lys Glu Gly Ala Arg Ala His Leu Lys
   770 775 780
   Val Arg Thr Ala Ala Arg Lys Leu Ile Gly Lys Val Arg Ile Lys Asn
   785 790 795 800
   Val Arg Tyr Ser Glu Ser Phe Arg Asn Ala Ser Ser Gln Glu Tyr Arg
   805 810 815
   Asp Phe Leu Glu Leu Phe Phe Arg Met Val Arg Gly Ser Leu Pro Ala
   820 825 830
   Thr Met Cys Gln His Met Asp Ala Gly Gly Val Arg Met Glu Val Val
   835 840 845
   Ser Val Thr Asn Gly Ser Ile Val Val Glu Phe His Leu Leu Ile Ile
   850 855 860
   Ala Asp Val Asp Val Gln Glu Val Ser Ala Ala Phe Leu Thr Ala Phe
   865 870 875 880
   Gln Thr Val Pro Leu Leu Glu Val Ile Arg Gly Asp Thr Phe Ile Gln
   885 890 895
   Asp Tyr Asp Glu Cys Glu Arg Lys Glu Asp Asp Cys Val Pro Gly Thr
   900 905 910
   Ser Cys Arg Asn Thr Leu Gly Ser Phe Thr Cys Ser Cys Glu Gly Gly
   915 920 925
   Ala Pro Asp Phe Pro Val Glu Tyr Ser Glu Arg Pro Cys Glu Gly Asp
   930 935 940
   Ser Pro Gly Asn Glu Thr Trp Ala Thr Ser Pro Glu Arg Pro Leu Thr
   945 950 955 960
   Thr Ala Gly Thr Lys Ala Ala Phe Val Gln Gly Thr Ser Pro Thr Pro
   965 970 975
   Gln Gly Leu Pro Gln Arg Leu Asn Leu Thr Gly Ala Val Arg Val Leu
   980 985 990
   Cys Glu Ile Glu Lys Val Val Val Ala Ile Gln Lys Arg Phe Leu Gln
   995 1000 1005
   Gln Glu Ser Ile Pro Glu Ser Ser Leu Tyr Leu Ser His Pro Ser
   1010 1015 1020
   Cys Asn Val Ser His Ser Asn Gly Thr His Val Leu Leu Glu Ala
   1025 1030 1035
   Gly Trp Ser Glu Cys Gly Thr Leu Met Gln Ser Asn Met Thr Asn
   1040 1045 1050
   Thr Val Val Arg Thr Thr Leu Arg Asn Asp Leu Ser Gln Glu Gly
   1055 1060 1065
   Ile Ile His His Leu Lys Ile Leu Ser Pro Ile Tyr Cys Ala Phe
   1070 1075 1080
   Gln Asn Asp Leu Leu Thr Ser Ser Gly Phe Thr Leu Glu Trp Gly
   1085 1090 1095
   Val Tyr Thr Ile Ile Glu Asp Leu His Gly Ala Gly Asn Phe Val
   1100 1105 1110
   Thr Glu Met Gln Leu Phe Ile Gly Asp Ser Pro Ile Pro Gln Asn
   1115 1120 1125
   Tyr Ser Val Ser Ala Ser Asp Asp Val Arg Ile Glu Val Gly Leu
   1130 1135 1140
   Tyr Arg Gln Lys Ser Asn Leu Lys Val Val Leu Thr Glu Cys Trp
   1145 1150 1155
   Ala Thr Pro Ser Ser Asn Ala Arg Asp Pro Ile Thr Phe Ser Phe
   1160 1165 1170
   Ile Asn Asn Ser Cys Pro Val Pro Asn Thr Tyr Thr Asn Val Ile
   1175 1180 1185
   Glu Asn Gly Asn Ser Asn Lys Ala Gln Phe Lys Leu Arg Ile Phe
   1190 1195 1200
   Ser Phe Ile Asn Asp Ser Ile Val Tyr Leu His Cys Lys Leu Arg
   1205 1210 1215
   Val Cys Met Glu Ser Pro Gly Ala Thr Cys Lys Ile Asn Cys Asn
   1220 1225 1230
   Asn Phe Arg Leu Leu Gln Asn Ser Glu Thr Ser Ala Thr His Gln
   1235 1240 1245
   Met Ser Trp Gly Pro Leu Ile Arg Ser Glu Gly Glu Pro Pro His
   1250 1255 1260
   Ala Glu Ala Gly Leu Gly Ala Gly Tyr Val Val Leu Ile Val Val
   1265 1270 1275
   Ala Ile Phe Val Leu Val Ala Gly Thr Ala Thr Leu Leu Ile Val
   1280 1285 1290
   Arg Tyr Gln Arg Met Asn Gly Arg Tyr Asn Phe Lys Ile Gln Ser
   1295 1300 1305
   Asn Asn Phe Ser Tyr Gln Val Phe Tyr Glu
   1310 1315
<210> 57
   <211> 3957
   <212> DNA
   <213> Homo sapiens
<400> 57
   atgctcagga cctcggggct ggcactgctg gctctggtca gtgctgtggg cccaagccag    60
   gccagcggct tcacagaaaa aggcctctcc ctgttgggct accagctatg cagccaccgt   120
   gtgacccaca ctgtacagaa ggtggaggcc gtgcagacgt cctacacgtc ctatgtgtcc   180
   tgcggcggct ggatcccctg gaggcggtgc cctaagatgg tttaccggac acagtacctg   240
   gtagtggagg tccccgagtc caggaacgtg actgactgct gtgagggcta tgaacagctc   300
   ggcctctact gtgtcttgcc cctgaatcag tccgggcagt tcacgtcaag acccggggcc   360
   tgccccgcag aggggcctga accatccacc tccccctgca gcttggacat cgactgtcct   420
   ggacttgaga agtgctgccc ctggtcaggg gggcgctact gcatggcccc tgcaccccaa   480
   gctccagaga gggaccctgt gggctcctgg tacaacgtca ccatactggt gaaaatggac   540
   ttcaaggaac tccagcaagt ggaccccagg ctcctgaacc acatgcgcct tctgcattcc   600
   ttggtcacca gcgccctgca accaatggcc tccaccgtcc accacctgca ctcagcccct   660
   gggaacgcct ccaccacagt gtcgcggctg ctactgggcc tgccacggcc actgcctgtg   720
   gctgacgtct ccaccctgct gggtgacatt gcgaagcgtg tctatgaagt gatcagcgtc   780
   caggtgcaag atgtcaatga gtgtttctat gaggagctca atgcctgctc tggaagggaa   840
   ctgtgcgcaa acctggaggg ctcgtactgg tgcgtctgtc accaggaagc tccagcgacg   900
   tctccacgga agctgaacct ggagtgggaa gattgtcctc cagtcagtga ctacgtggtc   960
   ctcaacgtca ccagtgacag ttttcaagta tcctggcgtt taaattctac acagaaccac  1020
   actttccatg tccgggttta ccggggtatg gagttgctca ggagcgccag gacacagagc  1080
   caggcactgg cagtggctgg gctggaggct ggagtgctgt acagggtgaa gaccagctac  1140
   caggggtgcg gggccgacgt ctccaccacg ctgaccatca aaaccaatgc ccaggtattt  1200
   gaagtcacaa taaagattgt aaaccacaac ctgacggaga agttactcaa ccgcagcagc  1260
   gtggagtacc aggacttttc tagacaactg cttcacgagg tcgagagctc cttcccacca  1320
   gtggtgtctg acttgtaccg aagtgggaag ctgagaatgc agatcgtgtc tctccaggcg 1380
   ggaagtgtgg tcgtgaggct caagctcacc gtgcaggacc ccgggtttcc catgggcatc 1440
   tccacgctgg cccccatact ccagcccctg ttggcaagca cagtgttcca gattgaccgg 1500
   caggggacac gcgtgcaaga ctgggacgag tgtgtggaca gcgcggaaca cgactgctca 1560
   ccggctgcct ggtgcatcaa cctggagggc tcctacacct gccagtgccg taccaccagg 1620
   gacgccaccc cctcccgcgc aggccgggcc tgtgagggtg acctggtgag ccccacgggc 1680
   ggtggactgt ctgcggcaac aggggtaacg gtcccaggtc ttggcacggg aacagcagcc 1740
   ctcggcctag agaacttcac cttgtcaccc agtcctgggt accctcaggg caccccggca 1800
   gcaggccagg cctggacccc agagccctca cccagaagag ggggcagcaa tgtggtcggg 1860
   tatgacagga acaacacagg aaaaggcgtg gagcaggagc tacagggaaa ctccaccatg 1920
   gagccaccct cctggccttc ccctactgag gaccccaccg gccacttcct gtggcatgcc 1980
   acccgttcca cccgggaaac acttctgaat cccacgtggc tgcgaaatga ggacagtgga 2040
   ccctccggtt ctgtagacct gccattgacc tccaccctca cagctctgaa gacccccgcc 2100
   tgtgttcctg tctccattgg gaggatcatg gtctccaatg tgaccagcac cggcttccac 2160
   ctggcatggg aggcggatct tgctatggac tccaccttcc agctcactct gacttccatg 2220
   tggagccctg ctgtggtcct agagacctgg aacacgagtg tgacactgtc ggggctggag 2280
   cctggggtct tgcacctggt tgagatcatg gccaaagcat gtgggaaaga aggtgccaga 2340
   gctcatctga aagtgaggac agcagcccgg aagctcattg gaaaggtcag aatcaaaaat 2400
   gtcaggtact cagaatcctt tcgcaacgca agcagccagg agtatcgaga tttcctagaa 2460
   ctattcttca ggatggtgcg gggctccctg ccagccacca tgtgtcagca catggacgct 2520
   ggtggggtca ggatggaagt cgtcagcgtc accaacggca gcatcgtggt ggagtttcac 2580
   ttgctgataa tcgcagatgt ggatgtccag gaggtgtcag ctgcatttct caccgccttc 2640
   cagaccgtgc ctctgctgga ggtgatcaga ggcgacacct tcatacagga ttacgatgag 2700
   tgtgaaagga aggaggacga ctgtgtgccg gggacatcct gtcgaaacac cctcgggtct 2760
   ttcacttgta gctgcgaggg aggagccccc gacttccctg tggaatattc tgagagaccc 2820
   tgtgaaggtg actctcctgg caatgaaacc tgggccacca gcccagagag gcctctcacc 2880
   acagcaggga ccaaggctgc ctttgtgcaa ggcaccagcc ccacccccca aggcctgccc 2940
   cagcggctga acctgaccgg agcagtcagg gtgctctgtg agatcgagaa ggtggttgtc 3000
   gccatccaga agcgcttcct gcagcaggaa tccatccccg agtcctcgtt gtacctcagc 3060
   cacccctcct gcaacgtgag ccacagcaat ggcacacacg tgctcctgga ggccggctgg 3120
   agcgagtgtg ggaccctcat gcagagcaac atgacgaaca ccgtggtgag gaccacgctg 3180
   aggaacgacc tgtcccagga gggcatcatc caccacctga agatcctgag ccccatctac 3240
   tgcgccttcc agaatgacct gctgacatcc tccggcttca ccctggagtg gggggtttac 3300
   accatcatcg aggacctcca cggcgctggg aattttgtta ccgaaatgca gttgtttatc 3360
   ggagactctc ccatacctca gaattatagc gtgtctgcca gtgacgatgt caggatcgaa 3420
   gtggggctct acaggcagaa aagcaacctc aaggtggtcc tgacggagtg ctgggcaacc 3480
   ccgtctagca acgcccggga ccccatcacc ttcagcttca ttaacaacag ctgccccgtg 3540
   cccaacacat acaccaacgt gattgagaac ggcaactcca ataaggccca gttcaagctg 3600
   aggatctttt cctttatcaa cgactccatc gtctacctgc actgcaaact ccgcgtctgc 3660
   atggaatccc ccggagccac gtgcaaaatc aattgcaata actttcggtt gctgcaaaat 3720
   agtgaaacct ctgccacaca ccagatgtcc tggggacccc tcatccggtc tgaaggtgag 3780
   cctccacatg cagaagcagg cctgggtgcc ggttatgtgg tccttattgt ggtggccatc 3840
   ttcgtgctgg tggcgggaac agccaccctt ctgatcgtgc gctaccagag aatgaatggg 3900
   agatacaact ttaaaatcca gtcccacaac ttcagctacc aggtgttcta cgaatag 3957
<210> 58
   <211> 1446
   <212> PRT
   <213> Homo sapiens
<400> 58
   Met Leu Arg Thr Ser Gly Leu Ala Leu Leu Ala Leu Val Ser Ala Val
   1               5                   10                  15
   Gly Pro Ser Gln Ala Ser Gly Phe Thr Glu Lys Gly Leu Ser Leu Leu
               20                  25                  30
   Gly Tyr Gln Leu Cys Ser His Arg Val Thr His Thr Val Gln Lys Val
           35                  40                  45
   Glu Ala Val Gln Thr Ser Tyr Thr Ser Tyr Val Ser Cys Gly Gly Trp
       50                  55                  60
   Ile Pro Trp Arg Arg Cys Pro Lys Met Val Tyr Arg Thr Gln Tyr Leu
   65                  70                  75                  80
   Val Val Glu Val Pro Glu Ser Arg Asn Val Thr Asp Cys Cys Glu Gly
                   85                  90                  95
   Tyr Glu Gln Leu Gly Leu Tyr Cys Val Leu Pro Leu Asn Gln Ser Gly
               100                 105                 110
   Gln Phe Thr Ser Arg Pro Gly Ala Cys Pro Ala Glu Gly Pro Glu Pro
           115                 120                 125
   Ser Thr Ser Pro Cys Ser Leu Asp Ile Asp Cys Pro Gly Leu Glu Lys
       130                 135                 140
   Cys Cys Pro Trp Ser Gly Gly Arg Tyr Cys Met Ala Pro Ala Pro Gln
   145                 150                 155                 160
   Ala Pro Glu Arg Asp Pro Val Gly Ser Trp Tyr Asn Val Thr Ile Leu
   165 170 175
   Val Lys Met Asp Phe Lys Glu Leu Gln Gln Val Asp Pro Arg Leu Leu
   180 185 190
   Asn His Met Arg Leu Leu His Ser Leu Val Thr Ser Ala Leu Gln Pro
   195 200 205
   Met Ala Ser Thr Val His His Leu His Ser Ala Pro Gly Asn Ala Ser
   210 215 220
   Thr Thr Val Ser Arg Leu Leu Leu Gly Leu Pro Arg Pro Leu Pro Val
   225 230 235 240
   Ala Asp Val Ser Thr Leu Leu Gly Asp Ile Ala Lys Arg Val Tyr Glu
   245 250 255
   Val Ile Ser Val Gln Val Gln Asp Val Asn Glu Cys Phe Tyr Glu Glu
   260 265 270
   Leu Asn Ala Cys Ser Gly Arg Glu Leu Cys Ala Asn Leu Glu Gly Ser
   275 280 285
   Tyr Trp Cys Val Cys His Gln Glu Ala Pro Ala Thr Ser Pro Arg Lys
   290 295 300
   Leu Asn Leu Glu Trp Glu Asp Cys Pro Pro Val Ser Asp Tyr Val Val
   305 310 315 320
   Leu Asn Val Thr Ser Asp Ser Phe Gln Val Ser Trp Arg Leu Asn Ser
   325 330 335
   Thr Gln Asn His Thr Phe His Val Arg Val Tyr Arg Gly Met Glu Leu
   340 345 350
   Leu Arg Ser Ala Arg Thr Gln Ser Gln Ala Leu Ala Val Ala Gly Leu
   355 360 365
   Glu Ala Gly Val Leu Tyr Arg Val Lys Thr Ser Tyr Gln Gly Cys Gly
   370 375 380
   Ala Asp Val Ser Thr Thr Leu Thr Ile Lys Thr Asn Ala Gln Val Phe
   385 390 395 400
   Glu Val Thr Ile Lys Ile Val Asn His Asn Leu Thr Glu Lys Leu Leu
   405 410 415
   Asn Arg Ser Ser Val Glu Tyr Gln Asp Phe Ser Arg Gln Leu Leu His
   420 425 430
   Glu Val Glu Ser Ser Phe Pro Pro Val Val Ser Asp Leu Tyr Arg Ser
           435                 440                 445
   Gly Lys Leu Arg Met Gln Ile Val Ser Leu Gln Ala Gly Ser Val Val
       450                 455                 460
   Val Arg Leu Lys Leu Thr Val Gln Asp Pro Gly Phe Pro Met Gly Ile
   465                 470                 475                 480
   Ser Thr Leu Ala Pro Ile Leu Gln Pro Leu Leu Ala Ser Thr Val Phe
                   485                 490                 495
   Gln Ile Asp Arg Gln Gly Thr Arg Val Gln Asp Trp Asp Glu Cys Val
               500                 505                 510
   Asp Ser Ala Glu His Asp Cys Ser Pro Ala Ala Trp Cys Ile Asn Leu
           515                 520                 525
   Glu Gly Ser Tyr Thr Cys Gln Cys Arg Thr Thr Arg Asp Ala Thr Pro
       530                 535                 540
   Ser Arg Ala Gly Arg Ala Cys Glu Gly Asp Leu Val Ser Pro Met Gly
   545                 550                 555                 560
   Gly Gly Leu Ser Ala Ala Thr Gly Val Thr Val Pro Gly Leu Gly Thr
                   565                 570                 575
   Gly Thr Ala Ala Leu Gly Leu Glu Asn Phe Thr Leu Ser Pro Ser Pro
               580                 585                 590
   Gly Tyr Pro Gln Gly Thr Pro Ala Ala Gly Gln Ala Trp Thr Pro Glu
           595                 600                 605
   Pro Ser Pro Arg Arg Gly Gly Ser Asn Val Val Gly Tyr Asp Arg Asn
       610                 615                 620
   Asn Thr Gly Lys Gly Val Glu Gln Glu Val Pro Ser Thr Ala Pro Gly
   625                 630                 635                 640
   Leu Gly Met Asp Gln Gly Ser Pro Ser Gln Val Asn Pro Ser Gln Gly
                   645                 650                 655
   Ser Pro Ser Gln Gly Ser Leu Arg Gln Glu Ser Thr Ser Gln Ala Ser
               660                 665                 670
   Pro Ser Gln Arg Ser Thr Ser Gln Gly Ser Pro Ser Gln Val Asn Pro
           675                 680                 685
   Ser Gln Gly Ser Thr Ser His Ala Asn Ser Ser Gln Gly Ser Pro Ser
       690                 695                 700
   Gln Gly Ser Pro Ser Gln Glu Ser Pro Ser Gln Gly Ser Thr Ser Gln
   705 710 715 720
   Ala Ser Pro Ser His Arg Asn Thr Ile Gly Val Ile Gly Thr Thr Ser
   725 730 735
   Ser Pro Lys Ala Thr Gly Ser Thr His Ser Phe Pro Pro Gly Ala Thr
   740 745 750
   Asp Gly Pro Leu Ala Leu Pro Gly Gln Leu Gln Gly Asn Ser Ile Met
   755 760 765
   Glu Pro Pro Ser Trp Pro Ser Pro Thr Glu Asp Pro Thr Gly His Phe
   770 775 780
   Leu Trp His Ala Thr Arg Ser Thr Arg Glu Thr Leu Leu Asn Pro Thr
   785 790 795 800
   Trp Leu Arg Asn Glu Asp Ser Gly Pro Ser Gly Ser Val Asp Leu Pro
   805 810 815
   Leu Thr Ser Thr Leu Thr Ala Leu Lys Thr Pro Ala Cys Val Pro Val
   820 825 830
   Ser Ile Gly Arg Ile Met Val Ser Asn Val Thr Ser Thr Gly Phe His
   835 840 845
   Leu Ala Trp Glu Ala Asp Leu Ala Met Asp Ser Thr Phe Gln Leu Thr
   850 855 860
   Leu Thr Ser Met Trp Ser Pro Ala Val Val Leu Glu Thr Trp Asn Thr
   865 870 875 880
   Ser Val Thr Leu Ser Gly Leu Glu Pro Gly Val Leu His Leu Val Glu
   885 890 895
   Ile Met Ala Lys Ala Cys Gly Lys Glu Gly Ala Arg Ala His Leu Lys
   900 905 910
   Val Arg Thr Ala Ala Arg Lys Leu Ile Gly Lys Val Arg Ile Lys Asn
   915 920 925
   Val Arg Tyr Ser Glu Ser Phe Arg Asn Ala Ser Ser Gln Glu Tyr Arg
   930 935 940
   Asp Phe Leu Glu Leu Phe Phe Arg Met Val Arg Gly Ser Leu Pro Ala
   945 950 955 960
   Thr Met Cys Gln His Met Asp Ala Gly Gly Val Arg Met Glu Val Val
   965 970 975
   Ser Val Thr Asn Gly Ser Ile Val Val Glu Phe His Leu Leu Ile Ile
   980 985 990
   Ala Asp Val Asp Val Gln Glu Val Ser Ala Ala Phe Leu Thr Ala Phe
   995 1000 1005
   Gln Thr Val Pro Leu Leu Glu Val Ile Arg Gly Asp Thr Phe Ile
   1010 1015 1020
   Gln Asp Tyr Asp Glu Cys Glu Arg Lys Glu Asp Asp Cys Val Pro
   1025 1030 1035
   Gly Thr Ser Cys Arg Asn Thr Leu Gly Ser Phe Thr Cys Ser Cys
   1040 1045 1050
   Glu Gly Gly Ala Pro Asp Phe Pro Val Glu Tyr Ser Glu Arg Pro
   1055 1060 1065
   Cys Glu Gly Asp Ser Pro Gly Asn Glu Thr Trp Ala Thr Ser Pro
   1070 1075 1080
   Glu Arg Pro Leu Thr Thr Ala Gly Thr Lys Ala Ala Phe Val Gln
   1085 1090 1095
   Gly Thr Ser Pro Thr Pro Gln Gly Leu Pro Gln Arg Leu Asn Leu
   1100 1105 1110
   Thr Gly Ala Val Arg Val Leu Cys Glu Ile Glu Lys Val Val Val
   1115 1120 1125
   Ala Ile Gln Lys Arg Phe Leu Gln Gln Glu Ser Ile Pro Glu Ser
   1130 1135 1140
   Ser Leu Tyr Leu Ser His Pro Ser Cys Asn Val Ser His Ser Asn
   1145 1150 1155
   Gly Thr His Val Leu Leu Glu Ala Gly Trp Ser Glu Cys Gly Thr
   1160 1165 1170
   Leu Met Gln Ser Asn Met Thr Asn Thr Val Val Arg Thr Thr Leu
   1175 1180 1185
   Arg Asn Asp Leu Ser Gln Glu Gly Ile Ile His His Leu Lys Ile
   1190 1195 1200
   Leu Ser Pro Ile Tyr Cys Ala Phe Gln Asn Asp Leu Leu Thr Ser
   1205 1210 1215
   Ser Gly Phe Thr Leu Glu Trp Gly Val Tyr Thr Ile Ile Glu Asp
   1220 1225 1230
   Leu His Gly Ala Gly Asn Phe Val Thr Glu Met Gln Leu Phe Ile
   1235 1240 1245
   Gly Asp Ser Pro Ile Pro Gln Asn Tyr Ser Val Ser Ala Ser Asp
   1250 1255 1260
   Asp Val Arg Ile Glu Val Gly Leu Tyr Arg Gln Lys Ser Asn Leu
   1265 1270 1275
   Lys Val Val Leu Thr Glu Cys Trp Ala Thr Pro Ser Ser Asn Ala
   1280 1285 1290
   Arg Asp Pro Ile Thr Phe Ser Phe Ile Asn Asn Ser Cys Pro Val
   1295 1300 1305
   Pro Asn Thr Tyr Thr Asn Val Ile Glu Asn Gly Asn Ser Asn Lys
   1310 1315 1320
   Ala Gln Phe Lys Leu Arg Ile Phe Ser Phe Ile Asn Asp Ser Ile
   1325 1330 1335
   Val Tyr Leu His Cys Lys Leu Arg Val Cys Met Glu Ser Pro Gly
   1340 1345 1350
   Ala Thr Cys Lys Ile Asn Cys Asn Asn Phe Arg Leu Leu Gln Asn
   1355 1360 1365
   Ser Glu Thr Ser Ala Thr His Gln Met Ser Trp Gly Pro Leu Ile
   1370 1375 1380
   Arg Ser Glu Gly Glu Pro Pro His Ala Glu Ala Gly Leu Gly Ala
   1385 1390 1395
   Gly Tyr Val Val Leu Ile Val Val Ala Ile Phe Val Leu Val Ala
   1400 1405 1410
   Gly Thr Ala Thr Leu Leu Ile Val Arg Tyr Gln Arg Met Asn Gly
   1415 1420 1425
   Arg Tyr Asn Phe Lys Ile Gln Ser Asn Asn Phe Ser Tyr Gln Val
   1430 1435 1440
   Phe Tyr Glu
   1445
<210> 59
   <211> 1246
   <212> PRT
   <213> Homo sapiens
<400> 59
   Met Val Tyr Arg Thr Gln Tyr Leu Val Val Glu Val Pro Glu Ser Arg
   1               5                   10                  15
   Asn Val Thr Asp Cys Cys Glu Gly Tyr Glu Gln Leu Gly Leu Tyr Cys
               20                  25                  30
   Val Leu Pro Leu Asn Gln Ser Gly Gln Phe Thr Ser Arg Pro Gly Ala
           35                  40                  45
   Cys Pro Ala Glu Gly Pro Glu Pro Ser Thr Ser Pro Cys Ser Leu Asp
       50                  55                  60
   Ile Asp Cys Pro Gly Leu Glu Lys Cys Cys Pro Trp Ser Gly Gly Arg
   65                  70                  75                  80
   Tyr Cys Met Ala Pro Ala Pro Gln Ala Pro Glu Arg Asp Pro Val Gly
                   85                  90                  95
   Ser Trp Tyr Asn Val Thr Ile Leu Val Lys Met Asp Phe Lys Glu Leu
               100                 105                 110
   Gln Gln Val Asp Pro Arg Leu Leu Asn His Met Arg Leu Leu His Ser
           115                 120                 125
   Leu Val Thr Ser Ala Leu Gln Pro Met Ala Ser Thr Val His His Leu
       130                 135                 140
   His Ser Ala Pro Gly Asn Ala Ser Thr Thr Val Ser Arg Leu Leu Leu
   145                 150                 155                 160
   Gly Leu Pro Arg Pro Leu Pro Val Ala Asp Val Ser Thr Leu Leu Gly
                   165                 170                 175
   Asp Ile Ala Lys Arg Val Tyr Glu Val Ile Ser Val Gln Val Gln Asp
               180                 185                 190
   Val Asn Glu Cys Phe Tyr Glu Glu Leu Asn Ala Cys Ser Gly Arg Glu
           195                 200                 205
   Leu Cys Ala Asn Leu Glu Gly Ser Tyr Trp Cys Val Cys His Gln Glu
       210                 215                 220
   Ala Pro Ala Thr Ser Pro Arg Lys Leu Asn Leu Glu Trp Glu Asp Cys
   225                 230                 235                 240
   Pro Pro Val Ser Asp Tyr Val Val Leu Asn Val Thr Ser Asp Ser Phe
                   245                 250                 255
   Gln Val Ser Trp Arg Leu Asn Ser Thr Gln Asn His Thr Phe His Val
               260                 265                 270
   Arg Val Tyr Arg Gly Met Glu Leu Leu Arg Ser Ala Arg Thr Gln Ser
   275 280 285
   Gln Ala Leu Ala Val Ala Gly Leu Glu Ala Gly Val Leu Tyr Arg Val
   290 295 300
   Lys Thr Ser Tyr Gln Gly Cys Gly Ala Asp Val Ser Thr Thr Leu Thr
   305 310 315 320
   Ile Lys Thr Asn Ala Gln Val Phe Glu Val Thr Ile Lys Ile Val Asn
   325 330 335
   His Asn Leu Thr Glu Lys Leu Leu Asn Arg Ser Ser Val Glu Tyr Gln
   340 345 350
   Asp Phe Ser Arg Gln Leu Leu His Glu Val Glu Ser Ser Phe Pro Pro
   355 360 365
   Val Val Ser Asp Leu Tyr Arg Ser Gly Lys Leu Arg Met Gln Ile Val
   370 375 380
   Ser Leu Gln Ala Gly Ser Val Val Val Arg Leu Lys Leu Thr Val Gln
   385 390 395 400
   Asp Pro Gly Phe Pro Met Gly Ile Ser Thr Leu Ala Pro Ile Leu Gln
   405 410 415
   Pro Leu Leu Ala Ser Thr Val Phe Gln Ile Asp Arg Gln Gly Thr Arg
   420 425 430
   Val Gln Asp Trp Asp Glu Cys Val Asp Ser Ala Glu His Asp Cys Ser
   435 440 445
   Pro Ala Ala Trp Cys Ile Asn Leu Glu Gly Ser Tyr Thr Cys Gln Cys
   450 455 460
   Arg Thr Thr Arg Asp Ala Thr Pro Ser Arg Ala Gly Arg Ala Cys Glu
   465 470 475 480
   Gly Asp Leu Val Ser Pro Met Gly Gly Gly Leu Ser Ala Ala Thr Gly
   485 490 495
   Val Thr Val Pro Gly Leu Gly Thr Gly Thr Ala Ala Leu Gly Leu Glu
   500 505 510
   Asn Phe Thr Leu Ser Pro Ser Pro Gly Tyr Pro Gln Gly Thr Pro Ala
   515 520 525
   Ala Gly Gln Ala Trp Thr Pro Glu Pro Ser Pro Arg Arg Gly Gly Ser
   530 535 540
   Asn Val Val Gly Tyr Asp Arg Asn Asn Thr Gly Lys Gly Val Glu Gln
   545                 550                 555                 560
   Glu Leu Gln Gly Asn Ser Ile Met Glu Pro Pro Ser Trp Pro Ser Pro
                   565                 570                 575
   Thr Glu Asp Pro Thr Gly His Phe Leu Trp His Ala Thr Arg Ser Thr
               580                 585                 590
   Arg Glu Thr Leu Leu Asn Pro Thr Trp Leu Arg Asn Glu Asp Ser Gly
           595                 600                 605
   Pro Ser Gly Ser Val Asp Leu Pro Leu Thr Ser Thr Leu Thr Ala Leu
       610                 615                 620
   Lys Thr Pro Ala Cys Val Pro Val Ser Ile Gly Arg Ile Met Val Ser
   625                 630                 635                 640
   Asn Val Thr Ser Thr Gly Phe His Leu Ala Trp Glu Ala Asp Leu Ala
                   645                 650                 655
   Met Asp Ser Thr Phe Gln Leu Thr Leu Thr Ser Met Trp Ser Pro Ala
               660                 665                 670
   Val Val Leu Glu Thr Trp Asn Thr Ser Val Thr Leu Ser Gly Leu Glu
           675                 680                 685
   Pro Gly Val Leu His Leu Val Glu Ile Met Ala Lys Ala Cys Gly Lys
       690                 695                 700
   Glu Gly Ala Arg Ala His Leu Lys Val Arg Thr Ala Ala Arg Lys Leu
   705                 710                 715                 720
   Ile Gly Lys Val Arg Ile Lys Asn Val Arg Tyr Ser Glu Ser Phe Arg
                   725                 730                 735
   Asn Ala Ser Ser Gln Glu Tyr Arg Asp Phe Leu Glu Leu Phe Phe Arg
               740                 745                 750
   Met Val Arg Gly Ser Leu Pro Ala Thr Met Cys Gln His Met Asp Ala
           755                 760                 765
   Gly Gly Val Arg Met Glu Val Val Ser Val Thr Asn Gly Ser Ile Val
       770                 775                 780
   Val Glu Phe His Leu Leu Ile Ile Ala Asp Val Asp Val Gln Glu Val
   785                 790                 795                 800
   Ser Ala Ala Phe Leu Thr Ala Phe Gln Thr Val Pro Leu Leu Glu Val
                   805                 810                 815
   Ile Arg Gly Asp Thr Phe Ile Gln Asp Tyr Asp Glu Cys Glu Arg Lys
   820 825 830
   Glu Asp Asp Cys Val Pro Gly Thr Ser Cys Arg Asn Thr Leu Gly Ser
   835 840 845
   Phe Thr Cys Ser Cys Glu Gly Gly Ala Pro Asp Phe Pro Val Glu Tyr
   850 855 860
   Ser Glu Arg Pro Cys Glu Gly Asp Ser Pro Gly Asn Glu Thr Trp Ala
   865 870 875 880
   Thr Ser Pro Glu Arg Pro Leu Thr Thr Ala Gly Thr Lys Ala Ala Phe
   885 890 895
   Val Gln Gly Thr Ser Pro Thr Pro Gln Gly Leu Pro Gln Arg Leu Asn
   900 905 910
   Leu Thr Gly Ala Val Arg Val Leu Cys Glu Ile Glu Lys Val Val Val
   915 920 925
   Ala Ile Gln Lys Arg Phe Leu Gln Gln Glu Ser Ile Pro Glu Ser Ser
   930 935 940
   Leu Tyr Leu Ser His Pro Ser Cys Asn Val Ser His Ser Asn Gly Thr
   945 950 955 960
   His Val Leu Leu Glu Ala Gly Trp Ser Glu Cys Gly Thr Leu Met Gln
   965 970 975
   Ser Asn Met Thr Asn Thr Val Val Arg Thr Thr Leu Arg Asn Asp Leu
   980 985 990
   Ser Gln Glu Gly Ile Ile His His Leu Lys Ile Leu Ser Pro Ile Tyr
   995 1000 1005
   Cys Ala Phe Gln Asn Asp Leu Leu Thr Ser Ser Gly Phe Thr Leu
   1010 1015 1020
   Glu Trp Gly Val Tyr Thr Ile Ile Glu Asp Leu His Gly Ala Gly
   1025 1030 1035
   Asn Phe Val Thr Glu Met Gln Leu Phe Ile Gly Asp Ser Pro Ile
   1040 1045 1050
   Pro Gln Asn Tyr Ser Val Ser Ala Ser Asp Asp Val Arg Ile Glu
   1055 1060 1065
   Val Gly Leu Tyr Arg Gln Lys Ser Asn Leu Lys Val Val Leu Thr
   1070 1075 1080
   Glu Cys Trp Ala Thr Pro Ser Ser Asn Ala Arg Asp Pro Ile Thr
   1085 1090 1095
   Phe Ser Phe Ile Asn Asn Ser Cys Pro Val Pro Asn Thr Tyr Thr
   1100 1105 1110
   Asn Val Ile Glu Asn Gly Asn Ser Asn Lys Ala Gln Phe Lys Leu
   1115 1120 1125
   Arg Ile Phe Ser Phe Ile Asn Asp Ser Ile Val Tyr Leu His Cys
   1130 1135 1140
   Lys Leu Arg Val Cys Met Glu Ser Pro Gly Ala Thr Cys Lys Ile
   1145 1150 1155
   Asn Cys Asn Asn Phe Arg Leu Leu Gln Asn Ser Glu Thr Ser Ala
   1160 1165 1170
   Thr His Gln Met Ser Trp Gly Pro Leu Ile Arg Ser Glu Gly Glu
   1175 1180 1185
   Pro Pro His Ala Glu Ala Gly Leu Gly Ala Gly Tyr Val Val Leu
   1190 1195 1200
   Ile Val Val Ala Ile Phe Val Leu Val Ala Gly Thr Ala Thr Leu
   1205 1210 1215
   Leu Ile Val Arg Tyr Gln Arg Met Asn Gly Arg Tyr Asn Phe Lys
   1220 1225 1230
   Ile Gln Ser Asn Asn Phe Ser Tyr Gln Val Phe Tyr Glu
   1235 1240 1245
<210> 60
   <211> 1374
   <212> PRT
   <213> Homo sapiens
<400> 60
   Met Val Tyr Arg Thr Gln Tyr Leu Val Val Glu Val Pro Glu Ser Arg
   1               5                   10                  15
   Asn Val Thr Asp Cys Cys Glu Gly Tyr Glu Gln Leu Gly Leu Tyr Cys
               20                  25                  30
   Val Leu Pro Leu Asn Gln Ser Gly Gln Phe Thr Ser Arg Pro Gly Ala
           35                  40                  45
   Cys Pro Ala Glu Gly Pro Glu Pro Ser Thr Ser Pro Cys Ser Leu Asp
       50                  55                  60
   Ile Asp Cys Pro Gly Leu Glu Lys Cys Cys Pro Trp Ser Gly Gly Arg
   65 70 75 80
   Tyr Cys Met Ala Pro Ala Pro Gln Ala Pro Glu Arg Asp Pro Val Gly
   85 90 95
   Ser Trp Tyr Asn Val Thr Ile Leu Val Lys Met Asp Phe Lys Glu Leu
   100 105 110
   Gln Gln Val Asp Pro Arg Leu Leu Asn His Met Arg Leu Leu His Ser
   115 120 125
   Leu Val Thr Ser Ala Leu Gln Pro Met Ala Ser Thr Val His His Leu
   130 135 140
   His Ser Ala Pro Gly Asn Ala Ser Thr Thr Val Ser Arg Leu Leu Leu
   145 150 155 160
   Gly Leu Pro Arg Pro Leu Pro Val Ala Asp Val Ser Thr Leu Leu Gly
   165 170 175
   Asp Ile Ala Lys Arg Val Tyr Glu Val Ile Ser Val Gln Val Gln Asp
   180 185 190
   Val Asn Glu Cys Phe Tyr Glu Glu Leu Asn Ala Cys Ser Gly Arg Glu
   195 200 205
   Leu Cys Ala Asn Leu Glu Gly Ser Tyr Trp Cys Val Cys His Gln Glu
   210 215 220
   Ala Pro Ala Thr Ser Pro Arg Lys Leu Asn Leu Glu Trp Glu Asp Cys
   225 230 235 240
   Pro Pro Val Ser Asp Tyr Val Val Leu Asn Val Thr Ser Asp Ser Phe
   245 250 255
   Gln Val Ser Trp Arg Leu Asn Ser Thr Gln Asn His Thr Phe His Val
   260 265 270
   Arg Val Tyr Arg Gly Met Glu Leu Leu Arg Ser Ala Arg Thr Gln Ser
   275 280 285
   Gln Ala Leu Ala Val Ala Gly Leu Glu Ala Gly Val Leu Tyr Arg Val
   290 295 300
   Lys Thr Ser Tyr Gln Gly Cys Gly Ala Asp Val Ser Thr Thr Leu Thr
   305 310 315 320
   Ile Lys Thr Asn Ala Gln Val Phe Glu Val Thr Ile Lys Ile Val Asn
   325 330 335
   His Asn Leu Thr Glu Lys Leu Leu Asn Arg Ser Ser Val Glu Tyr Gln
   340 345 350
   Asp Phe Ser Arg Gln Leu Leu His Glu Val Glu Ser Ser Phe Pro Pro
   355 360 365
   Val Val Ser Asp Leu Tyr Arg Ser Gly Lys Leu Arg Met Gln Ile Val
   370 375 380
   Ser Leu Gln Ala Gly Ser Val Val Val Arg Leu Lys Leu Thr Val Gln
   385 390 395 400
   Asp Pro Gly Phe Pro Met Gly Ile Ser Thr Leu Ala Pro Ile Leu Gln
   405 410 415
   Pro Leu Leu Ala Ser Thr Val Phe Gln Ile Asp Arg Gln Gly Thr Arg
   420 425 430
   Val Gln Asp Trp Asp Glu Cys Val Asp Ser Ala Glu His Asp Cys Ser
   435 440 445
   Pro Ala Ala Trp Cys Ile Asn Leu Glu Gly Ser Tyr Thr Cys Gln Cys
   450 455 460
   Arg Thr Thr Arg Asp Ala Thr Pro Ser Arg Ala Gly Arg Ala Cys Glu
   465 470 475 480
   Gly Asp Leu Val Ser Pro Met Gly Gly Gly Leu Ser Ala Ala Thr Gly
   485 490 495
   Val Thr Val Pro Gly Leu Gly Thr Gly Thr Ala Ala Leu Gly Leu Glu
   500 505 510
   Asn Phe Thr Leu Ser Pro Ser Pro Gly Tyr Pro Gln Gly Thr Pro Ala
   515 520 525
   Ala Gly Gln Ala Trp Thr Pro Glu Pro Ser Pro Arg Arg Gly Gly Ser
   530 535 540
   Asn Val Val Gly Tyr Asp Arg Asn Asn Thr Gly Lys Gly Val Glu Gln
   545 550 555 560
   Glu Val Pro Ser Thr Ala Pro Gly Leu Gly Met Asp Gln Gly Ser Pro
   565 570 575
   Ser Gln Val Asn Pro Ser Gln Gly Ser Pro Ser Gln Gly Ser Leu Arg
   580 585 590
   Gln Glu Ser Thr Ser Gln Ala Ser Pro Ser Gln Arg Ser Thr Ser Gln
   595 600 605
   Gly Ser Pro Ser Gln Val Asn Pro Ser Gln Gly Ser Thr Ser His Ala
   610 615 620
   Asn Ser Ser Gln Gly Ser Pro Ser Gln Gly Ser Pro Ser Gln Glu Ser
   625 630 635 640
   Pro Ser Gln Gly Ser Thr Ser Gln Ala Ser Pro Ser His Arg Asn Thr
   645 650 655
   Ile Gly Val Ile Gly Thr Thr Ser Ser Pro Lys Ala Thr Gly Ser Thr
   660 665 670
   His Ser Phe Pro Pro Gly Ala Thr Asp Gly Pro Leu Ala Leu Pro Gly
   675 680 685
   Gln Leu Gln Gly Asn Ser Ile Met Glu Pro Pro Ser Trp Pro Ser Pro
   690 695 700
   Thr Glu Asp Pro Thr Gly His Phe Leu Trp His Ala Thr Arg Ser Thr
   705 710 715 720
   Arg Glu Thr Leu Leu Asn Pro Thr Trp Leu Arg Asn Glu Asp Ser Gly
   725 730 735
   Pro Ser Gly Ser Val Asp Leu Pro Leu Thr Ser Thr Leu Thr Ala Leu
   740 745 750
   Lys Thr Pro Ala Cys Val Pro Val Ser Ile Gly Arg Ile Met Val Ser
   755 760 765
   Asn Val Thr Ser Thr Gly Phe His Leu Ala Trp Glu Ala Asp Leu Ala
   770 775 780
   Met Asp Ser Thr Phe Gln Leu Thr Leu Thr Ser Met Trp Ser Pro Ala
   785 790 795 800
   Val Val Leu Glu Thr Trp Asn Thr Ser Val Thr Leu Ser Gly Leu Glu
   805 810 815
   Pro Gly Val Leu His Leu Val Glu Ile Met Ala Lys Ala Cys Gly Lys
   820 825 830
   Glu Gly Ala Arg Ala His Leu Lys Val Arg Thr Ala Ala Arg Lys Leu
   835 840 845
   Ile Gly Lys Val Arg Ile Lys Asn Val Arg Tyr Ser Glu Ser Phe Arg
   850 855 860
   Asn Ala Ser Ser Gln Glu Tyr Arg Asp Phe Leu Glu Leu Phe Phe Arg
   865 870 875 880
   Met Val Arg Gly Ser Leu Pro Ala Thr Met Cys Gln His Met Asp Ala
                   885                 890                 895
   Gly Gly Val Arg Met Glu Val Val Ser Val Thr Asn Gly Ser Ile Val
               900                 905                 910
   Val Glu Phe His Leu Leu Ile Ile Ala Asp Val Asp Val Gln Glu Val
           915                 920                 925
   Ser Ala Ala Phe Leu Thr Ala Phe Gln Thr Val Pro Leu Leu Glu Val
       930                 935                 940
   Ile Arg Gly Asp Thr Phe Ile Gln Asp Tyr Asp Glu Cys Glu Arg Lys
   945                 950                 955                 960
   Glu Asp Asp Cys Val Pro Gly Thr Ser Cys Arg Asn Thr Leu Gly Ser
                   965                 970                 975
   Phe Thr Cys Ser Cys Glu Gly Gly Ala Pro Asp Phe Pro Val Glu Tyr
               980                 985                 990
   Ser Glu Arg Pro Cys Glu Gly Asp  Ser Pro Gly Asn Glu  Thr Trp Ala
           995                 1000                1005
   Thr Ser  Pro Glu Arg Pro Leu  Thr Thr Ala Gly Thr  Lys Ala Ala
       1010                 1015                1020
   Phe Val  Gln Gly Thr Ser Pro  Thr Pro Gln Gly Leu  Pro Gln Arg
       1025                 1030                1035
   Leu Asn  Leu Thr Gly Ala Val  Arg Val Leu Cys Glu  Ile Glu Lys
       1040                 1045                1050
   Val Val  Val Ala Ile Gln Lys  Arg Phe Leu Gln Gln  Glu Ser Ile
       1055                 1060                1065
   Pro Glu  Ser Ser Leu Tyr Leu  Ser His Pro Ser Cys  Asn Val Ser
       1070                 1075                1080
   His Ser  Asn Gly Thr His Val  Leu Leu Glu Ala Gly  Trp Ser Glu
       1085                 1090                1095
   Cys Gly  Thr Leu Met Gln Ser  Asn Met Thr Asn Thr  Val Val Arg
       1100                 1105                1110
   Thr Thr  Leu Arg Asn Asp Leu  Ser Gln Glu Gly Ile  Ile His His
       1115                 1120                1125
   Leu Lys  Ile Leu Ser Pro Ile  Tyr Cys Ala Phe Gln  Asn Asp Leu
       1130                 1135                1140
   Leu Thr  Ser Ser Gly Phe Thr  Leu Glu Trp Gly Val  Tyr Thr Ile
   1145 1150 1155
   Ile Glu Asp Leu His Gly Ala Gly Asn Phe Val Thr Glu Met Gln
   1160 1165 1170
   Leu Phe Ile Gly Asp Ser Pro Ile Pro Gln Asn Tyr Ser Val Ser
   1175 1180 1185
   Ala Ser Asp Asp Val Arg Ile Glu Val Gly Leu Tyr Arg Gln Lys
   1190 1195 1200
   Ser Asn Leu Lys Val Val Leu Thr Glu Cys Trp Ala Thr Pro Ser
   1205 1210 1215
   Ser Asn Ala Arg Asp Pro Ile Thr Phe Ser Phe Ile Asn Asn Ser
   1220 1225 1230
   Cys Pro Val Pro Asn Thr Tyr Thr Asn Val Ile Glu Asn Gly Asn
   1235 1240 1245
   Ser Asn Lys Ala Gln Phe Lys Leu Arg Ile Phe Ser Phe Ile Asn
   1250 1255 1260
   Asp Ser Ile Val Tyr Leu His Cys Lys Leu Arg Val Cys Met Glu
   1265 1270 1275
   Ser Pro Gly Ala Thr Cys Lys Ile Asn Cys Asn Asn Phe Arg Leu
   1280 1285 1290
   Leu Gln Asn Ser Glu Thr Ser Ala Thr His Gln Met Ser Trp Gly
   1295 1300 1305
   Pro Leu Ile Arg Ser Glu Gly Glu Pro Pro His Ala Glu Ala Gly
   1310 1315 1320
   Leu Gly Ala Gly Tyr Val Val Leu Ile Val Val Ala Ile Phe Val
   1325 1330 1335
   Leu Val Ala Gly Thr Ala Thr Leu Leu Ile Val Arg Tyr Gln Arg
   1340 1345 1350
   Met Asn Gly Arg Tyr Asn Phe Lys Ile Gln Ser Asn Asn Phe Ser
   1355 1360 1365
   Tyr Gln Val Phe Tyr Glu
   1370
<210> 61
   <211> 866
   <212> PRT
   <213> Homo sapiens
<400> 61
   Met Glu Gly Ala Gly Pro Arg Gly Ala Gly Pro Ala Arg Arg Arg Gly
   1               5                   10                  15
   Ala Gly Gly Pro Pro Ser Pro Leu Leu Pro Ser Leu Leu Leu Leu Leu
               20                  25                  30
   Leu Leu Trp Met Leu Pro Asp Thr Val Ala Pro Gln Glu Leu Asn Pro
           35                  40                  45
   Arg Gly Arg Asn Val Cys Arg Ala Pro Gly Ser Gln Val Pro Thr Cys
       50                  55                  60
   Cys Ala Gly Trp Arg Gln Gln Gly Asp Glu Cys Gly Ile Ala Val Cys
   65                  70                  75                  80
   Glu Gly Asn Ser Thr Cys Ser Glu Asn Glu Val Cys Val Arg Pro Gly
                   85                  90                  95
   Glu Cys Arg Cys Arg His Gly Tyr Phe Gly Ala Asn Cys Asp Thr Lys
               100                 105                 110
   Cys Pro Arg Gln Phe Trp Gly Pro Asp Cys Lys Glu Leu Cys Ser Cys
           115                 120                 125
   His Pro His Gly Gln Cys Glu Asp Val Thr Gly Gln Cys Thr Cys His
       130                 135                 140
   Ala Arg Arg Trp Gly Ala Arg Cys Glu His Ala Cys Gln Cys Gln His
   145                 150                 155                 160
   Gly Thr Cys His Pro Arg Ser Gly Ala Cys Arg Cys Glu Pro Gly Trp
                   165                 170                 175
   Trp Gly Ala Gln Cys Ala Ser Ala Cys Tyr Cys Ser Ala Thr Ser Arg
               180                 185                 190
   Cys Asp Pro Gln Thr Gly Ala Cys Leu Cys His Ala Gly Trp Trp Gly
           195                 200                 205
   Arg Ser Cys Asn Asn Gln Cys Ala Cys Asn Ser Ser Pro Cys Glu Gln
       210                 215                 220
   Gln Ser Gly Arg Cys Gln Cys Arg Glu Arg Thr Phe Gly Ala Arg Cys
   225                 230                 235                 240
   Asp Arg Tyr Cys Gln Cys Phe Arg Gly Arg Cys His Pro Val Asp Gly
                   245                 250                 255
   Thr Cys Ala Cys Glu Pro Gly Tyr Arg Gly Lys Tyr Cys Arg Glu Pro
               260                 265                 270
   Cys Pro Ala Gly Phe Tyr Gly Leu Gly Cys Arg Arg Arg Cys Gly Gln
           275                 280                 285
   Cys Lys Gly Gln Gln Pro Cys Thr Val Ala Glu Gly Arg Cys Leu Thr
       290                 295                 300
   Cys Glu Pro Gly Trp Asn Gly Thr Lys Cys Asp Gln Pro Cys Ala Thr
   305                 310                 315                 320
   Gly Phe Tyr Gly Glu Gly Cys Ser His Arg Cys Pro Pro Cys Arg Asp
                   325                 330                 335
   Gly His Ala Cys Asn His Val Thr Gly Lys Cys Thr Arg Cys Asn Ala
               340                 345                 350
   Gly Trp Ile Gly Asp Arg Cys Glu Thr Lys Cys Ser Asn Gly Thr Tyr
           355                 360                 365
   Gly Glu Asp Cys Ala Phe Val Cys Ala Asp Cys Gly Ser Gly His Cys
       370                 375                 380
   Asp Phe Gln Ser Gly Arg Cys Leu Cys Ser Pro Gly Val His Gly Pro
   385                 390                 395                 400
   His Cys Asn Val Thr Cys Pro Pro Gly Leu His Gly Ala Asp Cys Ala
                   405                 410                 415
   Gln Ala Cys Ser Cys His Glu Asp Thr Cys Asp Pro Val Thr Gly Ala
               420                 425                 430
   Cys His Leu Glu Thr Asn Gln Arg Lys Gly Val Met Gly Ala Gly Ala
           435                 440                 445
   Leu Leu Val Leu Leu Val Cys Leu Leu Leu Ser Leu Leu Gly Cys Cys
       450                 455                 460
   Cys Ala Cys Arg Gly Lys Asp Pro Thr Arg Arg Glu Leu Ser Leu Gly
   465                 470                 475                 480
   Arg Lys Lys Ala Pro His Arg Leu Cys Gly Arg Phe Ser Arg Ile Ser
                   485                 490                 495
   Met Lys Leu Pro Arg Ile Pro Leu Arg Arg Gln Lys Leu Pro Lys Val
               500                 505                 510
   Val Val Ala His His Asp Leu Asp Asn Thr Leu Asn Cys Ser Phe Leu
           515                 520                 525
   Glu Pro Pro Ser Gly Leu Glu Gln Pro Ser Pro Ser Trp Ser Ser Arg
       530                 535                 540
   Ala Ser Phe Ser Ser Phe Asp Thr Thr Asp Glu Gly Pro Val Tyr Cys
   545 550 555 560
   Val Pro His Glu Glu Ala Pro Ala Glu Ser Arg Asp Pro Glu Val Pro
   565 570 575
   Thr Val Pro Ala Glu Ala Pro Ala Pro Ser Pro Val Pro Leu Thr Thr
   580 585 590
   Pro Ala Ser Ala Glu Glu Ala Ile Pro Leu Pro Ala Ser Ser Asp Ser
   595 600 605
   Glu Arg Ser Ala Ser Ser Val Glu Gly Pro Gly Gly Ala Leu Tyr Ala
   610 615 620
   Arg Val Ala Arg Arg Glu Ala Arg Pro Ala Arg Ala Arg Gly Glu Ile
   625 630 635 640
   Gly Gly Leu Ser Leu Ser Pro Ser Pro Glu Arg Arg Lys Pro Pro Pro
   645 650 655
   Pro Asp Pro Ala Thr Lys Pro Lys Val Ser Trp Ile His Gly Lys His
   660 665 670
   Ser Ala Ala Ala Ala Gly Arg Ala Pro Ser Pro Pro Pro Pro Gly Ser
   675 680 685
   Glu Ala Ala Pro Ser Pro Ser Lys Arg Lys Arg Thr Pro Ser Asp Lys
   690 695 700
   Ser Ala His Thr Val Glu His Gly Ser Pro Arg Thr Arg Asp Pro Thr
   705 710 715 720
   Pro Arg Pro Pro Gly Leu Pro Glu Glu Ala Thr Ala Leu Ala Ala Pro
   725 730 735
   Ser Pro Pro Arg Ala Arg Ala Arg Gly Arg Gly Pro Gly Leu Leu Glu
   740 745 750
   Pro Thr Asp Ala Gly Gly Pro Pro Arg Ser Ala Pro Glu Ala Ala Ser
   755 760 765
   Met Leu Ala Ala Glu Leu Arg Gly Lys Thr Arg Ser Leu Gly Arg Ala
   770 775 780
   Glu Val Ala Leu Gly Ala Gln Gly Pro Arg Glu Lys Pro Ala Pro Pro
   785 790 795 800
   Gln Lys Ala Lys Arg Ser Val Pro Pro Ala Ser Pro Ala Arg Ala Pro
   805 810 815
   Pro Ala Thr Glu Thr Pro Gly Pro Glu Lys Ala Ala Thr Asp Leu Pro
   820 825 830
   Ala Pro Glu Thr Pro Arg Lys Lys Thr Pro Ile Gln Lys Pro Pro Arg
   835 840 845
   Lys Lys Ser Arg Glu Ala Ala Gly Glu Leu Gly Arg Ala Gly Ala Pro
   850 855 860
   Thr Leu
   865
<210> 62
   <211> 2601
   <212> DNA
   <213> Homo sapiens
<400> 62
   atggagggcg cagggccccg gggggccggg ccggcgcggc gccggggagc cggggggccg 60
   ccgtcaccgc tgctgccgtc gctgctgctg ctgctgctgc tctggatgct gccggacacc 120
   gtggcgcctc aggaactgaa ccctcgcggc cgcaacgtgt gccgtgctcc cggctcccag 180
   gtgcccacgt gctgcgctgg ctggaggcag caaggggacg agtgtgggat tgcggtgtgc 240
   gaaggcaact ccacgtgctc agagaacgag gtgtgcgtga ggcctggcga gtgccgctgc 300
   cgccacggct acttcggtgc caactgcgac accaagtgcc cgcgccagtt ctggggcccc 360
   gactgcaagg agctgtgtag ctgccaccca cacgggcagt gcgaggacgt gacaggccag 420
   tgtacttgtc acgcgcggcg ctggggcgcg cgctgcgagc atgcgtgcca gtgccagcac 480
   ggcacgtgcc acccgcggag cggcgcgtgc cgctgtgagc ccggctggtg gggcgcgcag 540
   tgcgccagcg cgtgctactg cagcgccacg tcgcgctgcg acccacagac cggcgcctgc 600
   ctgtgccacg caggctggtg gggccgcagc tgcaacaacc agtgcgcctg caactcgtct 660
   ccctgcgagc agcagagcgg ccgctgtcag tgccgcgagc gtacgttcgg cgcgcgctgc 720
   gatcgctact gccagtgctt ccgcggccgc tgccaccctg tggacggcac gtgtgcctgc 780
   gagccgggct accgcggcaa gtactgtcgc gagccgtgcc ccgccggctt ctacggcttg 840
   ggctgtcgcc gccggtgtgg ccagtgcaag ggccagcagc cgtgcacggt ggccgagggc 900
   cgctgcttga cgtgcgagcc cggctggaac ggaaccaagt gcgaccagcc ttgcgccacc 960
   ggtttctatg gcgagggctg cagccaccgc tgtccgccat gccgcgacgg gcatgcctgt 1020
   aaccatgtca ccggcaagtg tacgcgctgc aacgcgggct ggatcggcga ccggtgcgag 1080
   accaagtgta gcaatggcac ttacggcgag gactgcgcct tcgtgtgcgc cgactgcggc 1140
   agcggacact gcgacttcca gtcggggcgc tgcctgtgca gccctggcgt ccacgggccc 1200
   cactgtaacg tgacgtgccc gcccggactc cacggcgcgg actgtgctca ggcctgcagc 1260
   tgccacgagg acacgtgcga cccggtcact ggtgcctgcc acctagaaac caaccagcgc 1320
   aagggcgtga tgggcgcggg cgcgctgctc gtcctgctcg tctgcctgct gctctcgctg 1380
   ctcggctgct gctgcgcttg ccgcggcaag gaccctacgc gccgggagct ttcgcttggg 1440
   aggaagaagg cgccgcaccg actatgcggg cgcttcagtc gcatcagcat gaagctgccc 1500
   cggatcccgc tccggaggca gaaactaccc aaagtcgtag tggcccacca cgacctggat 1560
   aacacactca actgcagctt cctggagcca ccctcagggc tggagcagcc ctcaccatcc 1620
   tggtcctctc gggcctcctt ctcctcgttt gacaccactg atgaaggccc tgtgtactgt 1680
   gtaccccatg aggaggcacc agcggagagc cgggaccccg aagtccccac tgtccctgcc 1740
   gaggcgccgg cgccgtcccc tgtgcccttg accacgccag cctccgccga ggaggcgata 1800
   cccctccccg cgtcctccga cagcgagcgg tcggcgtcca gcgtggaggg gcccggaggg 1860
   gctctgtacg cgcgcgtggc ccgacgcgag gcccggccgg cccgggcccg gggcgagatt 1920
   gggggcctgt cgctgtcgcc atcgcccgag cgcaggaaac cgccgccacc tgaccccgcc 1980
   accaagccta aggtgtcctg gatccacggc aagcacagcg ccgctgcagc tggccgtgcg 2040
   ccctcaccac cgccgccagg ctccgaggcc gcgcccagcc ccagcaagag gaaacggacg 2100
   cccagcgaca aatcggcgca tacggtcgaa cacggcagcc cccggacccg cgacccaacg 2160
   ccgcggcccc ccgggctgcc cgaggaggcg acagccctcg ctgcgccctc gccgcccagg 2220
   gcccgagcgc ggggccgcgg ccccggcctc ttggagccca cggacgccgg cggtcccccg 2280
   cgaagcgcgc ccgaggctgc ctccatgttg gccgctgagc tgcgcggcaa gactcgcagc 2340
   ctgggccgcg ccgaggtggc cctgggcgcg cagggcccca gggaaaagcc ggcgccccca 2400
   cagaaagcca agcgctccgt gccgccagcc tcgcccgccc gcgcgccccc agcgaccgaa 2460
   accccggggc ctgagaaggc ggcgaccgac ttgcccgcgc ctgagacccc ccggaagaag 2520
   acccccatcc agaagccgcc gcgcaagaag agccgggagg cggcgggcga gctgggcagg 2580
   gcgggcgcac ccaccctgta g 2601
<210> 63
   <211> 822
   <212> PRT
   <213> Homo sapiens
<400> 63
   Met Lys Pro Ala Leu Leu Pro Trp Ala Leu Leu Leu Leu Ala Thr Ala
   1 5 10 15
   Leu Gly Pro Gly Pro Gly Pro Thr Ala Asp Ala Gln Glu Ser Cys Ser
   20 25 30
   Met Arg Cys Gly Ala Leu Asp Gly Pro Cys Ser Cys His Pro Thr Cys
   35 40 45
   Ser Gly Leu Gly Thr Cys Cys Leu Asp Phe Arg Asp Phe Cys Leu Glu
   50 55 60
   Ile Leu Pro Tyr Ser Gly Ser Met Met Gly Gly Lys Asp Phe Val Val
   65 70 75 80
   Arg His Phe Lys Met Ser Ser Pro Thr Asp Ala Ser Val Ile Cys Arg
   85 90 95
   Phe Lys Asp Ser Ile Gln Thr Leu Gly His Val Asp Ser Ser Gly Gln
   100 105 110
   Val His Cys Val Ser Pro Leu Leu Tyr Glu Ser Gly Arg Ile Pro Phe
   115 120 125
   Thr Val Ser Leu Asp Asn Gly His Ser Phe Pro Arg Ala Gly Thr Trp
   130 135 140
   Leu Ala Val His Pro Asn Lys Val Ser Met Met Glu Lys Ser Glu Leu
   145 150 155 160
   Val Asn Glu Thr Arg Trp Gln Tyr Tyr Gly Thr Ala Asn Thr Ser Gly
   165 170 175
   Asn Leu Ser Leu Thr Trp His Val Lys Ser Leu Pro Thr Gln Thr Ile
   180 185 190
   Thr Ile Glu Leu Trp Gly Tyr Glu Glu Thr Gly Met Pro Tyr Ser Gln
   195 200 205
   Glu Trp Thr Ala Lys Trp Ser Tyr Leu Tyr Pro Leu Ala Thr His Ile
   210 215 220
   Pro Asn Ser Gly Ser Phe Thr Phe Thr Pro Lys Pro Ala Pro Pro Ser
   225 230 235 240
   Tyr Gln Arg Trp Arg Val Gly Ala Leu Arg Ile Ile Asp Ser Lys Asn
   245 250 255
   Tyr Ala Gly Gln Lys Asp Val Gln Ala Leu Trp Thr Asn Asp His Ala
   260 265 270
   Leu Ala Trp His Leu Ser Asp Asp Phe Arg Glu Asp Pro Val Ala Trp
   275 280 285
   Ala Arg Thr Gln Cys Gln Ala Trp Glu Glu Leu Glu Asp Gln Leu Pro
   290 295 300
   Asn Phe Leu Glu Glu Leu Pro Asp Cys Pro Cys Thr Leu Thr Gln Ala
   305 310 315 320
   Arg Ala Asp Ser Gly Arg Phe Phe Thr Asp Tyr Gly Cys Asp Met Glu
   325 330 335
   Gln Gly Ser Val Cys Thr Tyr His Pro Gly Ala Val His Cys Val Arg
   340 345 350
   Ser Val Gln Ala Ser Leu Arg Tyr Gly Ser Gly Gln Gln Cys Cys Tyr
   355 360 365
   Thr Ala Asp Gly Thr Gln Leu Leu Thr Ala Asp Ser Ser Gly Gly Ser
   370 375 380
   Thr Pro Asp Arg Gly His Asp Trp Gly Ala Pro Pro Phe Arg Thr Pro
   385 390 395 400
   Pro Arg Val Pro Ser Met Ser His Trp Leu Tyr Asp Val Leu Ser Phe
   405 410 415
   Tyr Tyr Cys Cys Leu Trp Ala Pro Asp Cys Pro Arg Tyr Met Gln Arg
   420 425 430
   Arg Pro Ser Asn Asp Cys Arg Asn Tyr Arg Pro Pro Arg Leu Ala Ser
   435 440 445
   Ala Phe Gly Asp Pro His Phe Val Thr Phe Asp Gly Thr Asn Phe Thr
   450 455 460
   Phe Asn Gly Arg Gly Glu Tyr Val Leu Leu Glu Ala Ala Leu Thr Asp
   465 470 475 480
   Leu Arg Val Gln Ala Arg Ala Gln Pro Gly Thr Met Ser Asn Gly Thr
   485 490 495
   Glu Thr Arg Gly Thr Gly Leu Thr Ala Val Ala Val Gln Glu Gly Asn
   500 505 510
   Ser Asp Val Val Glu Val Arg Leu Ala Asn Arg Thr Gly Gly Leu Glu
   515 520 525
   Val Leu Leu Asn Gln Glu Val Leu Ser Phe Thr Glu Gln Ser Trp Met
   530 535 540
   Asp Leu Lys Gly Met Phe Leu Ser Val Ala Ala Gly Asp Arg Val Ser
   545 550 555 560
   Ile Met Leu Ala Ser Gly Ala Gly Leu Glu Val Ser Val Gln Gly Pro
   565 570 575
   Phe Leu Ser Val Ser Val Leu Leu Pro Glu Lys Phe Leu Thr His Thr
   580 585 590
   His Gly Leu Leu Gly Thr Leu Asn Asn Asp Pro Thr Asp Asp Phe Thr
   595 600 605
   Leu His Ser Gly Arg Val Leu Pro Pro Gly Thr Ser Pro Gln Glu Leu
   610 615 620
   Phe Leu Phe Gly Ala Asn Trp Thr Val His Asn Ala Ser Ser Leu Leu
   625 630 635 640
   Thr Tyr Asp Ser Trp Phe Leu Val His Asn Phe Leu Tyr Gln Pro Lys
   645 650 655
   His Asp Pro Thr Phe Glu Pro Leu Phe Pro Ser Glu Thr Thr Leu Asn
   660 665 670
   Pro Ser Leu Ala Gln Glu Ala Ala Lys Leu Cys Gly Asp Asp His Phe
   675 680 685
   Cys Asn Phe Asp Val Ala Ala Thr Gly Ser Leu Ser Thr Gly Thr Ala
   690 695 700
   Thr Arg Val Ala His Gln Leu His Gln Arg Arg Met Gln Ser Leu Gln
   705 710 715 720
   Pro Val Val Ser Cys Gly Trp Leu Ala Pro Pro Pro Asn Gly Gln Lys
   725 730 735
   Glu Gly Asn Arg Tyr Leu Ala Gly Ser Thr Ile Tyr Phe His Cys Asp
   740 745 750
   Asn Gly Tyr Ser Leu Ala Gly Ala Glu Thr Ser Thr Cys Gln Ala Asp
   755 760 765
   Gly Thr Trp Ser Ser Pro Thr Pro Lys Cys Gln Pro Gly Arg Ser Tyr
   770 775 780
   Ala Val Leu Leu Gly Ile Ile Phe Gly Gly Leu Ala Val Val Ala Ala
   785 790 795 800
   Val Ala Leu Val Tyr Val Leu Leu Arg Arg Arg Lys Gly Asn Thr His
   805 810 815
   Val Trp Gly Ala Gln Pro
   820
<210> 64
   <211> 2469
   <212> DNA
   <213> Homo sapiens
<400> 64
   atgaagccag ccctcctgcc ctgggccctg ctgctgctgg cgacagccct cggcccgggc 60
   cccggaccca cagcagatgc ccaagagagc tgctccatgc gctgtggcgc cctggacggg 120
   ccatgttcct gccacccgac gtgctctggc cttggcacct gctgcttgga tttccgggac 180
   ttctgcctgg agatattgcc ctactcagga tccatgatgg gcggcaagga ctttgtggtg 240
   cggcacttca agatgtccag ccccacagac gccagtgtga tctgcaggtt taaggacagc 300
   atccagaccc tcggccatgt ggactcctcc gggcaagtgc actgtgtgtc acctctgctc 360
   tatgagagcg gccgcatccc cttcactgtg tcactggaca acggccactc cttccctcgt 420
   gcgggcacct ggctggctgt gcaccccaac aaagtgtcaa tgatggagaa gagcgagttg 480
   gtgaacgaga cgcgttggca atactacggc accgccaaca cctcaggcaa cctcagcctg 540
   acctggcatg tcaagtcgct gcccacgcag accatcacca tcgaactgtg gggctacgag 600
   gagacaggaa tgccctactc acaggagtgg actgcaaagt ggtcgtacct gtaccccctg 660
   gccacacaca tccccaactc cggctctttc actttcaccc caaaacctgc tcctcccagc 720
   taccagagat ggcgagtggg tgcacttcgg atcatcgaca gcaaaaatta cgcagggcag 780
   aaggacgtgc aggcgctctg gaccaacgac cacgcactgg cctggcacct gagcgatgac 840
   ttccgagagg accctgtggc ctgggcacga actcagtgcc aggcctggga ggagctggag 900
   gatcagctgc ccaacttcct ggaggagctg ccggactgcc cctgcaccct gacccaggcc 960
   cgggctgact ccggccgctt cttcacggac tacggctgtg acatggagca gggcagcgtg 1020
   tgcacctacc accccggggc cgtgcactgt gtgcgttctg tgcaggccag cctccggtac 1080
   ggctcaggtc agcagtgctg ctacacagcg gacgggacgc agctcctgac agctgactcc 1140
   agcggcggca gcactcccga ccgcggccat gactggggcg cacccccgtt ccgcacgcca 1200
   ccccgagtgc ccagcatgtc ccactggctc tacgatgtcc tcagcttcta ttactgctgc 1260
   ctctgggcac ccgactgccc ccgctacatg caacggcggc cctccaatga ctgccgcaac 1320
   taccggcccc caagactggc ctccgccttc ggagacccac actttgtgac cttcgacggc 1380
   accaacttca cattcaatgg gcgcggagag tacgtgctgc tggaggcagc gctgaccgac 1440
   ctgagggtgc aggcgcgggc ccagcccggg acgatgtcca acggcacgga gacccgtggc 1500
   actgggctga ccgcagtggc cgtccaggag ggcaactcag atgtggtgga agtcaggctg 1560
   gccaacagga ccggaggtct ggaggtgctg ctgaaccagg aggtgctgag cttcaccgag 1620
   cagagctgga tggacctgaa aggaatgttc ctgtcggtgg ctgccgggga cagggtctcc 1680
   atcatgctgg catcaggggc cggcctggag gtcagcgtgc agggcccgtt cctgagtgtg 1740
   tccgtcctgc tgcctgagaa gttcctcacc cacacccacg gcctcctcgg gacactcaac 1800
   aacgacccca ccgacgactt caccctgcac agcgggcgcg tcctgccccc aggcaccagt 1860
   ccccaggagc tgttcctgtt tggggccaac tggaccgtgc acaatgcgtc ctccctgctc 1920
   acctacgatt cctggttcct ggtccacaac ttcctgtacc aacccaagca cgaccccacc 1980
   ttcgagcccc tcttccccag tgagaccacc ctcaacccca gcctggcaca agaggcagcc 2040
   aaactatgtg gggacgatca tttctgcaac tttgatgtgg cagccactgg gagcctgagc 2100
   acgggcactg ccactcgggt ggcccaccag ctgcaccagc gtcgcatgca gagcctgcag 2160
   ccagtggtgt cctgtggctg gctggcccca cctcccaacg gacaaaagga gggcaacagg 2220
   tacctggcgg gttccaccat ctacttccac tgtgacaacg gctacagcct ggccggggca 2280
   gagaccagca cctgccaggc tgacggcacc tggtcctcac ccaccccgaa gtgccagcca 2340
   ggacgcagct acgcggtgct gttgggcatc atctttgggg gcctcgcggt ggtggcggcg 2400
   gttgcgctcg tctatgtgct gctgcgccgc aggaagggca acacgcacgt ctggggtgca 2460
   cagccctga 2469
<210> 65
   <211> 188
   <212> PRT
   <213> Homo sapiens
<400> 65
   Met Ile Ile Tyr Arg Asp Leu Ile Ser His Asp Glu Met Phe Ser Asp
   1 5 10 15
   Ile Tyr Lys Ile Arg Glu Ile Ala Asp Gly Leu Cys Leu Glu Val Glu
   20 25 30
   Gly Lys Met Val Ser Arg Thr Glu Gly Asn Ile Asp Asp Ser Leu Ile
   35 40 45
   Gly Gly Asn Ala Ser Ala Glu Gly Pro Glu Gly Glu Gly Thr Glu Ser
   50 55 60
   Thr Val Ile Thr Gly Val Asp Ile Val Met Asn His His Leu Gln Glu
   65 70 75 80
   Thr Ser Phe Thr Lys Glu Ala Tyr Lys Lys Tyr Ile Lys Asp Tyr Met
   85 90 95
   Lys Ser Ile Lys Gly Lys Leu Glu Glu Gln Arg Pro Glu Arg Val Lys
   100 105 110
   Pro Phe Met Thr Gly Ala Ala Glu Gln Ile Lys His Ile Leu Ala Asn
   115 120 125
   Phe Lys Asn Tyr Gln Phe Phe Ile Gly Glu Asn Met Asn Pro Asp Gly
   130 135 140
   Met Val Ala Leu Leu Asp Tyr Arg Glu Asp Gly Val Thr Pro Tyr Met
   145 150 155 160
   Ile Phe Phe Lys Asp Gly Leu Glu Met Glu Lys Cys Val Ser Thr Arg
   165 170 175
   Lys Trp Val Lys Ile Asn Asn Val Lys Lys Thr Phe
   180 185
<210> 66
   <211> 567
   <212> DNA
   <213> Homo sapiens
<400> 66
   atgattatct accgggacct catcagccac gatgagatgt tctccgacat ctacaagatc    60
   cgggagatcg cggacgggtt gtgcctggag gtggagggga agatggtcag taggacagaa   120
   ggtaacattg atgactcgct cattggtgga aatgcctccg ctgaaggccc cgagggcgaa   180
   ggtaccgaaa gcacagtaat cactggtgtc gatattgtca tgaaccatca cctgcaggaa   240
   acaagtttca caaaagaagc ctacaagaag tacatcaaag attacatgaa atcaatcaaa   300
   gggaaacttg aagaacagag accagaaaga gtaaaacctt ttatgacagg ggctgcagaa   360
   caaatcaagc acatccttgc taatttcaaa aactaccagt tctttattgg tgaaaacatg   420
   aatccagatg gcatggttgc tctattggac taccgtgagg atggtgtgac cccatatatg   480
   attttcttta aggatggttt agaaatggaa aaatgtgtaa gtacaaggaa gtgggttaaa   540
   ataaataatg taaaaaagac attttag                                        567
<210> 67
   <211> 401
   <212> PRT
   <213> Homo sapiens
<400> 67
<210> 68
   <211> 1206
   <212> DNA
   <213> Homo sapiens
<400> 68
   atggttctct gttgtagagg ttctctgttg tggatggttc tctgttgtgg atggttctct      60
   gttatagagg gtctctgttg tggagggtct ctgttgtgga tggttctctg ttgtgggtgg     120
   gtctctgttg tgggtgggtc tctgttgtgg agggttctct gttgtggagg gttctctgtt     180
   gtggagggtt ctctgttgtg gagggtctct gttggggatg gttctctgtt gtggaggctc     240
   tctgttgtgg aggttctctg ttgtggagga tctctgttgt ggatggttct gtgttgtgga     300
   gggttctgtg ttgtggaggg ttctctgttg tggagggtct ctgttgtgga tggttctctg     360
   tcgtggaggc tctctgtcgt ggaggctccc tgtcgtggag gctctctgtc gtggagggtc     420
   tctgtcgtgg gtggttcttt gttgtggagg gtctctgttg tggatggttc tctgttgtgg     480
   agggtctctg ttgtggaggg tctctgttgt ggagggttct ctgttgtgga gggtctctgt     540
   tgtggagggt tctctgttgt ggagggttgt ctgttgtgga gggttctctg ttgtggagcg     600
   tctctgttgt ggagagtctc tgttgtggag ggttctctgt tgtggagagt ctgttgtgga     660
   gggttctctg ttgtggagag tctctgttgt ggatggttct ctgttgtgga gggttctctg     720
   ttgtggaggg ttctctgttg tggatggttc tctgttatgg agggttctct gttgtggagg     780
   gttctctctt gtggatggtt ctctgttgtg gatggttctc tgttatggag ggtctctgtt     840
   gtggagggtc tctgttgtgg agagtctgtt gtggatggtt ctctgttgtg gagagtctct     900
   gttgtggagg gtctctgttg tgggtggttc tttgttgtgg gtggttctct gttgtggatg     960
   gttctttgtt gtgggtggtt ctctgttgtg aatggttctt tgttgtggat gggtctctgt    1020
   tgtggagggt tctctgttgt ggatgggtct ctgttgtgga tgggtctctg ttgtggaggg    1080
   tctctgttgt ggatggttct ctgttttgga tgggtctctg ttgtggaggt tctctgttgt    1140
   ggaagttctt tgttgtattt gctgcaaata tgcatgataa gatgcttagg agcatctacc    1200
   cactag                                                               1206
<210> 69
   <211> 290
   <212> PRT
   <213> Homo sapiens
<400> 69
   Asp Arg Ser Arg Trp Arg Gly Arg Ala Gly Gln Gly Phe Gly Leu Arg
   1               5                   10                  15
   Arg Arg Glu Met Ala Ala Gly Gly Arg Met Glu Asp Gly Ser Leu Asp
               20                  25                  30
   Ile Thr Gln Ser Ile Glu Asp Asp Pro Leu Leu Asp Ala Gln Leu Leu
           35                  40                  45
   Pro His His Ser Leu Gln Ala His Phe Arg Pro Arg Phe His Pro Leu
       50                  55                  60
   Pro Thr Val Ile Ile Val Asn Leu Leu Trp Phe Ile His Leu Val Phe
   65                  70                  75                  80
   Val Val Leu Ala Phe Leu Thr Gly Val Leu Cys Ser Tyr Pro Asn Pro
                   85                  90                  95
   Asn Glu Asp Lys Cys Pro Gly Asn Tyr Thr Asn Pro Leu Lys Val Gln
               100                 105                 110
   Ala Val Ile Ile Leu Gly Lys Val Ile Leu Trp Ile Leu His Leu Leu
           115                 120                 125
   Leu Glu Cys Tyr Ile Gln Tyr His His Ser Lys Ile Arg Asn Arg Gly
       130                 135                 140
   Tyr Asn Leu Ile Tyr Arg Ser Thr Arg His Leu Lys Arg Leu Ala Leu
   145                 150                 155                 160
   Met Ile Gln Ser Ser Gly Asn Thr Val Leu Leu Leu Ile Leu Cys Met
                   165                 170                 175
   Gln His Ser Phe Pro Lys Pro Gly Arg Leu Tyr Leu Asp Leu Ile Leu
               180                 185                 190
   Ala Ile Leu Ala Leu Glu Leu Ile Cys Ser Leu Ile Cys Leu Leu Ile
           195                 200                 205
   Tyr Thr Val Lys Ile Arg Arg Phe Asn Lys Ala Lys Pro Glu Pro Asp
       210                 215                 220
   Ile Leu Glu Glu Glu Lys Ile Tyr Ala Tyr Pro Ser Asn Ile Thr Ser
   225                 230                 235                 240
   Glu Thr Gly Phe Arg Thr Ile Ser Ser Leu Glu Glu Ile Val Glu Lys
                   245                 250                 255
   Gln Gly Asp Thr Ile Glu Tyr Leu Lys Arg His Asn Ala Leu Leu Ser
               260                 265                 270
   Lys Arg Leu Leu Ala Leu Thr Ser Ser Asp Leu Gly Cys Gln Pro Ser
           275                 280                 285
   Arg Thr
       290
<210> 70
   <211> 873
   <212> DNA
   <213> Homo sapiens
<400> 70
   gacaggtcca ggtggagagg ccgggctggc cagggcttcg gcctccggcg tcgggaaatg      60
   gcggcggggg gcaggatgga ggacggttcc ttggatatca cccagagtat tgaagacgac     120
   ccacttctgg atgcccagct tctcccacac cactcattac aagctcactt tagaccccga     180
   ttccatcctc ttcctacagt catcatagtg aatcttctgt ggtttattca tctcgtgttt     240
   gttgttttag catttttaac aggtgtgctt tgttcttatc ctaatccaaa tgaggacaag     300
   tgcccaggaa attacacaaa cccattgaaa gttcaggcgg ttataatcct tgggaaagtt     360
   attttgtgga ttctccattt actccttgaa tgctacatcc agtatcacca cagcaaaatc     420
   agaaaccgag gctataactt gatctaccga tcaacaaggc atctcaagag acttgcgttg     480
   atgatacagt cctctggcaa cacagtgctt ctcctcatac tgtgcatgca gcactccttc     540
   ccaaagcctg gcagattgta tcttgacctc attctggcca tcttggcact ggaactcatc     600
   tgttccctga tatgtctcct catttacaca gtgaaaatcc ggagatttaa taaagctaaa     660
   ccagagcctg atatacttga agaagaaaaa atctatgctt accccagcaa tattacctcg     720
   gagactggat tcagaactat ttcaagccta gaagaaattg ttgaaaagca aggagacacc     780
   attgaatacc tgaagcgaca caatgcgctg ctgagtaagc gattgttggc tctcacttcc     840
   tcagacctgg gctgtcagcc aagtagaacg tga                                  873
<210> 71
   <211> 257
   <212> PRT
   <213> Homo sapiens
<400> 71
   Met Ala Ala Asp Gly Val Asp Glu Arg Ser Pro Leu Leu Ser Ala Ser
   1               5                   10                  15
   His Ser Gly Asn Val Thr Pro Thr Ala Pro Pro Tyr Leu Gln Glu Ser
               20                  25                  30
   Ser Pro Arg Ala Glu Leu Pro Pro Pro Tyr Thr Ala Ile Ala Ser Pro
           35                  40                  45
   Asp Ala Ser Gly Ile Pro Val Ile Asn Cys Arg Val Cys Gln Ser Leu
       50                  55                  60
   Ile Asn Leu Asp Gly Lys Leu His Gln His Val Val Lys Cys Thr Val
   65                  70                  75                  80
   Cys Asn Glu Ala Thr Pro Ile Lys Asn Pro Pro Thr Gly Lys Lys Tyr
                   85                  90                  95
   Val Arg Cys Pro Cys Asn Cys Leu Leu Ile Cys Lys Asp Thr Ser Arg
               100                 105                 110
   Arg Ile Gly Cys Pro Arg Pro Asn Cys Arg Arg Ile Ile Asn Leu Gly
           115                 120                 125
   Pro Val Met Leu Ile Ser Glu Glu Gln Pro Ala Gln Pro Ala Leu Pro
       130                 135                 140
   Ile Gln Pro Glu Gly Thr Arg Val Val Cys Gly His Cys Gly Asn Thr
   145                 150                 155                 160
   Phe Leu Trp Met Glu Leu Arg Phe Asn Thr Leu Ala Lys Cys Pro His
                   165                 170                 175
   Cys Lys Lys Ile Ser Ser Val Gly Ser Ala Leu Pro Arg Arg Arg Cys
               180                 185                 190
   Cys Ala Tyr Ile Thr Ile Gly Met Ile Cys Ile Phe Ile Gly Val Gly
           195                 200                 205
   Leu Thr Val Gly Thr Pro Asp Phe Ala Arg Arg Phe Arg Ala Thr Tyr
       210                 215                 220
   Val Ser Trp Ala Ile Ala Tyr Leu Leu Gly Leu Ile Cys Leu Ile Arg
   225                 230                 235                 240
   Ala Cys Tyr Trp Gly Ala Ile Arg Val Ser Tyr Pro Glu His Ser Phe
                   245                 250                 255
   Ala
<210> 72
   <211> 774
   <212> DNA
   <213> Homo sapiens
<400> 72
   atggctgctg atggggtgga cgaacgctcg cctctgctgt cagcatccca ctccggaaat     60
   gtcactccca ccgccccacc gtacttgcaa gaaagcagcc ccagagcgga gctcccacct    120
   ccatatacag ccattgccag tccagacgcc agtggtattc cagtaataaa ctgccgtgtg    180
   tgccaatcac taatcaattt ggatggcaag cttcaccagc atgtggttaa gtgcacagtt    240
   tgcaatgaag ctacgccaat caaaaacccc ccaacaggca agaaatatgt tagatgccct    300
   tgtaattgtc ttctcatttg taaggacaca tctcggcgaa taggatgccc aagacccaac    360
   tgtagacgga taattaacct tggcccagta atgcttattt ctgaagaaca accagctcag    420
   cctgcattgc caatccaacc agaaggtaca agggtcgtgt gtgggcactg tggaaacaca    480
   ttcctgtgga tggaactgag gttcaacact ctggcaaaat gcccacactg caaaaaaatc    540
   tcctcagtgg gtagtgcact tccacgaaga cgctgctgtg catatattac cattggaatg    600
   atatgtattt tcattggagt tgggttaact gttggcaccc cagattttgc aaggcgattt    660
   cgagcaacct atgtttcttg ggcaattgct tatctcctag gattgatctg ccttatccga    720
   gcttgttatt ggggagccat aagagtcagt tatccagaac acagttttgc ataa          774
<210> 73
   <211> 118
   <212> PRT
   <213> Homo sapiens
<400> 73
<210> 74
   <211> 357
   <212> DNA
   <213> Homo sapiens
<400> 74
   atggtgctta cagccatggt gggaaaaatc cataggaaaa gactcacata cacaaatgca 60
   ggcagaataa aaaaactcac acaaacaaac gttgcagatg ttgttaagtt acataaagga 120
   gatatgcatg gctgtgcatg gtggctcgtg cctgtaatct tagcacttgg gggggccggg 180
   gcgggtggat cacttgaggc caggagctca agaccagcct ggccaacatg gcgaagccct 240
   gtctctacta aaaatacaag agttggccag gcatggtggt ccatgcctgt gatctcggct 300
   acttgggaga ctgaggtggg aggatcgctt gggcccagga ggcagagggt acagtga 357
<210> 75
   <211> 608
   <212> PRT
   <213> Homo sapiens
<400> 75
   Met Ala Leu Arg Gly Pro Ala Gly Leu Gly Pro Gly Ser Arg Arg Pro
   1 5 10 15
   Leu Asp Glu Ala Val Ala Gly Ala Glu Gly Arg Glu Ala Pro Ala Leu
   20 25 30
   Val Ala Ala Gly Gly Ala Pro Glu Asp Asp Glu Glu Asp Asp Gly Arg
   35 40 45
   Gly Arg Gly Leu Leu Arg Trp Asp Ser Phe Ser Ala Trp Leu His Cys
   50 55 60
   Val Cys Val Val Gly Phe Asp Leu Glu Leu Gly Gln Ala Val Glu Val
   65 70 75 80
   Ile Tyr Pro Gln His Ser Lys Leu Thr Asp Arg Glu Lys Thr Asn Ile
   85 90 95
   Cys Tyr Leu Ser Phe Pro Asp Ser Asn Ser Gly Cys Leu Gly Asp Thr
   100 105 110
   Gln Phe Cys Phe Arg Phe Arg Gln Ser Ser Gly Arg Arg Val Ser Leu
   115 120 125
   His Cys Leu Leu Asp Gln Phe Asp Lys Asp Leu Pro Val Tyr Leu Lys
   130 135 140
   Lys Asp Pro Ala Tyr Phe Tyr Gly Tyr Val Tyr Phe Arg Gln Val Arg
   145 150 155 160
   Asp Lys Thr Leu Lys Arg Gly Tyr Phe Gln Lys Ser Leu Val Leu Ile
   165 170 175
   Ser Lys Leu Pro Tyr Ile His Phe Phe His Thr Val Leu Lys Gln Ile
   180 185 190
   Ala Pro Glu Tyr Phe Glu Lys Asn Glu Pro Tyr Leu Glu Ala Ala Cys
   195 200 205
   Asn Asp Val Asp Arg Trp Pro Ala Pro Val Pro Gly Lys Thr Leu His
   210 215 220
   Leu Pro Ile Met Gly Val Val Met Lys Val Arg Ile Pro Thr Cys His
   225 230 235 240
   Asp Lys Pro Gly Thr Thr Gln Ile Val Gln Leu Thr Gln Gln Val Asp
   245 250 255
   Thr Asn Ile Ser Val Ile Leu Pro Thr Val His Glu Val Asp Ile Phe
   260 265 270
   Arg Cys Phe Cys Pro Val Phe Leu His Ser Gln Met Leu Trp Glu Leu
   275 280 285
   Val Leu Leu Gly Glu Pro Leu Val Val Met Ala Pro Ser Pro Ser Glu
   290 295 300
   Ser Ser Glu Thr Val Leu Ala Leu Val Asn Cys Ile Ser Pro Leu Lys
   305 310 315 320
   Tyr Phe Ser Asp Phe Arg Pro Tyr Phe Thr Ile His Asp Ser Glu Phe
   325 330 335
   Lys Glu Tyr Thr Thr Arg Thr Gln Ala Pro Pro Ser Val Ile Leu Gly
   340 345 350
   Val Thr Asn Pro Phe Phe Ala Lys Thr Leu Gln His Trp Pro His Ile
   355 360 365
   Ile Arg Ile Gly Asp Leu Lys Pro Thr Gly Glu Ile Pro Lys Gln Val
   370 375 380
   Lys Val Lys Lys Leu Lys Asn Leu Lys Thr Leu Asp Ser Lys Pro Gly
   385 390 395 400
   Val Tyr Thr Ser Tyr Lys Pro Tyr Leu Asn Arg Asp Glu Glu Ile Ile
   405 410 415
   Lys Gln Leu Gln Lys Gly Val Gln Gln Lys Arg Pro Ser Glu Ala Gln
   420 425 430
   Ser Val Ile Leu Arg Arg Tyr Phe Leu Glu Leu Thr Gln Ser Phe Ile
   435 440 445
   Ile Pro Leu Glu Arg Tyr Val Ala Ser Leu Met Pro Leu Gln Lys Ser
   450 455 460
   Ile Ser Pro Trp Lys Ser Pro Pro Gln Leu Arg Gln Phe Leu Pro Glu
   465 470 475 480
   Glu Phe Met Lys Thr Leu Glu Lys Thr Gly Pro Gln Leu Thr Ser Arg
   485 490 495
   Ile Lys Gly Asp Trp Ile Gly Leu Tyr Arg His Phe Leu Lys Ser Pro
   500 505 510
   Asn Phe Asp Gly Trp Phe Lys Thr Arg Arg Lys Glu Met Thr Gln Lys
   515 520 525
   Leu Glu Ala Leu His Leu Glu Ala Leu Cys Glu Glu Asp Leu Leu Leu
   530 535 540
   Trp Ile Gln Lys His Thr Glu Val Glu Thr Val Asp Leu Val Leu Lys
   545                 550                 555                 560
   Leu Lys Asn Lys Leu Leu Gln Ala Asp Arg Glu His Leu Pro Val Lys
                   565                 570                 575
   Pro Asp Thr Met Glu Lys Leu Arg Thr His Ile Asp Ala Ile Ile Leu
               580                 585                 590
   Ala Leu Pro Glu Asp Leu Gln Gly Ile Leu Leu Lys Thr Gly Met Thr
           595                 600                 605
<210> 76
   <211> 1827
   <212> DNA
   <213> Homo sapiens
<400> 76
   atggctttga ggggccctgc gggcttgggg cccggctctc gaaggccgtt ggacgaagcg      60
   gtggcagggg ccgagggccg cgaggcgccg gcccttgtgg cggcgggagg cgcgccagag     120
   gacgatgaag aggacgatgg ccgtggccgg ggcctgctgc gctgggacag cttctccgcc     180
   tggctgcact gcgtgtgtgt ggtgggcttc gacctggagc tgggccaggc cgtggaggta     240
   atttatcctc agcattccaa acttactgac agagaaaaaa ccaatatttg ctatttgtct     300
   tttccagatt caaattcagg ttgtcttgga gatacccagt tttgttttag atttcgacag     360
   tcttctggga ggagggtgtc gctgcattgt ctcctggatc aatttgacaa agatttacca     420
   gtttacttaa agaaggatcc tgcttatttt tatggatatg tgtatttccg acaagttcga     480
   gataaaactc taaaaagagg ctactttcag aagtccttgg ttttgatcag caaactacct     540
   tatattcatt tttttcacac tgtgctcaaa cagatagcac cagagtattt tgaaaagaat     600
   gaaccttatt tggaagcagc ttgtaatgat gttgatcgat ggcctgcccc agtgccaggg     660
   aaaacattac acctgccaat catgggggtg gtaatgaagg tacggattcc cacatgtcat     720
   gacaagcctg ggacaactca aatagtgcag ttaactcagc aggtggacac aaatatatct     780
   gttattttac ctactgttca tgaggtggat attttcaggt gtttctgccc agttttcctt     840
   catagtcaga tgctctggga gctggtgctg ttgggggagc cccttgtggt tatggcgcca     900
   tcaccatcgg aatcatcaga gactgtattg gcacttgtta actgtatttc tccattaaag     960
   tacttcagtg atttccgacc ttatttcact attcatgata gtgaattcaa agaatatact    1020
   acccgtacgc aagctccgcc ctcagttata ttaggagtaa ccaacccttt ttttgctaag    1080
   acactccagc actggccaca cattattcga ataggagacc ttaaacctac aggtgaaatt    1140
   cctaagcagg ttaaagtgaa aaaactgaag aatctaaaga ctctggattc caaacctgga    1200
   gtttatactt catataagcc atatttaaat agagatgaag agatcataaa acaattacag    1260
   aagggtgtac aacagaaacg tccttctgag gctcaaagtg ttattcttcg acgctatttt    1320
   ttggaactga cacaaagttt catcattcca ttagaaagat atgtggcaag cttgatgcct    1380
   ttgcagaaaa gtatttcccc atggaagagt ccacctcaat taagacagtt tcttccagaa    1440
   gaatttatga aaacacttga gaaaacagga cctcagctaa cctctagaat aaaaggcgat    1500
   tggattggac tttaccggca tttcctaaag tctccaaatt ttgatggctg gtttaagacc    1560
   cggaggaagg aaatgaccca aaaattggag gcactccatc tagaagctct ttgtgaagag    1620
   gacttacttc tctggatcca gaaacacaca gaagtagaaa cagtagacct tgtcttgaag    1680
   ctgaaaaata agctgttgca ggctgatcga gagcacttac ctgtgaaacc tgacactatg    1740
   gaaaagttac ggacacacat agatgccatt atcttagcat tgccagagga cttgcaaggc    1800
   atactgctca aaacgggcat gacatga                                        1827
<210> 77
   <211> 175
   <212> PRT
   <213> Homo sapiens
<400> 77
   Met Gly Ala Leu Val Ile Arg Gly Ile Arg Asn Phe Asn Leu Glu Asn
   1               5                   10                  15
   Arg Ala Glu Arg Glu Ile Ser Lys Met Lys Pro Ser Val Ala Pro Arg
               20                  25                  30
   His Pro Ser Thr Asn Ser Leu Leu Arg Glu Gln Ile Ser Leu Tyr Pro
           35                  40                  45
   Glu Val Lys Gly Glu Ile Ala Arg Lys Asp Glu Lys Leu Leu Ser Phe
       50                  55                  60
   Leu Lys Asp Val Tyr Val Asp Ser Lys Asp Pro Val Ser Ser Leu Gln
   65                  70                  75                  80
   Val Lys Ala Ala Glu Thr Cys Gln Glu Pro Lys Glu Phe Arg Leu Pro
                   85                  90                  95
   Lys Asp His His Phe Asp Met Ile Asn Ile Lys Ser Ile Pro Lys Gly
               100                 105                 110
   Lys Ile Ser Ile Val Glu Ala Leu Thr Leu Leu Asn Asn His Lys Leu
           115                 120                 125
   Phe Pro Glu Thr Trp Thr Ala Glu Lys Ile Met Gln Glu Tyr Gln Leu
       130                 135                 140
   Glu Gln Lys Asp Val Asn Ser Leu Leu Lys Tyr Phe Val Thr Phe Glu
   145                 150                 155                 160
   Val Glu Ile Phe Pro Pro Glu Asp Lys Lys Ala Ile Arg Ser Lys
                   165                 170                 175
<210> 78
   <211> 528
   <212> DNA
   <213> Homo sapiens
<400> 78
   atgggagcac tagtgattcg cggtatcagg aatttcaacc tagagaaccg agcggaacgg     60
   gaaatcagca agatgaagcc ctctgtcgct cccagacacc cctctaccaa cagcctcctg    120
   cgagagcaga ttagtctcta tccagaagtt aaaggagaga ttgctcgtaa agatgaaaag    180
   ctgctgtcgt ttctaaaaga tgtgtatgtt gattccaaag atcctgtgtc ttccttgcag    240
   gtaaaagctg ctgaaacatg tcaagagccg aaggaattca gattgccgaa agaccatcat    300
   tttgatatga taaatattaa gagcattccc aaaggcaaaa tttccattgt agaagcattg    360
   acacttctca ataatcataa gcttttccca gaaacctgga ctgctgagaa aataatgcaa    420
   gaataccagt tagaacagaa agatgtgaat tctcttctta aatattttgt tacttttgaa    480
   gtcgaaatct tccctcctga agacaagaaa gcaatacgat caaaatga                 528
<210> 79
   <211> 132
   <212> PRT
   <213> Homo sapiens
<400> 79
<210> 80
   <211> 399
   <212> DNA
   <213> Homo sapiens
<400> 80
   atggtatttt attgtatgca cttaaagtat tattctgaga aggctccaaa ggggccacag 60
   ggcaaaaaca attacgaccc tattgggctc ggaacacaat accccaaagt atggcacttt 120
   ggcatgctga gtgctttaaa ccaaaggaga ttggagggcc tcagacgcaa agtctctctg 180
   aacctctcct actctcctgt ctctgacttc tttttcccct acaaaggaag tcatagaaac 240
   caaaattcct cctcagtcta ttaccatgag atcataccct tttgtccagt cacatttcta 300
   caaggctgcc cattcttcat ggaatgtaag cataaaaata gacagttttg gctgggcggg 360
   gtggctcggg cctgtaatcc cagcactttg ggtggctga 399
<210> 81
   <211> 186
   <212> PRT
   <213> Homo sapiens
<400> 81
   Met Ala Gly Ala Glu Trp Lys Ser Leu Glu Glu Cys Leu Glu Lys His
   1 5 10 15
   Leu Pro Leu Pro Asp Leu Gln Glu Val Lys Arg Val Leu Tyr Gly Lys
   20 25 30
   Glu Leu Arg Lys Leu Asp Leu Pro Arg Glu Ala Phe Glu Ala Ala Ser
   35 40 45
   Arg Glu Asp Phe Glu Leu Gln Gly Tyr Ala Phe Glu Ala Ala Glu Glu
   50 55 60
   Gln Leu Arg Arg Pro Arg Ile Val His Val Gly Leu Val Gln Asn Arg
   65 70 75 80
   Ile Pro Leu Pro Ala Asn Ala Pro Val Ala Glu Gln Val Ser Ala Leu
   85 90 95
   His Arg Arg Ile Lys Ala Ile Val Glu Val Ala Ala Met Cys Gly Val
   100 105 110
   Asn Ile Ile Cys Phe Gln Glu Ala Trp Ile Leu Arg Pro His His Gln
   115 120 125
   Glu Pro Arg Pro Pro Cys Cys Tyr Ala Pro Ser Cys Cys Leu Ile Pro
   130 135 140
   Ser Val Phe Pro His Arg Ser Glu Ser Ile Arg Ser Ser Pro Ser Leu
   145 150 155 160
   Tyr His His Leu Val Gln Ala Pro Gly Ser Ser Pro Leu Ala Phe Val
   165 170 175
   Ile Pro Ser Ser Trp Ser Pro Val Pro Ser
   180 185
<210> 82
   <211> 561
   <212> DNA
   <213> Homo sapiens
<400> 82
   atggcgggcg ctgagtggaa gtcgctggag gaatgcttgg agaagcacct gccgctcccc     60
   gacttgcagg aagtgaagcg cgttctctat ggcaaggaac tcaggaagct tgatctgccc    120
   agggaagctt tcgaagctgc ctccagagaa gactttgaac tgcagggata tgcctttgaa    180
   gcagcggagg agcagctgag acgaccccgc attgtgcacg tggggctggt tcagaacaga    240
   atccccctcc ccgcaaatgc ccctgtggca gaacaggtct ctgcccttca tagacgcata    300
   aaggctatcg tagaggtggc tgcaatgtgt ggagtcaaca tcatctgttt ccaggaagca    360
   tggattctcc gtccacatca ccaggaaccc cggcctccct gctgttacgc accgagctgt    420
   tgtctgattc cttcagtttt cccacatcgt tctgaatcca ttcgctcctc tccatctctc    480
   taccaccacc tggtccaagc ccctggcagc tcacctctgg cttttgtaat tccctccagc    540
   tggtctcctg tgccctcctg a                                              561
<210> 83
   <211> 384
   <212> PRT
   <213> Homo sapiens
<400> 83 <210> 84
   <211> 1155
   <212> DNA
   <213> Homo sapiens
<400> 84
<210> 85
   <211> 254
   <212> PRT
   <213> Homo sapiens
<400> 85
   Met Asn Ser Arg Gln Ala Trp Arg Leu Phe Leu Ser Gln Gly Arg Gly
   1               5                   10                  15
   Asp Arg Trp Val Ser Arg Pro Arg Gly His Phe Ser Pro Ala Leu Arg
               20                  25                  30
   Arg Glu Phe Phe Thr Thr Thr Thr Lys Glu Gly Tyr Asp Arg Arg Pro
           35                  40                  45
   Val Asp Ile Thr Pro Leu Glu Gln Arg Lys Leu Thr Phe Asp Thr His
       50                  55                  60
   Ala Leu Val Gln Asp Leu Glu Thr His Gly Phe Asp Lys Thr Gln Ala
   65                  70                  75                  80
   Glu Thr Ile Val Ser Ala Leu Thr Ala Leu Ser Asn Val Ser Leu Asp
                   85                  90                  95
   Thr Ile Tyr Lys Glu Met Val Thr Gln Ala Gln Gln Glu Ile Thr Val
               100                 105                 110
   Gln Gln Leu Met Ala His Leu Asp Ala Ile Arg Lys Asp Met Val Ile
           115                 120                 125
   Leu Glu Lys Ser Glu Phe Ala Asn Leu Arg Ala Glu Asn Glu Lys Met
       130                 135                 140
   Lys Ile Glu Leu Asp Gln Val Lys Gln Gln Leu Met His Glu Thr Ser
   145                 150                 155                 160
   Arg Ile Arg Ala Asp Asn Lys Leu Asp Ile Asn Leu Glu Arg Ser Arg
                   165                 170                 175
   Val Thr Asp Met Phe Thr Asp Gln Glu Lys Gln Leu Met Glu Thr Thr
               180                 185                 190
   Thr Glu Phe Thr Lys Lys Asp Thr Gln Thr Lys Ser Ile Ile Ser Glu
           195                 200                 205
   Thr Ser Asn Lys Ile Asp Ala Glu Ile Ala Ser Leu Lys Thr Leu Met
       210                 215                 220
   Glu Ser Asn Lys Leu Glu Thr Ile Arg Tyr Leu Ala Ala Ser Val Phe
   225                 230                 235                 240
   Thr Cys Leu Ala Ile Ala Leu Gly Phe Tyr Arg Phe Trp Lys
                   245                 250
<210> 86
   <211> 765
   <212> DNA
   <213> Homo sapiens
<400> 86
   atgaatagtc gccaggcttg gcggctcttt ctctcccaag gcagaggaga tcgttgggtt 60
   tcaaggcccc gcgggcattt ctcgccggcc ctgcggagag agttcttcac taccacaacc 120
   aaggagggat atgataggcg gccagtggat ataactcctt tagaacaaag gaaattaact 180
   tttgataccc atgcattggt tcaggacttg gaaactcatg gatttgacaa aacacaagca 240
   gaaacaattg tatcagcgtt aactgcttta tcaaatgtca gcctggatac tatctataaa 300
   gagatggtca ctcaagctca acaggaaata acagtacaac agctaatggc tcatttggat 360
   gctatcagga aagacatggt catcctagag aaaagtgaat ttgcaaatct gagagcagag 420
   aatgagaaaa tgaaaattga attagaccaa gttaagcaac aactaatgca tgaaaccagt 480
   cgaatcagag cagataataa actggatatc aacttagaaa ggagcagagt aacagatatg 540
   tttacagatc aagaaaagca acttatggaa acaactacag aatttacaaa aaaggatact 600
   caaaccaaaa gtattatttc agagaccagt aataaaattg acgctgaaat tgcttcctta 660
   aaaacactga tggaatctaa caaacttgag acaattcgtt atcttgcagc ttcggtgttt 720
   acttgcctgg caatagcatt gggattttat agattctgga agtag 765
<210> 87
   <211> 254
   <212> PRT
   <213> Homo sapiens
<400> 87
   Met Asn Ser Arg Gln Ala Trp Arg Leu Leu Leu Ser Gln Gly Arg Gly
   1 5 10 15
   Asp Arg Trp Val Ser Arg Pro Arg Gly His Phe Ser Pro Ala Leu Arg
   20 25 30
   Arg Glu Phe Phe Thr Thr Thr Thr Lys Glu Gly Tyr Asp Arg Arg Pro
   35 40 45
   Val Asp Ile Thr Pro Leu Glu Gln Arg Lys Leu Thr Phe Asp Thr His
   50 55 60
   Ala Leu Val Gln Asp Leu Glu Thr His Gly Phe Asp Lys Thr Gln Ala
   65 70 75 80
   Glu Thr Ile Val Ser Ala Leu Thr Ala Leu Ser Asn Val Ser Leu Asp
   85 90 95
   Thr Ile Tyr Lys Glu Met Val Thr Gln Ala Gln Gln Glu Ile Thr Val
   100 105 110
   Gln Gln Leu Met Ala His Leu Asp Ala Ile Arg Lys Asp Met Val Ile
   115 120 125
   Leu Glu Lys Ser Glu Phe Ala Asn Leu Arg Ala Glu Asn Glu Lys Met
   130 135 140
   Lys Ile Glu Leu Asp Gln Val Lys Gln Gln Leu Met His Glu Thr Ser
   145 150 155 160
   Arg Ile Arg Ala Asp Asn Lys Leu Asp Ile Asn Leu Glu Arg Ser Arg
   165 170 175
   Val Thr Asp Met Phe Thr Asp Gln Glu Lys Gln Leu Met Glu Thr Thr
   180 185 190
   Thr Glu Phe Thr Lys Lys Asp Thr Gln Thr Lys Ser Ile Ile Ser Glu
   195 200 205
   Thr Ser Asn Lys Ile Asp Ala Glu Ile Ala Ser Leu Lys Thr Leu Met
   210 215 220
   Glu Ser Asn Lys Leu Glu Thr Ile Arg Tyr Leu Ala Ala Ser Val Phe
   225 230 235 240
   Thr Cys Leu Ala Ile Ala Leu Gly Phe Tyr Arg Phe Trp Lys
   245 250
<210> 88
   <211> 765
   <212> DNA
   <213> Homo sapiens
<400> 88
   atgaatagtc gccaggcttg gcggctcttg ctctcccaag gcagaggaga tcgttgggtt     60
   tcaaggcccc gcgggcattt ctcgccggcc ctgcggagag agttcttcac taccacaacc    120
   aaggagggat atgataggcg gccagtggat ataactcctt tagaacaaag gaaattaact    180
   tttgataccc atgcattggt tcaggacttg gaaactcatg gatttgacaa aacacaagca    240
   gaaacaattg tatcagcgtt aactgcttta tcaaatgtca gcctggatac tatctataaa    300
   gagatggtca ctcaagctca acaggaaata acagtacaac agctaatggc tcatttggat    360
   gctatcagga aagacatggt catcctagag aaaagtgaat ttgcaaatct gagagcagag    420
   aatgagaaaa tgaaaattga attagaccaa gttaagcaac aactaatgca tgaaaccagt    480
   cgaatcagag cagataataa actggatatc aacttagaaa ggagcagagt aacagatatg    540
   tttacagatc aagaaaagca acttatggaa acaactacag aatttacaaa aaaggatact    600
   caaaccaaaa gtattatttc agagaccagt aataaaattg acgctgaaat tgcttcctta    660
   aaaacactga tggaatctaa caaacttgag acaattcgtt atcttgcagc ttcggtgttt    720
   acttgcctgg caatagcatt gggattttat agattctgga agtag                    765
<210> 89
   <211> 353
   <212> PRT
   <213> Homo sapiens
<400> 89
   Met Arg Leu Pro Arg Arg Ala Ala Leu Gly Leu Leu Pro Leu Leu Leu
   1               5                   10                  15
   Leu Leu Pro Pro Ala Pro Glu Ala Ala Lys Lys Pro Thr Pro Cys His
               20                  25                  30
   Arg Cys Arg Gly Leu Val Asp Lys Phe Asn Gln Gly Met Val Asp Thr
   35 40 45
   Ala Lys Lys Asn Phe Gly Gly Gly Asn Thr Ala Trp Glu Glu Lys Thr
   50 55 60
   Leu Ser Lys Tyr Glu Ser Ser Glu Ile Arg Leu Leu Glu Ile Leu Glu
   65 70 75 80
   Gly Leu Cys Glu Ser Ser Asp Phe Glu Cys Asn Gln Met Leu Glu Ala
   85 90 95
   Gln Glu Glu His Leu Glu Ala Trp Trp Leu Gln Leu Lys Ser Glu Tyr
   100 105 110
   Pro Asp Leu Phe Glu Trp Phe Cys Val Lys Thr Leu Lys Val Cys Cys
   115 120 125
   Ser Pro Gly Thr Tyr Gly Pro Asp Cys Leu Ala Cys Gln Gly Gly Ser
   130 135 140
   Gln Arg Pro Cys Ser Gly Asn Gly His Cys Ser Gly Asp Gly Ser Arg
   145 150 155 160
   Gln Gly Asp Gly Ser Cys Arg Cys His Met Gly Tyr Gln Gly Pro Leu
   165 170 175
   Cys Thr Asp Cys Met Asp Gly Tyr Phe Ser Ser Leu Arg Asn Glu Thr
   180 185 190
   His Ser Ile Cys Thr Ala Cys Asp Glu Ser Cys Lys Thr Cys Ser Gly
   195 200 205
   Leu Thr Asn Arg Asp Cys Gly Glu Cys Glu Val Gly Trp Val Leu Asp
   210 215 220
   Glu Gly Ala Cys Val Asp Val Asp Glu Cys Ala Ala Glu Pro Pro Pro
   225 230 235 240
   Cys Ser Ala Ala Gln Phe Cys Lys Asn Ala Asn Gly Ser Tyr Thr Cys
   245 250 255
   Glu Glu Cys Asp Ser Ser Cys Val Gly Cys Thr Gly Glu Gly Pro Gly
   260 265 270
   Asn Cys Lys Glu Cys Ile Ser Gly Tyr Ala Arg Glu His Gly Gln Cys
   275 280 285
   Ala Asp Val Asp Glu Cys Ser Leu Ala Glu Lys Thr Cys Val Arg Lys
   290 295 300
   Asn Glu Asn Cys Tyr Asn Thr Pro Gly Ser Tyr Val Cys Val Cys Pro
   305 310 315 320
   Asp Gly Phe Glu Glu Thr Glu Asp Ala Cys Val Pro Pro Ala Glu Ala
   325 330 335
   Glu Ala Thr Glu Gly Glu Ser Pro Thr Gln Leu Pro Ser Arg Glu Asp
   340 345 350
   Leu
<210> 90
   <211> 1062
   <212> DNA
   <213> Homo sapiens
<400> 90
   atgcgcctgc cgcgccgggc cgcgctgggg ctcctgccgc ttctgctgct gctgccgccc      60
   gcgccggagg ccgccaagaa gccgacgccc tgccaccggt gccgggggct ggtggacaag     120
   tttaaccagg ggatggtgga caccgcaaag aagaactttg gcggcgggaa cacggcttgg     180
   gaggaaaaga cgctgtccaa gtacgagtcc agcgagattc gcctgctgga gatcctggag     240
   gggctgtgcg agagcagcga cttcgaatgc aatcagatgc tagaggcgca ggaggagcac     300
   ctggaggcct ggtggctgca gctgaagagc gaatatcctg acttattcga gtggttttgt     360
   gtgaagacac tgaaagtgtg ctgctctcca ggaacctacg gtcccgactg tctcgcatgc     420
   cagggcggat cccagaggcc ctgcagcggg aatggccact gcagcggaga tgggagcaga     480
   cagggcgacg ggtcctgccg gtgccacatg gggtaccagg gcccgctgtg cactgactgc     540
   atggacggct acttcagctc gctccggaac gagacccaca gcatctgcac agcctgtgac     600
   gagtcctgca agacgtgctc gggcctgacc aacagagact gcggcgagtg tgaagtgggc     660
   tgggtgctgg acgagggcgc ctgtgtggat gtggacgagt gtgcggccga gccgcctccc     720
   tgcagcgctg cgcagttctg taagaacgcc aacggctcct acacgtgcga agagtgtgac     780
   tccagctgtg tgggctgcac aggggaaggc ccaggaaact gtaaagagtg tatctctggc     840
   tacgcgaggg agcacggaca gtgtgcagat gtggacgagt gctcactagc agaaaaaacc     900
   tgtgtgagga aaaacgaaaa ctgctacaat actccaggga gctacgtctg tgtgtgtcct     960
   gacggcttcg aagaaacgga agatgcctgt gtgccgccgg cagaggctga agccacagaa    1020
   ggagaaagcc cgacacagct gccctcccgc gaagacctgt aa                       1062
<210> 91
   <211> 321
   <212> PRT
   <213> Homo sapiens
<400> 91
   Met Arg Leu Pro Arg Arg Ala Ala Leu Gly Leu Leu Pro Leu Leu Leu
   1 5 10 15
   Leu Leu Pro Pro Ala Pro Glu Ala Ala Lys Lys Pro Thr Pro Cys His
   20 25 30
   Arg Cys Arg Gly Leu Val Asp Lys Phe Asn Gln Gly Met Val Asp Thr
   35 40 45
   Ala Lys Lys Asn Phe Gly Gly Gly Asn Thr Ala Trp Glu Glu Lys Thr
   50 55 60
   Leu Ser Lys Tyr Glu Ser Ser Glu Ile Arg Leu Leu Glu Ile Leu Glu
   65 70 75 80
   Gly Leu Cys Glu Ser Ser Asp Phe Glu Cys Asn Gln Met Leu Glu Ala
   85 90 95
   Gln Glu Glu His Leu Glu Ala Trp Trp Leu Gln Leu Lys Ser Glu Tyr
   100 105 110
   Pro Asp Leu Phe Glu Trp Phe Cys Val Lys Thr Leu Lys Val Cys Cys
   115 120 125
   Ser Pro Gly Thr Tyr Gly Pro Asp Cys Leu Ala Cys Gln Gly Gly Ser
   130 135 140
   Gln Arg Pro Cys Ser Gly Asn Gly His Cys Ser Gly Asp Gly Ser Arg
   145 150 155 160
   Gln Gly Asp Gly Ser Cys Arg Cys His Met Gly Tyr Gln Gly Pro Leu
   165 170 175
   Cys Thr Asp Cys Met Asp Gly Tyr Phe Ser Ser Leu Arg Asn Glu Thr
   180 185 190
   His Ser Ile Cys Thr Ala Cys Asp Glu Ser Cys Lys Thr Cys Ser Gly
   195 200 205
   Leu Thr Asn Arg Asp Cys Gly Glu Cys Glu Val Gly Trp Val Leu Asp
   210 215 220
   Glu Gly Ala Cys Val Asp Val Asp Glu Cys Ala Ala Glu Pro Pro Pro
   225 230 235 240
   Cys Ser Ala Ala Gln Phe Cys Lys Asn Ala Asn Gly Ser Tyr Thr Cys
   245 250 255
   Glu Asp Val Asp Glu Cys Ser Leu Ala Glu Lys Thr Cys Val Arg Lys
   260 265 270
   Asn Glu Asn Cys Tyr Asn Thr Pro Gly Ser Tyr Val Cys Val Cys Pro
   275 280 285
   Asp Gly Phe Glu Glu Thr Glu Asp Ala Cys Val Pro Pro Ala Glu Ala
   290 295 300
   Glu Ala Thr Glu Gly Glu Ser Pro Thr Gln Leu Pro Ser Arg Glu Asp
   305 310 315 320
   Leu
<210> 92
   <211> 284
   <212> PRT
   <213> Homo sapiens
<400> 92
   Met Arg Leu Pro Arg Arg Ala Ala Leu Gly Leu Leu Pro Leu Leu Leu
   1 5 10 15
   Leu Leu Pro Pro Ala Pro Glu Ala Ala Lys Lys Pro Thr Pro Cys His
   20 25 30
   Arg Cys Arg Gly Leu Val Asp Lys Phe Asn Gln Gly Met Val Asp Thr
   35 40 45
   Ala Lys Lys Asn Phe Gly Gly Gly Asn Thr Ala Trp Glu Glu Lys Thr
   50 55 60
   Leu Ser Lys Tyr Glu Ser Ser Glu Ile Arg Leu Leu Glu Ile Leu Glu
   65 70 75 80
   Gly Leu Cys Glu Ser Ser Asp Phe Glu Cys Asn Gln Met Leu Glu Ala
   85 90 95
   Gln Glu Glu His Leu Glu Ala Trp Trp Leu Gln Leu Lys Ser Glu Tyr
   100 105 110
   Pro Asp Leu Phe Glu Trp Phe Cys Val Lys Thr Leu Lys Val Cys Cys
   115 120 125
   Ser Pro Gly Thr Tyr Gly Pro Asp Cys Leu Ala Cys Gln Gly Gly Ser
   130 135 140
   Gln Arg Pro Cys Ser Gly Asn Gly His Cys Ser Gly Asp Gly Ser Arg
   145 150 155 160
   Gln Gly Asp Gly Ser Cys Arg Cys His Met Gly Tyr Gln Gly Pro Leu
   165 170 175
   Cys Thr Asp Cys Met Asp Gly Tyr Phe Ser Ser Leu Arg Asn Glu Thr
   180 185 190
   His Ser Ile Cys Thr Ala Cys Asp Glu Ser Cys Lys Thr Cys Ser Gly
   195 200 205
   Leu Thr Asn Arg Asp Cys Gly Glu Cys Glu Val Gly Trp Val Leu Asp
   210 215 220
   Glu Gly Ala Cys Val Asp Val Asp Glu Cys Ala Ala Glu Pro Pro Pro
   225 230 235 240
   Cys Ser Ala Ala Gln Phe Cys Lys Asn Ala Asn Gly Ser Tyr Thr Cys
   245 250 255
   Glu Gly Gly Pro Gly Gly Arg Val Cys Thr Pro Gly Pro Ala Gly Phe
   260 265 270
   Arg Cys Cys Leu Cys Gln His Ser Phe Met Ala Ser
   275 280
<210> 93
   <211> 325
   <212> PRT
   <213> Homo sapiens
<400> 93
   Met Arg Leu Pro Arg Arg Ala Ala Leu Gly Leu Leu Pro Leu Leu Leu
   1               5                   10                  15
   Leu Leu Pro Pro Ala Pro Glu Ala Ala Lys Lys Pro Thr Pro Cys His
               20                  25                  30
   Arg Cys Arg Gly Leu Val Asp Lys Phe Asn Gln Gly Met Val Asp Thr
           35                  40                  45
   Ala Lys Lys Asn Phe Gly Gly Gly Asn Thr Ala Trp Glu Glu Lys Thr
       50                  55                  60
   Leu Ser Lys Tyr Glu Ser Ser Glu Ile Arg Leu Leu Glu Ile Leu Glu
   65                  70                  75                  80
   Gly Leu Cys Glu Ser Ser Asp Phe Glu Cys Asn Gln Met Leu Glu Ala
                   85                  90                  95
   Gln Glu Glu His Leu Glu Ala Trp Trp Leu Gln Leu Lys Ser Glu Tyr
               100                 105                 110
   Pro Asp Leu Phe Glu Trp Phe Cys Val Lys Thr Leu Lys Val Cys Cys
           115                 120                 125
   Ser Pro Gly Thr Tyr Gly Pro Asp Cys Leu Ala Cys Gln Gly Gly Ser
       130                 135                 140
   Gln Arg Pro Cys Ser Gly Asn Gly His Cys Ser Gly Asp Gly Ser Arg
   145 150 155 160
   Gln Gly Asp Gly Ser Cys Arg Cys His Met Gly Tyr Gln Gly Pro Leu
   165 170 175
   Cys Thr Asp Cys Met Asp Gly Tyr Phe Ser Ser Leu Arg Asn Glu Thr
   180 185 190
   His Ser Ile Cys Thr Ala Cys Asp Glu Ser Cys Lys Thr Cys Ser Gly
   195 200 205
   Leu Thr Asn Arg Asp Cys Gly Glu Cys Glu Val Gly Trp Val Leu Asp
   210 215 220
   Glu Gly Ala Cys Val Glu Cys Asp Ser Ser Cys Val Gly Cys Thr Gly
   225 230 235 240
   Glu Gly Pro Gly Asn Cys Lys Glu Cys Ile Ser Gly Tyr Ala Arg Glu
   245 250 255
   His Gly Gln Cys Ala Asp Val Asp Glu Cys Ser Leu Ala Glu Lys Thr
   260 265 270
   Cys Val Arg Lys Asn Glu Asn Cys Tyr Asn Thr Pro Gly Ser Tyr Val
   275 280 285
   Cys Val Cys Pro Asp Gly Phe Glu Glu Thr Glu Asp Ala Cys Val Pro
   290 295 300
   Pro Ala Glu Ala Glu Ala Thr Glu Gly Glu Ser Pro Thr Gln Leu Pro
   305 310 315 320
   Ser Arg Glu Asp Leu
   325
<210> 94
   <211> 402
   <212> PRT
   <213> Homo sapiens
<400> 94
   Met Arg Leu Pro Arg Arg Ala Ala Leu Gly Leu Leu Pro Leu Leu Leu
   1 5 10 15
   Leu Leu Pro Pro Ala Pro Glu Ala Ala Lys Lys Pro Thr Pro Cys His
   20 25 30
   Arg Cys Arg Gly Leu Val Asp Lys Phe Asn Gln Gly Met Val Asp Thr
   35 40 45
   Ala Lys Lys Asn Phe Gly Gly Gly Asn Thr Ala Trp Glu Glu Lys Thr
        50                  55                  60
   Leu Ser Lys Tyr Glu Ser Ser Glu Ile Arg Leu Leu Glu Ile Leu Glu
   65                  70                  75                  80
   Gly Leu Cys Glu Ser Ser Asp Phe Glu Cys Asn Gln Met Leu Glu Ala
                   85                  90                  95
   Gln Glu Glu His Leu Glu Ala Trp Trp Leu Gln Leu Lys Ser Glu Tyr
               100                 105                 110
   Pro Asp Leu Phe Glu Trp Phe Cys Val Lys Thr Leu Lys Val Cys Cys
           115                 120                 125
   Ser Pro Gly Thr Tyr Gly Pro Asp Cys Leu Ala Cys Gln Gly Gly Ser
       130                 135                 140
   Gln Arg Pro Cys Ser Gly Asn Gly His Cys Ser Gly Asp Gly Ser Arg
   145                 150                 155                 160
   Gln Gly Asp Gly Ser Cys Arg Cys His Met Gly Tyr Gln Gly Pro Leu
                   165                 170                 175
   Cys Thr Asp Cys Met Asp Gly Tyr Phe Ser Ser Leu Arg Asn Glu Thr
               180                 185                 190
   His Ser Ile Cys Thr Val Arg Thr Gly Leu Ser Asp Ser Tyr Pro Pro
           195                 200                 205
   Cys Cys Leu Ser Leu Gly Cys Trp Arg Gly Val Gly His Ala Trp Ile
       210                 215                 220
   Arg Gly Arg Asn Thr His Thr Gln Pro Gly Tyr Ser Ser Arg Val Trp
   225                 230                 235                 240
   Ile Ala Ala Phe Ser Pro Ala Cys Asp Glu Ser Cys Lys Thr Cys Ser
                   245                 250                 255
   Gly Leu Thr Asn Arg Asp Cys Gly Glu Cys Glu Val Gly Trp Val Leu
               260                 265                 270
   Asp Glu Gly Ala Cys Val Asp Val Asp Glu Cys Ala Ala Glu Pro Pro
           275                 280                 285
   Pro Cys Ser Ala Ala Gln Phe Cys Lys Asn Ala Asn Gly Ser Tyr Thr
       290                 295                 300
   Cys Glu Glu Cys Asp Ser Ser Cys Val Gly Cys Thr Gly Glu Gly Pro
   305                 310                 315                 320
   Gly Asn Cys Lys Glu Cys Ile Ser Gly Tyr Ala Arg Glu His Gly Gln
   325 330 335
   Cys Ala Asp Val Asp Glu Cys Ser Leu Ala Glu Lys Thr Cys Val Arg
   340 345 350
   Lys Asn Glu Asn Cys Tyr Asn Thr Pro Gly Ser Tyr Val Cys Val Cys
   355 360 365
   Pro Asp Gly Phe Glu Glu Thr Glu Asp Ala Cys Val Pro Pro Ala Glu
   370 375 380
   Ala Glu Ala Thr Glu Gly Glu Ser Pro Thr Gln Leu Pro Ser Arg Glu
   385 390 395 400
   Asp Leu
<210> 95
   <211> 373
   <212> PRT
   <213> Homo sapiens
<400> 95
   Met Arg Leu Pro Arg Arg Ala Ala Leu Gly Leu Leu Pro Leu Leu Leu
   1 5 10 15
   Leu Leu Pro Pro Ala Pro Glu Ala Ala Lys Lys Pro Thr Pro Cys His
   20 25 30
   Arg Cys Arg Gly Leu Val Asp Lys Phe Asn Gln Gly Met Val Asp Thr
   35 40 45
   Ala Lys Lys Asn Phe Gly Gly Gly Asn Thr Ala Trp Glu Glu Lys Thr
   50 55 60
   Leu Ser Lys Tyr Glu Ser Ser Glu Ile Arg Leu Leu Glu Ile Leu Glu
   65 70 75 80
   Gly Leu Cys Glu Ser Ser Asp Phe Glu Cys Asn Gln Met Leu Glu Ala
   85 90 95
   Gln Glu Glu His Leu Glu Ala Trp Trp Leu Gln Leu Lys Ser Glu Tyr
   100 105 110
   Pro Asp Leu Phe Glu Trp Phe Cys Val Lys Thr Leu Lys Val Cys Cys
   115 120 125
   Ser Pro Gly Thr Tyr Gly Pro Asp Cys Leu Ala Cys Gln Gly Gly Ser
   130 135 140
   Gln Arg Pro Cys Ser Gly Asn Gly His Cys Ser Gly Asp Gly Ser Arg
   145 150 155 160
   Gln Gly Asp Gly Ser Cys Arg Cys His Met Gly Tyr Gln Gly Pro Leu
   165 170 175
   Cys Thr Asp Cys Met Asp Gly Tyr Phe Ser Ser Leu Arg Asn Glu Thr
   180 185 190
   His Ser Ile Cys Thr Ala Cys Asp Glu Ser Cys Lys Thr Cys Ser Gly
   195 200 205
   Leu Thr Asn Arg Asp Cys Gly Glu Cys Glu Val Gly Trp Val Leu Asp
   210 215 220
   Glu Gly Ala Cys Val Asp Val Asp Glu Cys Ala Ala Glu Pro Pro Pro
   225 230 235 240
   Cys Ser Ala Ala Gln Phe Cys Lys Asn Ala Asn Gly Ser Tyr Thr Cys
   245 250 255
   Glu Glu Cys Asp Ser Ser Cys Val Gly Cys Thr Gly Glu Gly Pro Gly
   260 265 270
   Asn Cys Lys Glu Cys Ile Ser Gly Tyr Ala Arg Glu His Gly Gln Cys
   275 280 285
   Ala Asp Val Asp Glu Cys Ser Leu Ala Glu Lys Thr Cys Val Arg Lys
   290 295 300
   Asn Glu Asn Cys Tyr Asn Thr Pro Gly Ser Tyr Val Cys Val Cys Pro
   305 310 315 320
   Asp Gly Phe Glu Glu Thr Glu Asp Ala Cys Val Pro Pro Ala Glu Ala
   325 330 335
   Gly Glu Trp His Gly Cys Pro Pro His Arg Leu Pro Ser Pro Gly Pro
   340 345 350
   Gln Gly Leu His Val Asp Trp Leu Leu Gly Leu Lys Ser Thr Gln Met
   355 360 365
   Val Ala Leu Arg Trp
   370
<210> 96
   <211> 2204
   <212> PRT
   <213> Homo sapiens
<400> 96
<210> 97
   <211> 6615
   <212> DNA
   <213> Homo sapiens
<400> 97
   atgcctctgt tcttctccgc gctgttggtc ttgctgctag ttgcgcttag cgccctcttt    60
   ctaggccggt ggcttgtggt ccggttggcc accaagtggt gtcagcggaa gctgcaggcg   120
   gagctaaaga ttggctcctt ccgctttttt tggatccaga atgtcagtct taagtttcag   180
   caacaccagc aaacagtgga aattgataac ctgtggattt ccagcaaacc ccttagccat   240
   gatcttccac actatgtggc attgtgcttt ggagaagtgc gtatcagaac ggacctacag   300
   aaagtttctg acctgtctgc cccattctcc cagagcgctg gggtggatca aaaggaactg   360
   tccttcagcc catccttatt gaagatcttc tgccaactat tctccattca tgtagatgct   420
   ataaacatca tggttctcaa ggtggatacc tctgagtcct tatggcatat tcagatcagt   480
   agaagcagat ttcttttgga tagtgatggg aaaaggctaa tctgtgaggt gagcttatgt   540
   aagatcaaca gcaaagttct aaagagtggt cagctggagg acacctgcct agtggagctt   600
   tcactggccc tggacctgtg tctaaaggtg ggcattagca gtcggcatct cactgctatc   660
   actgtggatg tgtggacact ccatgctgaa ctgcatgagg gcctcttcca gagccaactg   720
   ctgtgccagg gcccaagcct agcatctaag cctgttccct gttcagaggt gacagaaaac   780
   ttagttgagc caactctgcc tggcctattc cttctccagc agctgccaga ccaggtcaag   840
   gttaagatgg agaacacaag cgtggtattg tccatgaata gtcaaaagag gcacctgact   900
   tggactctga agctgctgca gttcctgtac caccgtgatg aggatcagct gccccttcga   960
   agcttcacag caaactctga tatggcacag atgagcactg aactgctgct ggaagatggg  1020
   ttgttgttgt cccagagtcg ccaacgcatt gtctgcctca actccctcaa ggctagtgtg  1080
   caggtgacca ccattgacct ctcagcctcc ctagttctga acacttgcat cattcactac 1140
   cggcaccagg aattctctca ctggctgcac ctgctagcac tggaaaccca agggtctagt 1200
   tcacctgttc taaagcaaag gaaaaaaaga accttccccc aaatcctggc tcccatcatc 1260
   tttagcacct ccatctccaa tgtcaacatt tccattcaac ttggagatac accacctttt 1320
   gccttgggat tcaattctat ctctctggat taccagcacc tcaggccaca aagcatccat 1380
   cagcggggcg tcctaactgt ggaccacctc tgctggcgtg tgggcagtga ctcccacatt 1440
   cagcgggcgc cacacccacc caatatgcat gtttggggtg aggcacttgt tctggactcc 1500
   ttcacactac agggtagcta taaccagcct ctggtcctgt ccagcaccca gtcagatacc 1560
   ctttttcttg attgtaccat tcgaggactt caggtggaag catcagatac ctgtgcccaa 1620
   tgtctgtctc gtatcttatc cctgatgggt ccacaatctg ggaagtcagc tgtctctagg 1680
   cactcttcat ttggggaatc tgtgtcatta ctgtggaagg tggacttgaa ggtcgaagac 1740
   atgaacttgt ttaccctttc tgccttggtt ggtgcttcag aggtacgact ggacacccta 1800
   gctatcctgg gcagtgcaga gacgtccact gtggggattc aaggacttgt gttagcgctg 1860
   gtgaaatcag tcacggagaa gatgcaaccc tgttgcaagg cccctgacat ccctacccca 1920
   gtgctcagcc tttccatgct ctccatcacc tatcacagca gcatccgctc tctggaggtt 1980
   cagtgtggtg cagggctgac cttactttgg agccccccag atcacatgta cctgtaccag 2040
   catgtcctgg ccactctaca gtgccgagac ctactaagag ccactgtgtt tcctgagact 2100
   gtaccatccc ttgcactaga gacttcagga actacttctg agctagaagg ccgtgcccct 2160
   gagccattac ccccaaagcg gctgctaaac ctaaccctgg aggtgagcac agccaagctc 2220
   acagcttttg tagctgagga caagttcatt accctggctg cagagagtgt gtcactgagc 2280
   cggcatggag gttccctgca ggcatactgt ccagagctgg ctgctggctt tgatggcaat 2340
   agtatcttca acttcaagga ggtggaggtg cagctgctac ctgagctgga gtttccttat 2400
   cagtatgact tttctcgaac tctagatgag gctgtgggag ttcagaagtg gctgaaggga 2460
   ctacatcaag ggactcgtgc ttgggcctct ccaagccctg tcccactccc acctgatcta 2520
   ctcttaaagg ttgagcactt ctcatgggtt ttcttggatg atgtttttga ggtgaaactt 2580
   catgataact acgagctgat gaaggatgaa agtaaggaga gtgccaaaag actacagcta 2640
   ctggatgcta aagtggccgc ccttcggaag cagcatgggg agttgttgcc tgcccgcaaa 2700
   attgaggagc tctatgcctc tttggaacgc aaaaacattg aaatctacat ccagcgttcc 2760
   cgtcgtctct atggcaacac acccatgcgc cgggcactgc ttacttggag cttagcaggg 2820
   ctagaactgg tagctctggc agatgcctcc ttccatggtc ctgagcatgt ggtagaacag 2880
   gttcaagagc ttgatccagg cagccctttt ccccctgagg gattagatct tgtcattcag 2940
   tggtgtcgaa tgctcaagtg caatgtcaag agctttctgg ttcggatcag ggactatcca 3000
   cggtacctgt ttgagatccg tgactggcgg ctaatgggtc gacttgtggg caccgagcag 3060
   agtggtcagc cttgctcccg tcggcgtcag atcttgcact tggggcttcc gtggggtaac 3120
   gtggcagtgg agaggaacat gcccccactc aaattctacc atgactttca ctcggaaata 3180
   ttccagtaca cagtggtgtg gggcccatgc tgggatccag cctggacact aattggccag 3240
   tgtgtggacc tcttgaccaa gccctcagct gaccccagcc cacctttgcc ctggtgggac 3300
   aagagccgtc ttctgttcca tggagactgg cacatggaca ttgaacaggc gaacctgcac 3360
   cagctggcca ctgaggatcc atacaacaca actgaaaata tgcactggga gtggagccac 3420
   ctgtcttttc attggaaacc tggtcagttt gtgttcaagg gtgacttgga tatcaacgtg 3480
   agaacagcct ctaagtatga cgactgctgc ttccttcacc tgcctgacct ctgcatgaca 3540
   ctggacctgc agtggctgtg ccatgggaac ccccatgatc accatagtgt cactctgcgg 3600
   gccccagagt tcctgcctga ggtgcccttg ggccagcttc atgactccta ccgggccttt 3660
   cgctcggaga acctcaatct ctccatcaag atggatctgg ctcggcacag tggaacaata 3720
   tcccagcccc gaattctgct atatagtagt accctgcgct ggatgcaaaa cttctgggca 3780
   acttggacaa gtgtcacaag gcctatctgc aggggaaagc tcttcaataa cctgaaaccc 3840
   agcaagaaga aacttggtca gcactacaag caactttcct atacagccct ctttccccag 3900
   ctgcaggtac attattgggc ctcatttgcc cagcaacggg gcatccagat tgagtgcagt 3960
   cagggccatg tcttcactcg ggggactcag cggcttatac ctcaagcagg cacagtgatg 4020
   cggcgcctta tctctgattg gagtgttacc cagatggtga gtgacctaag tcaggtgacc 4080
   gttcacctga tggcctcacc cactgaagag aatgctgatc actgtcttga tcccttggta 4140
   acaaagaccc acctgctgag cttgtcctcc ctcacctacc aacggcatag caatcgcaca 4200
   gctgaggagg agctctctgc tcgtgatggg gatcctacct ttcatacaca tcagctgcac 4260
   ttagtagatt tacggatttc ctggacaact accaatcgag acattgcctt tgggttatat 4320
   gatgggtaca aaaaggcagc tgtactcaaa cgtaatcttt ctactgaggc cctgaagggg 4380
   ttaaagattg atccacagat gccagccaaa aagccaaagc ggggtgtccc aactagtgcc 4440
   tcagccccac ctcgtgttaa cactcccagc ttcagtggac aacctgataa ggggtcatca 4500
   ggaggtgctt acatgttgca gaagctaatt gaagagacag ataggcttgt agtgttcaca 4560
   gaagaggaat caggcatgag tgaccagttg tgtggcattg ctgcctgcca gacggatgac 4620
   atatacaacc gaaactgcct tattgaattg gtcaactgtc agatggttct tcgtggagca 4680
   gagacagaag gctgtgtcat tgtgtcagct gccaaagccc aactgctgca gtgccagcac 4740
   catccagcct ggtatggtga tacattgaag caaaagacat cctggacttg cctcttggat 4800
   ggcatgcagt actttgccac cactgaaagc agccccacag agcaggatgg ccgacagctc 4860
   tggttagagg tgaagaatat cgaggagcac cggcagcgta gtctggactc tgtgcaggag 4920
   ctgatggaga gtgggcaggc agtgggcggc atggttacca caaccacaga ttggaaccag 4980
   ccagctgagg cacagcaagc ccagcaagtc cagcggatca tttcgcgttg caactgccga 5040
   atgtactata ttagttacag ccatgacatt gatcctgaac tagcaactca gattaagcca 5100
   cctgaagttc ttgagaacca ggaaaaggaa gatctcctaa agaagcagga aggggctgtg 5160
   gataccttca cacttatcca ccatgagctg gaaatttcca acccagctca gtatgccatg 5220
   atcctggaca ttgtcaacaa cctgctgctc catgtagaac ctaagcggaa ggaacatagt 5280
   gagaagaagc aacgggtcag gttccagctt gagatctcta gcaatccaga ggagcaacgc 5340
   agcagcatac tgcatttgca ggaggctgtg cggcagcatg tggcccaaat acgacagctg 5400
   gagaagcaga tgtattctac catgaagtct ttgcaggatg acagcaagaa tgagaatctg 5460
   cttgacctga accagaagct tcagttgcag ctaaaccagg agaaggccaa cctgcagctg 5520
   gaaagtgaag aactgaatat cctcatcagg tgttttaagg atttccaact gcagcgggct 5580
   aacaagatgg agctgcgaaa gcagcaagaa gatgtgagtg tggtccgtcg cactgagttt 5640
   tactttgctc aggcacggtg gcgcctgaca gaggaagatg gacagctggg aattgctgaa 5700
   ttagaactgc agaggttcct ctacagcaag gtgaataagt ctgatgacac agcagaacat 5760
   cttctggagt tgggctggtt taccatgaac aacctcctcc ccaatgctgt ctataaggta 5820
   gtactgcggc cccagagctc ctgccagtct gggcgacagc tagctctccg cctcttcagc 5880
   aaagttcggc cccctgttgg gggtatctct gttaaggagc attttgaggt aaatgtggtg 5940
   cctctcacca tccagctgac acaccagttc ttccacagaa tgatgggctt tttctttcct 6000
   ggccgaagtg tggaagatga tgaagttggt gatgaagagg ataagtccaa actggtgact 6060
   actggaatac cagtggtgaa gcctcggcag ctgattgcaa cagatgatgc agtaccactg 6120
   ggccctggga agggtgtggc acagggtttg actcggagtt ctggggtcag aaggtcattt 6180
   cgcaaatcgc cagagcaccc tgtggatgac attgacaaga tgaaagagcg agctgccatg 6240
   aacaactcct tcatctacat aaagattcca caggttccac tgtgtgtcag ctacaagggt 6300
   gagaagaaca gtgtggactg ggcgaccttt aacctggtgc tgccctgtct ggagtaccac 6360
   aacaacacat ggacatggct agactttgcc atggctgtca aaagggacag ccgcaaagcc 6420
   ctggttgccc aggtaatcaa agagaagcta aggctgaagt ctgcaacggg ctctgaggtc 6480
   cggggaaagc tagaaactaa atcggacctg aacatgcaac agcaggaaga ggaggagaaa 6540
   gcccggctcc tcattggttt aagtgtgggc gacaagaacc ctggcaagaa gtccatcttt 6600
   ggcaggcgca aatga 6615
<210> 98
   <211> 989
   <212> PRT
   <213> Homo sapiens
<400> 98
<210> 99
   <211> 2970
   <212> DNA
   <213> Homo sapiens
<400> 99
   atgaccatgg ctgggggcag gaggggacta gtggcccctc aaaacacgtt tctggagaat    60
   attgttcggc ggtccaatga tactaatttt gtgttgggga atgctcagat agtggactgg   120
   cctattgtgt acagcaatga tggattttgc aagctgtctg gctatcacag ggcagaagtg   180
   atgcaaaaaa gcagcacctg cagttttatg tatggggagc tgactgataa agacacgatt   240
   gaaaaagtgc ggcaaacatt tgagaactat gagatgaatt cctttgaaat tctgatgtac   300
   aagaagaaca ggacacctgt gtggttcttt gtgaaaattg ctccaattcg aaacgaacag   360
   gataaagtgg ttttatttct ttgcactttc agtgacataa cagctttcaa acagccaatt   420
   gaggatgatt catgtaaagg ctgggggaag tttgctcggc tgacaagagc actgacaagc   480
   agcaggggtg tcctgcagca gctggctcca agcgtgcaaa aaggcgagaa tgtccacaag   540
   cactcccgcc tggcagaggt cctacagctg ggctcagaca tccttcccca gtacaagcaa   600
   gaggcaccaa agactccccc tcacatcatc ttacattatt gtgtttttaa gaccacgtgg   660
   gattggatca tcttgatctt gaccttctat acagccatct tggtccctta taatgtctcc   720
   ttcaaaacca ggcagaataa tgtggcctgg ctggttgttg atagcatcgt ggatgttatc   780
   tttttggtgg acattgtgct caattttcat accacctttg ttggaccagc aggggaggtg   840
   atttctgacc ccaaacttat ccgcatgaac tacctgaaga cgtggtttgt gattgacctt 900
   ctgtcctgtt tgccatatga tgtcatcaac gcttttgaga acgtggatga ggttagtgcc 960
   tttatgggtg atccagggaa gattggtttt gctgatcaga ttccaccacc actggagggg 1020
   agagagagtc agggcatcag cagcctgttc agctctctaa aagttgtccg gctgctccgt 1080
   cttgggcgag tggcccgtaa gctggaccac tacattgaat atggagctgc tgtgctggtc 1140
   ctgctggtgt gtgtgtttgg gctggctgca cactggatgg cctgcatctg gtacagcatt 1200
   ggggactatg agatctttga cgaggacacc aagacaatcc gcaacaacag ctggctgtac 1260
   caactagcga tggacattgg caccccttac cagtttaatg ggtctggctc agggaagtgg 1320
   gaaggtggtc ccagcaagaa ttctgtctac atctcctcgt tgtatttcac aatgaccagc 1380
   ctcaccagtg tgggctttgg gaacatcgcc ccatccacag acattgagaa gatctttgca 1440
   gtggccatca tgatgattgg ctcacttctc tatgccacca tcttcgggaa tgtgacgact 1500
   attttccaac agatgtatgc caacaccaac agataccatg agatgctcaa cagtgttcgg 1560
   gacttcctga agctctacca ggtgccaaaa ggattgagtg agcgagtaat ggattatatt 1620
   gtgtccactt ggtccatgtc cagaggcatt gacacagaga aggtcctgca gatctgcccc 1680
   aaggacatga gagccgacat ctgcgtgcac ctgaaccgca aggtgttcaa ggagcacccg 1740
   gccttccggc tggccagtga tggctgcctc cgggcactgg ccatggagtt ccagacggtg 1800
   cactgtgccc caggggacct catctaccat gcaggagaga gcgttgacag cctctgcttt 1860
   gtggtttctg gctccctgga ggtgatccaa gatgatgagg tggtggccat tctaggaaaa 1920
   ggagacgtgt ttggagatgt gttctggaag gaagccaccc ttgcccagtc ctgtgccaat 1980
   gttagggcct tgacctactg tgatctgcat gtgatcaagc gggatgccct gcagaaagtg 2040
   ctggaattct acacggcctt ctcccattcc ttctcccgga acctgattct gacgtacaac 2100
   ttgaggaaga ggattgtgtt ccggaagatc agcgatgtga aacgtgaaga ggaagaacgc 2160
   atgaaacgaa agaatgaggc ccccctgatc ttgcccccgg accaccctgt ccggcgcctc 2220
   ttccagagat tccgacagca gaaagaggcc aggctggcag ctgagagagg gggccgggac 2280
   ctggatgacc tagatgtgga gaagggcaat gtccttacag agcatgcctc cgccaaccac 2340
   agcctcgtga aggccagcgt ggtcaccgtg cgtgagagtc ctgccacgcc cgtatccttc 2400
   caggcagcct ccacctccgg ggtgccagac cacgcaaagc tacaggcgcc agggtccgag 2460
   tgcctgggcc ccaagggggg cgggggcgat tgtgccaagc gcaaaagctg ggcccgcttc 2520
   aaagatgctt gcgggaagag tgaggactgg aacaaggtgt ccaaggctga gtcgatggag 2580
   acacttcccg agaggacaaa agcgtcaggc gaggccacac tgaagaagac agactcgtgt 2640
   gacagtggca tcaccaagag cgacttgcgc ctggacaacg tgggtgaggc caggagtccc 2700
   caggatcgga gtcccatcct ggcagaggtc aagcattcgt tctaccccat ccctgagcag 2760
   acgctgcagg ccacagtcct ggaggtgagg cacgagctga aggaggacat caaggcctta 2820
   aacgccaaaa tgaccaatat tgagaaacag ctctctgaga tactcaggat attaacttcc 2880
   agaagatcct ctcagtctcc tcaggagttg tttgaaatat cgaggccaca gtccccagaa 2940
   tcagagagag acatttttgg agccagctga 2970
<210> 100
   <211> 962
   <212> PRT
   <213> Homo sapiens
<400> 100
   Met Thr Met Ala Gly Gly Arg Arg Gly Leu Val Ala Pro Gln Asn Thr
   1               5                   10                  15
   Phe Leu Glu Asn Ile Val Arg Arg Ser Asn Asp Thr Asn Phe Val Leu
               20                  25                  30
   Gly Asn Ala Gln Ile Val Asp Trp Pro Ile Val Tyr Ser Asn Asp Gly
           35                  40                  45
   Phe Cys Lys Leu Ser Gly Tyr His Arg Ala Glu Val Met Gln Lys Ser
       50                  55                  60
   Ser Thr Cys Ser Phe Met Tyr Gly Glu Leu Thr Asp Lys Asp Thr Ile
   65                  70                  75                  80
   Glu Lys Val Arg Gln Thr Phe Glu Asn Tyr Glu Met Asn Ser Phe Glu
                   85                  90                  95
   Ile Leu Met Tyr Lys Lys Asn Arg Thr Pro Val Trp Phe Phe Val Lys
               100                 105                 110
   Ile Ala Pro Ile Arg Asn Glu Gln Asp Lys Val Val Leu Phe Leu Cys
           115                 120                 125
   Thr Phe Ser Asp Ile Thr Ala Phe Lys Gln Pro Ile Glu Asp Asp Ser
       130                 135                 140
   Cys Lys Gly Trp Gly Lys Phe Ala Arg Leu Thr Arg Ala Leu Thr Ser
   145                 150                 155                 160
   Ser Arg Gly Val Leu Gln Gln Leu Ala Pro Ser Val Gln Lys Gly Glu
                   165                 170                 175
   Asn Val His Lys His Ser Arg Leu Ala Glu Val Leu Gln Leu Gly Ser
               180                 185                 190
   Asp Ile Leu Pro Gln Tyr Lys Gln Glu Ala Pro Lys Thr Pro Pro His
           195                 200                 205
   Ile Ile Leu His Tyr Cys Val Phe Lys Thr Thr Trp Asp Trp Ile Ile
       210                 215                 220
   Leu Ile Leu Thr Phe Tyr Thr Ala Ile Leu Val Pro Tyr Asn Val Ser
   225                 230                 235                 240
   Phe Lys Thr Arg Gln Asn Asn Val Ala Trp Leu Val Val Asp Ser Ile
                   245                 250                 255
   Val Asp Val Ile Phe Leu Val Asp Ile Val Leu Asn Phe His Thr Thr
               260                 265                 270
   Phe Val Gly Pro Ala Gly Glu Val Ile Ser Asp Pro Lys Leu Ile Arg
           275                 280                 285
   Met Asn Tyr Leu Lys Thr Trp Phe Val Ile Asp Leu Leu Ser Cys Leu
       290                 295                 300
   Pro Tyr Asp Val Ile Asn Ala Phe Glu Asn Val Asp Glu Gly Ile Ser
   305                 310                 315                 320
   Ser Leu Phe Ser Ser Leu Lys Val Val Arg Leu Leu Arg Leu Gly Arg
                   325                 330                 335
   Val Ala Arg Lys Leu Asp His Tyr Ile Glu Tyr Gly Ala Ala Val Leu
               340                 345                 350
   Val Leu Leu Val Cys Val Phe Gly Leu Ala Ala His Trp Met Ala Cys
           355                 360                 365
   Ile Trp Tyr Ser Ile Gly Asp Tyr Glu Ile Phe Asp Glu Asp Thr Lys
       370                 375                 380
   Thr Ile Arg Asn Asn Ser Trp Leu Tyr Gln Leu Ala Met Asp Ile Gly
   385                 390                 395                 400
   Thr Pro Tyr Gln Phe Asn Gly Ser Gly Ser Gly Lys Trp Glu Gly Gly
                   405                 410                 415
   Pro Ser Lys Asn Ser Val Tyr Ile Ser Ser Leu Tyr Phe Thr Met Thr
               420                 425                 430
   Ser Leu Thr Ser Val Gly Phe Gly Asn Ile Ala Pro Ser Thr Asp Ile
           435                 440                 445
   Glu Lys Ile Phe Ala Val Ala Ile Met Met Ile Gly Ser Leu Leu Tyr
       450                 455                 460
   Ala Thr Ile Phe Gly Asn Val Thr Thr Ile Phe Gln Gln Met Tyr Ala
   465                 470                 475                 480
   Asn Thr Asn Arg Tyr His Glu Met Leu Asn Ser Val Arg Asp Phe Leu
                   485                 490                 495
   Lys Leu Tyr Gln Val Pro Lys Gly Leu Ser Glu Arg Val Met Asp Tyr
   500 505 510
   Ile Val Ser Thr Trp Ser Met Ser Arg Gly Ile Asp Thr Glu Lys Val
   515 520 525
   Leu Gln Ile Cys Pro Lys Asp Met Arg Ala Asp Ile Cys Val His Leu
   530 535 540
   Asn Arg Lys Val Phe Lys Glu His Pro Ala Phe Arg Leu Ala Ser Asp
   545 550 555 560
   Gly Cys Leu Arg Ala Leu Ala Met Glu Phe Gln Thr Val His Cys Ala
   565 570 575
   Pro Gly Asp Leu Ile Tyr His Ala Gly Glu Ser Val Asp Ser Leu Cys
   580 585 590
   Phe Val Val Ser Gly Ser Leu Glu Val Ile Gln Asp Asp Glu Val Val
   595 600 605
   Ala Ile Leu Gly Lys Gly Asp Val Phe Gly Asp Val Phe Trp Lys Glu
   610 615 620
   Ala Thr Leu Ala Gln Ser Cys Ala Asn Val Arg Ala Leu Thr Tyr Cys
   625 630 635 640
   Asp Leu His Val Ile Lys Arg Asp Ala Leu Gln Lys Val Leu Glu Phe
   645 650 655
   Tyr Thr Ala Phe Ser His Ser Phe Ser Arg Asn Leu Ile Leu Thr Tyr
   660 665 670
   Asn Leu Arg Lys Arg Ile Val Phe Arg Lys Ile Ser Asp Val Lys Arg
   675 680 685
   Glu Glu Glu Glu Arg Met Lys Arg Lys Asn Glu Ala Pro Leu Ile Leu
   690 695 700
   Pro Pro Asp His Pro Val Arg Arg Leu Phe Gln Arg Phe Arg Gln Gln
   705 710 715 720
   Lys Glu Ala Arg Leu Ala Ala Glu Arg Gly Gly Arg Asp Leu Asp Asp
   725 730 735
   Leu Asp Val Glu Lys Gly Asn Val Leu Thr Glu His Ala Ser Ala Asn
   740 745 750
   His Ser Leu Val Lys Ala Ser Val Val Thr Val Arg Glu Ser Pro Ala
   755 760 765
   Thr Pro Val Ser Phe Gln Ala Ala Ser Thr Ser Gly Val Pro Asp His
       770                 775                     780
   Ala Lys Leu Gln Ala Pro Gly Ser Glu Cys Leu Gly Pro Lys Gly Gly
   785                 790                 795                 800
   Gly Gly Asp Cys Ala Lys Arg Lys Ser Trp Ala Arg Phe Lys Asp Ala
                   805                 810                 815
   Cys Gly Lys Ser Glu Asp Trp Asn Lys Val Ser Lys Ala Glu Ser Met
               820                 825                 830
   Glu Thr Leu Pro Glu Arg Thr Lys Ala Ser Gly Glu Ala Thr Leu Lys
           835                 840                 845
   Lys Thr Asp Ser Cys Asp Ser Gly Ile Thr Lys Ser Asp Leu Arg Leu
       850                 855                 860
   Asp Asn Val Gly Glu Ala Arg Ser Pro Gln Asp Arg Ser Pro Ile Leu
   865                 870                 875                 880
   Ala Glu Val Lys His Ser Phe Tyr Pro Ile Pro Glu Gln Thr Leu Gln
                   885                 890                 895
   Ala Thr Val Leu Glu Val Arg His Glu Leu Lys Glu Asp Ile Lys Ala
               900                 905                 910
   Leu Asn Ala Lys Met Thr Asn Ile Glu Lys Gln Leu Ser Glu Ile Leu
           915                 920                 925
   Arg Ile Leu Thr Ser Arg Arg Ser Ser Gln Ser Pro Gln Glu Leu Phe
       930                 935                 940
   Glu Ile Ser Arg Pro Gln Ser Pro Glu Ser Glu Arg Asp Ile Phe Gly
   945                 950                 955                 960
   Ala Ser